# EUROPEAN PATENT APPLICATION

(11) **EP 1 892 238 A1**
(43) Date of publication of application: **27.02.2008**
(21) Application number: 06766669.3
(22) Date of filing: 14.06.2006
(51) Int. Cl.: C07D 207/08, A61K 31/495, A61K 31/496, A61K 31/5377, A61K 31/538, A61K 31/54, A61P 1/04, A61P 11/06, A61P 19/02, A61P 25/00, A61P 29/00, A61P 37/02, A61P 37/08, A61P 43/00, C07D 207/27, C07D 207/404, C07D 211/46, C07D 211/58

(54) **1, 2-DI(CYCLIC GROUP)SUBSTITUTED BENZENE DERIVATIVE**

(30) Priority: 14.06.2005 JP 2005174072
(71) Applicant: Eisai R&D Management Co., Ltd., Tokyo 112-8088 (JP)
(72) Inventor: KAWAHARA, Tetsuya, EISAI Co., Ltd., Tsukuba-shi, Ibaraki 300-2635 (JP); KOTAKE, Makoto, EISAI Co., Ltd., Tsukuba-shi, Ibaraki 300-2635 (JP); YONEDA, Naoki, EISAI Co., Ltd., Tsukuba-shi, Ibaraki 300-2635 (JP); HIROTA, Shinsuke, EISAI Co., Ltd., Tsukuba-shi, Ibaraki 300-2635 (JP); OHKURO, Masayoshi, EISAI Co., Ltd., Tsukuba-shi, Ibaraki 300-2635 (JP)
(74) Representative: HOFFMANN EITLE
(86) International application number: PCT/JP2006/311900
(87) International publication number: WO 2006/134955

(57) **Abstract**

A compound represented by the following general formula (1) or (100), a salt thereof or a hydrate of the foregoing has excellent cell adhesion inhibitory action and cell infiltration inhibitory action. 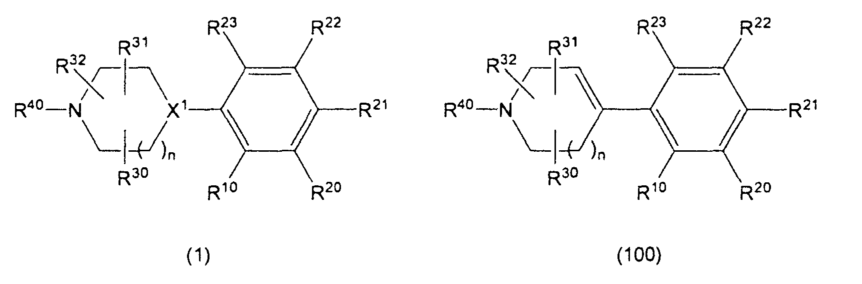 wherein R¹⁰ represents 5- to 10-membered cycloalkyl etc. optionally substituted with hydroxyl etc., R³⁰, R³¹ and R³² may be the same or different and each represents hydrogen etc., R⁴⁰ represents C1-10 alkyl etc. optionally substituted with hydroxyl etc., n represents an integer of 0, 1 or 2, X¹ represents CH or nitrogen, and R²⁰, R²¹, R²² and R²³ may be the same or different and each represents hydrogen etc.

## Description

### Technical Field

The present invention relates to 1,2-di(cyclic)substituted benzene derivatives which are useful as cell adhesion inhibitors or cell infiltration inhibitors, as well as to their salts and to hydrates of the foregoing.
The present invention relates to 1,2-di(cyclic)substituted benzene derivatives which are useful as therapeutic or prophylactic agents for inflammatory diseases and autoimmune diseases, as well as to their salts and to hydrates of the foregoing.
The invention further relates to 1,2-di(cyclic)substituted benzene derivatives which are useful as therapeutic or prophylactic agents for various diseases associated with adhesion and infiltration of leukocyte, such as inflammatory bowel disease (particularly ulcerative colitis or Crohn's disease), irritable bowel syndrome, rheumatoid arthritis, psoriasis, multiple sclerosis, asthma and atopic dermatitis, as well as to their salts and to hydrates of the foregoing.

### Background Art

Inflammatory reaction is accompanied by infiltration of leukocytes, typically neutrophils and lymphocytes, into inflammatory sites.
Infiltration of leukocytes is defined as migration of leukocytes such as neutrophils and lymphocytes out of vessels and into the surrounding tissues as a consequence of initiation and activation by cytokines, chemokines, lipids and complement to interact called "rolling" or "tethering" with vascular endothelial cells activated by cytokines such as IL-1 or TNFα, followed by adhesion to the vascular endothelial cells.

As explained below, relationship between leukocyte adhesion or infiltration and various inflammatory diseases and autoimmune diseases was reported. Such reports have raised the possibility that compounds having cell adhesion inhibitory action or cell infiltration inhibitory action may serve as therapeutic or prophylactic agents for such diseases.
(1) Therapeutic or prophylactic agents for inflammatory bowel disease (ulcerative colitis, Crohn's disease and the like) (see Non-patent documents 1, 2 and 3)
(2) Therapeutic or prophylactic agents for irritable bowel syndrome (see Non-patent document 4)
(3) Therapeutic or prophylactic agents for rheumatoid arthritis (see Non-patent document 5)
(4) Therapeutic or prophylactic agents for psoriasis (see Non-patent document 6)
(5) Therapeutic or prophylactic agents for multiple sclerosis (see Non-patent document 7)
(6) Therapeutic or prophylactic agents for asthma (see Non-patent document 8)
(7) Therapeutic or prophylactic agents for atopic dermatitis (see Non-patent document 9)

Thus, substances which inhibit cell adhesion or cell infiltration are expected to be useful as therapeutic or prophylactic agents for inflammatory diseases and autoimmune diseases and as therapeutic or prophylactic agents for various diseases associated with adhesion and infiltration of leukocytes, such as inflammatory bowel disease (particularly ulcerative colitis or Crohn's disease), irritable bowel syndrome, rheumatoid arthritis, psoriasis, multiple sclerosis, asthma and atopic dermatitis.

Compounds are also known which have anti-inflammatory action based on inhibition of adhesion of leukocyte and vascular endothelial cell, or anti-inflammatory action based on inhibition of leukocyte infiltration (these will hereinafter be referred to as cell adhesion inhibitors and cell infiltration inhibitors, respectively), such as the following compound:

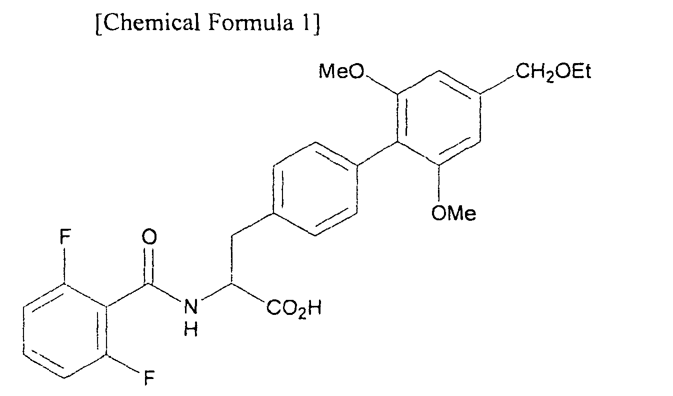

(see Patent document 1).

However, the compounds represented by general formula (1) or general formula (100) according to the present invention are characterized by including a partial chemical structure such as piperazine, piperidine or tetrahydropyridine at the ortho position of a benzene ring bonded to an aliphatic carbocyclic group such as cyclohexyl, therefore differ in their structures from the aforementioned cell adhesion inhibitors or cell infiltration inhibitors.

The known compound comprising a partial chemical structure such as having piperazine, piperidine or tetrahydropyridine at the ortho position of a benzene ring bonded to an aliphatic carbocyclic group such as cyclohexyl, as a chemical structural feature of the compounds represented by general formula (1) or general formula (100) according to the present invention, is the compound represented by the following formula:

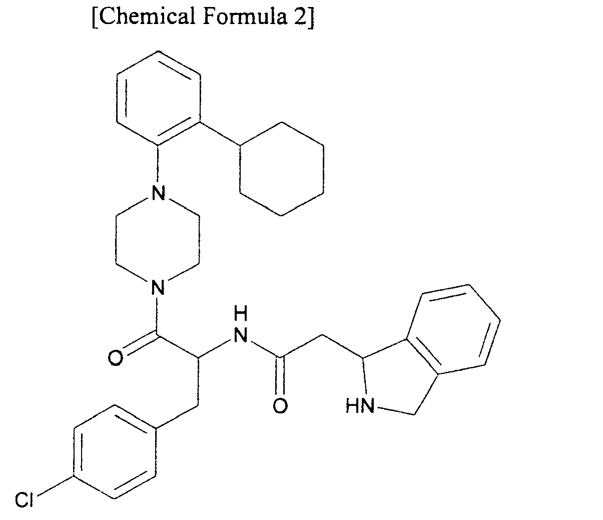

(see Patent document 2).
However, the patent application discloses only its use as an anti-obesity agent and diabetes treatment based on the melanocortin receptor agonistic activity of the compound, while it neither discloses nor suggests its use as an anti-inflammatory agent based on inhibitory action of leukocyte adhesion or infiltration.
Other than the above compound, the compound represented by the following formula:

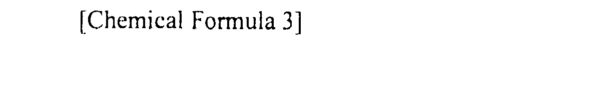

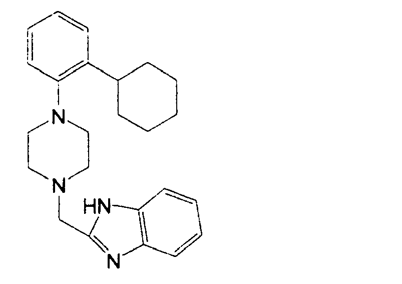

is known (see Non-patent document 10, compound number 45).

[Patent document 1] WO 2002/018320
[Patent document 2] WO 2002/059108
[Non-patent document 1] Inflammatory Bowel Disease (N. Engl. J. Med., 347:417-429(2002))
[Non-patent document 2] Natalizumab for active Crohn's disease (N. Engl. J. Med., 348:24-32(2003))
[Non-patent document 3] Granulocyte adsorption therapy in active period of ulcerative colitis (Japanese Journal of Apheresis 18:117-131 (1999))
[Non-patent document 4] A role for inflammation in irritable bowel syndrome (Gut., 51: i41-i44 (2002))
[Non-patent document 5] Rheumatoid arthritis (Int. J. Biochem. Cell Biol., 36:372-378(2004))
[Non-patent document 6] Psoriasis (Lancet, 361:1197-1204(2003))
[Non-patent document 7] New and emerging treatment options for multiple sclerosis (Lancet Neurology, 2:563-566(2003))
[Non-patent document 8] The role of T lymphocytes in the pathogenesis of asthma (J. Allergy Clin. Immunol., 111:450-463(2003)
[Non-patent document 9] The molecular basis of lymphocyte recruitment to the skin (J. Invest. Dermatol., 121:951-962(2003))
[Non-patent document 10] Discovery of 2-(4-pyridin-2-ylpiperazin-1-ylmethyl)-1H-benzimidazole (ABT-724), a dopaminergic agent with a novel mode of action for the potential treatment of erectile dysfunction (J. Med. Chem., 47: 3853-3864(2004))

### Disclosure of the Invention

### Problems to be Solved by the Invention

It is an object of the present invention to provide novel compounds having excellent cell adhesion inhibitory action and cell infiltration inhibitory action, which are useful as therapeutic or prophylactic agents for various inflammatory diseases and autoimmune diseases associated with adhesion and infiltration of leukocytes, such as inflammatory bowel disease (particularly ulcerative colitis or Crohn's disease), irritable bowel syndrome, rheumatoid arthritis, psoriasis, multiple sclerosis, asthma and atopic dermatitis.

### Means for Solving the Problems

As a result of intensive research, the present inventors have discovered that 1,2-di(cyclic)-substituted benzene derivatives having a specific chemical structure exhibit excellent cell adhesion inhibitory action or cell infiltration inhibitory action, and are particularly useful as therapeutic or prophylactic agents for various inflammatory diseases and autoimmune diseases associated with adhesion and infiltration of leukocytes, such as inflammatory bowel disease (particularly ulcerative colitis or Crohn's disease), irritable bowel syndrome, rheumatoid arthritis, psoriasis, multiple sclerosis, asthma and atopic dermatitis, and the present invention was completed on the basis of this discovery.

Specifically, the invention is a compound according to the following [1] or [2], a salt thereof or a hydrate of the foregoing.

### [1] A compound represented by the following general formula (1), a salt thereof or a hydrate of the foregoing:

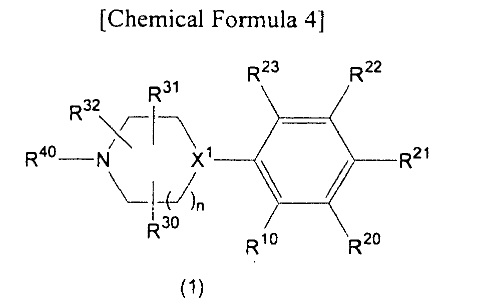

wherein R¹⁰ represents 5- to 10-membered cycloalkyl optionally substituted with a substituent selected from Group A1 or 5- to 10-membered cycloalkenyl optionally substituted with a substituent selected from Group A1,
R³⁰, R³¹ and R³² may be the same or different and each represents hydrogen, hydroxyl, halogen, cyano, carboxyl, C1-6 alkyl, C1-6 alkoxy or C2-7 alkoxycarbonyl, or
two of R³⁰, R³¹ and R³² bond together to form oxo (=O) or methylene (-CH₂-) and the other represents hydrogen, hydroxyl, halogen, cyano, carboxyl, C 1-6 alkyl, C 1-6 alkoxy or C2-7 alkoxycarbonyl,
R 40 represents C 1-10 alkyl optionally substituted with a substituent selected from Group D1, 3- to 8-membered cycloalkyl optionally substituted with a substituent selected from Group E1, a 4- to 8-membered heterocyclic group optionally substituted with a substituent selected from Group E1, C2-7 alkenyl optionally substituted with a substituent selected from Group F1, C2-7 alkynyl optionally substituted with a substituent selected from Group F1, C2-7 alkylcarbonyl optionally substituted with a substituent selected from Group G1, mono(C1-6 alkyl)aminocarbonyl, 4- to 8-membered heterocyclic carbonyl, C2-7 alkoxycarbonyl or C1-6 alkylsulfonyl,
n represents an integer of 0, 1 or 2,
X¹ represents CH or nitrogen, and
R²⁰, R²¹, R²² and R²³ may be the same or different and each represents hydrogen, hydroxyl, halogen, nitro, cyano, carboxyl, C1-6 alkylthio optionally substituted with a substituent selected from Group F1, C2-7 alkoxycarbonyl, phenoxy, -SO₃H, C1-6 alkyl optionally substituted with a substituent selected from Group W1, C1-6 alkyl optionally substituted with a substituent selected from Group K1, C1-6 alkoxy optionally substituted with a substituent selected from Group W1, a 4- to 8-membered heterocyclic group optionally substituted with a substituent selected from Group W1, a 4- to 8-membered heterocyclic group optionally substituted with a substituent selected from Group V1, a 5- to 10-membered heteroaryl ring group optionally substituted with a substituent selected from Group W1, a 6- to 10-membered aryl ring group optionally substituted with a substituent selected from Group W1, C2-7 alkenyl optionally substituted with a substituent selected from Group W1, C2-7 alkynyl optionally substituted with a substituent selected from Group W1, 3- to 8-membered cycloalkyl optionally substituted with a substituent selected from Group W1, 5- to 8-membered cycloalkenyl optionally substituted with a substituent selected from Group W1, -NR^{1X}R^{2X}, -CO-R^{1X}, -CO-NR^{1X}R^{2X}, -NR^{1X}-CO-R^{2X}, -SO₂-R^{3X} or -O-SO₂-R^{3X},
wherein R^{1X} and R^{2X} may be the same or different and each represents hydrogen, C1-6 alkyl optionally substituted with a substituent selected from Group U1 or a 4- to 8-membered heterocyclic group, and
R^{3X} represents C1-6 alkyl optionally substituted with a substituent selected from Group F1; or
(i) R²⁰ and R²¹, (ii) R²¹ and R²² or (iii) R²² and R²³ bond together to form a ring selected from Group Z1,
wherein Group A1 consists of hydroxyl, halogen, cyano, C1-6 alkoxy, phenyl optionally substituted with a substituent selected from Group C1, C1-6 alkyl, C1-6 haloalkyl and C2-7 alkylene where C2-7 alkylene is permissible only in the case that a spiro union is formed together with the substituted 5- to 10-membered cycloalkyl or the substituted 5- to 10-membered cycloalkenyl,
Group C1 consists of cyano, halogen, C1-6 alkyl and C1-6 alkoxy,
Group D1 consists of hydroxyl, halogen, cyano, C1-6 alkoxy, C1-6 alkylthio, C1-6 alkylsulfonyl, C1-6 alkylsulfinyl, mono(C1-6 alkyl)amino, di(C1-6 alkyl)amino, C2-7 alkylcarbonylamino, 3- to 8-membered cycloalkyl optionally substituted with a substituent selected from Group H1, C2-7 alkoxycarbonyl, carboxyl, a 4- to 8-membered heterocyclic group, a 5- to 10-membered heteroaryl ring group, a 6- to 10-membered aryl ring group, C2-7 alkylcarbonyl, 6- to 10-membered aryl ring carbonyl, aminocarbonyl, mono(C1-6 alkyl)aminocarbonyl optionally substituted with halogen, mono(3- to 8-membered cycloalkyl)aminocarbonyl, mono(C2-7 alkoxyalkyl)aminocarbonyl, di(C1-6 alkyl)aminocarbonyl, mono(5- to 10-membered heteroaryl ring)aminocarbonyl, 4-to 8-membered heterocyclic carbonyl optionally substituted with C1-6 alkyl, and 5-to 10-membered heteroaryl ring carbonyl,
Group E 1 consists of halogen, C1-6 alkoxy, oxo (=O) and C1-6 alkyl,
Group F 1 consists of halogen and C1-6 alkoxy,
Group G1 consists of 3- to 8-membered cycloalkyl,
Group H1 consists of hydroxyl, C1-6 haloalkyl, C1-6 alkyl, C2-7 alkoxyalkyl, mono(C1-6 alkyl)aminocarbonyl, di(C1-6 alkyl)aminocarbonyl, C2-7 alkoxycarbonyl, carboxyl and C2-7 cyanoalkyl,
Group W1 consists of halogen, hydroxyl, cyano, carboxyl, C1-6 alkyl, C2-7 alkoxyalkyl, C1-6 alkoxy optionally substituted with a substituent selected from Group T1, phenoxy, C2-7 alkoxycarbonyl, C2-7 alkylcarbonyl, -NR^{6X}R^{7X} and -CO-NR^{6X}R^{7X} wherein R^{6X} and R^{7X} may be the same or different and each represents hydrogen or C1-6 alkyl,
Group T1 consists of C1-6 alkoxy, carboxyl, C2-7 alkoxycarbonyl and -CONR^{4X}R^{5X} wherein R^{4X} and R^{5X} may be the same or different and each represents hydrogen or C1-6 alkyl,
Group V1 consists of oxo (=O) and ethylenedioxy (-O-CH₂CH₂-O-) where ethylenedioxy is permissible only in the case that a spiro union is formed together with the substituted 4- to 8-membered heterocyclic group,
Group K1 consists of a 4- to 8-membered heterocyclic group,
Group U1 consists of carboxyl, C1-6 alkoxy, C2-7 alkoxycarbonyl, halogen, a 6- to 10-membered aryl ring group and -CO-NR^{8x}R^{9x} wherein R^{8x} and R^{9x} may be the same or different and each represents hydrogen or C1-6 alkyl, and
Group Z1 consists of

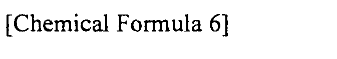

[Chemical Formula 6] 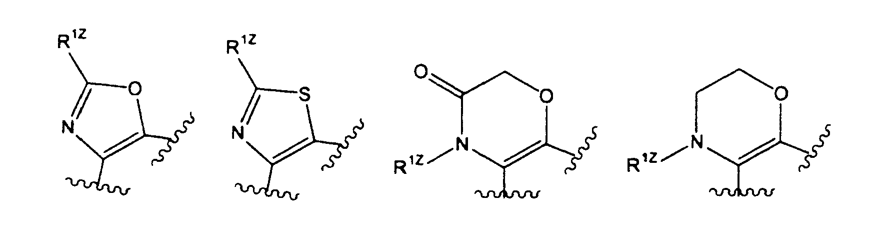 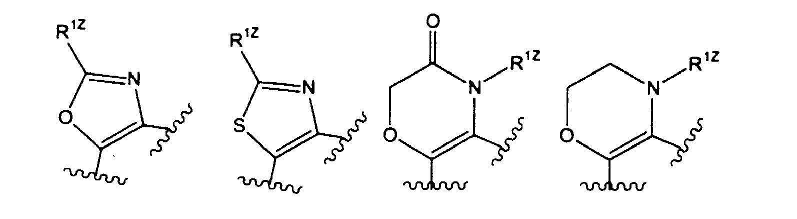 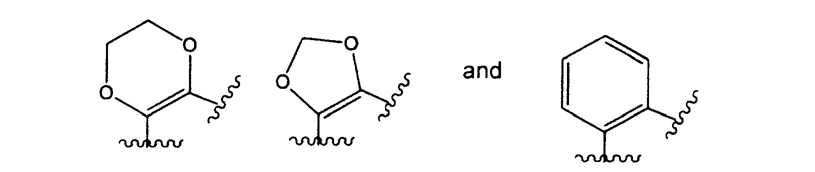 wherein R^{1Z} represents hydrogen, C1-6 alkyl or benzyl,
with the proviso that a compound represented by the following formula: 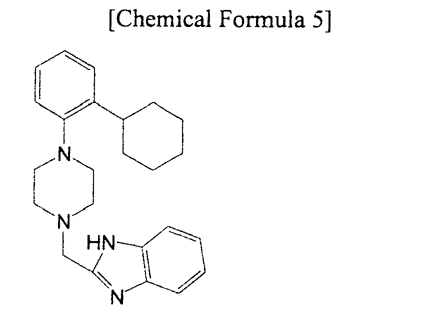 is excepted.

### [2] A compound represented by the following general formula (100), a salt thereof or a hydrate of the foregoing:

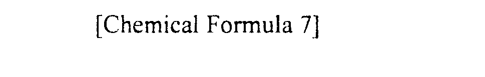

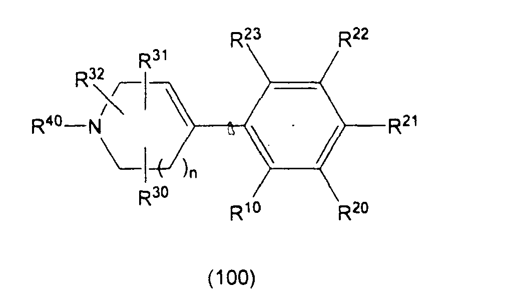

wherein R¹⁰, R²⁰, R²¹, R²², R²³, R³⁰, R³¹, R³², R⁴⁰ and n have the same respective definitions as R¹⁰, R²⁰, R²¹, R²², R²³, R³⁰, R³¹, R³², R⁴⁰ and n in [1] above.

As examples of the "5- to 10-membered cycloalkyl" of the "5- to 10-membered cycloalkyl optionally substituted with a substituent selected from Group A1" for R¹⁰, there may be mentioned cyclopentyl, cyclohexyl, cycloheptyl, cyclooctyl, cyclononyl or cyclodecyl, among which cyclopentyl, cyclohexyl, cycloheptyl or cyclooctyl is preferred, and cyclohexyl is particularly preferred.

The "5- to 10-membered cycloalkenyl" of the "5- to 10-membered cycloalkenyl optionally substituted with a substituent selected from Group A1" for R¹⁰ may have multiple double bonds, and as examples there may be mentioned cyclopentenyl (1-cyclopentenyl, 2-cyclopentenyl or 3-cyclopentenyl), cyclohexenyl (1-cyclohexenyl, 2-cyclohexenyl or 3-cyclohexenyl), cycloheptenyl (1-cycloheptenyl, 2-cycloheptenyl, 3-cycloheptenyl or 4-cycloheptenyl), cyclooctenyl (1-cyclooctenyl, 2-cyclooctenyl, 3-cyclooctenyl or 4-cyclooctenyl), cyclononenyl (1-cyclononenyl, 2-cyclononenyl, 3-cyclononenyl, 4-cyclononenyl or 5-cyclononenyl) or cyclodecenyl (1-cyclodecenyl, 2-cyclodecenyl, 3-cyclodecenyl, 4-cyclodecenyl or 5-cyclodecenyl), among which cyclopentenyl, cyclohexenyl, cycloheptenyl or cyclooctenyl is preferred, cyclohexenyl is more preferred, and 1-cyclohexenyl is most preferred.

As examples of the "halogen" for R²⁰, R²¹, R²² and R²³, there may be mentioned fluorine, chlorine, bromine, iodine or the like, among which bromine, fluorine or chlorine is preferred.

The "C1-6 alkylthio" of the "C1-6 alkylthio optionally substituted with a substituent selected from Group F1" for R²⁰, R²¹ R²² and R²³ is a thio group having the "C1-6 alkyl" described below bonded thereto, and as examples there may be mentioned straight-chain or branched-chain groups such as methylthio, ethylthio, propylthio, isopropylthio, carbonylbutylthio, isobutylthio, s-butylthio, t-butylthio, pentylthio, isopentylthio, 2-methylbutylthio, neopentylthio, 1-ethylpropylthio, hexylthio, isohexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio or 2-ethylbutylthio, among which C1-4 groups are preferred, and methylthio is particularly preferred.

The "C2-7 alkoxycarbonyl" for R²⁰, R²¹, R²² and R²³ is a carbonyl group having the "C1-6 alkoxy" described below bonded thereto, and as examples there may be mentioned straight-chain or branched-chain groups such as methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentoxycarbonyl, isopentoxycarbonyl, 2-methylbutoxycarbonyl, neopentoxycarbonyl, hexyloxycarbonyl, 4-methylpentoxycarbonyl, 3-methylpentoxycarbonyl, 2-methylpentoxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl or 2,3-dimethylbutoxycarbonyl, among which methoxycarbonyl or ethoxycarbonyl is preferred.

As examples of the "C1-6 alkyl" of the "C1-6 alkyl optionally substituted with a substituent selected from Group W1" for R²⁰ , R²¹, R²² and R²³ there may be mentioned straight-chain or branched-chain groups such as methyl, ethyl, n-propyl, isopropyl, n-butyl, isobutyl, s-butyl, t-butyl, n-pentyl, isopentyl, s-pentyl, t-pentyl, 2-methylbutyl, 1-methylbutyl, 2-methylbutyl, neopentyl, 1,1-dimethylpropyl, 1,2-dimethylpropyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, 2-ethylbutyl, 1,1,2-trimethylpropyl, 1,2,2,-trimethylpropyl, 1-ethyl-1-methylpropyl or 1-ethyl-2-methylpropyl, among which C1-4 groups are preferred, methyl, ethyl or t-butyl is more preferred, and methyl is most preferred.

As examples of the "C1-6 alkyl" of the "C1-6 alkyl optionally substituted with a substituent selected from Group K1" for R²⁰, R²¹, R²² and R²³ there may be mentioned the same ones listed above, among which C1-4 groups are preferred, and methyl is particularly preferred.

As examples of the "C1-6 alkoxy" of the "C1-6 alkoxy optionally substituted with a substituent selected from Group W1" for R²⁰, R²¹, R²² and R²³ there may be mentioned straight-chain or branched-chain groups such as methoxy, ethoxy, propoxy, isopropoxy, butoxy, isobutoxy, s-butoxy, t-butoxy, pentoxy, isopentoxy, 2-methylbutoxy, neopentoxy, hexyloxy, 4-methylpentoxy, 3-methylpentoxy, 2-methylpentoxy, 3,3-dimethylbutoxy, 2,2-dimethylbutoxy, 1,1-dimethylbutoxy, 1,2-dimethylbutoxy, 1,3-dimethylbutoxy or 2,3-dimethylbutoxy, among which methoxy, ethoxy, propoxy or isopropoxy is preferred, methoxy or ethoxy is more preferred, and methoxy is most preferred.

The "4- to 8-membered heterocyclic group" of the "4- to 8-membered heterocyclic group optionally substituted with a substituent selected from Group W1" for R²⁰, R²¹, R²² and R²³ is a monovalent group obtained by removing one hydrogen from any desired position of a "4- to 8-membered heterocycle" as described below.
The "4- to 8-membered heterocycle" is a non-aromatic ring (either a completely saturated ring or a partially unsaturated ring) having 4-8 atoms forming the ring and containing one or more heteroatoms among the atoms forming the ring, and as example there may be mentioned azetidine ring, a pyrrolidine ring, a piperidine ring, an azepane ring, an azocane ring, a tetrahydrofuran ring, a tetrahydropyran ring, a tetrahydrothiopyran ring, a morpholine ring, a thiomorpholine ring, a piperazine ring, a diazepane ring, a thiazolidine ring, an isoxazolidine ring, an imidazolidine ring, a pyrazolidine ring, a dioxane ring, a 1,3-dioxolane ring, an oxathiane ring, a dithiane ring, a pyran ring, a dihydropyran ring, a pyrroline ring, a dihydropyridine ring, a pyrazoline ring, an oxazoline ring, an imidazoline ring or a thiazoline ring. Preferable "4- to 8-membered heterocyclic groups" are completely saturated 4- to 8-membered heterocyclic groups, completely saturated 4- to 8-membered heterocyclic group derived by eliminating one hydrogen linked to nitrogen constituting the ring are more preferred, piperazin-1-yl, pyrrolidin-1-yl, azetidin-1-yl, thiomorpholin-4-yl, piperidin-1-yl or morpholin-4-yl is further preferred, and pyrrolidin-1-yl, piperidin-1-yl or morpholin-4-yl is most preferred.

As examples of the "4- to 8-membered heterocyclic group" of the "4- to 8-membered heterocyclic group optionally substituted with a substituent selected from Group V1" for R²⁰, R²¹, R²² and R²³ there may be mentioned the same ones listed above, among which pyrrolidine ring, piperidine ring, thiomorpholin-4-yl or 1,6-dihydropyridin-2-yl is preferred, pyrrolidin-1-yl or piperidin-1-yl is more preferred, and piperidin-1-yl is most preferred.
The "4- to 8-membered heterocyclic group optionally substituted with a substituent selected from Group V1" for R²⁰, R²¹, R²² and R²³ is not particularly restricted so long as it is the aforementioned "4- to 8-membered heterocyclic group" optionally substituted with a substituent selected from Group V1, but it is preferably a group represented by the following formula:

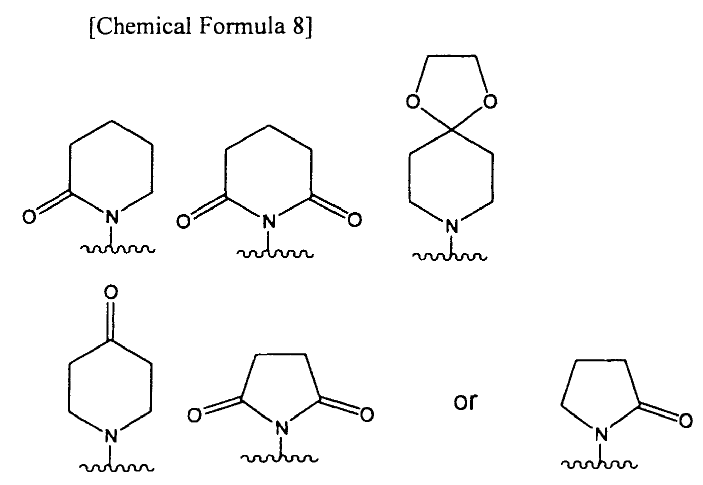

The "5- to 10-membered heteroaryl ring group" of the "5- to 10-membered heteroaryl ring group optionally substituted with a substituent selected from Group W1" for R²⁰, R²¹, R²² and R²³ is a monovalent group obtained by eliminating one hydrogen from any desired position of a "5- to 10-membered heteroaryl ring" as described below.
The "5- to 10-membered heteroaryl ring" is an aromatic ring having 5-10 atoms forming the ring and containing one or more heteroatoms among the atoms forming the ring (with regard to fused rings, at least one of the rings are aromatic), and as examples there may be mentioned a pyridine ring, a thiophene ring, a furan ring, a pyrrole ring, an oxazole ring, an isoxazole ring, a thiazole ring, a thiadiazole ring, an isothiazole ring, an imidazole ring, a triazole ring, a tetrazole ring, a pyrazole ring, a furazan ring, a thiadiazole ring, an oxadiazole ring, a pyridazine ring, a pyrimidine ring, a pyrazine ring, a triazine ring, an indole ring, an isoindole ring, an indazole ring, a quinoline ring, an isoquinoline ring, a cinnoline ring, a quinazoline ring, a quinoxaline ring, a naphthylidine ring, a phthalazine ring, a purine ring, a pteridine ring, a thienofuran ring, an imidazothiazole ring, a benzofuran ring, a benzothiophene ring, a benzoxazole ring, a benzothiazole ring, a benzothiadiazole ring, a benzimidazole ring, an imidazopyridine ring, a pyrrolopyridine ring, a pyrrolopyrimidine ring, a pyridopyrimidine ring, a coumaran ring, a chromene ring, a chromane ring, an isochromane ring, an indoline ring or an isoindoline ring. Preferable "5- to 10-membered heteroaryl ring" is a 5-to 6-membered ring, an isoxazole ring, an oxadiazole ring, a tetrazole ring, a pyridine ring, a thiazole ring or a thiophene ring is more preferred, and a pyridine ring, a thiazole ring, a thiophene ring or a tetrazole ring is most preferred.

The "6- to 10-membered aryl ring group" of the "6- to 10-membered aryl ring group optionally substituted with a substituent selected from Group W1" for R²⁰, R²¹, R²² and R²³ is an aromatic hydrocarbon ring group of 6 to 10 carbon (with regard to fused rings, at lease one of the rings are aromatic), as examples there may be mentioned phenyl, 1-naphthyl, 2-naphthyl, indenyl, indanyl, azulenyl or heptalenyl, among which phenyl is preferred.

The "C2-7 alkenyl" of the "C2-7 alkenyl optionally substituted with a substituent selected from Group W1" for R²⁰, R²¹, R²² and R²³ is straight-chain or branched-chain alkenyl groups of 2 to 7 carbon which may contain 1 or 2 double bonds, and as examples there may be mentioned ethenyl, 1-propenyl, 2-propenyl, 1-butenyl, 2-butenyl, 3-butenyl, 2-methyl-1-propenyl, 2-methyl-2-propenyl, 1-pentenyl, 1-hexenyl, 1,6-hexanedienyl or 1-heptenyl, among which C2-5 groups containing one double bond are preferred, and ethenyl is particularly preferred.

The "C2-7 alkynyl" of the "C2-7 alkynyl optionally substituted with a substituent selected from Group W1" for R²⁰, R²¹, R²² and R²³ is straight-chain or branched-chain alkynyl groups of 2 to 7 carbon which may contain 1 or 2 triple bonds, and as examples there may be mentioned ethynyl, 1-propynyl, 2-propynyl, 1-butynyl, 2-butynyl, 3-butynyl, 1-pentynyl, 1-hexynyl, 1,6-hexanediynyl or 1-heptynyl, among which C2-5 groups containing one triple bond are preferred, and ethynyl or 1-propynyl are particularly preferred.

As examples of the "3- to 8-membered cycloalkyl" of the "3- to 8-membered cycloalkyl optionally substituted with a substituent selected from Group W1" for R²⁰, R²¹, R²² and R²³, there may be mentioned the same ones listed below, among which cyclopropyl, cyclobutyl, cyclopentyl or cyclohexyl is preferred, and cyclopropyl, cyclopentyl or cyclohexyl is particularly preferred.

The "5- to 8-membered cycloalkenyl" of the "5- to 8-membered cycloalkenyl optionally substituted with a substituent selected from Group W1" for R²⁰, R²¹, R²² and R²³ may have multiple double bonds, and as examples there may be mentioned cyclopentenyl (1-cyclopentenyl, 2-cyclopentenyl or 3-cyclopentenyl), cyclohexenyl (1-cyclohexenyl, 2-cyclohexenyl or 3-cyclohexenyl), cycloheptenyl (1-cycloheptenyl, 2-cycloheptenyl, 3-cycloheptenyl or 4-cycloheptenyl) and cyclooctenyl (1-cyclooctenyl, 2-cyclooctenyl, 3-cyclooctenyl or 4-cyclooctenyl), among which cyclopentenyl or cyclohexenyl is preferred, and 1-cyclopentenyl or 1-cyclohexenyl is particularly preferred.

As examples of the "C1-6 alkyl" of the "C1-6 alkyl optionally substituted with a substituent selected from Group U1" for R^{1X} and R^{2X} there may be mentioned the same ones listed above.

As examples of the "4- to 8-membered heterocyclic group" for R^{1X} and R^{2X} there may be mentioned the same ones listed above, among which completely saturated 4- to 8-membered heterocyclic groups are preferred, a tetrahydropyran ring group, a morpholine ring group, a piperidine ring group or a pyrrolidine ring group is more preferred, and tetrahydropyran-4-yl or morpholin-4-yl is most preferred.

As examples of the "C1-6 alkyl" of the "C1-6 alkyl optionally substituted with a substituent selected from Group F1" for R^{3X} there may be mentioned the same ones listed above, among which C1-4 groups are preferred, and methyl or ethyl is particularly preferred.

"-NR^{1X}R^{2X}" for R²⁰, R²¹, R²² and R²³ is an amino group (-NH₂) having two hydrogen replaced with R^{1X} and R^{2X}.
When neither R^{1X} nor R^{2X} of "-NR^{1X}R^{2X}" for R²⁰, R²¹, R²² and R²³ is a 4- to 8-membered heterocyclic group, then "-NR^{1x}R^{2x}" is preferably methylamino, ethylamino, dimethylamino, methylethylamino, diethylamino, (methoxyethyl)amino, di(methoxyethyl)amino or methyl (methoxyethyl)amino.

"-CO-R^{1X}" for R²⁰, R²¹, R²² and R²³ is a carbonyl group having R^{1X} bonded thereto, and morpholin-4-ylcarbonyl, methoxyethylcarbonyl, ethylcarbonyl or acetyl is preferred.

"-CO-NR^{1X}R^{2X} " for R²⁰, R²¹, R²² and R²³ is a carbonyl group having -NR^{1X}R^{2X} bonded thereto.

"-NR^{1X}-CO-R^{2X}" for R²⁰, R²¹, R²² and R²³ is an amino group (-NH₂) having two hydrogen replaced with "a carbonyl group having R^{2X} bonded thereto" and R^{1X}.

"-SO₂-R^{3X}" for R²⁰, R²¹, R²² and R²³ is a sulfonyl group having R^{3X} bonded thereto.

"-O-SO₂-R^{3X}" for R²⁰, R²¹, R²² and R²³ is a sulfonyloxy group having R^{3X} bonded thereto.

As examples of the "halogen" for R³⁰, R³¹ and R³² there may be mentioned the same ones listed above, among which fluorine or chlorine is preferred.

As examples of the "C1-6 alkyl" for R³⁰, R³¹ and R³² there may be mentioned the same ones listed above, among which C1-4 groups are preferred, and methyl is particularly preferred.

As examples of the "C1-6 alkoxy" for R³⁰, R³¹ and R³² there may be mentioned the same ones listed above, among which C1-4 groups are preferred, and methoxy is particularly preferred.

The "C2-7 alkoxycarbonyl" for R³⁰, R³¹ and R³² is a carbonyl group having the aforementioned "C1-6 alkoxy" bonded thereto, and as examples there may be mentioned straight-chain or branched-chain groups such as methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentoxycarbonyl, isopentoxycarbonyl, 2-methylbutoxycarbonyl, neopentoxycarbonyl, hexyloxycarbonyl, 4-methylpentoxycarbonyl, 3-methylpentoxycarbonyl, 2-methylpentoxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl or 2,3-dimethylbutoxycarbonyl, among which methoxycarbonyl or ethoxycarbonyl is preferred.

Two of R³⁰, R³¹ and R³² "bond together to form oxo (=O)" means that two of R³⁰, R³¹ and R³² bond to the same carbon atom to form an oxo group.

Two of R³⁰, R³¹ and R³² "bond together to form methylene (-CH₂-)" means that two of R³⁰, R³¹ and R³² bond to different carbon atoms to form a methylene group.

As examples of the "C1-10 alkyl" of the "C1-10 alkyl optionally substituted with a substituent selected from Group D1" for R⁴⁰ there may be mentioned the same examples as listed for "C1-6 alkyl" above, as well as C7-10 straight-chain or branched-chain alkyl groups such as heptyl, 3-methylhexyl, octyl, nonyl or decyl, among which C1-6 groups are preferred, and methyl, ethyl, propyl, isopropyl, isobutyl, butyl or pentyl is particularly preferred.

As examples of the "3- to 8-membered cycloalkyl" of the "3- to 8-membered cycloalkyl substituted with a substituent selected from Group E1" for R⁴⁰ there may be mentioned cyclopropyl, cyclobutyl, cyclopentyl, cyclohexyl, cycloheptyl or cyclooctyl, among which cyclopropyl, cyclobutyl, cyclopentyl, cyclohexyl or cycloheptyl is preferred, and cyclobutyl, cyclopentyl or cyclohexyl is particularly preferred.

As exampled of the "4- to 8-membered heterocyclic group" of the "4- to 8-membered heterocyclic group optionally substituted with a substituent selected from Group E1" for R⁴⁰ may be mentioned the same ones listed above, among which a pyrrolidine ring group, a piperidine ring group, a tetrahydrothiopyran ring group or a tetrahydropyran ring group is preferred.

The "C2-7 alkenyl" of the "C2-7 alkenyl optionally substituted with a substituent selected from Group F1" for R⁴⁰ is straight-chain or branched-chain alkenyl groups of 2 to 7 carbon which may contain 1 or 2 double bonds, and as examples there may be mentioned ethenyl, 1-propenyl, 2-propenyl, 1-butenyl, 2-butenyl, 3-butenyl, 2-methyl-1-propenyl, 2-methyl-2-propenyl, 1-pentenyl, 1-hexenyl, 1,6-hexanedienyl or 1-heptenyl, among which C2-5 groups are preferred, and 2-propenyl or 2-methyl-2-propenyl is particularly preferred.

The "C2-7 alkynyl" of the "C2-7 alkynyl optionally substituted with a substituent selected from Group F1" for R⁴⁰ is straight-chain or branched-chain alkynyl groups of 2 to 7 carbon which may contain 1 or 2 triple bonds, and as examples there may be mentioned ethynyl, 1-propynyl, 2-propynyl, 1-butynyl, 2-butynyl, 3-butynyl, 1-pentynyl, 1-hexynyl, 1,6-hexanediynyl or 1-heptynyl, among which C2-5 groups are preferred, and 2-butynyl or 2-propynyl is particularly preferred.

The "C2-7 alkylcarbonyl group" of the "C2-7 alkylcarbonyl group optionally substituted with a substituent selected from Group G1" for R⁴⁰ is a carbonyl having the aforementioned "C1-6 alkyl" bonded thereto, and as examples there may be mentioned straight-chain or branched-chain groups such as acetyl, ethylcarbonyl, n-propylcarbonyl, isopropylcarbonyl, n-butylcarbonyl, isobutylcarbonyl, s-butylcarbonyl, t-butylcarbonyl, pentylcarbonyl, isopentylcarbonyl, 2-methylbutylcarbonyl, neopentylcarbonyl, 1-ethylpropylcarbonyl, hexylcarbonyl, isohexylcarbonyl, 4-methylpentylcarbonyl, 3-methylpentylcarbonyl, 2-methylpentylcarbonyl, 1-methylpentylcarbonyl, 3,3-dimethylbutylcarbonyl, 2,2-dimethylbutylcarbonyl, 1,1-dimethylbutylcarbonyl, 1,2-dimethylbutylcarbonyl, 1,3-dimethylbutylcarbonyl, 2,3-dimethylbutylcarbonyl, 1-ethylbutylcarbonyl or 2-ethylbutylcarbonyl, among which C2-5 groups are preferred, and acetyl or propylcarbonyl is particularly preferred.

The "mono(C1-6 alkyl)aminocarbonyl" for R⁴⁰ is a carbonyl group having "mono(C1-6 alkyl)amino" bonded thereto, and as examples there may be mentioned straight-chain or branched-chain groups such as methylaminocarbonyl, ethylaminocarbonyl, propylaminocarbonyl, isopropylaminocarbonyl, butylaminocarbonyl, isobutylaminocarbonyl, s-butylaminocarbonyl, t-butylaminocarbonyl, pentylaminocarbonyl, isopentylaminocarbonyl, 2-methylbutylaminocarbonyl, neopentylaminocarbonyl, 1-ethylpropylaminocarbonyl, hexylaminocarbonyl, isohexylaminocarbonyl, 4-methylpentylaminocarbonyl, 3-methylpentylaminocarbonyl, 2-methylpentylaminocarbonyl, 1-methylpentylaminocarbonyl, 3,3-dimethylbutylaminocarbonyl, 2,2-dimethylbutylaminocarbonyl, 1,1-dimethylbutylaminocarbonyl, 1,2-dimethylbutylaminocarbonyl, 1,3-dimethylbutylaminocarbonyl, 2,3-dimethylbutylaminocarbonyl, 1-ethylbutylaminocarbonyl or 2-ethylbutylaminocarbonyl, among which C2-5 (total number of carbon) groups are preferred, and ethylaminocarbonyl is particularly preferred.

The "4- to 8-membered heterocyclic carbonyl" for R⁴⁰ is a carbonyl group having the aforementioned "4- to 8-membered heterocyclic group" bonded thereto, among which piperidin-1-ylcarbonyl or morpholin-4-ylcarbonyl is preferred.

As examples of the "C2-7 alkoxycarbonyl" for R⁴⁰ there may be mentioned the same ones listed above, among which methoxycarbonyl or ethoxycarbonyl is preferred.

The "C1-6 alkylsulfonyl" for R⁴⁰ is a sulfonyl group having the aforementioned "C1-6 alkyl" bonded thereto, as examples there may be mentioned straight-chain or branched-chain groups such as methylsulfonyl, ethylsulfonyl, propylsulfonyl, isopropylsulfonyl, carbonylbutylsulfonyl, isobutylsulfonyl, s-butylsulfonyl, t-butylsulfonyl, pentylsulfonyl, isopentylsulfonyl, 2-methylbutylsulfonyl, neopentylsulfonyl, 1-ethylpropylsulfonyl, hexylsulfonyl, isohexylsulfonyl, 4-methylpentylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, 1,3-dimethylbutylsulfonyl, 2,3-dimethylbutylsulfonyl, 1-ethylbutylsulfonyl or 2-ethylbutylsulfonyl, among which propylsulfonyl is preferred.

As examples of the "halogen" for Group A1 may be mentioned the same ones listed above, among which bromine, fluorine or chlorine is preferred.

As examples of the "C1-6 alkoxy" of Group A1 there may be mentioned the same ones listed above, among which C1-4 groups are preferred, and methoxy is particularly preferred.

As examples of the "C1-6 alkyl" of Group A1 and Group A2 (mentioned below) may be mentioned the same ones listed above, among which C1-4 groups are preferred, and methyl, ethyl, n-butyl or t-butyl is particularly preferred.

The "C1-6 haloalkyl" of Group A1 and Group A2 (mentioned below) is aforementioned "C1-6 alkyl" having 1-6 aforementioned "halogen" bonded thereto, as examples there may be mentioned straight-chain or branched-chain alkyl groups such as methyl, ethyl, n-propyl, isopropyl, n-butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl or 2-ethylbutyl, having fluorine or chlorine bonded thereto, among which C1-4 alkyl having 1 to 3 fluorine or chlorine bonded thereto is preferred, and trifluoromethyl is particularly preferred.

As examples of the "C2-7 alkylene", where C2-7 alkylene is permissible only in the case that a spiro union is formed together with the substituted 5- to 10-membered cycloalkyl or the substituted 5- to 10-membered cycloalkenyl, of Group A1 and Group A2 (mentioned below), there may be mentioned straight-chain or branched-chain groups such as 1,2-ethylene, trimethylene, propylene, ethylethylene, tetramethylene, pentamethylene, hexamethylene or heptamethylene, among which 1,2-ethylene, trimethylene, tetramethylene or pentamethylene is preferred, and 1,2-ethylene, tetramethylene or pentamethylene is particularly preferred.

As examples of the "halogen" of Group W1 there may be mentioned the same ones listed above, among which fluorine or chlorine is preferred.

As examples of the "C1-6 alkyl" of Group W1 there may be mentioned the same ones listed above, among which C1-4 groups are preferred, and methyl or ethyl is particularly preferred.

The "C2-7 alkoxyalkyl" of Group W1 is aforementioned "C1-6 alkyl" having the aforementioned "C1-6 alkoxy" bonded thereto, of 2 to 7 carbon (total number of carbon), and as examples there may be mentioned methoxymethyl, methoxyethyl, ethoxyethyl, methoxypropyl or propoxyethyl, among which methoxymethyl is preferred.

As examples of the "C1-6 alkoxy" of the "C1-6 alkoxy optionally substituted with a substituent selected from Group T1" of Group W 1 there may be mentioned the same ones listed above, among which C1-4 groups are preferred, and methoxy, ethoxy, isopropoxy or propoxy is particularly preferred.

As examples of the "C2-7 alkoxycarbonyl" of Group W1 there may be mentioned the same ones listed above, among which methoxycarbonyl or ethoxycarbonyl is preferred.

As examples of the "C2-7 alkylcarbonyl" of Group W1 there may be mentioned the same ones listed above, among which C2-5 groups are preferred, and acetyl is particularly preferred.

As examples of the "C1-6 alkyl" for R^{6X} and R^{7X} there may be mentioned the same ones listed above.

As examples of the "halogen" of Group Cl there may be mentioned the same ones listed above, among which bromine, fluorine or chlorine is preferred.

As examples of the "C1-6 alkyl" of Group C1 there may be mentioned the same ones listed above, among which C1-4 groups are preferred, and methyl is particularly preferred.

As examples of the "C1-6 alkoxy" of Group C 1 there may be mentioned the same ones listed above, among which C1-4 groups are preferred, and methoxy or ethoxy is particularly preferred.

As examples of the "halogen" of Group D1 and Group D2 (mentioned below) there may be mentioned the same ones listed above, among which fluorine or chlorine is preferred.

As examples of the "C1-6 alkoxy" of Group D1 and Group D2 (mentioned below) there may be mentioned the same ones listed above, among which C1-4 groups are preferred, and methoxy or ethoxy is particularly preferred.

The "C1-6 alkylthio" of Group D1 is a thio group having the aforementioned "C1-6 alkyl" bonded thereto, and as examples there may be mentioned straight-chain or branched-chain groups such as methylthio, ethylthio, propylthio, isopropylthio, carbonylbutylthio, isobutylthio, s-butylthio, t-butylthio, pentylthio, isopentylthio, 2-methylbutylthio, neopentylthio, 1-ethylpropylthio, hexylthio, isohexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio or 2-ethylbutylthio, among which C1-4 groups are preferred, and methylthio or ethylthio is particularly preferred.

As examples of the "C1-6 alkylsulfonyl" of Group D1 there may be mentioned the same ones listed above, among which C1-4 groups are preferred, and methylsulfonyl or ethylsulfonyl is particularly preferred.

The "C1-6 alkylsulfinyl" of Group D1 is a sulfinyl group having the aforementioned "C1-6 alkyl" bonded thereto, as examples there may be mentioned straight-chain or branched-chain groups such as methylsulfinyl, ethylsulfinyl, propylsulfinyl, isopropylsulfinyl, carbonylbutylsulfinyl, isobutylsulfinyl, s-butylsulfinyl, t-butylsulfinyl, pentylsulfinyl, isopentylsulfinyl, 2-methylbutylsulfinyl, neopentylsulfinyl, 1-ethylpropylsulfinyl, hexylsulfinyl, isohexylsulfinyl, 4-methylpentylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, 1,3-dimethylbutylsulfinyl, 2,3-dimethylbutylsulfinyl, 1-ethylbutylsulfinyl or 2-ethylbutylsulfinyl, among which C1-4 groups are preferred, and methylsulfinyl or ethylsulfinyl is particularly preferred.

As examples of the "mono(C1-6 alkyl)amino" of Group D1 there may be mentioned straight-chain or branched-chain groups such as methylamino, ethylamino, propylamino, isopropylamino, butylamino, isobutylamino, s-butylamino, t-butylamino, pentylamino, isopentylamino, 2-methylbutylamino, neopentylamino, 1-ethylpropylamino, hexylamino, isohexylamino, 4-methylpentylamino, 3-methylpentylamino, 2-methylpentylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino, 1-ethylbutylamino or 2-ethylbutylamino, among which methylamino or ethylamino is preferred, and methylamino is particularly preferred.

The "di(C1-6 alkyl)amino" of Group D1 may be either symmetric or asymmetric, and as examples there may be mentioned straight-chain or branched-chain groups such as dimethylamino, methylethylamino, diethylamino, methylpropylamino, ethylpropylamino, dipropylamino, diisopropylamino, dibutylamino, diisobutylamino, di(s-butyl)amino, di(t-butyl)amino, methylpentylamino, dipentylamino, diisopentylamino, di(2-methylbutyl)amino, di(neopentyl)amino, di(1-ethylpropyl)amino, dihexylamino, methylisohexylamino, diisohexylamino, di(4-methylpentyl)amino, di(3-methylpentyl)amino, di(2-methylpentyl)amino, di(1-methylpentyl)amino, di(3,3-dimethylbutyl)amino, di(2,2-dimethylbutyl)amino, di(1,1-dimethylbutyl)amino, di(1,2-dimethylbutyl)amino, di(1,3-dimethylbutyl)amino, di(2,3-dimethylbutyl)amino, di(1-ethylbutyl)amino or di(2-ethylbutyl)amino, among which dimethylamino, methylethylamino or diethylamino is preferred, and dimethylamino is particularly preferred.

The "C2-7 alkylcarbonylamino" of Group D1 is an amino group having the aforementioned "C2-7 alkylcarbonyl" bonded thereto, and as examples there may be mentioned straight-chain or branched-chain groups such as acetylamino, ethylcarbonylamino, n-propylcarbonylamino, isopropylcarbonylamino, n-butylcarbonylamino, isobutylcarbonylamino, s-butylcarbonylamino, t-butylcarbonylamino, pentylcarbonylamino, isopentylcarbonylamino, 2-methylbutylcarbonylamino, neopentylcarbonylamino, 1-ethylpropylcarbonylamino, hexylcarbonviamino, isohexylcarbonylamino, 4-methylpentylcarbonylamino, 3-methylpentylcarbonylamino, 2-methylpentylcarbonylamino, 1-methylpentylcarbonylamino, 3,3-dimethylbutylcarbonylamino, 2,2-dimethylbutylcarbonylamino, 1,1-dimethylbutylcarbonylamino, 1,2-dimethylbutylcarbonylamino, 1,3-dimethylbutylcarbonylamino, 2,3-dimethylbutylcarbonylamino, 1-ethylbutylcarbonylamino or 2-ethylbutylcarbonylamino, among which amino groups having C2-5 alkylcarbonyl bonded thereto are preferred, and acetylamino or ethylcarbonylamino is particularly preferred.

As examples of the "3- to 8-membered cycloalkyl" of the "3- to 8-membered cycloalkyl optionally substituted with a substituent selected from Group H1" of Group D1 and the "3- to 8-membered cycloalkyl" of Group D2 (mentioned below) there may be mentioned the same ones listed above, among which cyclopropyl, cyclobutyl, cyclopentyl or cyclohexyl is preferred, cyclopropyl or cyclobutyl is more preferred, and cyclopropyl is most preferred.

As exampled of the "C2-7 alkoxycarbonyl" of Group D1 there may be mentioned the same ones listed above, among which methoxycarbonyl or ethoxycarbonyl is preferred.

As examples of the "4- to 8-membered heterocyclic group" of Group D1 and Group D2 (mentioned below) there may be mentioned the same ones listed above, among which a tetrahydropyran ring group or a tetrahydrofuran ring group is preferred, and tetrahydropyran-4-yl is particularly preferred.

As examples of the "5- to 10-membered heteroaryl ring group" of Group D1 there may be mentioned the same ones listed above, among which furyl, thienyl, pyridyl, pyrazyl, pyrimidinyl or pyridazinyl is preferred, and furyl, thienyl or pyridyl is particularly preferred.

The "6- to 10-membered aryl ring group" of Group D1 is an aromatic hydrocarbon ring group of 6 to 10 carbon (with regard to fused rings, at least one of the rings are aromatic), as examples there may be mentioned phenyl, 1-naphthyl, 2-naphthyl, indenyl, indanyl, azulenyl or heptalenyl, among which phenyl, 1-naphthyl or 2-naphthyl is preferred, and phenyl is particularly preferred.

As examples of the "C2-7 alkylcarbonyl" of Group D1 and Group D2 (mentioned below) there may be mentioned the same ones listed above, among which C2-5 groups are preferred, and acetyl or ethylcarbonyl is particularly preferred.

The "6- to 10-membered aryl ring carbonyl group" of Group D1 is a carbonyl group having the aforementioned "6- to 10-membered aryl ring group" bonded thereto, as examples there may be mentioned benzoyl, 1-naphthoyl. 2-naphthoyl, indenylcarbonyl, indanylcarbonyl, azulenylcarbonyl or heptalenylcarbonyl, among which benzoyl, 1-naphthoyl or 2-naphthoyl is preferred, and benzoyl is particularly preferred.

As examples of the "mono(C1-6 alkyl)aminocarbonyl" of the "mono(C1-6 alkyl)aminocarbonyl optionally substituted with halogen" of Group D1 and the "mono(C1-6 alkyl)aminocarbonyl" of Group D2 (mentioned below) there may be mentioned the same ones listed above, among which C2-5 (total number of carbon) groups are preferred, methylaminocarbonyl, ethylaminocarbonyl, propylaminocarbonyl, isopropylaminocarbonyl or butylaminocarbonyl is more preferred, and methylaminocarbonyl, ethylaminocarbonyl, propylaminocarbonyl or isopropylaminocarbonyl is most preferred.

As examples of the "mono(3- to 8-membered cycloalkyl)aminocarbonyl" of Group D1, there may be mentioned cyclopropylaminocarbonyl, cyclobutylaminocarbonyl, cyclopentylaminocarbonyl, cyclohexylaminocarbonyl, cycloheptylaminocarbonyl or cyclooctylaminocarbonyl, among which cyclopropylaminocarbonyl, cyclobutylaminocarbonyl, cyclopentylaminocarbonyl or cyclohexylaminocarbonyl is preferred, and cyclopropylaminocarbonyl is particularly preferred.

The "mono(C2-7 alkoxyalkyl)aminocarbonyl" of Group D1 is an aminocarbonyl group having "C2-7 alkoxyalkyl" bonded thereto, where "C2-7 alkoxyalkyl" is aforementioned "C1-6 alkyl" having the aforementioned "C1-6 alkoxy" bonded thereto, of 2 to 7 carbon (total number of carbon). As examples of the "mono(C2-7 alkoxyalkyl)aminocarbonyl" there may be mentioned methoxymethylaminocarbonyl, methoxyethylaminocarbonyl, ethoxyethylaminocarbonyl, methoxypropylaminocarbonyl or propoxyethylaminocarbonyl, among which methoxyethylaminocarbonyl is preferred.

The "di(C1-6 alkyl)aminocarbonyl" of Group D1 and Group D2 (mentioned below) is a carbonyl group having the aforementioned "di(C1-6 alkyl)amino" bonded thereto, and as preferred examples there may be mentioned straight-chain or branched-chain groups such as dimethylaminocarbonyl, methylethylaminocarbonyl, diethylaminocarbonyl, methylpropylaminocarbonyl, ethylpropylaminocarbonyl, dipropylaminocarbonyl, diisopropylaminocarbonyl, dibutylaminocarbonyl, diisobutylaminocarbonyl, di(s-butyl)aminocarbonyl, di(t-butyl)aminocarbonyl, methylpentylaminocarbonyl, dipentylaminocarbonyl, diisopentylaminocarbonyl, di(2-methylbutyl)aminocarbonyl, di(neopentyl)aminocarbonyl, di(1-ethylpropyl)aminocarbonyl, dihexylaminocarbonyl, methylisohexylaminocarbonyl, diisohexylaminocarbonyl, di(4-methylpentyl)aminocarbonyl, di(3-methylpentyl)aminocarbonyl, di(2-methylpentyl)aminocarbonyl, di(1-methylpentyl)aminocarbonyl, di(3,3-dimethylbutyl)aminocarbonyl, di(2,2-dimethylbutyl)aminocarbonyl, di(1,1-dimethylbutyl)aminocarbonyl, di(1,2-dimethylbutyl)aminocarbonyl, di(1,3-dimethylbutyl)aminocarbonyl, di(2,3-dimethylbutyl)aminocarbonyl, di(1-ethylbutyl)aminocarbonyl or di(2-ethylbutyl)aminocarbonyl, among which dimethylaminocarbonyl, methylethylaminocarbonyl or diethylaminocarbonyl is preferred, and dimethylaminocarbonyl is particularly preferred.

The "mono(5- to 10-membered heteroaryl ring)aminocarbonyl" of Group D1 is an aminocarbonyl group (carbamoyl) having one hydrogen replaced with the aforementioned "5- to 10-membered heteroaryl ring", and as examples of the "5- to 10-membered heteroaryl ring" there may be mentioned a pyridine ring, a thiophene ring, a furan ring, a pyrrole ring, an oxazole ring, an isoxazole ring, a thiazole ring, a thiadiazole ring, an isothiazole ring, an imidazole ring, a triazole ring, a tetrazole ring, a pyrazole ring, a furazan ring, a thiadiazole ring, an oxadiazole ring, a pyridazine ring, a pyrimidine ring, a pyrazine ring, a triazine ring, an indole ring, an isoindole ring, an indazole ring, a quinoline ring, an isoquinoline ring, a cinnoline ring, a quinazoline ring, a quinoxaline ring, a naphthylidine ring, a phthalazine ring, a purine ring, a pteridine ring, a thienofuran ring, an imidazothiazole ring, a benzofuran ring, a benzothiophene ring, a benzoxazole ring, a benzothiazole ring, a benzothiadiazole ring, a benzimidazole ring, an imidazopyridine ring, a pyrrolopyridine ring, a pyrrolopyrimidine ring, a pyridopyrimidine ring, a coumaran ring, a chromene ring, a chromane ring, an isochromane ring, an indoline ring or an isoindoline ring. Preferable mono(5- to 10-membered heteroaryl ring)aminocarbonyl group is pyridin-2-ylaminocarbonyl.

As examples of the "4- to 8-membered heterocyclic carbonyl" of the "4- to 8-membered heterocyclic carbonyl optionally substituted with C1-6 alkyl" of Group D1 and the "4- to 8-membered heterocyclic carbonyl" of Group D2 (mentioned below) there may be mentioned the same ones listed above, among which pyrrolidin-1-ylcarbonyl, azepan-1-ylcarbonyl, azocan-1-ylcarbonyl, piperidin-1-ylcarbonyl or morpholin-4-ylcarbonyl is preferred, and pyrrolidin-1-ylcarbonyl, piperidin-1-ylcarbonyl or morpholin-4-ylcarbonyl is particularly preferred.

The "5- to 10-membered heteroaryl ring carbonyl" of Group D1 is a carbonyl group having the aforementioned "5- to 10-membered heteroaryl ring group" bonded thereto.

The "5-membered heteroaryl ring group" of Group D2 (mentioned below) is a group, the number of atoms constituting the ring is 5 in the aforementioned "5- to 10-membered heteroaryl ring group" of Group D1, and as examples there may be mentioned thienyl, furyl, pyrrolyl, oxazolyl, isoxazolyl, thiazolyl, thiadiazolyl, isothiazolyl, imidazolyl, triazolyl, pyrazolyl, furazanyl or oxadiazolyl, among which thienyl or furyl is preferred.

As examples of the "halogen" of Group E1 there may be mentioned the same ones listed above, among which bromine, fluorine or chlorine is preferred.

As examples of the "C1-6 alkoxy" of Group E1 there may be mentioned the same ones listed above, among which C1-4 groups are preferred, and methoxy is particularly preferred.

As examples of the "C1-6 alkyl" of Group E1 there may be mentioned the same ones listed above, among which C1-4 groups are preferred, and methyl is particularly preferred.

As examples of the "halogen" of Group F1 there may be mentioned the same ones listed above, among which fluorine or chlorine is preferred.

As examples of the "C1-6 alkoxy" of Group F1 there may be mentioned the same ones listed above, among which C1-4 groups are preferred, and methoxy is particularly preferred.

As examples of the "3- to 8-membered cycloalkyl" of Group G1 there may be mentioned the same ones listed above, among which cyclohexyl or cyclopropyl is particularly preferred.

As examples of the "C1-6 haloalkyl" of Group H1 there may be mentioned the same ones listed above, among which chloromethyl or fluoromethyl is particularly preferred.

As examples of the "C1-6 alkyl" of Group H1 there may be mentioned the same ones listed above, among which C1-4 groups are preferred, and methyl is particularly preferred.

As examples of the "C2-7 alkoxyalkyl" of Group H1 there may be mentioned the same ones listed above, among which methoxymethyl is preferred.

As examples of the "mono(C1-6 alkyl)aminocarbonyl" of Group H1 there may be mentioned the same ones listed above, among which methylaminocarbonyl is preferred.

As examples of the "di(C1-6 alkyl)aminocarbonyl''' of Group H1 there may be mentioned the same ones listed above, among which dimethylaminocarbonyl or diethylaminocarbonyl is preferred, and dimethylaminocarbonyl is particularly preferred.

As examples of the "C2-7 alkoxycarbonyl" of Group H1 there may be mentioned the same ones listed above, among which methoxycarbonyl or ethoxycarbonyl is preferred.

The "C2-7 cyanoalkyl" of Group H1 is aforementioned "C1-6 alkyl" having a cyano group bonded thereto, as examples there may be mentioned straight-chain or branched-chain alkyl groups such as methyl, ethyl, n-propyl, isopropyl, n-butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl or 2-ethylbutyl, having cyano groups bonded thereto, among which C1-4 alkyl having cyano group bonded thereto is preferred, and cyanomethyl is particularly preferred.

As examples of the "C1-6 alkoxy" of Group T1 there may be mentioned the same ones listed above.

As examples of the "C2-7 alkoxycarbonyl" of Group T1 there may be mentioned the same ones listed above.

As examples of the "C1-6 alkyl" for R^{4X} and R^{5X} there may be mentioned the same ones listed above.

As examples of the "4- to 8-membered heterocyclic group" of Group K1 there may be mentioned the same ones listed above, among which a piperidine ring group, a tetrahydropyran ring group or a morpholine ring group is preferred, and tetrahydropyran-4-yl or morpholin-4-yl is particularly preferred.

As examples of the "C1-6 alkoxy" of Group U 1 there may be mentioned the same ones listed above.

As examples of the "C2-7 alkoxycarbonyl" of Group U1 there may be mentioned the same ones listed above.

As examples of the "halogen" of Group U1 there may be mentioned the same ones listed above.

As examples of the "6- to 10-membered aryl ring group" of Group U1 there may be mentioned the same ones listed above.

As examples of the "C1-6 alkyl" for R^{8X} and R^{9X} there may be mentioned the same ones listed above.

As examples of the "C1-6 alkyl" for R^{1Z} there may be mentioned the same ones listed above, among which C1-4 groups are preferred, and methyl is particularly preferred.

The term "optionally substituted with a substituent" as used herein has the same meaning as "optionally substituted with 1-6 substituents of 1 or 2 or more kinds at any desired combination at substitutable positions", so long as the number and kind of substituents is not particularly restricted.

Several of the structural formulas for the compounds throughout the present specification represent only one isomeric form for convenience, but the invention encompasses any and all of the geometric isomers as well as optical isomers based on asymmetric carbons, stereoisomers and tautomers, and mixtures of those isomers, which are implied by the structures of the compounds, without being limited to any of the structural formulas, and they may be in the form of one of the isomers or a mixture thereof. The compounds of the invention therefore include all those having asymmetric carbons in the molecule and existing in optically active or racemic form. Some of the compounds may exist in polymorphic crystalline forms, and the compounds may be in one crystalline form or a mixture of different crystalline forms. The invention also encompasses anhydrides and hydrates of the compounds of the invention and their salts. Also encompassed within the scope of the invention are metabolites of the compounds of general formulas (1) and (100) of the invention, produced by decomposition of the compounds *in vivo.* In addition, the invention further encompasses compounds which are metabolized *in vivo* by oxidation, reduction, hydrolysis, conjugation or the like to produce the compounds of general formulas (1) and (100) of the invention (i.e., "prodrugs").

The term "salt" as used herein is not particularly restricted so long as a salt is formed with a compound of the invention, and the salt is pharmacologically acceptable, and as examples there may be mentioned inorganic acid salts, organic acid salts, inorganic base salts, organic base salts, and acidic or basic amino acid salts.
As preferred examples of inorganic acid salts there may be mentioned hydrochloride, hydrobromide, sulfate, nitrate and phosphate, and as preferred examples of organic acid salts there may be mentioned acetate, succinate, fumarate, maleate, tartarate, citrate, lactate, stearate, benzoate, methanesulfonate, ethanesulfonate, benzenesulfonate and p-toluenesulfonate.
As preferred examples of inorganic base salts there may be mentioned alkali metal salts (sodium salts and potassium salts, etc.), alkaline earth metal salts (calcium and magnesium salts, etc.), aluminum salts and ammonium salts, and as preferred examples of organic base salts there may be mentioned diethylamine salts, diethanolamine salts, meglumine salts and N,N'-dibenzylethylenediamine salts.
As preferred examples of acidic amino acid salts there may be mentioned aspartate and glutamate, and as preferred examples of basic amino acid salts there may be mentioned arginine salts, lysine salts and ornithine salts.

As preferred aspects of the compound of [1] or [2] above, the salt thereof or the hydrate of the foregoing, there may be mentioned compounds according to the following [2-2] and [3]-[23], as well as the salts thereof and the hydrates of the foregoing.

[2-2] The compound according to [1] or [2], the salt thereof or the hydrate of the foregoing, with the exception of compounds wherein R²⁰, R²¹, R²² and R²³ are all hydrogen.

[3] The compound according to [1] or [2], the salt thereof or the hydrate of the foregoing, wherein R¹⁰ is 5- to 10-membered cycloalkyl optionally substituted with a substituent from Group A2. or 5- to 10-membered cycloalkenyl optionally substituted with a substituent from Group A2,
wherein Group A2 consists of hydroxyl, phenyl, C1-6 alkyl, C1-6 haloalkyl and C2-7 alkylene where C2-7 alkylene is permissible only in the case that a spiro union is formed together with the substituted 5- to 10-membered cycloalkyl or the substituted 5- to 10-membered cycloalkenyl.

[4] The compound according to [1] or [2], the salt thereof or the hydrate of the foregoing, wherein R¹⁰ is 5- to 10-membered cycloalkyl optionally substituted with hydroxyl, phenyl, C1-6 alkyl, C1-6 haloalkyl, 1,2-ethylene, trimethylene, tetramethylene or pentamethylene, or 5- to 10-membered cycloalkenyl optionally substituted with hydroxyl, phenyl, C1-6 alkyl, C1-6 haloalkyl, 1,2-ethylene, trimethylene, tetramethylene or pentamethylene, where 1,2-ethylene, trimethylene, tetramethylene or pentamethylene is permissible only in the case that a spiro union is formed together with the 5- to 10-membered cycloalkyl or 5- to 10-membered cycloalkenyl.

[5] The compound according to [1] or [2], the salt thereof or the hydrate of the foregoing, wherein R¹⁰ is cyclohexyl, 4-t-butylcyclohexyl, 4,4-dimethylcyclohexyl, 4,4-diethylcyclohexyl, 3,3,5,5-tetramethylcyclohexyl, 3,5-dimethylcyclohexyl, 4-phenylcyclohexyl, 4-trifluoromethylcyclohexyl, 4-n-butylcyclohexyl, cyclopentyl, 3,3,4,4-tetramethylcyclopentyl, cycloheptyl, cyclooctyl, or a group represented by the following formula:

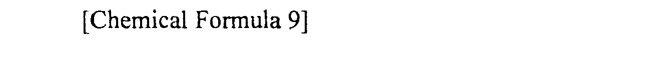

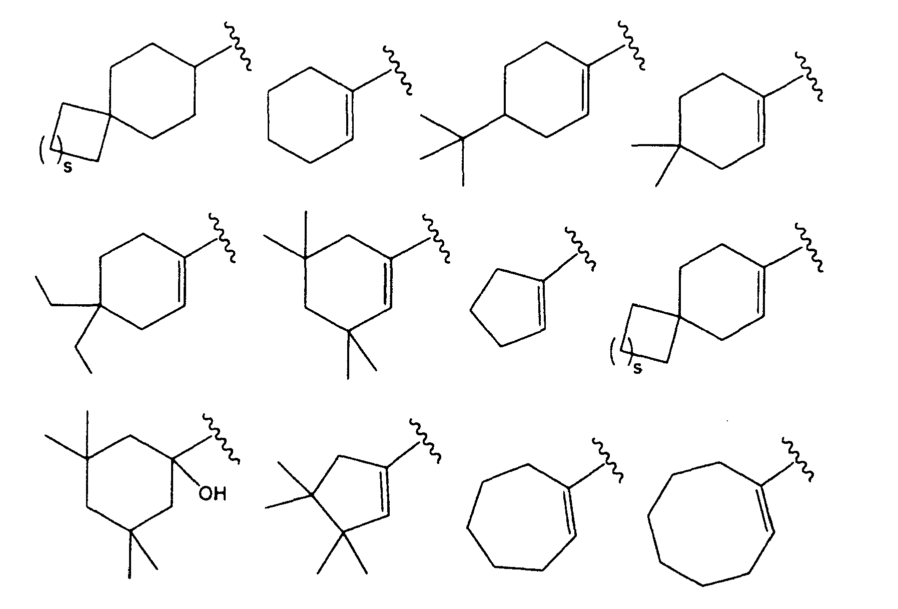

or 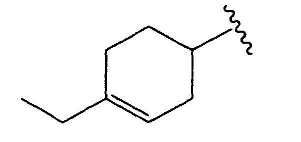 wherein s is an integer of 0, 1, 2 or 3.

[6] The compound according to any one of [1] to [5], the salt thereof or the hydrate of the foregoing, wherein R³⁰, R³¹ and R³² may be the same or different and each represents hydrogen or C1-6 alkyl, or R³⁰ and R³¹ bond together to form oxo (=O) and R³² is hydrogen or C 1-6 alkyl.

[7] The compound according to any one of [1] to [5], the salt thereof or the hydrate of the foregoing, wherein R³⁰, R³¹ and R³² may be the same or different and each represents hydrogen or methyl, or R³⁰ and R³¹ bond together to form oxo (=O) and R³² is hydrogen or methyl.

[8] The compound according to any one of [1] to [5], the salt thereof or the hydrate of the foregoing, wherein R³⁰, R³¹ and R³² are all hydrogen.

[9] The compound according to any one of [1] to [8], the salt thereof or the hydrate of the foregoing, wherein R⁴⁰ is C1-6 alkyl optionally substituted with a substituent selected from Group D1, 3- to 8-membered cycloalkyl optionally substituted with a substituent selected from Group E1, C2-7 alkenyl, C2-7 alkynyl or C2-7 alkylcarbonyl wherein Group D1 and Group E1 have the same respective definitions as Group D1 and Group E1 in [1].

[10] The compound according to any one of [1] to [8], the salt thereof or the hydrate of the foregoing, wherein R⁴⁰ is C1-6 alkyl optionally substituted with a substituent selected from Group D2.
wherein Group D2 consists of hydroxyl, halogen, cyano, C1-6 alkoxy, 3- to 8-membered cycloalkyl, a 4- to 8-membered heterocyclic group, mono(C1-6 alkyl)aminocarbonyl, di(C1-6 alkyl)aminocarbonyl, C2-7 alkylcarbonyl, a 5-membered heteroaryl ring group, 4- to 8-membered heterocyclic carbonyl and phenyl.

[11] The compound according to any one of [1] to [8], the salt thereof or the hydrate of the foregoing, wherein R⁴⁰ is n-propyl, n-butyl, n-pentyl, isobutyl, ethylcarbonylmethyl, methoxyethyl, ethoxyethyl, cyclopropylmethyl or tetrahydropyran-4-ylmethyl.

[12] The compound according to any one of [1] to [11], the salt thereof or the hydrate of the foregoing, wherein n is an integer of 1.

[13] The compound according to any one of [1] and [3] to [12], the salt thereof or the hydrate of the foregoing, wherein X¹ is nitrogen.

[14] The compound according to any one of [1] to [13], the salt thereof or the hydrate of the foregoing, wherein (i) R²⁰ and R²¹, (ii) R²¹ and R²² or (iii) R²² and R²³ bond together to form a ring selected from Group Z1 wherein Group Z1 has the same definition as Group Z1 in [1].

[15] The compound according to any one of [1] to [13], the salt thereof or the hydrate of the foregoing, wherein (i) R²¹ and R²² or (ii) R²² and R²³ bond together to form a ring selected from Group Z2,
wherein Group Z2 consists of

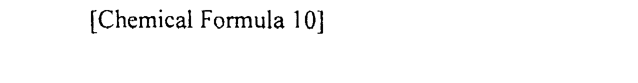

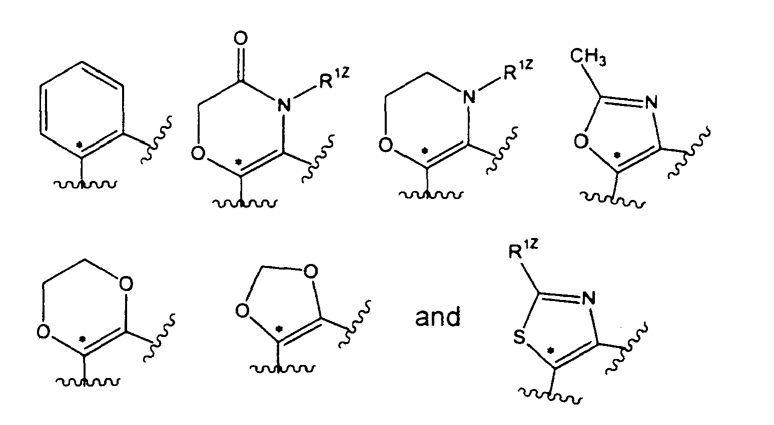

wherein R^{1Z} represents hydrogen, C1-6 alkyl or benzyl, and the carbon atom indicated by "*" is the carbon atom on benzene ring to which R²² is bonded.

[16] The compound according to any one of [1] to [13], the salt thereof or the hydrate of the foregoing, wherein (i) R²¹ and R²² or (ii) R²² and R²³ bond together to form a ring selected from Group Z3,
wherein Group Z3 consists of

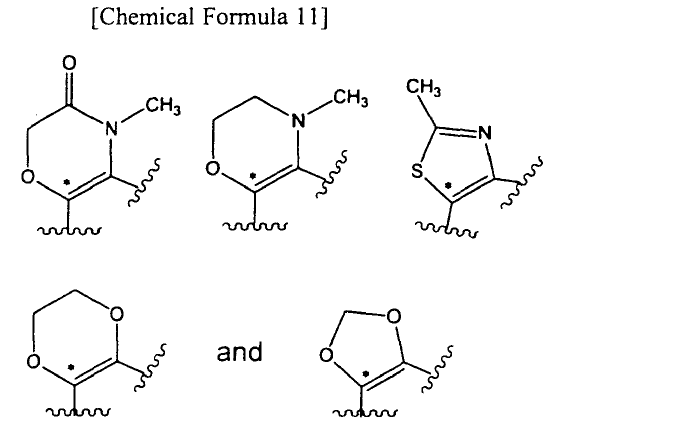

wherein the carbon atom indicated by "*" is the carbon atom on benzene ring to which R²² is bonded.

[17] The compound according to any one of [14] to [16], the salt thereof or the hydrate of the foregoing, wherein R²⁰ and R²³ are hydrogen.

[18] The compound according to any one of [1] to [13], the salt thereof or the hydrate of the foregoing, wherein at least one of R²⁰, R²¹, R²² and R²³ is carboxyl, C 1-6 alkylthio optionally substituted with a substituent selected from Group F1, C2-7 alkoxycarbonyl, phenoxy, -SO₃H, C1-6 alkyl substituted with a substituent selected from Group W2, C1-6 alkyl substituted with a substituent selected from Group K1, C1-6 alkoxy substituted with a substituent selected from Group W2, a 4- to 8-membered heterocyclic group substituted with a substituent selected from Group W3, a 4- to 8-membered heterocyclic group substituted with a substituent selected from Group V1, a 5- to 10-membered heteroaryl ring group substituted with a substituent selected from Group W3, a 6- to 10-membered aryl ring group optionally substituted with a substituent selected from Group W1; C2-7 alkenyl optionally substituted with substituent selected from Group W1, C2-7 alkynyl optionally substituted with a substituent selected from Group W1, 3- to 8-membered cycloalkyl optionally substituted with a substituent selected from Group W1, 5- to 8-membered cycloalkenyl optionally substituted with a substituent selected from Group W1, -NR^{10X}R^{2X}, -CO-R^{11X}, -CO-NR^{1X}R²X,
- NR^{1X}-CO-R^{2X}, -SO₂-R^{3X} or -O-SO₂-R^{3X},
   R^{1X} and R^{2X} may be the same or different and each represents hydrogen, C1-6 alkyl optionally substituted with a substituent selected from Group U1 or a 4-to 8-membered heterocyclic group,
   R^{3X} represents C1-6 alkyl optionally substituted with a substituent selected from Group F1,
   R^{10X} is C1-6 alkyl substituted with a substituent selected from Group U1, or a 4- to 8-membered heterocyclic group, and
   R^{11X} is hydrogen, C1-6 alkyl substituted with a substituent selected from Group U1, or a 4- to 8-membered heterocyclic group,
wherein Group W2 consists of hydroxyl, cyano, C1-6 alkyl, C2-7 alkoxyalkyl, C1-6 alkoxy optionally substituted with a substituent selected from Group T1, phenoxy, C2-7 alkylcarbonyl, -NR^{6X}R^{7X} and -CO-NR^{6X}R^{7X} wherein R^{6X} and R^{7X} may be the same or different and each represents hydrogen or C1-6 alkyl,
Group W3 consists of hydroxyl, carboxyl, C2-7 alkoxyalkyl, C1-6 alkoxy substituted with a substituent selected from Group T1, phenoxy, C2-7 alkoxycarbonyl, C2-7 alkylcarbonyl, -NR^{6X}R^{7X} and -CO-NR^{6X}R^{7X} wherein R^{6X} and R^{7X} may be the same or different and each represents hydrogen or C1-6 alkyl. and
Group F1, Group G1, Group H1, Group W1, Group T1, Group V1, Group K1 and Group U1 have the same respective definitions as Group F1, Group G1, Group H1, Group W1, Group T1, Group V1, Group K1 and Group U1 in [1].

[19] The compound according to any one of [1] to [13], the salt thereof or the hydrate of the foregoing, wherein one of R²⁰, R²¹, R²² and R²³ represents phenyl optionally substituted with one substituent selected from Group P, C2-7 alkenyl optionally substituted with one substituent selected from Group P, C2-7 alkynyl optionally substituted with one substituent selected from Group P, carboxyl, C1-6 alkylsulfonyloxy optionally having 1-3 fluorine, C1-6 alkylthio, C2-7 alkoxycarbonyl, C1-6 alkoxy-C1-6 alkoxy, C2-7 alkoxyalkyl, morpholin-4-yl-C1-6 alkyl, pyrrolidin-1-yl optionally substituted with one substituent selected from Group Q, piperidin-1-yl optionally substituted with one substituent selected from Group Q, a group represented by the following formula:

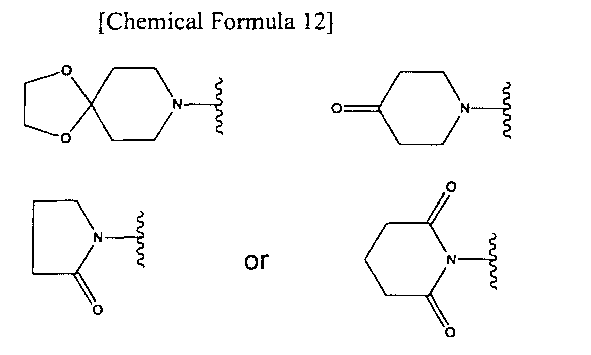

-NR⁸⁰R⁸¹, -CO-R⁸², -CO-NR⁸³R⁸⁴ or -NR⁸⁵CO-R⁸⁶,
wherein R⁸⁰ represents hydrogen, C1-6 alkyl, C2-7 alkoxyalkyl or tetrahydropyran-4-yl,
R⁸¹ represents C2-7 alkoxyalkyl or tetrahydropyran-4-yl,
R⁸² represents C2-7 alkoxyalkyl or morpholin-4-yl,
R⁸³ and R⁸⁴ may be the same or different and each represents hydrogen, C1-6 alkyl, tetrahydropyran-4-yl or C2-7 alkoxyalkyl,
R⁸⁵ represents hydrogen or C1-6 alkyl, and
R⁸⁶ represents C1-6 alkyl,
wherein Group P consists of carboxyl, C2-7 alkoxycarbonyl, C2-7 alkoxyalkyl, C 1-6 alkoxy and cyano, and
Group Q consists of carboxyl, C2-7 alkoxycarbonyl, C1-6 alkoxy-C1-6 alkoxy, carboxyl-C1-6 alkoxy, C2-7 alkoxyalkyl and hydroxyl.

[20] The compound according to any one of [1] to [13], the salt thereof or the hydrate of the foregoing, wherein one of R²⁰, R²¹, R²² and R²³ represents phenyl, C2-7 alkoxycarbonyl, C1-6 alkoxy-C1-6 alkoxy, C2-7 alkoxyalkyl, morpholin-4-yl-C1-6 alkyl, pyrrolidin-1-yl optionally having one substituent selected from Group R, piperidin-1-yl optionally having one substituent selected from Group R, a group represented by the following formula:

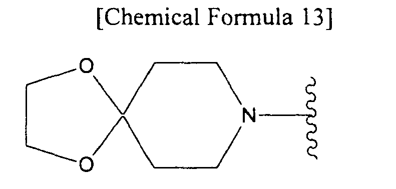

-NR⁹⁰R⁹¹, CO-R⁹² or -CO-NR⁹³R⁹⁴_{'}
wherein R⁹⁰ represents hydrogen, C1-6 alkyl, C2-7 alkoxyalkyl or tetrahydropyran-4-yl,
R⁹¹ represents C2-7 alkoxyalkyl or tetrahydropyran-4-yl,
R⁹² represents C2-7 alkoxyalkyl, and
R⁹³ and R⁹⁴ may be the same or different and each represents hydrogen or C1-6 alkyl,
wherein Group R consists of C1-6 alkoxy-C1-6 alkoxy and C2-7 alkoxyalkyl.

[21] The compound according to any one of [18] to [20], the salt thereof or the hydrate of the foregoing, wherein R²⁰ is hydrogen.

[22] The compound according to any one of [18] to [20], the salt thereof or the hydrate of the foregoing, wherein two of R²⁰, R²¹, R²² and R²³ are hydrogen, and one of the other two is hydrogen or C1-6 alkoxy.

[23] The compound according to any one of [18] to [20], the salt thereof or the hydrate of the foregoing, wherein three of R²⁰, R²¹, R²² and R²³ are hydrogen.

The "C1-6 alkyl" of Group W2, the "C2-7 alkylcarbonyl" of Group W2 and Group W3, the "C2-7 alkoxycarbonyl" of Group W3, Group P and Group Q and the "C2-7 alkoxyalkyl" of Group W2, Group W3, Group P, Group Q and Group R all have the same definitions as in Group W1.

The "C1-6 alkoxy" of the "C1-6 alkoxy optionally substituted with a substituent selected from Group T1" of Group W2, the "C1-6 alkoxy" of the "C1-6 alkoxy optionally substituted with a substituent selected from Group T1" of Group W3, the "C1-6 alkoxy" of Group P, the "C1-6 alkoxy" of the "C1-6 alkoxy-C1-6 alkoxy" and "carboxyl-C1-6 alkoxy" of Group Q, and the "C1-6 alkoxy" of the "C1-6 alkoxy-C1-6 alkoxy" of Group R, have the same definitions as the "C1-6 alkoxy" of the "C1-6 alkoxy optionally substituted with a substituent selected from Group T1" in Group W1.

As examples of the "C1-6 alkyl" of the "C1-6 alkyl optionally substituted with a substituent selected from Group U1" for R^{10X} there may be mentioned the same ones listed above.

As examples of the "4- to 8-membered heterocyclic group" for R^{10X} there may be mentioned the same ones listed above.

As examples of the "C1-6 alkyl" of the "C1-6 alkyl optionally substituted with a substituent selected from Group U1" for R^{11X} there may be mentioned the same ones listed above.

As examples of the "4- to 8-membered heterocyclic group" for R^{11X} there may be mentioned the same ones listed above.

As examples of the "C1-6 alkyl" for R⁸⁰ there may be mentioned the same ones listed above.

As examples of the "C2-7 alkoxyalkyl" for R⁸⁰ there may be mentioned the same ones listed above.

As examples of the "C2-7 alkoxyalkyl" for R⁸¹ there may be mentioned the same ones listed above.

As examples of the "C2-7 alkoxyalkyl" for R⁸² there may be mentioned the same ones listed above.

As examples of the "C1-6 alkyl" for R⁸³ and R⁸⁴ there may be mentioned the same ones listed above.

As examples of the "C2-7 alkoxyalkyl" for R⁸³ and R⁸⁴ there may be mentioned the same ones listed above.

As examples of the "C1-6 alkyl" for R⁸⁵ there may be mentioned the same ones listed above.

As examples of the "C1-6 alkyl" for R⁸⁶ there may be mentioned the same ones listed above.

As examples of the "C1-6 alkyl" for R⁹⁰ there may be mentioned the same ones listed above.

As examples of the "C2-7 alkoxyalkyl" for R⁹⁰ there may be mentioned the same ones listed above.

As examples of the "C2-7 alkoxyalkyl" for R⁹¹ there may be mentioned the same ones listed above.

As examples of the "C2-7 alkoxyalkyl" for R⁹² there may be mentioned the same ones listed above.

As examples of the "C1-6 alkyl" for R⁹³ and R⁹⁴ there may be mentioned the same ones listed above.

As preferred examples of compounds for the invention there may be mentioned the following:
1-[2-(4,4-dimethylcyclohexyl)-5-methoxyphenyl]-4-pentylpiperazine,
1-butyl-4-[2-(4-t-butylcyclohex-1-enyl)-4-(4-methoxypiperidin-1-yl)phenyl]piperazine,
1-butyl-4-[2-(3,3,5,5-tetramethylcyclohexyl)phenyl]piperazine,
1-cyclopropylmethyl-4-[2-(3,3,5,5-tetramethylcyclohexyl)phenyl]piperazine,
2-{4-[2-(4-t-butylcyclohexyl)phenyl]piperazin-1-yl}-N-ethylacetamide,
*cis-*4-(4-t-butylcyclohexyl)-3-(4-butylpiperazin-1-yl)benzonitrile,
*trans*-4-(4-t-butylcyclohexyl)-3-(4-butylpiperazin-1-yl)benzonitrile,
1-butyl-4-(2-cyclohexylphenyl)piperazine,
1-butyl-4-[2-(4-t-butylcyclohexyl)phenyl]piperazine,
1-{4-[2-(4,4-dimethylcyclohexyl)phenyl]piperazin-1-yl}butan-2-one,
4-[3-(4-t-butylcyclohex-1-enyl)-4-(4-butylpiperazin-1-yl)phenyl]morpholine,
1-[2-(4-t-butylcyclohexyl)phenyl]-4-(2-methoxyethyl)piperazine,
1-[2-(4-t-butylcyclohex-1-enyl)-4-(4-methoxypiperidin-1-yl)phenyl]-4-cyclopropylmethylpiperazine,
1-(tetrahydropyran-4-ylmethyl)-4-[2-(3,3,5,5-tetramethylcyclohex-1-enyl)phenyl]piperazine,
4-[4-(4-propylpiperazin-1-yl)-3-(3,3,5,5-tetramethylcyclohex-1-enyl)phenyl]morpholine,
1-{4-[2-(4,4-diethylcyclohex-1-enyl)-4-morpholin-4-ylphenyl]piperazin-1-yl}butan-2-one,
1-propyl-4-[2-(3,3,5,5-tetramethylcyclohexyl)phenyl]piperazine,
1-butyl-4-[4-(4-methoxypiperidin-1-yl)-2-(3,3,5,5-tetramethylcyclohexyl)phenyl]piperazine,
1-butyl-4-[2-(3,5-dimethylcyclohexyl)phenyl]piperazine,
1-[2-(4,4-diethylcyclohexyl)phenyl]-4-(tetrahydropyran-4-ylmethyl)piperazine,
4-[4-(4-butylpiperazin-1-yl)-3-(3,3,5,5-tetramethylcyclohex-1-enyl)phenyl]morpholine,
4-[4-(4-butylpiperazin-1-yl)-3-(3,3,5,5-tetramethylcyclohexyl)phenyl]morpholine,
1-[4-(4-ethoxypiperidin-1-yl)-2-(3,3,5,5-tetramethylcyclohexyl)phenyl]-4-propylpiperazine,
cis-4-[4-(4-butylpiperazin-1-yl)-3-(4,4-dimethylcyclohexyl)phenyl]-2,6-dimethylmorpholine,
4-{4-(4-pentylpiperazin-1-yl)-3-spiro[2.5]oct-6-ylphenyl}morpholine,
1-[3-fluoro-2-(3,3,5,5-tetramethylcyclohex-1-enyl)phenyl]-4-propylpiperazine,
1-cyclopropylmethyl-4-[2-(3,3,5,5-tetramethylcyclohexyl)phenyl]-1,2,3,6-tetrahydropyridine,
1-butyl-4-{2-(3,3,4,4-tetramethylcyclopentyl)phenyl}piperazine,
1-butyl-4-[2-(4,4-dimethylcyclohexyl)-4-(4-ethoxypiperidin-1-yl)phenyl]piperazine,
1-butyl-4-[2-(3,3,5,5-tetramethylcyclohex-1-enyl)phenyl]piperazine,
1-cyclopropylmethyl-4-[2-(3,3,5,5-tetramethylcyclohex-1-enyl)phenyl]piperazine,
1- {4-[2-(3,3,5,5-tetramethy)cyclohex-1-enyl)phenyl]piperazin-1-yl} butan-2-one,
1-(2-methoxyethyl)-4-[2-(3,3,5,5-tetramethylcyclohex-1-enyl)phenyl]piperazine,
1- {4-[2-(3,3,5,5-tetramethylcyclohexyl)phenyl]piperazin-l-yl}butan-2-one,
1-(2-methoxyethyl)-4-[2-(3,3,5,5-tetramethylcyclohexyl)phenyl]piperazine,
4-[4-(4-butylpiperazin-1-yl)-5-(4,4-diethylcyclohexyl)-2-methoxyphenyl]-morpholine,
1-butyl-4-(2-spiro[4.5]dec-8-ylphenyl)piperazine,
1-[2-(4,4-dimethylcyclohex-1-enyl)phenyl]-4-isobutylpiperazine,
1-cyclopropylmethyl-4-[2-(4,4-diethylcyclohexyl)-4-(4-methoxypiperidin-1-yl)phenyl]piperazine,
4-[3-(4,4-dimethylcyclohexyl)-4-(4-isobutylpiperazin-1-yl)phenyl]morpholine,
{4-[2-(3,3,5,5-tetramethylcyclohexyl)phenyl]piperazin-1-yl}acetonitrile,
1-(2-ethoxyethyl)-4-[2-(3,3,5,5-tetramethylcyclohexyl)phenyl]piperazine,
(R)-1-butyl-4-[2-(4,4-diethylcyclohexyl)-4-(3-methoxypyrrolidin-1-yl)phenyl]piperazine,
1-[4-methyl-2-(3,3,5,5-tetramethylcyclohexyl)phenyl]-4-propylpiperazine,
1-[4-methoxy-2-(3,3,5,5-tetramethylcyclohexyl)phenyl]-4-(tetrahydropyran-4-ylmethyl)piperazine,
1-butyl-4-[2-(3,3,5,5-tetramethylcyclohexyl)phenyl]piperidine,
1-isobutyl-4-[2-(3,3,4,4-tetramethylcyclopent-1-enyl)phenyl]piperazine, and
1-[2-(4-cyclopropylmethylpiperazin-1-yl)phenyl]-3,3,5,5-tetramethylcyclohexanol.

The compounds according to [1] to [23] above, or the salts thereof, or the hydrates of the foregoing, exhibit excellent cell adhesion inhibitory action or cell infiltration inhibitory action, and can be utilized as medicaments. More specifically, they can be utilized as therapeutic or prophylactic agents for inflammatory diseases and autoimmune diseases, and particularly for various diseases associated with adhesion and infiltration of leukocytes, such as inflammatory bowel disease (especially ulcerative colitis or Crohn's disease), irritable bowel syndrome, rheumatoid arthritis, psoriasis, multiple sclerosis, asthma and atopic dermatitis.

In other words, the invention also provides the following [24] to [29].
[24] A medicament comprising the compound according to any one of [1] to [23], the salt thereof or the hydrate of the foregoing.
[25] A cell adhesion or cell infiltration inhibitor comprising the compound according to any one of [1] to [23], the salt thereof or the hydrate of the foregoing.
[26] A therapeutic or prophylactic agent for an inflammatory disease or an autoimmune disease, comprising the compound according to any one of [1] to [23], the salt thereof or the hydrate of the foregoing.
[27] A therapeutic or prophylactic agent for an inflammatory bowel disease, irritable bowel syndrome, rheumatoid arthritis, psoriasis, multiple sclerosis, asthma or atopic dermatitis, comprising the compound according to any one of [1] to [23], the salt thereof or the hydrate of the foregoing.
[28] A therapeutic or prophylactic agent for an inflammatory bowel disease, comprising the compound according to any one of [1] to [23], the salt thereof or the hydrate of the foregoing.
[29] A therapeutic or prophylactic agent for ulcerative colitis or Crohn's disease, comprising the compound according to any one of [1] to [23], the salt thereof or the hydrate of the foregoing.

### Effect of the Invention

The compounds of the invention have excellent cell adhesion inhibitory action or cell infiltration inhibitory action, and are therefore useful as therapeutic or prophylactic agents for inflammatory diseases and autoimmune diseases, particularly as therapeutic or prophylactic agents for various diseases associated with adhesion and infiltration of leukocytes, such as inflammatory bowel disease (particularly ulcerative colitis or Crohn's disease), irritable bowel syndrome, rheumatoid arthritis, psoriasis, multiple sclerosis, asthma and atopic dermatitis.

### Best Mode for Carrying Out the Invention

### (General production methods)

Compounds (1) and (100) of the invention may be produced by the methods described below. However, it is to be understood that the production methods for the compounds of the invention are not limited to those described below.

Compound (1) of the invention may be produced by the following Method A, Method B, Method C, Method D, Method E, Method N, Method P or Method V.
Compound (1A) of the invention (the compound (1) wherein X¹ is nitrogen) may be produced by the following Method F, Method G, Method H, Method K, Method M, Method Q or Method R.
Compound (1B) of the invention (the compound (1) wherein X¹ is CH) and compound (100) of the invention may be produced by the following Method A, Method B, Method C, Method D, Method E, Method K, Method M, Method S, Method T or Method U.
Each of these methods will now be explained in detail.

### (Method A)

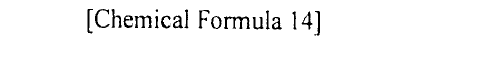

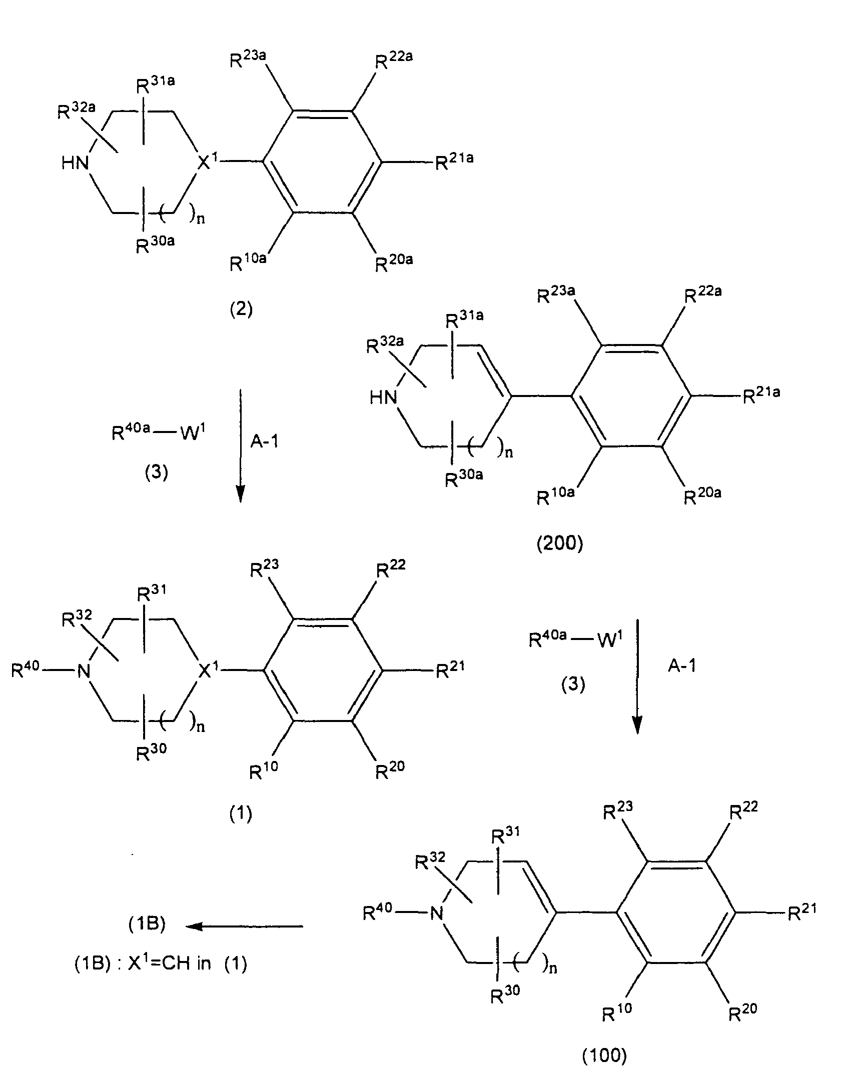

Method A is a method of producing compound (1) of the invention by reacting compound (2) with an alkylating agent (3), carbonylating agent (3) or sulfonylating agent (3) in an inert solvent, in the presence or in the absence of a base, in the presence or in the absence of an additive, and optionally removing any protecting groups on the resultant compound, or a method of producing compound (100) of the invention by reacting compound (200) in a similar manner.
In this scheme, R¹⁰, R²⁰, R²¹, R²², R²³, R³⁰, R³¹, R³², R⁴⁰, X¹ and n have the same definitions as above. R^{10a}, R^{20a}, R^{21a}, R^{22a}, R^{23a}, R^{30a}, R^{31a}, R^{32a} and R^{40a} have the same definitions as the corresponding R¹⁰, R²⁰, R²¹, R²², R²³, R³⁰, R³¹, R³² and R⁴⁰, or are the corresponding groups R¹⁰, R²⁰, R²¹, R²², R²³, R³⁰, R³¹, R³² and R⁴⁰ with the respective substituents on the groups protected, and W¹ represents a leaving group which is chlorine, bromine or iodine, alkylsulfonyloxy such as methanesulfonyloxy or ethanesulfonyloxy, haloalkanesulfonyloxy such as trifluoromethanesulfonyloxy or nonafluorobutanesulfonyloxy, or arylsulfonyloxy such as benzenesulfonyloxy or *p*-toluenesulfonyloxy, among which chlorine, bromine, iodine, methanesulfonyloxy, *p*-toluenesulfonyloxy, nonafluorobutanesulfonyloxy or trifluoromethanesulfonyloxy is preferred.

### (Alkylation)

There are no particular restrictions on the solvent used so long as it dissolves the starting compound to some extent and does not inhibit the reaction of this step, and specifically there may be mentioned amides such as formamide, dimethylformamide, dimethylacetamide, hexamethylphosphoric triamide and N-methylpyrrolidone, nitriles such as acetonitrile and isobutyronitrile, aromatic hydrocarbons such as toluene, benzene and xylene, ethers such as diethyl ether, diisopropyl ether, tetrahydrofuran, dioxane, dimethoxyethane and diethyleneglycol dimethyl ether or sulfoxides such as dimethyl sulfoxide, as well as mixtures of these solvents, among which dimethylformamide, acetonitrile, toluene or tetrahydrofuran is preferred.
There are no particular restrictions on the base used so long as it can yield the target compound and does not produce any unseparable by-products, and specifically there may be mentioned organic bases such as triethylamine and pyridine or inorganic bases such as potassium carbonate, sodium carbonate, potassium hydrogencarbonate, sodium hydrogencarbonate and cesium carbonate, among which potassium carbonate or triethylamine is preferred.
Sodium iodide or potassium iodide is used as an additive to accelerate the reaction if necessary.
The reaction temperature will differ depending on the starting materials, solvent and reagents, but will usually be between -30°C and 180°C, and is preferably between 0°C and 120°C.
The reaction time will differ depending on the starting materials, solvent. reagents and reaction temperature, but will usually be 0.5 to 100 hours, and is preferably 0.5 to 24 hours.

### (Carbonylation or sulfonylation)

There are no particular restrictions on the solvent used so long as it dissolves the starting compound to some extent and does not inhibit the reaction of this step, and specifically there may be mentioned halogenated hydrocarbons such as chloroform, dichloromethane, 1,2-dichloroethane and carbon tetrachloride, ethers such as diethyl ether, diisopropyl ether, tetrahydrofuran, dioxane, dimethoxyethane and diethyleneglycol dimethyl ether, amides such as formamide, dimethylformamide, dimethylacetamide, hexamethylphosphoric triamide and N-methylpyrrolidone, organic bases such as pyridine, or water, as well as mixtures of these solvents, among which dichloromethane, tetrahydrofuran, dioxane, dimethylformamide, pyridine, water, and mixtures thereof are preferred.
There are no particular restrictions on the base used so long as it can yield the target compound and does not produce any unseparable by-products, and specifically there may be mentioned organic bases such as triethylamine and pyridine or inorganic bases such as potassium carbonate, sodium carbonate, potassium hydrogencarbonate, sodium hydrogencarbonate, cesium carbonate and sodium hydroxide, among which potassium carbonate or triethylamine is preferred.
4-Dimethylaminopyridine is used as an additive to accelerate the reaction if necessary.
The reaction temperature will differ depending on the starting materials, solvent and reagents, but will usually be between -70°C and 120°C, and is preferably between -70°C and 60°C.
The reaction time will differ depending on the starting materials, solvent, reagents and reaction temperature, but will usually be 0.5 to 48 hours, and is preferably 0.5 to 12 hours.

Compounds (1) and (100) of the invention may be isolated or purified from the reaction mixture obtained above, by the method described below.
When R¹⁰ of the resultant compound (1) or (100) is optionally substituted 5-to 10-membered cycloalkenyl, it may be subjected to hydrogenation to yield compound (1) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).

### The hydrogenation may be carried out as follows.

Specifically, hydrogenation reaction may be carried out using a metal catalyst in an inert solvent, under a hydrogen atmosphere or in the presence of hydrogen-donating reagent, in the presence or in the absence of an acid.
There are no particular restrictions on the solvent used so long as it dissolves the starting compound to some extent and does not inhibit the reaction of this step, and specifically there may be mentioned water, alcohols such as methanol and ethanol, ethers such as diethyl ether, diisopropyl ether, tetrahydrofuran, dioxane, dimethoxyethane and diethyleneglycol dimethyl ether, esters such as methyl acetate, ethyl acetate, propyl acetate, butyl acetate and diethyl carbonate, amides such as formamide, dimethylformamide, dimethylacetamide, hexamethylphosphoric triamide and N-methylpyrrolidone, aliphatic hydrocarbons such as hexane, heptane, ligroin and petroleum ether or organic acids such as acetic acid, or mixtures of these solvents, among which methanol, ethanol, ethyl acetate, tetrahydrofuran, a mixed solvent of methanol and tetrahydrofuran, or a mixed solvent of ethanol and tetrahydrofuran is preferred.
There are no particular restrictions on the metal catalyst used so long as it can yield the target compound and does not produce any unseparable by-products, and specifically there may be mentioned heterogeneous rare metal catalysts such as palladium, palladium hydroxide, platinum, platinum oxide, rhodium, ruthenium and nickel (preferably supported on a carrier such as activated carbon, alumina, silica or zeolite) and homogeneous metal complex catalysts such as chlorotris(triphenylphosphine) rhodium(I) (Wilkinson's complex), among which heterogeneous rare metal catalysts (especially 5 to 10% palladium-activated carbon or platinum oxide, optionally wetted with water) are preferred.
The number of equivalents of the metal catalyst used (including the carrier) will differ depending on the starting materials, solvent and reagents, but will usually be a proportion of 0.05 to 10 and preferably 0.05 to 3, in terms of the weight ratio with respect to the starting material.
There are no particular restrictions on the acid used so long as it can yield the target compound and does not produce any unseparable by-products, and specifically there may be mentioned organic acids such as methanesulfonic acid, trifluoromethanesulfonic acid, acetic acid and trifluoroacetic acid, or inorganic acids such as hydrochloric acid and hydrobromic acid.
The reaction temperature will differ depending on the starting materials, solvent and reagents, but will usually be between -10°C and 80°C, and is preferably between 0°C and 50°C.
The reaction pressure of the hydrogen will also differ depending on the starting materials, solvent and reagents, but will usually be between I and 100 atmospheres, and preferably between 1 and 5 atmospheres.
The reaction time will differ depending on the starting materials, solvent, reagents and reaction temperature, but will usually be 0.5 to 200 hours, and is preferably 0.5 to 100 hours.

When the resultant compound is to be converted to an acid salt, this may be accomplished by a conventional method. The step of producing the salt and the step of hydrogenation described above may be carried out in a different order where appropriate.

### (Method B)

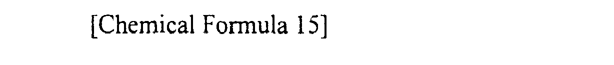

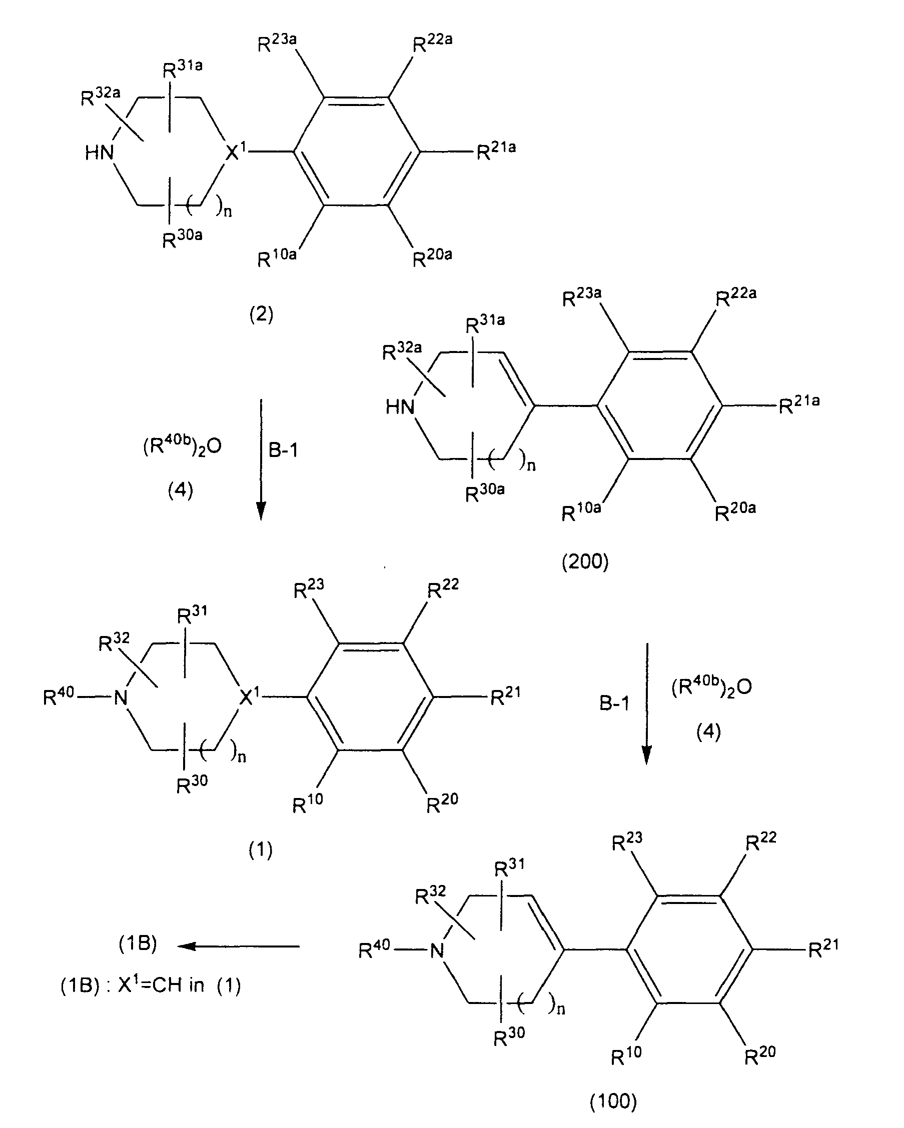

Method B is a method of producing compound (1) of the invention by reacting compound (2) with an acid anhydride (4) in an inert solvent, in the presence or in the absence of a base, and optionally removing any protecting groups on the resultant compound, or a method of producing compound (100) of the invention by reacting compound (200) in a similar manner.
In this scheme, R¹⁰, R²⁰, R²¹, R²², R²³, R³⁰, R³¹, R³², R⁴⁰, X¹, n, R^{10a}, R^{20a}, R^{21a}, R^{22a}, R^{23a}, R^{30a}, R^{31a} and R^{32a} have the same definitions as above. Also, R^{40b} is a group comprising carbonyl or sulfonyl, which is suitable for obtaining R⁴⁰ and can form an acid anhydride. Substituents on R^{40b} may also be protected.
This method may also be carried out in a manner similar to the carbonylation or sulfonylation step of Method A above.

Compounds (1) and (100) of the invention may be isolated or purified from the reaction mixture obtained above, by the method described below.
When R¹⁰ of the resultant compound (1) or (100) is optionally substituted 5-to 10-membered cycloalkenyl, it may be subjected to the hydrogenation described for Method A above to yield compound (1) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method C)

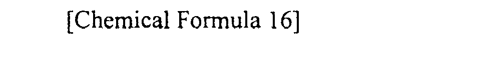

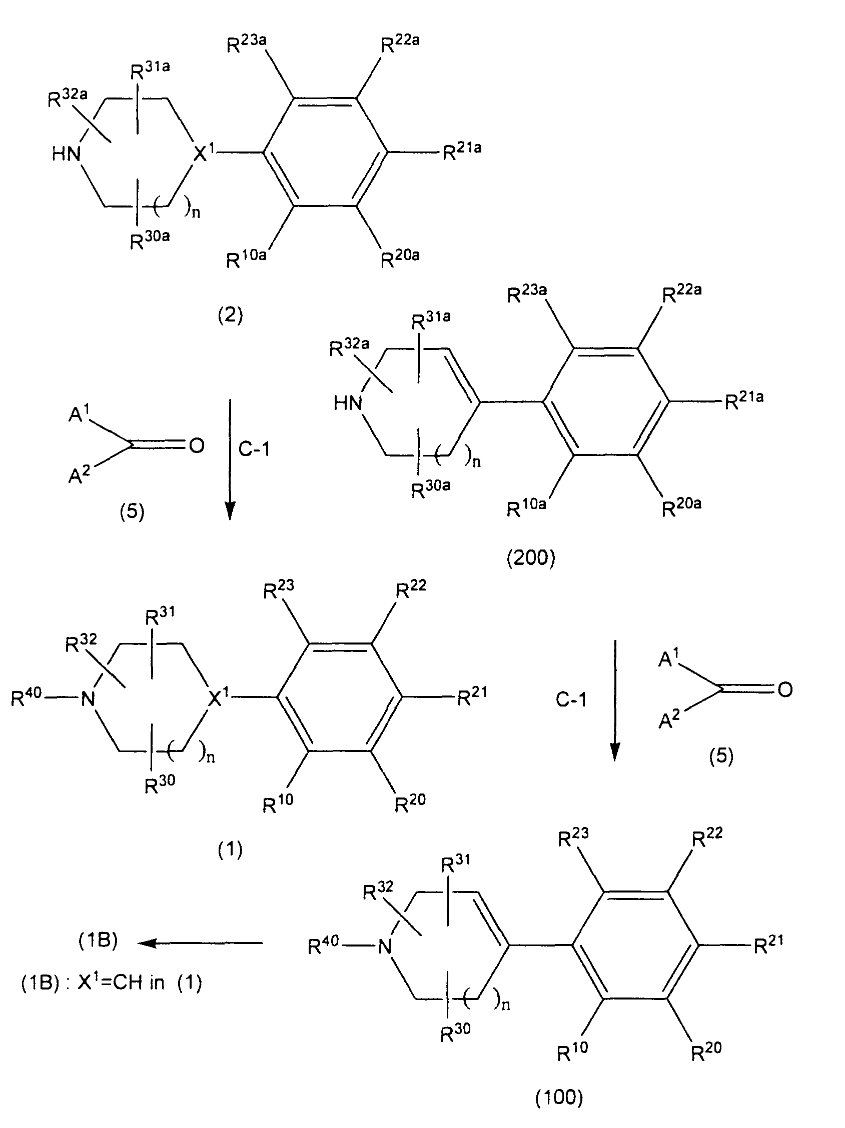

Method C is a method of producing compound (1) of the invention by reacting compound (2) with an aldehyde (5) or ketone (5) in an inert solvent, in the presence of a reducing agent, in the presence or in the absence of an acid, in the presence or in the absence of an additive, and optionally removing any protecting groups on the resultant compound, or a method of producing compound (100) of the invention by reacting compound (200) in a similar manner.
In this scheme, R¹⁰, R²⁰, R²¹, R²², R²³, R³⁰, R³¹, R³², R⁴⁰, X¹, n, R^{10a}, R^{20a}, R^{21a}, R²²a, R^{23a}, R^{30a}, R^{31a} and R^{32a} have the same definitions as above. Also, A¹ and A² are groups suitable for obtaining R⁴⁰. Substituents on A¹ or A² may also be protected.

There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, halogenated hydrocarbons such as chloroform, dichloromethane, 1,2-dichloroethane and carbon tetrachloride, nitriles such as acetonitrile and isobutyronitrile, aromatic hydrocarbons such as toluene, benzene and chlorobenzene or alcohols such as methanol and ethanol, among which ethers (particularly tetrahydrofuran) and halogenated hydrocarbons (particularly dichloroethane) are preferred.
There are no particular restrictions on the reducing agent used so long as it can yield the target compound and does not produce any unseparable by-products, and specifically there may be mentioned boron reducing agents such as sodium triacetoxyborohydride, sodium cyanoborohydride and borane-pyridine, and metal catalyst-hydrogen gas, among which sodium triacetoxyborohydride is preferred.
There are no particular restrictions on the acid used so long as it can yield the target compound and does not produce any unseparable by-products, and specifically there may be mentioned organic acids such as acetic acid and trifluoroacetic acid or Lewis acids such as titanium tetraisopropoxide and zinc chloride, among which organic acids (particularly acetic acid) are preferred.
There are no particular restrictions on the use of an additive so long as it can yield the target compound and does not produce any unseparable by-products, and specifically there may be mentioned molecular sieve or magnesium sulfate, among which Molecular Sieve 4Å is preferred.
The reaction temperature will differ depending on the starting materials, solvent and reagents, but will usually be between -70°C and 120°C, and is preferably between 0°C and 50°C.
The reaction time will differ depending on the starting materials, solvent, reagents and reaction temperature, but will usually be 0.1 to 200 hours, and is preferably 0.1 to 24 hours.
As supplementary literature to be used as reference for carrying out this method, there may be mentioned, but not limited to, Ahmed F. Abdel-Magid et al., J. Org. Chem. (1996), 61, 3849.

Compounds (1) and (100) of the invention may be isolated or purified from the reaction mixture obtained above, by the method described below.
When R¹⁰ of the resultant compound (1) or (100) is optionally substituted 5-to 10-membered cycloalkenyl, it may be subjected to the hydrogenation described for Method A above to yield compound (1) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method D)

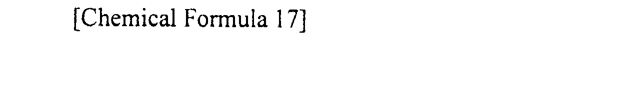

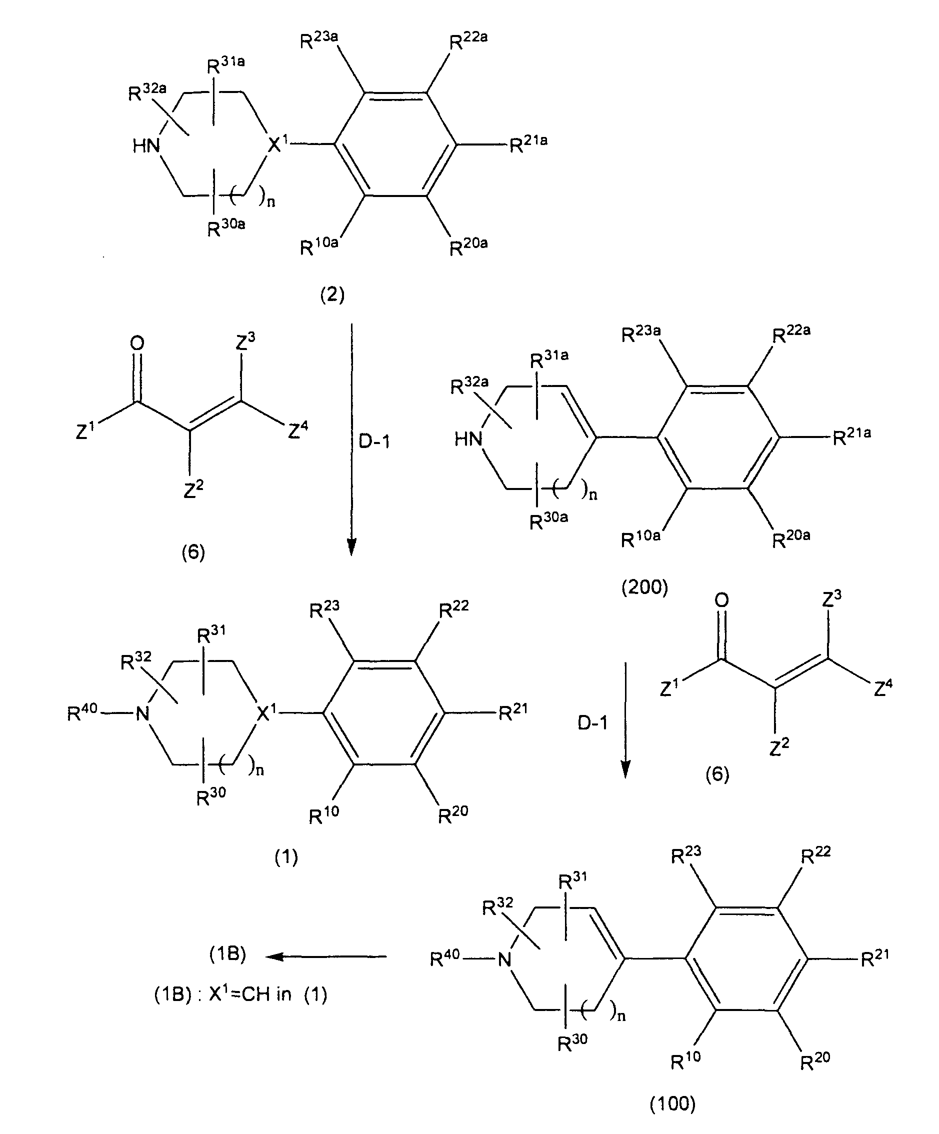

Method D is a method of producing compound (1) of the invention by reacting compound (2) with a conjugated carbonyl compound (6) by Michael addition reaction in an inert solvent, and optionally removing protecting groups on the resultant compound, or a method of producing compound (100) of the invention by reacting compound (200) in a similar manner.
In this scheme. R¹⁰, R²⁰, R²¹, R²², R²³, R³⁰ R³¹, R³², R⁴⁰ X¹, n, R^{10a}, R^{20a}, R^{21a}, R^{22a}, R^{23a}, R^{30a}, R^{31a} and R^{32a} have the same definitions as above. Also, Z¹, Z², Z³ and Z⁴ are groups suitable for obtaining R⁴⁰. Substituents on Z¹, Z², Z³ and Z⁴ may also be protected.

There are no particular restrictions on the solvent used so long as it dissolves the starting compound to some extent and does not inhibit the reaction of this step, and specifically there may be mentioned alcohols such as methanol, ethanol, n-propanol, isopropanol, n-butanol, isobutanol, t-butanol, isoamyl alcohol, diethylene glycol and glycerin, halogenated hydrocarbons such as chloroform, dichloromethane, 1,2-dichloroethane and carbon tetrachloride, ethers such as diethyl ether, diisopropyl ether, tetrahydrofuran, dioxane, dimethoxyethane and diethyleneglycol dimethyl ether, amides such as formamide, dimethylformamide, dimethylacetamide, hexamethylphosphoric triamide and N-methylpyrrolidone, and aromatic hydrocarbons such as benzene, toluene and xylene, among which halogenated hydrocarbons (particularly chloroform) or ethers (particularly tetrahydrofuran) are preferred.
The reaction temperature will differ depending on the starting materials, solvent and reagents, but will usually be between -30°C and 150°C, and is preferably between 0°C and 120°C.
The reaction time will differ depending on the starting materials, solvent, reagents and reaction temperature, but will usually be 0.5 to 48 hours, and is preferably 0.5 to 24 hours.

Compounds (1) and (100) of the invention may be isolated or purified from the reaction mixture obtained above, by the method described below.
When R¹⁰ of the resultant compound (1) or (100) is optionally substituted 5-to 10-membered cycloalkenyl, it may be subjected to the hydrogenation described for Method A above to yield compound (1) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method E)

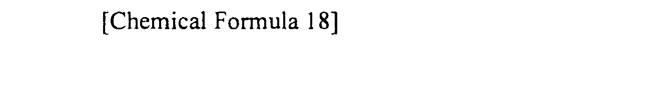

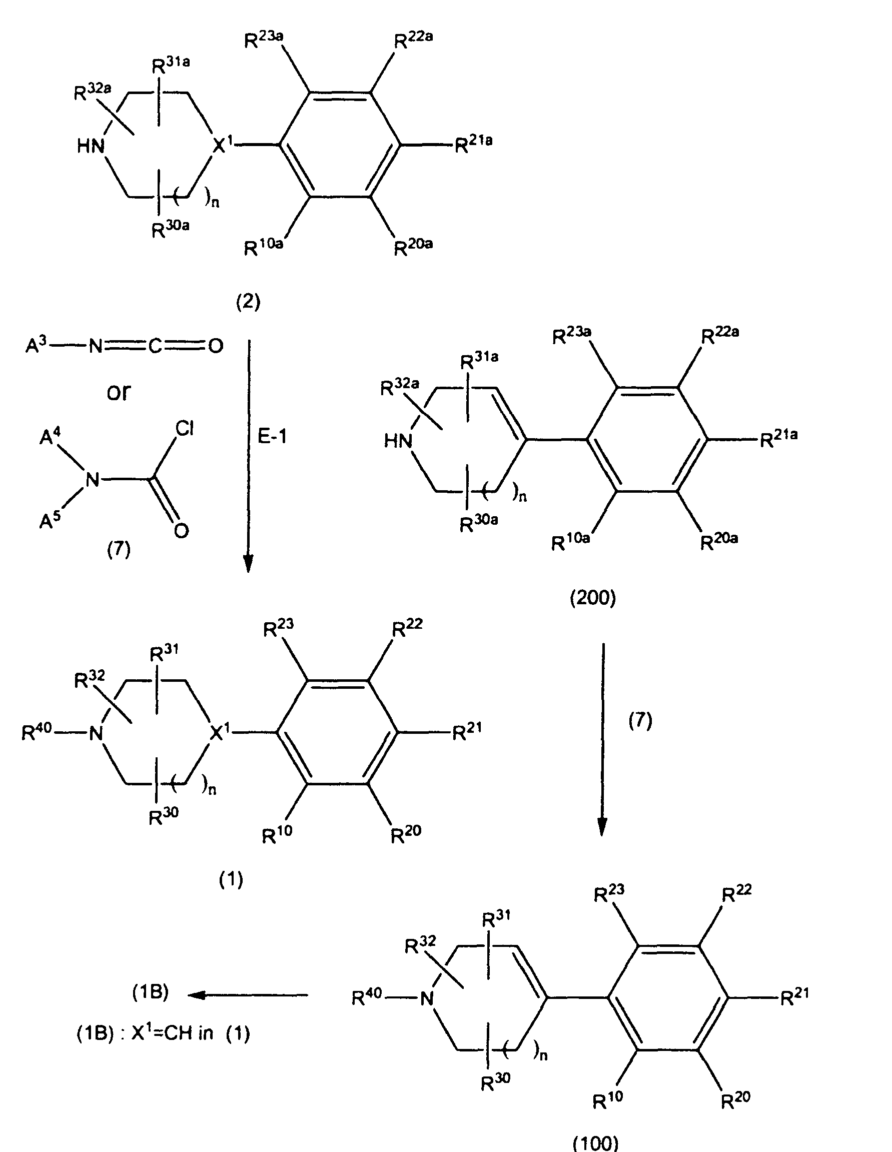

Method E is a method of producing compound (1) of the invention by reacting compound (2) with an isocyanate compound (7) or a substituted aminocarbonylchloride compound (7) in an inert solvent, in the presence or in the absence of a base, and optionally removing protecting groups on the resultant compound, or a method of producing compound (100) of the invention by reacting compound (200) in a similar manner.
In this scheme, R¹⁰, R²⁰, R²¹, R²², R²³, R³⁰, R³¹, R³², R⁴⁰, X¹, n, R^{10a}, R^{20a}, R^{21a}, R^{22a}, R^{23a}, R^{30a}, R^{31a} and R^{32a} have the same definitions as above. Also, A³, A⁴ and A⁵ are groups suitable for obtaining R⁴⁰, Substituents on A³, A⁴ and A⁵ may also be protected.

There are no particular restrictions on the solvent used so long as it dissolves the starting compound to some extent and does not inhibit the reaction of this step, and specifically there may be mentioned halogenated hydrocarbons such as chloroform, dichloromethane, 1,2-dichloroethane and carbon tetrachloride, aromatic hydrocarbons such as benzene, toluene and chlorobenzene, and ethers such as diethyl ether, diisopropyl ether, tetrahydrofuran, dioxane, dimethoxyethane and diethyleneglycol dimethyl ether, among which dichloromethane or tetrahydrofuran is preferred.
There are no particular restrictions on the base used so long as it can yield the target compound and does not produce any unseparable by-products, and specifically there may be mentioned organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), among which triethylamine or pyridine is preferred.
The reaction temperature will differ depending on the starting materials, solvent and reagents, but will usually be between -70°C and 100°C.
The reaction time will differ depending on the starting materials, solvent, reagents and reaction temperature, but will usually be 1 to 24 hours.

Compounds (1) and (100) of the invention may be isolated or purified from the reaction mixture obtained above, by the method described below.
When R¹⁰ of the resultant compound (1) or (100) is optionally substituted 5-to 10-membered cycloalkenyl, it may be subjected to the hydrogenation described for Method A above to yield compound (1) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method F)

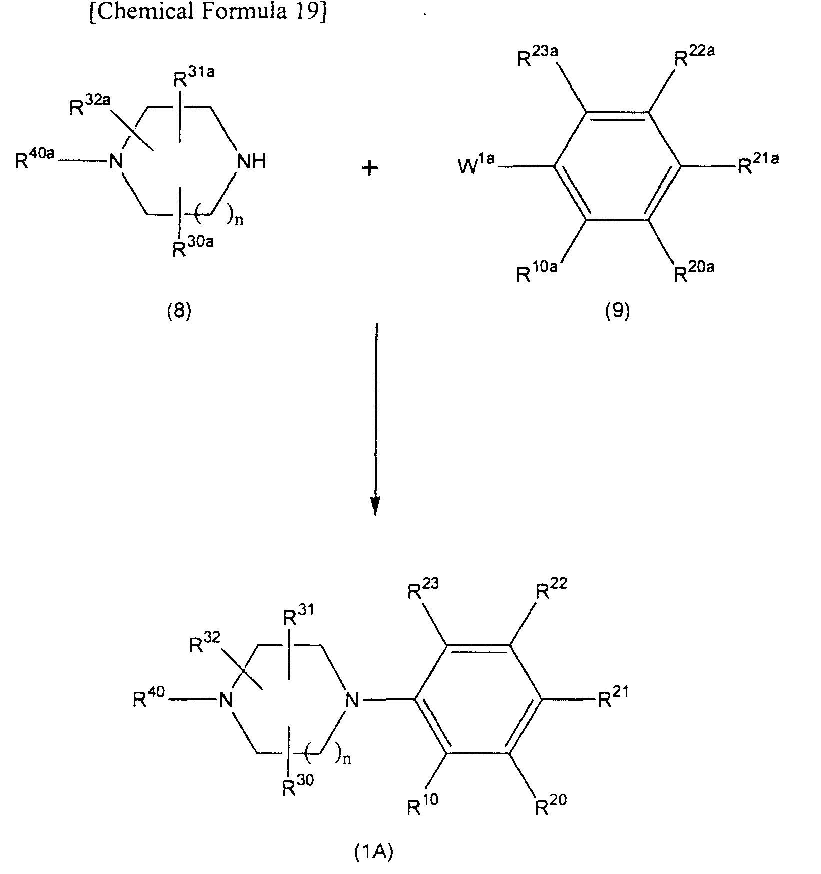

Method F is a method of producing compound (1A) of the invention (the compound (1) wherein X¹ is nitrogen) by reacting compound (8) with compound (9) (amination or amidation) in an inert solvent, in the presence of a palladium(0) catalyst or copper catalyst, in the presence or in the absence of a base, in the presence or in the absence of an additive, under or not under an inert gas atmosphere, and optionally removing protecting groups on the resultant compound.
In this scheme, R¹⁰, R²⁰, R²¹, R²², R²³, R³⁰, R³¹, R³², R⁴⁰, n, R^{10a}, R^{20a}, R^{21a}, R^{22a}, R^{23a}, R^{30a}, R^{31a} and R^{32a} have the same definitions as above. Also, W^{1a} represents chlorine, bromine or iodine, or a trifluoromethanesulfonyloxy group.

### (Reaction in the presence of palladium(0) catalyst)

There are no particular restrictions on the solvent used so long as it dissolves the starting compound to some extent and does not inhibit the reaction of this step, and specifically there may be mentioned amides such as dimethylformamide, dimethylacetamide, hexamethylphosphoric triamide and N-methylpyrrolidone, aromatic hydrocarbons such as toluene, benzene, xylene and mesitylene, ethers such as diethyl ether, diisopropyl ether, tetrahydrofuran, dioxane, dimethoxyethane and diethyleneglycol dimethyl ether, alcohols such as methanol, ethanol, n-propanol, isopropanol, n-butanol, isobutanol, t-butanol, isoamyl alcohol, diethylene glycol, glycerin, octanol and cyclohexanol, nitriles such as acetonitrile and isobutyronitrile, or mixtures of these solvents, among which dimethylformamide, toluene, xylene, tetrahydrofuran, dioxane or dimethoxyethane is preferred.
There are no particular restrictions on the palladium(0) catalyst used so long as it can yield the target compound and does not produce any unseparable by-products, and specifically there may be mentioned tetrakis(triphenylphosphine)palladium, tris(dibenzylideneacetone)dipalladium, bis(dibenzylideneacetone)palladium, bis(tri-t-butylphosphine)palladium and palladium black, or palladium(0) catalysts produced in the reaction system by combination of the palladium complexes which can be palladium(0) precursors mentioned below and various ligands mentioned below.
There are no particular restrictions on various palladium complexes which can be used as palladium(0) precursors, so long as they can yield the target compound without producing any unseparable by-products, and specifically there may be mentioned palladium acetate, [1,1'-bis(diphenylphosphino)ferrocene] dichloropalladium, dichlorobis(tri-o-tolylphosphine)palladium. dichlorobis(triscyclohexylphosphine)palladium, and the like. There are no particular restrictions on ligands used so long as they can yield the target compound without producing any unseparable by-products, and specifically there may be mentioned 2,2'-bis(diphenylphosphino)-1,1'-binaphthyl (BINAP), 9,9-dimethyl-4,5-bis(diphenylphosphino)xanthene (Xantphos), tri-t-butylphosphine, tri(4-methylphenyl)phosphine, tri-2-furylphosphine, 2-(di-t-butylphosphino)biphenyl, 2-(dicyclohexylphosphino)biphenyl, tricyclohexylphosphine, 2-dicyclohexylphosphino-2'-(N,N-dimethylamino)biphenyl, 1,1'-bis(diphenylphosphino)ferrocene, di-t-butylphosphonium tetrafluoroborate and 1,3-bis(2,4,6-trimethylphenyl)imidazol-2-ylidene.
There are no particular restrictions on the base used so long as it can yield the target compound and does not produce any unseparable by-products, and specifically there may be mentioned inorganic bases such as sodium t-butoxide, potassium t-butoxide, tripotassium phosphate, trisodium phosphate, cesium carbonate, potassium carbonate, sodium carbonate, cesium bicarbonate, potassium hydrogencarbonate, sodium hydrogencarbonate, sodium acetate, potassium acetate, cesium acetate, potassium fluoride, cesium fluoride, sodium hydroxide and potassium hydroxide, or organic bases such as triethylamine, 1,8-bis(dimethylamino)naphthalene, 1,4-diazabicyclo[2.2.2]octane (DABCO) and 1,8-diazabicyclo[5.4.0]undec-7-ene (DBU).
There are no particular restrictions on the additive used so long as it can yield the target compound and does not produce any unseparable by-products, and specifically there may be mentioned lithium fluoride, sodium fluoride, lithium chloride, sodium chloride, lithium bromide, sodium bromide, 1,4,7,10,13,16-hexaoxacyclooctadecane(18-Crown-6), 1,4,7,10,13-pentaoxacyclopentadecane(15-Crown-5), tetrabutylammonium fluoride and tetrabutylammonium bromide.
The reaction temperature will differ depending on the starting materials, solvent and reagents, but will usually be between 0°C and 150°C, and is preferably between 20°C and 110°C.
The reaction time will differ depending on the starting materials, solvent, reagents and reaction temperature, but will usually be 0.1 to 100 hours, and is preferably 0.5 to 48 hours.
When the reaction is carried out in an inert gas atmosphere, the inert gas is not particularly restricted so long as it does not inhibit the reaction of this step, and specifically it may be argon or nitrogen gas.
As supplementary literature to be used as reference for carrying out this method, there may be mentioned, but not limited to: D. Prim et al., Tetrahedron (2002), 58, 2041; and L. Buchwald et al., J. Organomet. Chem. (1999), 576, 125.

### (Reaction in the presence of copper catalyst)

There are no particular restrictions on the solvent used so long as it dissolves the starting compound to some extent and does not inhibit the reaction of this step, and specifically there may be mentioned amides such as formamide, dimethylformamide, dimethylacetamide, hexamethylphosphoric triamide and N-methylpyrrolidone, aromatic hydrocarbons such as toluene, benzene, xylene, mesitylene and nitrobenzene, ethers such as diethyl ether, diisopropyl ether, tetrahydrofuran, dioxane, dimethoxyethane and diethyleneglycol dimethyl ether, alcohols such as methanol, ethanol, n-propanol, isopropanol, n-butanol, isobutanol, t-butanol, isoamyl alcohol, diethylene glycol, glycerin, octanol, cyclohexanol and methyl cellosolve, or mixtures of these solvents, among which isopropanol, N-methylpyrrolidone, toluene and dimethylformamide are preferred.
There are no particular restrictions on the copper catalyst used so long as it can yield the target compound and does not produce any unseparable by-products, and specifically there may be mentioned copper (powder), copper(I) chloride, copper(II) chloride, copper(I) iodide, copper(I) oxide, copper(II) oxide, copper(II) acetate, copper(II) sulfate pentahydrate, copper(II) acetylacetonate, copper(I) thiocyanate and the like, among which copper (powder), copper(I) iodide and copper(I) chloride are preferred.
There are no particular restrictions on the ligand used so long as it can yield the target compound and does not produce any unseparable by-products, and specifically there may be mentioned ethylene glycol, diethylene glycol, cresol, 2,6-dimethylphenol, 1-naphthol, 2-naphthol, ethylenediamine, *N,N'-*dimethylethylenediamine and diisopropylamine, among which ethylene glycol and ethylenediamine are preferred.
There are no particular restrictions on the base used so long as it can yield the target compound and does not produce any unseparable by-products, and specifically there may be mentioned inorganic bases such as sodium t-butoxide, potassium t-butoxide, tripotassium phosphate, trisodium phosphate, cesium carbonate, potassium carbonate, sodium carbonate and sodium hydride, or organic bases such as potassium bis(trimethylsilyl)amide, among which potassium carbonate and tripotassium phosphate are preferred.
The reaction temperature will differ depending on the starting materials, solvent and reagents, but will usually be between 0°C and 250°C, and is preferably between 80°C and 150°C.
The reaction time will differ depending on the starting materials, solvent, reagents and reaction temperature, but will usually be 0.1 to 100 hours, and is preferably 0.5 to 48 hours.
When the reaction is carried out in an inert gas atmosphere, the inert gas is not particularly restricted so long as it does not inhibit the reaction of this step, and specifically it may be argon or nitrogen gas.
As supplementary literature to be used as reference for carrying out this method, there may be mentioned, but not limited to, L. Buchwald et al., Org. Lett. (2002), 4, 581.

Compound (1A) of the invention may be isolated or purified from the reaction mixture obtained above, by the method described below.
When R¹⁰ of the resultant compound (1A) is optionally substituted 5- to 10-membered cycloalkenyl, it may be subjected to the hydrogenation described for Method A above to yield compound (1A) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method G)

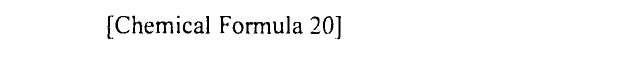

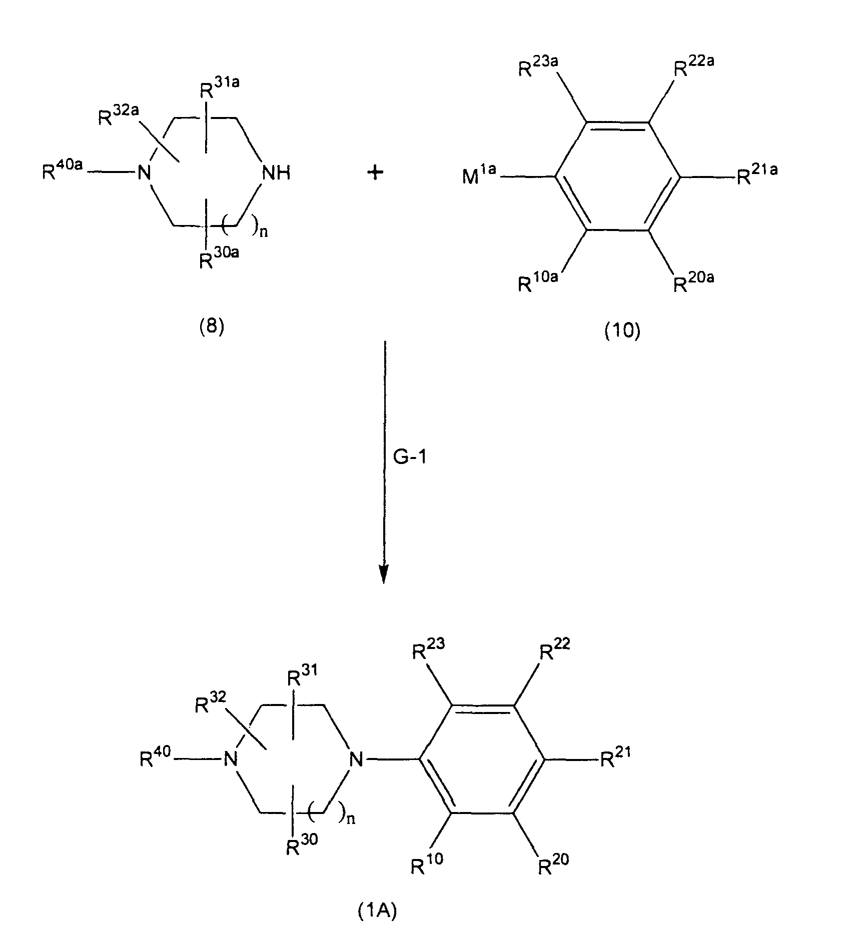

Method G is a method of producing compound (1 A) of the invention (the compound (1) wherein X¹ is nitrogen) by reacting compound (8) with compound (10) in an inert solvent, in the presence of a copper catalyst, in the presence of a base, in the presence or in the absence of oxygen, and optionally removing protecting groups on the resultant compound.
In this scheme, R¹⁰, R²⁰ R²¹, R²², R²³, R³⁰, R³¹, R³², R⁴⁰, n, R^{10a}, R^{20a}, R^{21a}, R^{22a}, R^{23a}, R^{30a}, R^{31a}, R^{32a} and R^{40a} have the same definitions as above. M^{1a} is a group represented by the formula -B(OH)₂.

There are no particular restrictions on the solvent used so long as it dissolves the starting compound to some extent and does not inhibit the reaction of this step, and specifically there may be mentioned amides such as formamide, dimethylformamide, dimethylacetamide, hexamethylphosphoric triamide and N-methylpyrrolidone, halogenated hydrocarbons such as chloroform, dichloromethane, 1,2-dichloroethane and carbon tetrachloride, aromatic hydrocarbons such as toluene, benzene and xylene or ethers such as diethyl ether, diisopropyl ether, tetrahydrofuran, dioxane, dimethoxyethane and diethyleneglycol dimethyl ether, or mixtures of these solvents, among which halogenated hydrocarbons (particularly dichloromethane) are preferred.
There are no particular restrictions on the copper catalyst used so long as it can yield the target compound and does not produce any unseparable by-products, and specifically there may be mentioned copper(II) acetate, copper(I) acetate, copper(II) trifluoromethanesulfonate and copper(II) isobutyrate, among which copper(II) acetate is preferred.
There are no particular restrictions on the base used so long as it can yield the target compound and does not produce any unseparable by-products, and specifically there may be mentioned organic bases such as triethylamine, pyridine, 2,6-lutidine, N-methylmorpholine and 1,8-diazabicyclo[5.4.0]undec-7-ene, among which triethylamine or pyridine is preferred.
There are no particular restrictions on the additive used to accelerate the reaction so long as it can yield the target compound and does not produce any unseparable by-products, and specifically there may be mentioned molecular sieve, pyridine-N-oxide and 2,2,6,6-tetramethylpiperidinooxy, among which molecular sieve (particularly 4Å) is preferred.
The reaction temperature will differ depending on the starting materials, solvent and reagents, but will usually be between 0°C and 80°C, and is preferably between 10°C and 50°C.
The reaction time will differ depending on the starting materials, solvent, reagents and reaction temperature, but will usually be 1 to 100 hours, and is preferably 24 to 48 hours.

Compound (1A) of the invention may be isolated or purified from the reaction mixture obtained in the manner described above, by the following method.
When R¹⁰ of the resultant compound (1A) is optionally substituted 5- to 10-membered cycloalkenyl, it may be subjected to the hydrogenation described for Method A above to yield compound (1A) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method H)

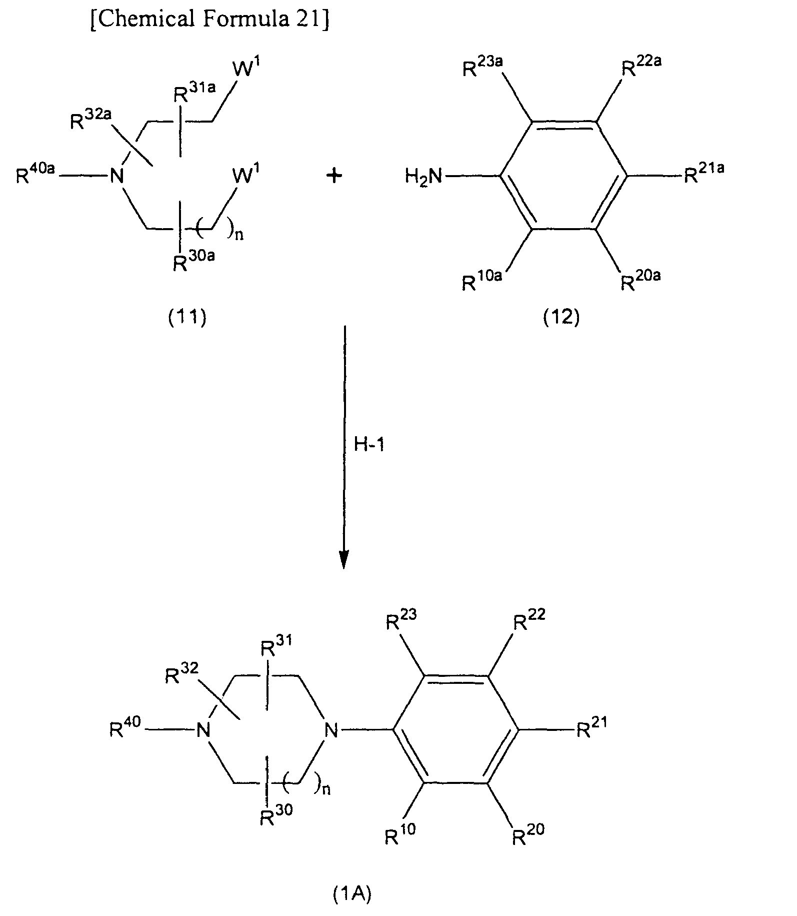

Method H is a method of producing compound (1A) of the invention (the compound (1) wherein X¹ is nitrogen) by reacting compound (11) with compound (12) in an inert solvent or in the absence of a solvent, under or not under an inert gas atmosphere, in the presence or in the absence of a base, in the presence or in the absence of an additive, and optionally removing protecting groups on the resultant compound.
In this scheme, R¹⁰, R²⁰, R²¹, R²², R²³, R³⁰, R³¹, R³², R⁴⁰, n, W¹, R^{10a}, R^{20a}, R^{21a}, R^{22a}, R^{23a}, R^{30a}, R^{31a}, R^{32a} and R^{40a} have the same definitions as above.

There are no particular restrictions on the solvent used so long as it dissolves the starting compound to some extent and does not inhibit the reaction of this step, and specifically there may be mentioned alcohols such as methanol, ethanol, n-propanol, isopropanol, n-butanol, isobutanol, t-butanol, isoamyl alcohol, diethylene glycol, glycerin, octanol, cyclohexanol and methyl cellosolve, aromatic hydrocarbons such as benzene, chlorobenzene, 1,2-dichlorobenzene, toluene and xylene, ethers such as diethyl ether, diisopropyl ether, tetrahydrofuran, dioxane, dimethoxyethane and diethyleneglycol dimethyl ether, and amides such as dimethylformamide, dimethylacetamide, hexamethylphosphoric triamide and N-methylpyrrolidone, among which butanol, 1,2-dichlorobenzene, toluene, xylene, tetrahydrofuran, dioxane, dimethylformamide or hexamethylphosphoric triamide is preferred.
When no solvent is used, the reaction may be carried out using a microwave reactor or with alumina or silica gel as a carrier.
When the reaction is carried out under an atmosphere of an inert gas, there are no particular restrictions on the inert gas so long as it does not inhibit the reaction of this step, and specifically there may be mentioned argon or nitrogen gas.
There are no particular restrictions on the base used so long as it can yield the target compound and does not produce any unseparable by-products, and specifically there may be mentioned organic bases such as triethylamine, pyridine, diisopropylethylamine, 4-dimethylaminopyridine, DBU and DABCO, or inorganic bases such as potassium carbonate, sodium carbonate and sodium hydrogencarbonate.
There are no particular restrictions on the additive used to accelerate the reaction so long as it can yield the target compound and does not produce any unseparable by-products, and specifically there may be mentioned alkali metal iodides such as sodium iodide and potassium iodide.
The reaction temperature will differ depending on the starting materials, solvent and reagents, but will usually be between 0°C and 270°C.
The reaction time will differ depending on the starting materials, solvent, reagents and reaction temperature, but will usually be 0.5 to 100 hours.

Compound (1A) of the invention may be isolated or purified from the reaction mixture obtained above, by the method described below.
When R¹⁰ of the resultant compound (1A) is optionally substituted 5- to 10-membered cycloalkenyl, it may be subjected to the hydrogenation described for Method A above to yield compound (1A) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method K)

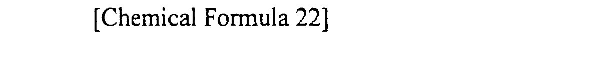

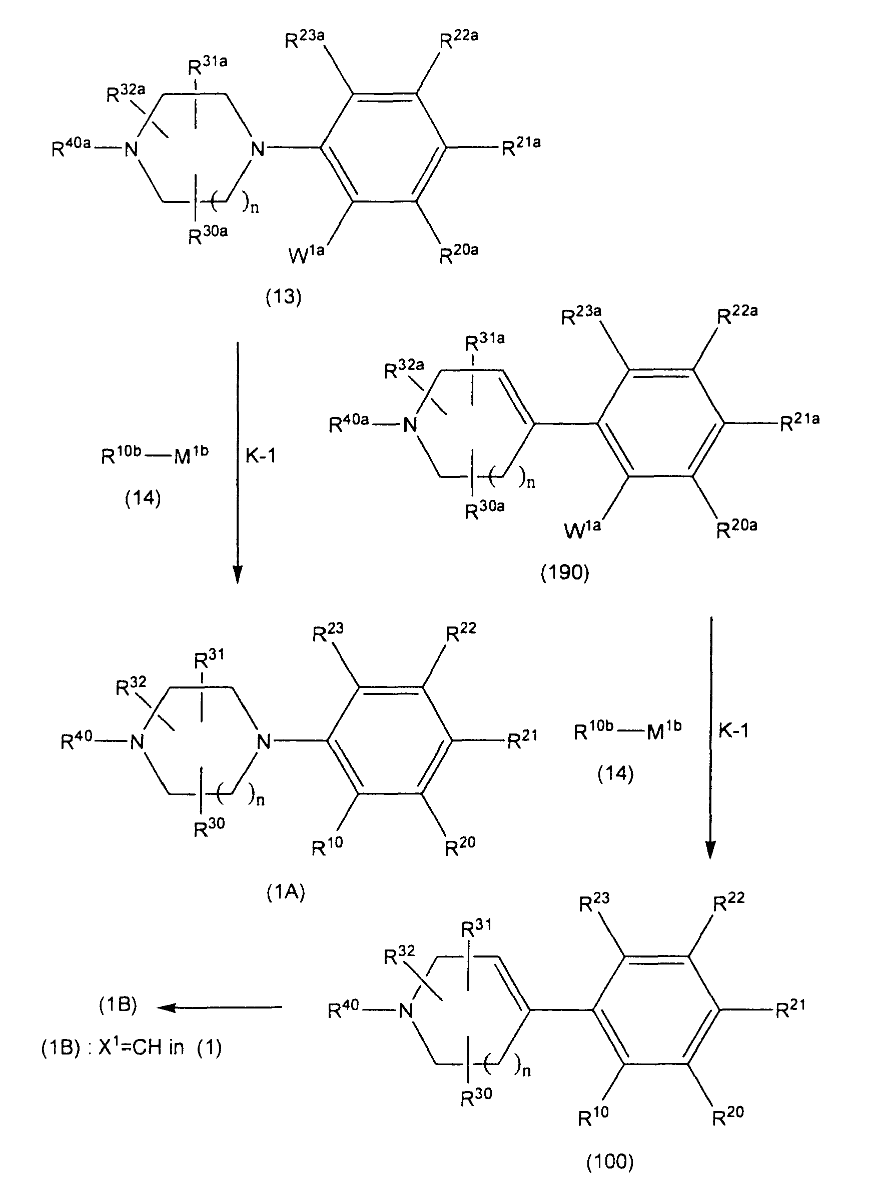

Method K is a method of producing compound (1A) of the invention (the compound (1) wherein X¹ is nitrogen) by reacting compound (13) with a boron metal reagent (14) or a tin metal reagent (14) (Suzuki reaction or Stille reaction) in an inert solvent, in the presence of a palladium(0) catalyst, under or not under an inert gas atmosphere, in the presence or in the absence of a base, in the presence or in the absence of an additive, and optionally removing protecting groups on the resultant compound, or a method of producing compound (100) of the invention by reacting compound (190) in a similar manner.
In this scheme, R¹⁰, R²⁰, R²¹, R²², R²³, R³⁰, R³¹, R³², R⁴⁰, n, W^{1a}, R^{20a}, R^{21a}, R^{22a}, R^{23a}, R^{30a}, R^{31a}, R^{32a} and R^{40a} have the same definitions as above. R^{10b} represents optionally substituted 5- to 10-membered cycloalkenyl, where the substituent has the same definition as the substituent of the "optionally substituted 5- to 10-membered cycloalkenyl" for R¹⁰, with the proviso that this substituent may be protected.
Also, M^{1b} represents B(OE^{10c})₂ or Sn(E^{10b})₃, wherein E^{10c} represents C1-6 alkyl or the two of E^{10c} bond together to form C2-3 alkylene optionally substituted with methyl, and E^{10b} represents C1-6 alkyl.
This method will differ depending on the nature of M^{1b}.

### (Suzuki coupling reaction)

This method is a method of producing compound (1A) of the invention (the compound (1) wherein X¹ is nitrogen) by reacting compound (13) with compound (14) in an inert solvent, in the presence of a palladium(0) catalyst, in the presence of a base, in the presence or in the absence of an additive, under or not under an inert gas atmosphere, and optionally removing protecting groups on the resultant compound, or a method of producing compound (100) of the invention by reacting compound (190) in a similar manner.
There are no particular restrictions on the solvent used so long as it dissolves the starting compound to some extent and does not inhibit the reaction of this step, and specifically there may be mentioned amides such as formamide, dimethylformamide, dimethylacetamide, hexamethylphosphoric triamide and N-methylpyrrolidone, aromatic hydrocarbons such as toluene, benzene, xylene and mesitylene, ethers such as diethyl ether, diisopropyl ether, tetrahydrofuran, dioxane, dimethoxyethane and diethyleneglycol dimethyl ether, alcohols such as methanol, ethanol, n-propanol, isopropanol, n-butanol, isobutanol, t-butanol, isoamyl alcohol, diethylene glycol, glycerin, octanol, cyclohexanol and methyl cellosolve, nitriles such as acetonitrile and isobutyronitrile, sulfoxides such as dimethylsulfoxide and sulfolane, or water, or mixtures of these solvents, among which dimethylformamide, toluene, xylene, tetrahydrofuran, dioxane, dimethoxyethane or water, or mixtures of these solvents, are preferred.
There are no particular restrictions on the palladium(0) catalyst used so long as it can yield the target compound and does not produce any unseparable by-products, and specifically there may be mentioned tetrakis(triphenylphosphine)palladium, tris(dibenzylideneacetone)dipalladium, bis(dibenzylideneacetone)palladium, bis(tri-t-butylphosphine)palladium, palladium black and the like, or palladium(0) catalysts produced in the reaction system by combination of the palladium complexes which can be palladium(0) precursors mentioned below and various ligands mentioned below.
There are no particular restrictions on various palladium complexes which can be used as palladium(0) precursor, so long as they can yield the target compound without producing any unseparable by-products, and specifically there may be mentioned palladium acetate, [1,1'-bis(diphenylphosphino)ferrocene] dichloropalladium, dichlorobis(tri-o-tolylphosphine)palladium, dichlorobis(triscyclohexylphosphine)palladium, and the like.
There are no particular restrictions on ligands used so long as they can yield the target compound without producing any unseparable by-products, and specifically there may be mentioned triphenylphosphine, tri-t-butylphosphine, tri(4-methylphenyl)phosphine, 2-(di-t-butylphosphino)biphenyl, 2-(dicyclohexylphosphino)biphenyl, tricyclohexylphosphine, 1,1'-bis(diphenylphosphino)ferrocene and di-t-butylphosphonium tetrafluoroborate.
There are no particular restrictions on the base used so long as it can yield the target compound and does not produce any unseparable by-products, and specifically there may be mentioned inorganic bases such as tripotassium phosphate, trisodium phosphate, cesium carbonate, potassium carbonate, sodium carbonate, cesium bicarbonate, potassium hydrogencarbonate, sodium hydrogencarbonate, sodium acetate, barium hydroxide, potassium hydroxide, potassium fluoride and cesium fluoride, metal alkoxides such as sodium ethoxide and sodium-t-butoxide, alkali metal acetate such as sodium acetate or potassium acetate, or organic bases such as triethylamine.
There are no particular restrictions on the additive used so long as it can yield the target compound and does not produce any unseparable by-products, and specifically there may be mentioned lithium chloride, sodium chloride, lithium bromide, sodium bromide and tetrabutylammonium bromide.
The reaction temperature will differ depending on the starting materials, solvent and reagents, but will usually be between 0°C and 150°C, and is preferably between 20°C and 120°C.
The reaction time will differ depending on the starting materials, solvent, reagents and reaction temperature, but will usually be 0.5 to 100 hours, and is preferably 0.5 to 48 hours.
When the reaction is carried out in an inert gas atmosphere, the inert gas is not particularly restricted so long as it does not inhibit the reaction of this step, and specifically it may be argon or nitrogen gas.
As supplementary literature to be used as reference for carrying out this method, there may be mentioned, but not limited to: S. P. Stanforth, Tetrahedron (1998), 54, 263; and N. Miyaura, A. Suzuki, Chem. Rev. (1995), 95, 2457.

### (Stille coupling reaction)

This method is a method of producing compound (1A) of the invention (the compound (1) wherein X¹ is nitrogen) by reacting compound (13) with compound (14) in an inert solvent, in the presence of a palladium(0) catalyst, in the presence or in the absence of an additive, under or not under an inert gas atmosphere, and optionally removing protecting groups on the resultant compound, or a method of producing compound (100) of the invention by reacting compound (190) in a similar manner.
There are no particular restrictions on the solvent used so long as it dissolves the starting compound to some extent and does not inhibit the reaction of this step, and specifically there may be mentioned amides such as dimethylformamide, dimethylacetamide, hexamethylphosphoric triamide and N-methylpyrrolidone, aromatic hydrocarbons such as toluene, benzene, xylene and mesitylene, ethers such as diethyl ether, diisopropyl ether, tetrahydrofuran, dioxane, dimethoxyethane and diethyleneglycol dimethyl ether, or mixtures of these solvents, among which dimethylformamide, toluene, xylene, tetrahydrofuran, dioxane and dimethoxyethane are preferred.
There are no particular restrictions on the palladium(0) catalyst used so long as it can yield the target compound and does not produce any unseparable by-products, and specifically there may be mentioned tetrakis(triphenylphosphine)palladium, tris(dibenzylideneacetone)dipalladium, bis(dibenzylideneacetone)palladium, bis(tri-t-butylphosphine)palladium, palladium black and the like, or palladium(0) catalysts produced in the reaction system by combination of the palladium complexes which can be palladium(0) precursors mentioned below and various ligands mentioned below.
There are no particular restrictions on various palladium complexes which can be used as palladium(0) precursors, so long as they can yield the target compound without producing any unseparable by-products, and specifically there may be mentioned palladium acetate, [1,1'-bis(diphenylphosphino)ferrocene] dichloropalladium, dichlorobis(tri-o-tolylphosphine)palladium, dichlorobis(acetonitrile)palladium and dichlorobis(triscyclohexylphosphine)palladium. There are no particular restrictions on ligands used so long as they can yield the target compound without producing any unseparable by-products, and specifically there may be mentioned triphenylphosphine, tri-t-butylphosphine, tri(4-methylphenyl)phosphine, 2-(di-t-butylphosphino)biphenyl, 2-(dicyclohexylphosphino)biphenyl, tricyclohexylphosphine, tri-2-furylphosphine, 1,1'-bis(diphenylphosphino)ferrocene, di-t-butylphosphonium tetrafluoroborate and triphenylarsine.
There are no particular restrictions on the additive used so long as it can yield the target compound and does not produce any unseparable by-products, and specifically there may be mentioned cesium fluoride, potassium fluoride, lithium chloride, lithium bromide, sodium bromide, tetrabutylammonium fluoride, copper iodide, copper oxide and zinc chloride.
The reaction temperature will differ depending on the starting materials, solvent and reagents, but will usually be between 0°C and 150°C, and is preferably between 20°C and 110°C.
The reaction time will differ depending on the starting materials, solvent, reagents and reaction temperature, but will usually be 0.1 to 100 hours, and is preferably 0.5 to 48 hours.
When the reaction is carried out in an inert gas atmosphere, the inert gas is not particularly restricted so long as it does not inhibit the reaction of this step, and specifically it may be argon or nitrogen gas.
As supplementary literature to be used as reference for carrying out this method, there may be mentioned, but not limited to: S .P. Stanforth, Tetrahedron (1998), 54, 263; and J.K. Stille, Angew. Chem. Int. Ed. Engl. (1986), 25, 508.

Compounds (1A) and (100) of the invention may be isolated or purified from the reaction mixture obtained above, by the method described below. When R¹⁰ of the resultant compound (1A) or (100) is optionally substituted 5- to 10-membered cycloalkenyl, it may be subjected to the hydrogenation described for Method A above to yield compound (1A) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method M)

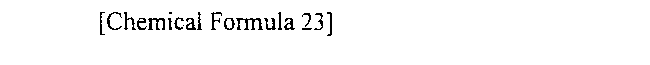

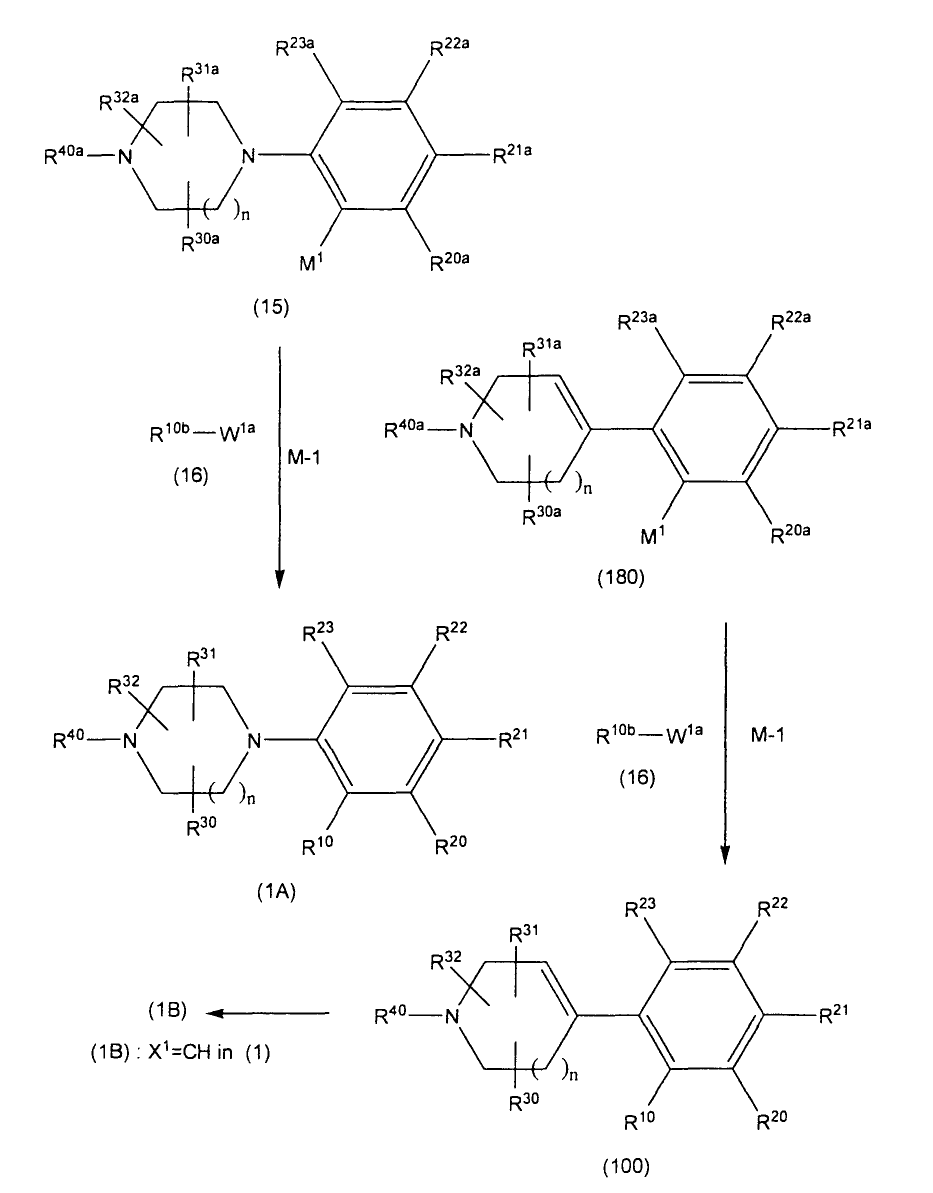

Method M is a method of producing compound (1A) of the invention (the compound (1) wherein X¹ is nitrogen) by reacting compound (16) with a boron metal reagent (15) or tin metal reagent (15) (Suzuki reaction or Stille reaction) in an inert solvent, in the presence of a palladium(0) catalyst, under or not under an inert gas atmosphere, in the presence or in the absence of a base, in the presence or in the absence of an additive, and optionally removing protecting groups on the resultant compound, or a method of producing compound (100) of the invention by reacting compound (180) in a similar manner.
In this scheme, R¹⁰, R²⁰, R²¹, R²², R²³, R³⁰, R³¹, R³², R⁴⁰, n, W^{1a}, R^{20a}, R^{21a}, R^{22a}, R^{23a}, R^{30a}, R^{31a}, R^{32a} and R^{40a} have the same definitions as above. R^{10b} also has the same definition as above.
M¹ represents B(OE^{10a})₂ or Sn(E^{10b})₃, wherein E^{10a} represents hydrogen, C1-6 alkyl or the two of E^{10a} bond together to form C2-3 alkylene optionally substituted with methyl, and E^{10b} represents C 1-6 alkyl.
This method may be carried out in a manner similar to Method K above.

Compounds (1A) and (100) of the invention may be isolated or purified from the reaction mixture obtained above, by the method described below.
When R¹⁰ of the resultant compound (1A) or (100) is optionally substituted 5- to 10-membered cycloalkenyl, it may be subjected to the hydrogenation described for Method A above to yield compound (1A) of the invention or compound (1B) of the invention (the compound (1) wherein X¹ is CH), wherein R¹⁰ is 5- to 10-membered cycloalkyl optionally substituted with the corresponding substituent.
When R¹⁰ of the resultant compound (100) is optionally substituted 5- to 10-membered cycloalkyl, it may be subjected to hydrogenation to yield compound (1B) of the invention (the compound (1) wherein X¹ is CH).
When the resultant compound is to be converted to an acid salt, this may be carried out by a conventional method. The step of producing the salt and the step of hydrogenation described above may be carried out in a different order where appropriate.

### (Method N)

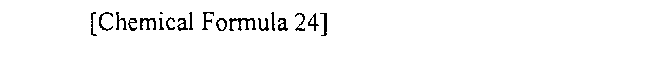

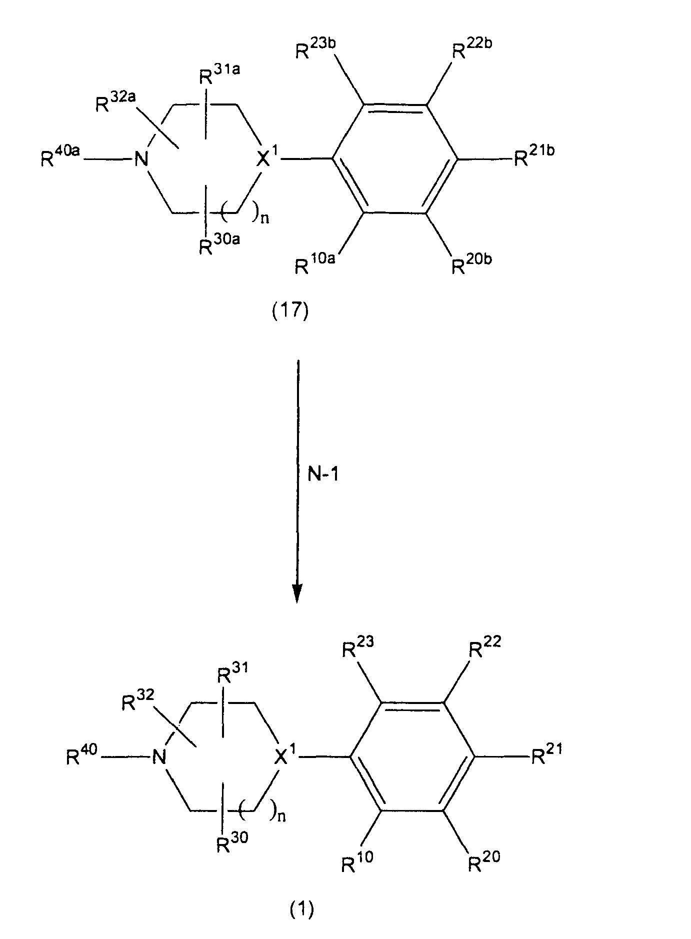

Method N is a method of producing compound (1) of the invention by reacting compound (17) with a halogenating reagent in an inert solvent, in the presence or in the absence of an additive, in the presence or in the absence of an inert gas, to yield a compound halogenated on the benzene ring to which R^{10a} is bonded (Step N-1-1), and optionally removing protecting groups on the resultant compound. Alternatively, Step N-1-1 may be followed by reaction of the halogenated compound with a compound which can introduce a desired substituent, or a reactive derivative thereof, in the presence of a transition metal catalyst, in an inert solvent, in the presence or in the absence of an additive, in the presence or in the absence of an inert gas (Step N-1-2), and optionally removing any protecting groups on the resultant compound, to produce compound (1) of the invention.
In this scheme, R¹⁰, R²⁰, R²¹, R²², R²³, R³⁰, R³¹, R³², R⁴⁰, n, X¹, R^{10a}, R^{30a}, R^{31a}, R^{32a} and R^{40a} have the same definitions as above. Also, at least one of R^{20b}, R^{21b}, R^{22b} and R^{23b} is hydrogen, and the remaining groups each have the same definition as the corresponding group of R^{20a}, R^{21a}, R^{22a} and R^{23a}.

### (Step N-1-1)

This is a halogenating step.
There are no particular restrictions on the solvent used so long as it dissolves the starting compound to some extent and does not inhibit the reaction of this step, and specifically there may be mentioned alcohols such as methanol, ethanol, n-propanol, isopropanol, n-butanol, isobutanol, t-butanol, isoamyl alcohol, diethylene glycol, glycerin, octanol, cyclohexanol and methyl cellosolve, aliphatic hydrocarbons such as hexane, heptane, ligroin and petroleum ether, ethers such as dioxane, dimethoxyethane and diethyleneglycol dimethyl ether, halogenated hydrocarbons such as chloroform, dichloromethane, 1,2-dichloroethane and carbon tetrachloride, ethers such as diethyl ether, diisopropyl ether, tetrahydrofuran, dioxane, dimethoxyethane and diethyleneglycol dimethyl ether, amides such as formamide, dimethylformamide, dimethylacetamide and hexamethylphosphoric triamide, and organic acids such as acetic acid, among which alcohols (particularly methanol) are preferred.
There are no particular restrictions on the halogenating agent used so long as it can yield the target compound and does not produce any unseparable by-products, and specifically there may be mentioned chlorine (Cl₂), bromine (Br₂), iodine (I₂), N-chlorosuccinimide, bromosuccinimide, N-iodosuccinimide, iodine monochloride and thionyl chloride, among which chlorine, bromine and iodine are preferred.
As additives to be used there may be mentioned alkali metal acetate such as sodium acetate and potassium acetate, among which sodium acetate is preferred.
The reaction temperature will differ depending on the starting materials, solvent and reagents, but will usually be between -20°C and 100°C, and is preferably between 20°C and 50°C.
The reaction time will differ depending on the starting materials, solvent, reagents and reaction temperature, but will usually be 0.25 to 48 hours, and is preferably 12 to 24 hours.

Compound (1) of the invention may be isolated or purified from the reaction mixture obtained above, by the method described below.
When R¹⁰ of the resultant compound (1) is optionally substituted 5- to 10-membered cycloalkenyl, the hydrogenation described for Method A above may be carried out by selecting the reaction conditions so as to avoid reducing the introduced halogen, to yield compound (1) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Step N-1-2)

In this step, the halogenated compound obtained in Step N-1-1 is converted to a compound having a desired substituent in the presence of a transition metal catalyst, and protecting groups on the resultant compound are optionally removed by the method described below, to produce compound (1) of the invention.
The aryl halide compound obtained in Step N-1-1 may then be subjected to cross coupling reaction with a compound which can introduce a desired substituent or a reactive derivative thereof, in the presence of a transition metal such as palladium, copper, nickel, zinc or zirconium, or a catalyst produced by combination of any of these metals with a ligand. The bond formation reaction type may be carbon-carbon bond formation, carbon-nitrogen bond formation or carbon-oxygen bond formation. Method F and Method K are examples of these reactions.
As supplementary literature to be used as reference for carrying out this step there may be mentioned, but not limited to: John F. Hartwig, Angew. Chem. Int. Ed., (1998), 37, 2046; Steven P. Nolan, et al., Org. Lett. (2001), 3, 10, 1511; Stephen L. Buchwald and Gregory C. Fu, et al., Org. Lett. (2000), 2, 12, 1729; Stephen P. Stanforth, Tetrahedron (1998), 54, 263; Karen, E. et. al., J.A.C.S. (2001), 123, 10770; Stephen L. Buchwald, et. al., J.A.C.S. (1999), 121, 4369; D. M. Tschaen and R. Desmond, et al., Synth. Comm. (1994), 24, 6, 887; John F. Hartwig, et. al., J.A.C.S. (2001), 123. 8410; Gregory C. Fu, et al., Org. Lett. (2001), 3, 26, 4295; and Damien Prim, et al., Tetrahedron (2002), 58, 2041.
For example, introduction of morpholine as a substituent in a carbon-nitrogen bond formation reaction may be carried out in the following manner.
There are no particular restrictions on the solvent used so long as it dissolves the starting compound to some extent and does not inhibit the reaction of this step, and specifically there may be mentioned aromatic hydrocarbons such as benzene, toluene and xylene, ethers such as dioxane, dimethoxyethane and tetrahydrofuran, and amides such as dimethylformamide, among which xylene is preferred.
There are no particular restrictions on the additive used so long as it can yield the target compound and does not produce any unseparable by-products, and it may be an appropriate combination of palladium catalysts such as palladium(II) acetate, bases such as potassium t-butoxide, sodium t-butoxide and cesium carbonate, and phosphines such as 2,2'-bis(diphenylphosphino)-1,1'-binaphthyl and tri-t-butylphosphonium tetrafluoroborate, among which a combination of palladium(II) acetate, sodium t-butoxide and tri-t-butylphosphonium tetrafluoroborate is preferred.
The reaction temperature will differ depending on the starting materials, solvent and reagents, but will usually be between 50°C and 200°C, and is preferably between 70°C and 150°C.
The reaction time will differ depending on the starting materials, solvent, reagents and reaction temperature, but will usually be 0.5 to 72 hours, and is preferably 2 to 24 hours.

Compound (1) of the invention may be isolated or purified from the reaction mixture obtained above, by the following method.
When R¹⁰ of the resultant compound (1) is optionally substituted 5- to 10-membered cycloalkenyl, it may be subjected to the hydrogenation described for Method A above to yield compound (1) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method P)

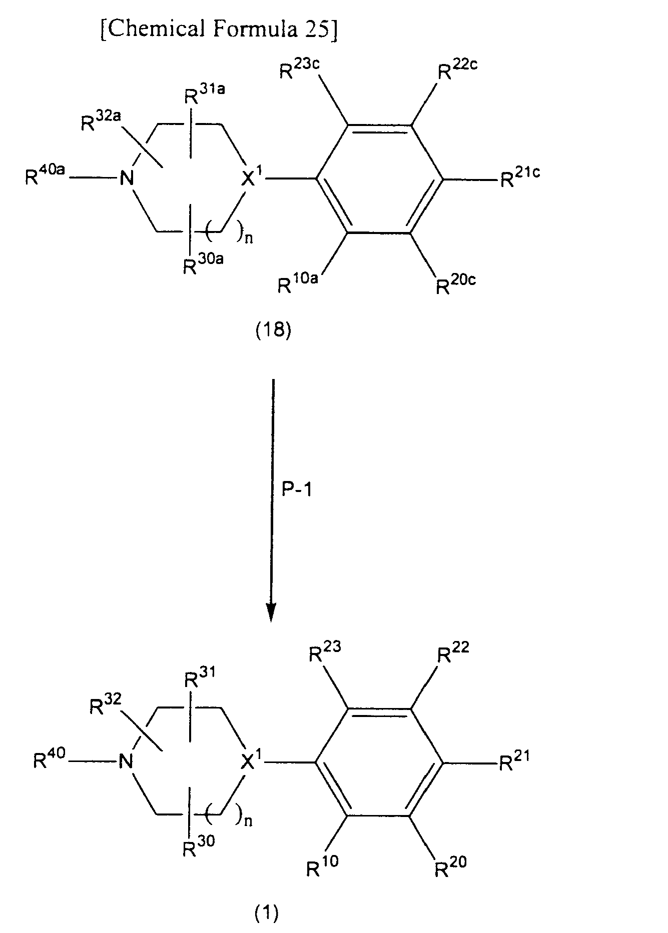

Method P is a method of producing compound (1) of the invention by reacting compound (18) with a trifluoromethanesulfonylating agent in an inert solvent, to yield a compound wherein the phenolic hydroxyl has been trifluoromethanesulfonylated (Step P-1-1), and optionally removing protecting groups on the resultant compound. Alternatively. Step P-1-1 may be followed by reaction with a compound which can introduce a desired substituent into the phenyltriflate compound, or a reactive derivative thereof (Step P-1-2), and optionally removal of protecting groups on the resultant compound, to produce compound (1) of the invention.
This method may be carried out when a phenolic hydroxyl group is present on the benzene ring to which R^{10a} is bonded.
In this scheme, R¹⁰, R²⁰, R²¹, R²², R²³, R³⁰, R³¹, R³², R⁴⁰ n, X¹. R^{10a}, R^{30a}, R^{31a}, R^{32a} and R^{40a} have the same definitions as above. Also, one of R^{20c}, R^{21c}, R^{22c} and R^{23c} is a phenolic hydroxyl group, and the remaining groups each have the same definition as the corresponding group of R^{20a}, R^{21a}, R^{22a} and R^{23a}.

### (Step P-1-1)

This step may be carried out in a manner similar to the sulfonylation described for Method A or Method B above. Trifluoromethanesulfonylation may be replaced by nonafluorobutanesulfonylation or toluenesulfonylation.

Compound (1) of the invention may be isolated or purified from the reaction mixture obtained above, by the method described below.
When R¹⁰ of the resultant compound (1) is optionally substituted 5- to 10-membered cycloalkenyl, it may be subjected to the hydrogenation described for Method A above to yield compound (1) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Step P-1-2)

This is a step of converting the trifluoromethanesulfonyloxy group of the compound obtained in Step P-1-1 to a desired substituent.
This step may be carried out in a manner similar to Method N-1-2 above. As supplementary literature to be used as reference for carrying out this method, there may be mentioned, but not limited to, Kurt Ritter, Synthesis, (1993), 735.

Compound (1) of the invention may be isolated or purified from the reaction mixture obtained above, by the method described below.
When R¹⁰ of the resultant compound (1) is optionally substituted 5- to 10-membered cycloalkenyl, it may be subjected to the hydrogenation described for Method A above to yield compound (1) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method Q)

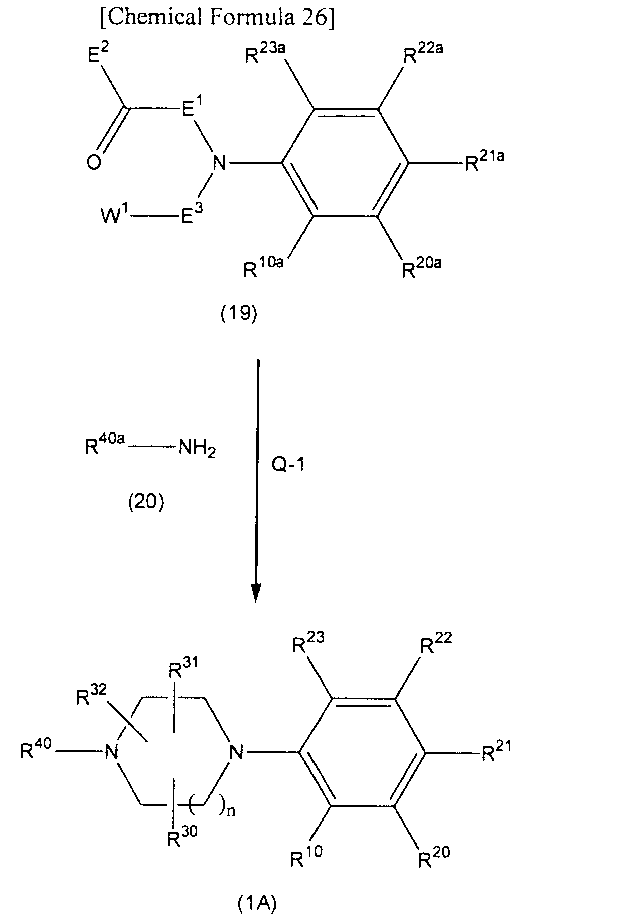

Method Q is a method of producing compound (1A) of the invention (the compound (1) wherein X¹ is nitrogen) by reacting compound (19) with compound (20) in an inert solvent, in the presence of a reducing agent, in the presence or in the absence of an acid, in the presence of an additive, and optionally removing protecting groups on the resultant compound.
In this scheme, R¹⁰, R²⁰, R²¹, R²², R²³, R³⁰, R³¹, R³², R⁴⁰, n, W¹, R^{10a}, R^{20a}, R^{21a}, R^{22a}, R^{23a} and R^{40a} have the same definitions as above. Also, E¹, E² and E³ are groups suitable for obtaining the desired group of the formula:

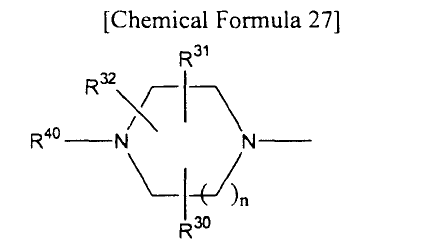

Substituents on E¹, E² or E³ may optionally be protected.
This method may be carried out in a manner similar to Method C above.

Compound (1A) of the invention may be isolated or purified from the reaction mixture obtained above, by the method described below.
When R¹⁰ of the resultant compound (1A) is optionally substituted 5- to 10-membered cycloalkenyl, it may be subjected to the hydrogenation described for Method A above to yield compound (1A) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method R)

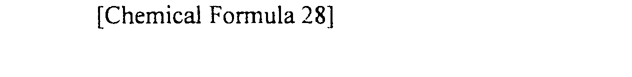

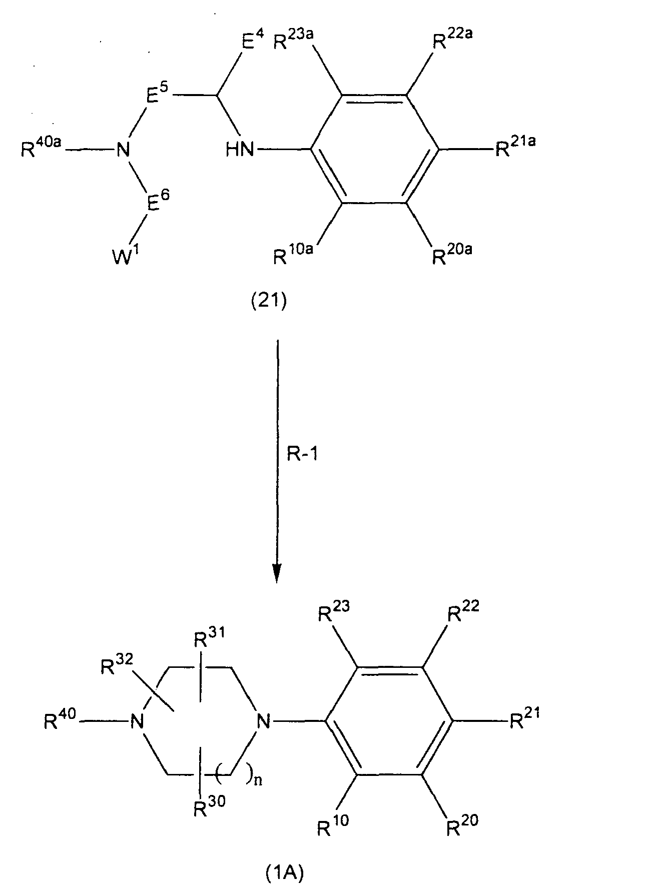

Method R is a method of producing compound (1A) of the invention (the compound (1) wherein X¹ is nitrogen) by reacting compound (21) with a base in an inert solvent, and optionally removing protecting groups on the resultant compound.
In this scheme, R¹⁰, R²⁰, R²¹, R²², R²³, R³⁰, R³¹, R³², R⁴⁰, n, W¹, R^{10a} R^{20a}, R^{21a}, R^{22a}, R^{23a} and R^{40a} have the same definitions as above. Also, E⁴, E⁵ and E⁶ are groups suitable for obtaining the desired group of the formula:

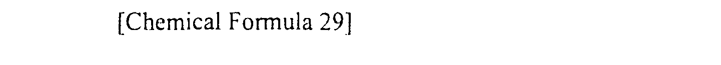

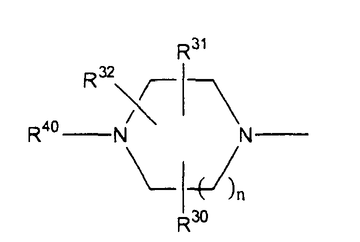

Substituents on E⁴, E⁵ or E⁶ may optionally be protected.
This method may be carried out in a manner similar to Method A above.

Compound (1A) of the invention may be isolated or purified from the reaction mixture obtained above, by the method described below.
When R¹⁰ of the resultant compound (1A) is optionally substituted 5- to 10-membered cycloalkenyl, it may be subjected to the hydrogenation described for Method A above to yield compound (1A) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method S)

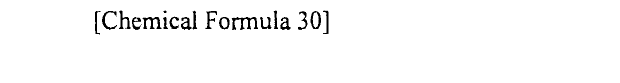

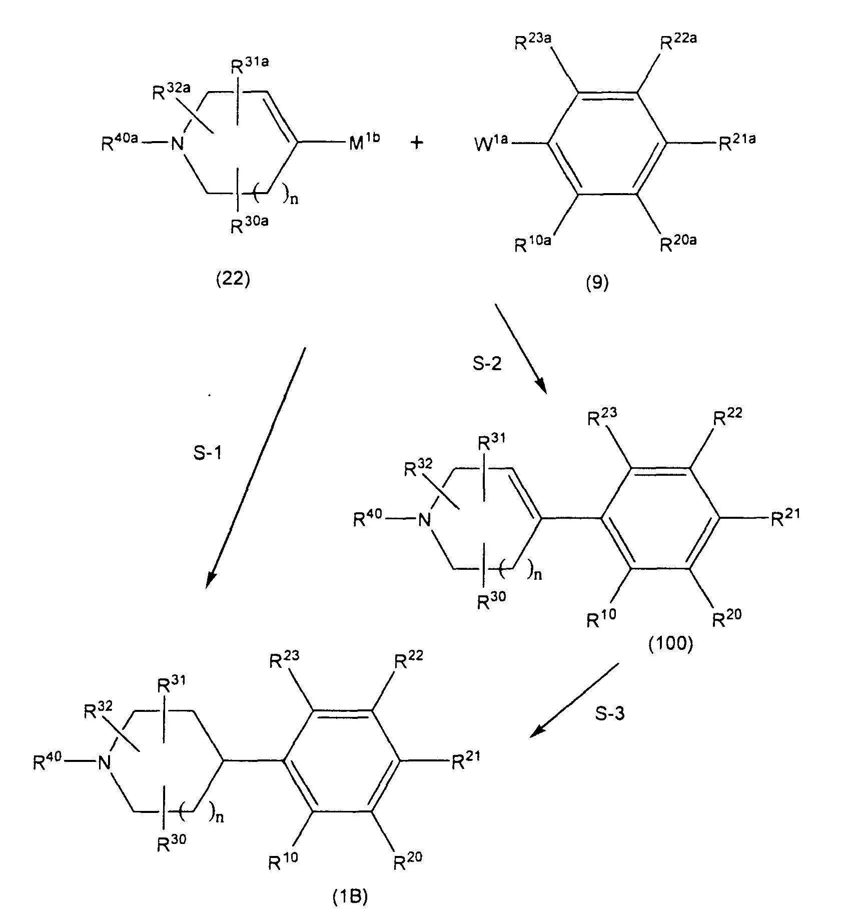

Method S is a method of producing compound (1B) of the invention (the compound (1) wherein X¹ is CH) by reacting compound (22) with compound (9) in an inert solvent, in the presence of a palladium(0) catalyst, and then hydrogenating the product and optionally removing protecting groups on the compound (Method S-1), or a method of producing compound (100) of the invention by reacting compound (22) with compound (9) in the same manner and optionally removing protecting groups on the resultant compound (Method S-2), and if necessary, further leading it to compound (1B) of the invention by hydrogenation (Method S-3).
In this scheme, R¹⁰, R²⁰, R²¹, R²², R²³, R³⁰, R³¹, R³², R⁴⁰, n, W^{1a}, M^{1b}, R^{10a}, R^{20a}, R^{21a}, R^{22a}, R^{23a}, R^{30a}, R^{31a}, R^{32a} and R^{40a} have the same definitions as above.
This method may be carried out in a manner similar to a combination of Method K above and the hydrogenation reaction in Method A above.

Compounds (1B) and (100) of the invention may be isolated or purified from the reaction mixture obtained above, by the method described below.
When the resultant compound is to be converted to an acid salt, this may be carried out by a conventional method. The step of producing the salt and the step of hydrogenation after reaction between compound (22) and compound (9) may be carried out in a different order where appropriate.

### (Method T)

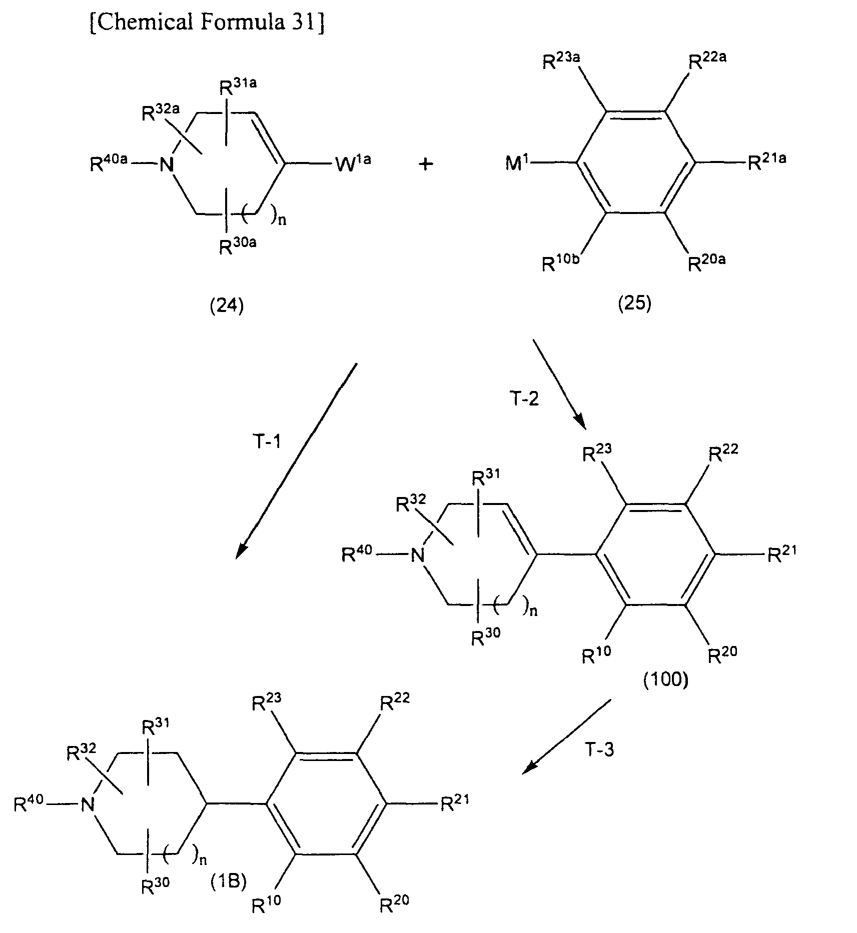

Method T is a method of producing compound (1B) of the invention (the compound (1) wherein X¹ is CH) by reacting compound (24) with compound (25) in an inert solvent, in the presence of a palladium(0) catalyst, and then hydrogenating the product and optionally removing protecting groups on the resultant compound (Method T-1), or a method of producing compound (100) of the invention by reacting compound (24) with compound (25) in the same manner and optionally removing protecting groups on the resultant compound (Method T-2), and if necessary, further leading it to compound (1B) of the invention by hydrogenation (Method T-3).
In this scheme, R¹⁰, R²⁰, R²¹, R²², R²³, R³⁰, R³¹, R³², R⁴⁰, n, W^{1a}, M¹, R^{10b} R^{20a}, R^{21a}, R^{22a} R^{23a}, R^{30a}, R^{31a}, R^{32a} and R^{40a} have the same definitions as above.
This method may be carried out in a manner similar to Method K above and the hydrogenation reaction in Method A above.

Compounds (1B) and (100) of the invention may be isolated or purified from the reaction mixture obtained above, by the method described below.
When the resultant compound is to be converted to an acid salt, this may be carried out by a conventional method. The step of producing the salt and the step of hydrogenation after reaction between compound (24) and compound (25) may be carried out in a different order where appropriate.

### (Method U)

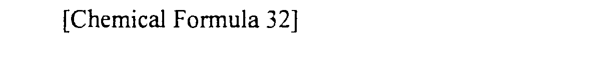

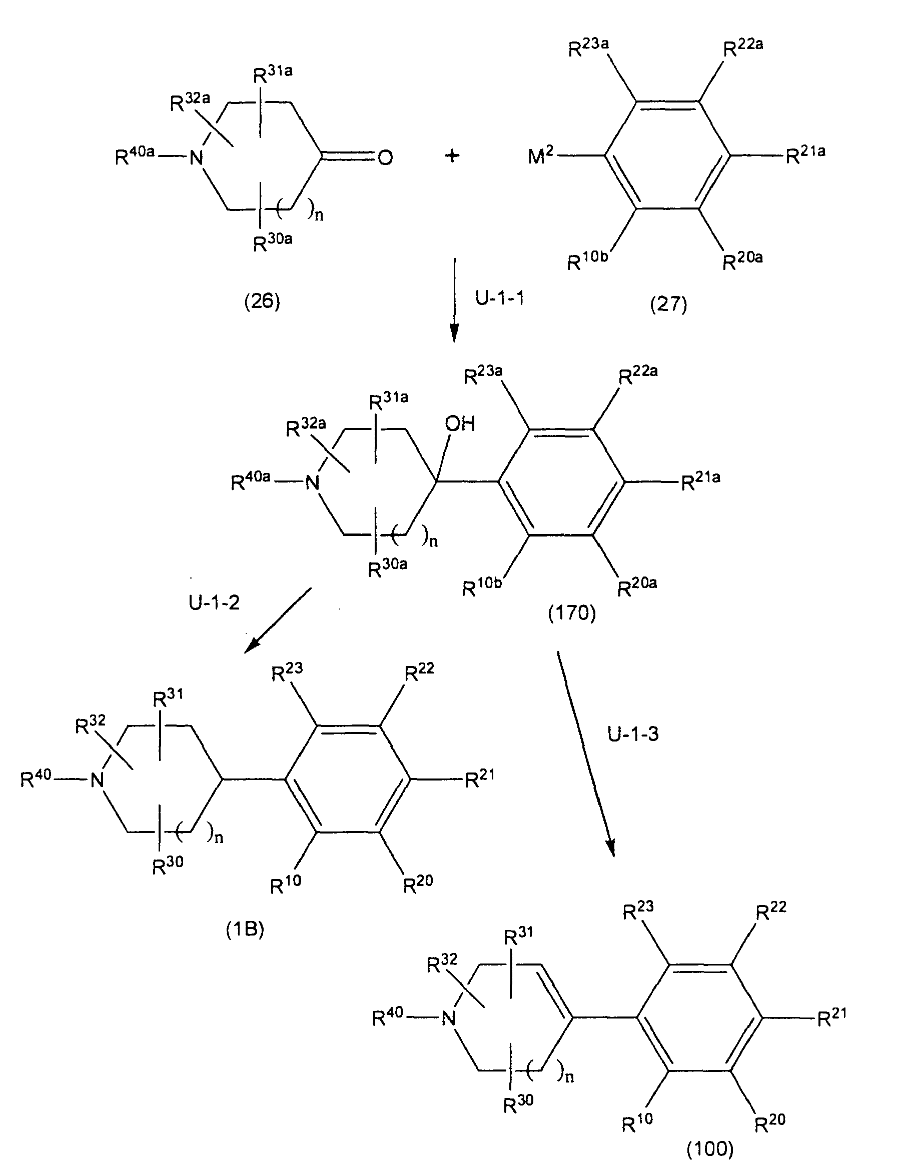

Method U is a method of producing compound (1B) of the invention (the compound (1) wherein X¹ is CH) by reacting compound (26) with compound (27) (i.e., a lithium reagent or Grignard reagent) in an inert solvent to yield an adduct (170) (Step U-1-1), and then reducing the hydroxyl at the benzyl position of the resultant adduct (170) (Step U-1-2), and further optionally removing protecting groups, or a method of producing compound (100) of the invention by the reaction in the same manner to yield an adduct (170) (Step U-1-1), then dehydrating the hydroxyl of the adduct (170) in the presence of or in the absence of acid (Step U-1-3), and optionally removing protecting groups.
In this scheme, R¹⁰, R²⁰, R²¹ R²², R²³, R³⁰, R³¹, R³², R⁴⁰, n, R^{10b}, R^{20a}, R^{21a}, R^{22a}, R^{23a}, R^{30a}, R^{31a}, R^{32a} and R^{40a} have the same definitions as above. M² is a lithium or magnesium halide.

### (Step U-1-1)

There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, among which tetrahydrofuran is preferred.
The reaction temperature will differ depending on the starting materials, solvent and reagents, but will usually be between -80°C and 30°C.
The reaction time will differ depending on the starting materials, solvent, reagents and reaction temperature, but will usually be 0.25 to 6 hours.

### (Step U-1-2)

This step may be carried out by reduction in a manner similar to the hydrogenation method described for Method A above, or by reduction using a trialkylsilyl hydride described below, although there is no limitation to these methods.
There are no particular restrictions on the solvent used so long as it dissolves the starting compound to some extent and does not inhibit the reaction of this step, and specifically there may be mentioned, in the case of reduction reaction using a trialkylsilyl hydride, halogenated hydrocarbons such as chloroform, dichloromethane, 1,2-dichloroethane and carbon tetrachloride (particularly dichloromethane).
There are no particular restrictions on the reducing agents used so long as it can yield the target compound and does not produce any unseparable by-products, and specifically there may be mentioned trialkylsilyl hydrides such as triethylsilyl hydride and triisopropylsilyl hydride, among which triethylsilyl hydride is preferred.
There are no particular restrictions on the additive used so long as it can yield the target compound and does not produce any unseparable by-products, and specifically there may be mentioned halo-substituted acetic acids such as trifluoroacetic acid, and Lewis acids such as boron trifluoride.
The reaction temperature will differ depending on the starting materials, solvent and reagents, but will usually be between -70°C and 50°C.
The reaction time will differ depending on the starting materials, solvent, reagents and reaction temperature, but will usually be 0.5 to 48 hours.

### (Step U-1-3)

There are no particular restrictions on the solvent used so long as it dissolves the starting compound to some extent and does not inhibit the reaction of this step, and specifically there may be mentioned, ethers such as diethyl ether, tetrahydrofuran, dioxane, dimethoxyethane and diethyleneglycol dimethylether, aromatic hydrocarbons such as toluene, benzene and xylene, halogenated hydrocarbons such as chloroform, dichloromethane, 1,2-dichloroethane and carbon tetrachloride, water, and the mixed solvent thereof, among which tetrahydrofuran, toluene, dichloromethane, chloroform or water is preferred. This step may be carried out without solvent.
There are no particular restrictions on the acid additive used so long as it can yield the target compound and does not produce any unseparable by-products, and specifically there may be mentioned halo-substituted acetic acids such as trifluoroacetic acid, Lewis acids such as boron trifluoride, organic sulfonic acids such as toluenesulfonic acid and camphor sulfonic acid, and inorganic acids such as hydrochloric acid and hydrogen bromide.
The reaction temperature will differ depending on the starting materials, solvent and reagents, but will usually be between -80°C and 180°C.
The reaction time will differ depending on the starting materials, solvent, reagents and reaction temperature, but will usually be 0.25 to 24 hours.

Compounds (1B) and (100) of the invention may be isolated or purified from the reaction mixture obtained above, by the method described below.
When R¹⁰ of the resultant compound (1B) is optionally substituted 5- to 10-membered cycloalkenyl, it may be subjected to the hydrogenation described for Method A above to yield compound (1B) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

### (Method V)

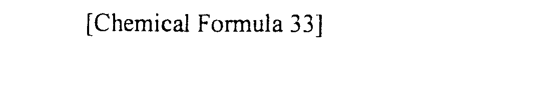

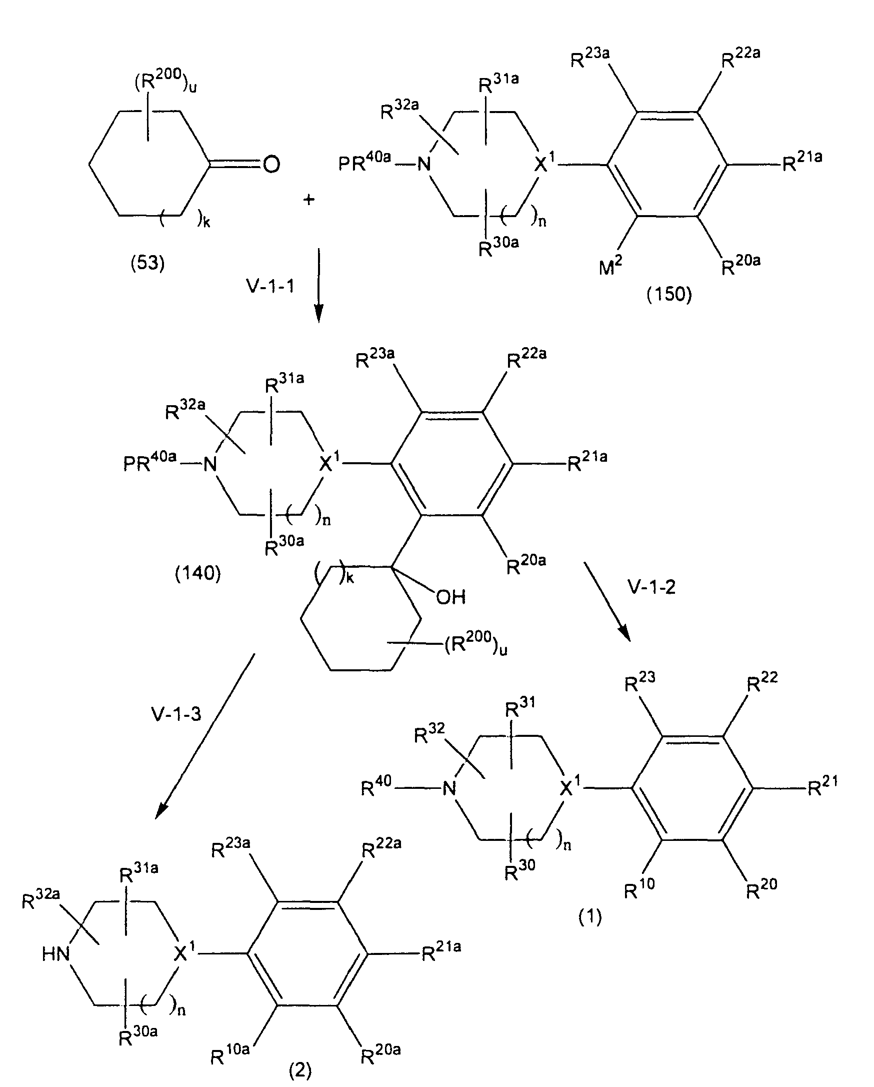

Method V is a method of producing compound (1) of the invention by reacting compound (53) with compound (150) (i.e., a lithium reagent or Grignard reagent) in an inert solvent to yield an adduct (140) (Step V-1-1), and then, if necessary, reducing or dehydrating the hydroxyl at the benzyl position of the resultant adduct (140) (Step V-1-2), and optionally removing protecting groups, or a method of producing compound (2) by the reaction in the same manner to yield an adduct (140) (Step V-1-1), then, if necessary, reducing or dehydrating the hydroxyl at the benzyl position of the adduct (140) (Step V-1-3), and further removing protecting groups.
In this scheme, R¹⁰, R²⁰, R²¹, R²², R²³, R³⁰, R³¹, R³², R⁴⁰, X¹, n, R^{10a}, R^{20a}, R^{21a}, R^{22a}, R^{23a}, R^{30a}, R^{31a} and R^{32a} have the same definitions as above. M² is a lithium or magnesium halide. R²⁰⁰ is a substituent in Group A1, or a substituent in Group A1, which is protected. u is an integer of 0, 1, 2, 3, 4 or 5. k is an integer of 0, 1, 2, 3, 4 or 5. PR^{40a} has the same definition as R^{40a} above or represents protecting group for amino (preferably, t-butoxycarbonyl or benzyl).
This method may be performed in a manner similar to Method U above and the hydrogenation reaction in Method A above.

Compounds (1) and (2) of the invention may be isolated or purified from the reaction mixture obtained above, by the method described below.
When R¹⁰ of the resultant compound (1) is optionally substituted 5- to 10-membered cycloalkenyl, it may be subjected to the hydrogenation described for Method A above to yield compound (1) of the invention wherein R¹⁰ is 5- to 10-membered cycloalkyl optionally substituted with the corresponding substituent.
When the resultant compound is to be converted to an acid salt, this may be carried out by a conventional method. The step of producing the salt and the step of hydrogenation described above may be carried out in a different order where appropriate.

Removal of the protecting group(s) will differ depending on their types, and it may be carried out in the following manner, according to protocols commonly known in the field of synthetic organic chemistry such as the protocol described in, for example: T.W. Greene, (Protective Groups in Organic Synthesis) John Wiley & Sons; or J.F.W. McOmis, (Protective Groups in Organic Chemistry), Plenum Press.

When the amino-protecting group is an optionally substituted silyl group such as trimethylsilyl, triethylsilyl, triisopropylsilyl, t-butyldimethylsilyl or t-butyldiphenylsilyl, it will usually be removed by treatment with a fluoride anion-generating compound such as tetrabutylammonium fluoride, hydrofluoric acid, hydrofluoric acid-pyridine or potassium fluoride.
The inert solvent used for the reaction is not particularly restricted so long as it does not inhibit the reaction, and for example, ethers such as diethyl ether, diisopropyl ether, tetrahydrofuran, dioxane, dimethoxyethane and diethyleneglycol dimethyl ether are preferred.
There are no particular restrictions on the reaction temperature and reaction time, but normally the reaction temperature will be between 0°C and 50°C, and the reaction time will be between 10 to 18 hours.

When the amino-protecting group is an optionally substituted aliphatic acyl group, an optionally substituted aromatic acyl group, an optionally substituted alkoxycarbonyl group or a substituted methylene group which forms a Schiff base, it may be removed by treatment with an acid or base in the presence of an aqueous solvent.
The acid used for this reaction is not particularly restricted so long as it is an acid which is ordinarily used for removal of the amino-protecting group, and for example, it may be an inorganic acid such as hydrobromic acid, hydrochloric acid, sulfuric acid, perchloric acid, phosphoric acid or nitric acid, or an organic acid such as trifluoroacetic acid and trifluoromethanesulfonic acid, among which hydrochloric acid or trifluoroacetic acid is preferred.
The base used for this reaction is not particularly restricted so long as it is a base which is ordinarily used for removal of the amino-protecting group, but there are preferably used alkali metal carbonic acid salts such as lithium carbonate, sodium carbonate and potassium carbonate; alkali metal hydroxides such as lithium hydroxide, sodium hydroxide and potassium hydroxide; metal alkoxides such as lithium methoxide, sodium methoxide, sodium ethoxide and potassium-t-butoxide; and ammonia mixtures such as ammonia water and concentrated ammonia-methanol.
The solvent used for the reaction may be, for example, an alcohol such as methanol, ethanol, n-propanol, isopropanol, n-butanol, isobutanol, t-butanol, isoamyl alcohol, diethylene glycol, glycerin, octanol, cyclohexanol or methyl cellosolve; an ether such as diethyl ether, diisopropyl ether, tetrahydrofuran, dioxane, dimethoxyethane or diethyleneglycol dimethyl ether; water; or a mixture of water and any of the aforementioned solvents, among which alcohols (most preferably ethanol) are preferred.
The reaction temperature and the reaction time will differ depending on the starting compounds, the solvent and the acid or base used and are not particularly restricted, but in order to inhibit by-products, the reaction temperature will usually be between 0°C and 150°C and the reaction time will usually be 1 to 10 hours.

When the amino-protecting group is an optionally substituted aralkyl group or an optionally substituted aralkyloxycarbonyl group, a method of contact with a reducing agent in an inert solvent (preferably catalytic reduction at ordinary temperature in the presence of a catalyst) or a method of removal by oxidation is generally preferred.

The inert solvent used for removal by catalytic reduction is not particularly restricted so long as it is inert to the reaction, and for example, it may be an aliphatic hydrocarbon such as hexane, heptane, ligroin or petroleum ether; an aromatic hydrocarbon such as toluene, benzene or xylene; an ester such as methyl acetate, ethyl acetate, propyl acetate, butyl acetate or diethyl carbonate; an ether such as diethyl ether, diisopropyl ether, tetrahydrofuran, dioxane, dimethoxyethane or diethyleneglycol dimethyl ether; an alcohol such as methanol, ethanol, n-propanol, isopropanol, n-butanol, isobutanol, t-butanol, isoamyl alcohol, diethylene glycol, glycerin, octanol, cyclohexanol or methylcellosolve; an organic acid such as acetic acid; water; or a mixture of any of these solvents with water, among which alcohols, ethers, organic acids and water (most preferably alcohols and organic acids) are preferred.
The catalyst used for removal by catalytic reduction is preferably palladium-carbon, Raney nickel, platinum oxide, platinum-black, rhodium-aluminum oxide, triphenylphosphine-rhodium chloride or palladium-barium sulfate.
There are no particular restrictions on the pressure, but it will ordinarily be from 1 to 10 atmospheres.
The reaction temperature and the reaction time will differ depending on the starting materials, catalyst and insert solvent, but usually the reaction temperature will be between 0°C and 100°C, and the reaction time will be between 5 minutes and 72 hours.

The inert solvent used for removal by oxidation is not particularly restricted so long as it does not participate in the reaction, but water-containing organic solvents are preferred. Such organic solvents include, for example, halogenated hydrocarbons such as chloroform, dichloromethane, 1,2-dichloroethane and carbon tetrachloride; nitriles such as acetonitrile; ethers such as diethyl ether, diisopropyl ether, tetrahydrofuran, dioxane, dimethoxyethane and diethyleneglycol dimethyl ether; ketones such as acetone; amides such as formamide, dimethylformamide, dimethylacetamide and hexamethylphosphoric triamide; and sulfoxides such as dimethylsulfoxide and sulfolane, among which halogenated hydrocarbons, ethers or sulfoxides (most preferably halogenated hydrocarbons and sulfoxides) are preferred.
The oxidizing agent used for this reaction is not particularly restricted so long as it is an oxidizing agent used for removal of the amino-protecting group, but it is preferably potassium persulfate, sodium persulfate, ammonium cerium nitrate (CAN) or 2,3-dichloro-5,6-dicyano-p-benzoquinone (DDQ).
The reaction temperature and the reaction time will differ depending on the starting compounds, oxidizing agent and solvent, but usually the reaction temperature will be between 0°C and 150°C, and the reaction time will be between 10 minutes and 24 hours.

When the amino-protecting group is an optionally substituted aralkyl group, the protecting group may be removed using an acid or base.
The acid used for this reaction is not particularly restricted so long as it is an acid used for removal of the optionally substituted aralkyl group as the amino-protecting group, and for example, it may be a Bronsted acid, e.g. an inorganic acid such as hydrochloric acid, hydrobromic acid, sulfuric acid, perchloric acid or phosphoric acid, or an organic acid such as acetic acid, formic acid, oxalic acid, methanesulfonic acid, p-toluenesulfonic acid, camphor sulfonic acid, trifluoroacetic acid or trifluoromethanesulfonic acid; a Lewis acid such as zinc chloride, tin tetrachloride, boron trichloride, boron trifluoride or boron tribromide; or an acidic ion-exchange resin, among which inorganic acids and organic acids (most preferably hydrochloric acid, acetic acid and trifluoroacetic acid) are preferred.
The base used for the reaction is not particularly restricted so long as it is a base ordinarily used for removal of the optionally substituted aralkyl group as the amino-protecting group, but it is preferably an alkali metal carbonate such as lithium carbonate, sodium carbonate or potassium carbonate; an alkali metal hydroxide such as lithium hydroxide, sodium hydroxide or potassium hydroxide; a metal alkoxide such as lithium methoxide, sodium methoxide, sodium ethoxide or potassium-t-butoxide; or an ammonia mixture such as aqueous ammonia or concentrated ammonia-methanol.
The inert solvent used for the first stage of the reaction is not particularly restricted so long as it is inert to the reaction, and as examples there may be mentioned aliphatic hydrocarbons such as hexane, heptane, ligroin and petroleum ether; aromatic hydrocarbons such as benzene, toluene and xylene; halogenated hydrocarbons such as chloroform, dichloromethane, 1,2-dichloroethane and carbon tetrachloride; esters such as methyl acetate, ethyl acetate, propyl acetate, butyl acetate and diethyl carbonate; ethers such as diethyl ether, diisopropyl ether, tetrahydrofuran, dioxane, dimethoxyethane and diethyleneglycol dimethyl ether; alcohols such as methanol, ethanol, n-propanol, isopropanol, n-butanol, isobutanol, t-butanol, isoamyl alcohol, diethylene glycol, glycerin, octanol, cyclohexanol and methyl cellosolve; amides such as formamide, dimethylformamide, dimethylacetamide and hexamethylphosphoric triamide; water; and mixtures of the aforementioned solvents, among which ethers, alcohols and water (most preferably dioxane, tetrahydrofuran, ethanol or water) are preferred.
The reaction temperature will differ depending on the starting compounds and the acid and solvent used, but will ordinarily be between -20°C and the boiling temperature (preferably between 0°C and 100°C).
The reaction time will differ depending on the starting compounds, the acid and inert solvent used and the reaction temperature, but will ordinarily be between 15 minutes and 48 hours (preferably between 30 minutes and 20 hours).

When the amino-protecting group is an optionally substituted alkenyloxycarbonyl group, usually the removal may be accomplished by treatment with an acid or base, under the same conditions as removal reaction when the amino-protecting group is an optionally substituted aliphatic acyl group, an optionally substituted aromatic acyl group, an optionally substituted alkoxycarbonyl group or a substituted methylene group which forms a Schiff base.
In the case of an allyloxycarbonyl group, it is particularly convenient to employ a method of removal using palladium and triphenylphosphine or nickel-tetracarbonyl, as the removal can be carried out with few side reactions.

When the amino-protecting group is an optionally substituted alkyl group, optionally substituted alkenyl group or optionally substituted sulfonyl group, usually the removal may be accomplished by treatment with an acid or base, under the same conditions as removal reaction when the amino-protecting group is an aliphatic acyl group, an aromatic acyl group, an alkoxycarbonyl group or a substituted methylene group which forms a Schiff base.

When the hydroxyl-protecting group is, for example, an optionally substituted silyl group such as trimethylsilyl, triethylsilyl, triisopropylsilyl, t-butyldimethylsilyl or t-butyldiphenylsilyl, it will usually be removed by treatment with a fluoride anion-generating compound such as tetrabutylammonium fluoride, hydrofluoric acid, hydrofluoric acid-pyridine or potassium fluoride, or with an inorganic acid such as hydrochloric acid, hydrobromic acid, sulfuric acid, perchloric acid or phosphoric acid, or an organic acid such as acetic acid, formic acid, oxalic acid, methanesulfonic acid, p-toluenesulfonic acid, camphor sulfonic acid, trifluoroacetic acid or trifluoromethanesulfonic acid.
For removal with fluoride anion, an organic acid such as formic acid, acetic acid or propionic acid may be added to accelerate the reaction.
The inert solvent used for the reaction is not particularly restricted so long as it is inert to the reaction, but it is preferably an ether such as diethyl ether, diisopropyl ether, tetrahydrofuran, dioxane, dimethoxyethane or diethyleneglycol dimethyl ether; a nitrile such as acetonitrile or isobutyronitrile; an organic acid such as acetic acid; water; or a mixture of these solvents.
The reaction temperature and the reaction time will differ depending on the starting compounds, catalyst and inert solvent used, but ordinarily the reaction temperature will be between 0°C and 100°C (preferably between 10°C and 50°C), and the reaction time will be 1 to 24 hours.

When the hydroxyl-protecting group is an optionally substituted aralkyl group or an optionally substituted aralkyloxycarbonyl group, a method of contact with a reducing agent in an inert solvent (preferably catalytic reduction at ordinary temperature in the presence of a catalyst) or a method of removal using an oxidizing agent is generally preferred.

The inert solvent used for removal by catalytic reduction is not particularly restricted so long as it does not participate in the reaction, and as examples there may be mentioned aliphatic hydrocarbons such as hexane, heptane, ligroin and petroleum ether; aromatic hydrocarbons such as toluene, benzene and xylene; esters such as ethyl acetate and propyl .acetate; ethers such as diethyl ether, diisopropyl ether, tetrahydrofuran, dioxane, dimethoxyethane and diethyleneglycol dimethyl ether; alcohols such as methanol, ethanol, n-propanol, isopropanol, n-butanol, isobutanol, t-butanol, isoamyl alcohol, diethylene glycol, glycerin, octanol, cyclohexanol and methyl cellosolve; amides such as formamide, dimethylformamide, dimethylacetamide, N-methyl-2-pyrrolidone and hexamethylphosphoric triamide; aliphatic acids such as formic acid and acetic acid; water; and mixtures of these solvents, among which alcohols (most preferably methanol and ethanol) are preferred.
There are no particular restrictions on the catalyst used for removal by catalytic reduction so long as it is one commonly used for removal of the hydroxyl-protecting group by catalytic reduction, and as examples there may be mentioned palladium-carbon, palladium black, Raney nickel, platinum oxide, platinum black, rhodium-aluminum oxide, triphenylphosphine-rhodium chloride or palladium-barium sulfate, among which palladium-carbon is preferred.
There are no particular restrictions on the pressure, but it will ordinarily be from I to 10 atmospheres.
The reaction temperature and the reaction time will differ depending on the starting compounds, catalyst and insert solvent, but usually the reaction temperature will be between 0°C and 100°C (preferably between 20°C and 70°C), and the reaction time will be between 5 minutes and 48 hours (preferably between 1 hour and 24 hours).

The inert solvent used for removal by oxidation is not particularly restricted so long as it does not participate in the reaction, but it is preferably a water-containing solvent, and there may be mentioned as examples ketones such as acetone; halogenated hydrocarbons such as methylene chloride, chloroform and carbon tetrachloride; nitriles such as acetonitrile; ethers such as diethyl ether, tetrahydrofuran and dioxane; amides such as dimethylformamide, dimethylacetamide and hexamethylphosphoric triamide; and sulfoxides such as dimethylsulfoxide.
The oxidizing agent used for this reaction is not particularly restricted so long as it is an oxidizing agent used for removal of the hydroxyl-protecting group, but it is preferably potassium persulfate, sodium persulfate, ammonium cerium nitrate (CAN) or 2,3-dichloro-5,6-dicyano-p-benzoquinone (DDQ).
The reaction temperature and the reaction time will differ depending on the starting compounds, oxidizing agent and inert solvent, but usually the reaction temperature will be between 0°C and 150°C, and the reaction time will be between 10 minutes and 24 hours.

The removal can also be accomplished by reaction with an alkali metal such as lithium metal or sodium metal in liquid ammonia or an alcohol such as methanol, ethanol, n-propanol, isopropanol, n-butanol, isobutanol, t-butanol, isoamyl alcohol, diethylene glycol, glycerin, octanol, cyclohexanol or methyl cellosolve, at a temperature of between -78°C and 0°C.

The removal can also be accomplished using aluminum chloride-sodium iodide or an alkylsilyl iodide such as trimethylsilyl iodide, in an inert solvent.
The inert solvent used in this reaction is not particularly restricted so long as it does not participate in the reaction, but it is preferably a halogenated hydrocarbon such as methylene chloride, chloroform or carbon tetrachloride; a nitrile such as acetonitrile; or a mixture of these solvents.
The reaction temperature and the reaction time will differ depending on the starting compounds and the inert solvent, but usually the reaction temperature will be between 0°C and 50°C, and the reaction time will be between 5 minutes and 72 hours.

When the hydroxyl-protecting group is an aliphatic acyl group, an aromatic acyl group or an optionally substituted alkoxycarbonyl group, it may be removed by treatment with a base in an inert solvent.
There are no particular restrictions on the base used for this reaction so long as it is a base ordinarily used for removal of the hydroxyl-protecting group, and for example, it may be an alkali metal carbonic acid salt such as lithium carbonate, sodium carbonate or potassium carbonate; an alkali hydrogencarbonate such as lithium hydrogencarbonate, sodium hydrogencarbonate or potassium hydrogencarbonate; an alkali metal hydroxide such as lithium hydroxide, sodium hydroxide or potassium hydroxide; a metal alkoxide such as lithium methoxide, sodium methoxide, sodium ethoxide or potassium-t-butoxide; or an ammonia mixture such as aqueous ammonia or concentrated ammonia-methanol, among which alkali metal hydroxides, metal alkoxides and ammonia mixtures (most preferably alkali metal hydroxides and metal alkoxides) are preferred.
The inert solvent used for this reaction is not particularly restricted so long as it is ordinarily used for hydrolysis reaction, but it is preferably an ether such as diethyl ether, diisopropyl ether, tetrahydrofuran, dioxane, dimethoxyethane or diethyleneglycol dimethyl ether; an alcohol such as methanol, ethanol, n-propanol, isopropanol, n-butanol, isobutanol, t-butanol, isoamyl alcohol, diethylene glycol, glycerin, octanol, cyclohexanol or methyl cellosolve; water; or a mixture of these solvents.
The reaction temperature and the reaction time will differ depending on the starting compounds and the base and inert solvent used, but in order to inhibit by-products, the reaction temperature will usually be between -20°C and 150°C, and the reaction time will usually be 1-10 hours.

When the hydroxyl-protecting group is optionally substituted alkoxymethyl, optionally substituted alkylthiomethyl, tetrahydropyranyl, tetrahydrothiopyranyl, tetrahydrofuranyl, tetrahydrothiofuranyl or optionally substituted ethyl such as 1-ethoxyethyl, it will usually be removed by treatment with an acid in an inert solvent.
There are no particular restrictions on the acid used for this reaction so long as it is an acid used for removal of the hydroxyl-protecting group, but usually compounds ordinarily used as Bronsted acids or Lewis acids may be mentioned, and preferred are Bronsted acids including hydrogen chloride; inorganic acids such as hydrochloric acid, sulfuric acid and nitric acid; and organic acids such as acetic acid, trifluoroacetic acid, methanesulfonic acid and p-toluenesulfonic acid; or Lewis acids such as boron trifluoride, while strong acid cation exchange resins such as DOWEX 50W may also be used.
There are no particular restrictions on the inert solvent used for this reaction so long as it is inert to the reaction, and for example, there may be mentioned aliphatic hydrocarbons such as hexane, heptane, ligroin and petroleum ether; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethyleneglycol dimethyl ether; alcohols such as methanol, ethanol, n-propanol, isopropanol. n-butanol, isobutanol, t-butanol, isoamyl alcohol, diethylene glycol, glycerin, octanol, cyclohexanol and methyl cellosolve; ketones such as acetone, methyl ethyl ketone, methyl isobutyl ketone, isophorone and cyclohexanone; water; and mixtures of these solvents, among which ethers and alcohols (most preferably tetrahydrofuran and methanol) are preferred.
The reaction temperature and the reaction time will differ depending on the starting compound and the acid and inert solvent used, but usually the reaction temperature will be between -10°C and 200°C (preferably between 0°C and 150°C), and the reaction time will be between 5 minutes and 48 hours (preferably between 30 minutes and 10 hours).

When the hydroxyl-protecting group is an optionally substituted alkenyloxycarbonyl group or optionally substituted sulfonyl group, usually the removal may be accomplished by treatment with a base, under the same conditions as removal reaction when the hydroxyl-protecting group is the aforementioned optionally substituted aliphatic acyl group, optionally substituted aromatic acyl group or optionally substituted alkoxycarbonyl group.
In the case of an allyloxycarbonyl group, it is particularly convenient to employ a method of removal using palladium and triphenylphosphine or bis(methyldiphenylphosphine)(1,5-cyclooctadiene)iridium (I) hexafluorophosphate, as the removal can be carried out with few side reactions.

When the carboxyl-protecting group is a lower alkyl group, a lower alkenyl group or a lower alkynyl group, or an optionally substituted silyl group, or when the compound has been converted to an ortho ester for the purpose of protection, a method of removal by treatment with an acid or base, or using an enzyme, is preferred.
There are no particular restrictions on the acid used for this reaction so long as it is used for removal of the carboxyl-protecting group, and for example, it may be hydrochloric acid, sulfuric acid, phosphoric acid or hydrobromic acid.
There are no particular restrictions on the base used for this reaction so long as it is used for removal of the carboxyl-protecting group, and for example, it may be an alkali metal carbonate such as sodium carbonate or potassium carbonate; an alkali metal hydroxide such as sodium hydroxide or potassium hydroxide; or concentrated ammonia-methanol solution, among which sodium hydroxide is preferred.
Isomerization may occur with hydrolysis using a base.
There are no particular restrictions on the enzyme used for the reaction so long as it is an enzyme used for removal of the carboxyl-protecting group, and for example, it may be a lipase or an esterase.
The solvent used for this reaction may be, for example, water, an alcohol such as methanol, ethanol or n-propanol; an ether such as tetrahydrofuran or dioxane; or a mixture of any of these solvents with water, among which an alcohol (most preferably methanol) is preferred.
The reaction temperature and the reaction time will differ depending on the starting compounds, the solvent and the reagents used and are not particularly restricted, but in order to inhibit by-products, the reaction temperature will usually be between 0°C and 220°C, and the reaction time will usually be between 30 minutes and 10 hours.

When the carboxyl-protecting group is an optionally substituted aralkyl group or halogeno lower alkyl group, it will usually be removed by reduction in a solvent.
The reduction method is preferably a method by chemical reduction with zinc-acetic acid when the carboxyl-protecting group is a halogeno lower alkyl group, and when it is an optionally substituted aralkyl group, the method may be one of catalytic reduction using a catalyst such as palladium-carbon or platinum, or a method of chemical reduction using an alkali metal sulfide such as potassium sulfide or sodium sulfide.
The solvent used is not particularly restricted so long as it does not participate in the reaction, but there are preferred alcohols such as methanol and ethanol; ethers such as tetrahydrofuran and dioxane; aliphatic acids such as acetic acid; and mixtures of these solvents with water.
The reaction temperature and the reaction time will differ depending on the starting compounds, the solvent and the reduction method, but usually the reaction temperature will be between 0°C and approximately room temperature and the reaction time will be between 5 minutes and 12 hours.

If the carbonyl group has been protected by conversion to a cyclic or acyclic ketal formed using, for example, an alcohol such as methanol, isopropanol or diethylene glycol or a thiol such as methanethiol, ethanethiol or propanedithiol, an acid may be used for reconversion to a carbonyl group.
The acid used for this reaction is not particularly restricted so long as it is an acid ordinarily used for reconversion to a carbonyl group from a cyclic or acyclic ketal formed for the purpose of protecting the carbonyl group, and for example, it may be a Bronsted acid, e.g. an inorganic acid such as hydrochloric acid, hydrobromic acid, sulfuric acid, perchloric acid or phosphoric acid, or an organic acid such as acetic acid, formic acid, oxalic acid, methanesulfonic acid, p-toluenesulfonic acid, camphor sulfonic acid, trifluoroacetic acid or trifluoromethanesulfonic acid; a Lewis acid such as zinc chloride, tin tetrachloride, boron trichloride, boron trifluoride or boron tribromide; or an acidic ion exchange resin, among which inorganic acids and organic acids (most preferably hydrochloric acid and p-toluenesulfonic acid) are preferred.
There are no particular restrictions on the inert solvent used for the first stage of the reaction so long as it is inert to the reaction, and as examples there may be mentioned aliphatic hydrocarbons such as hexane, heptane, ligroin and petroleum ether; aromatic hydrocarbons such as benzene, toluene and xylene; halogenated hydrocarbons such as chloroform, dichloromethane, 1,2-dichloroethane and carbon tetrachloride; esters such as methyl acetate, ethyl acetate, propyl acetate, butyl acetate and diethyl carbonate; ethers such as diethyl ether, diisopropyl ether, tetrahydrofuran, dioxane, dimethoxyethane and diethyleneglycol dimethyl ether; alcohols such as methanol, ethanol, n-propanol, isopropanol, n-butanol, isobutanol, t-butanol, isoamyl alcohol, diethylene glycol, glycerin, octanol, cyclohexanol and methyl cellosolve; amides such as formamide, dimethylformamide, dimethylacetamide and hexamethylphosphoric triamide; water; and mixtures of these solvents, among which ethers, alcohols and water (most preferably acetone, tetrahydrofuran and water) are preferred.
The reaction temperature will differ depending on the starting compounds and the acid and solvent used, but usually it will be between -20°C and the boiling point (preferably between 0°C and 100°C).
The reaction time will differ depending on the starting compounds, the acid and inert solvent used and the reaction temperature, but usually it will be between 5 minutes and 48 hours, (preferably between 10 minutes and 24 hours).
In the case of a cyclic or acyclic ketal formed using a thiol, it is particularly convenient to employ a method of removal using a substance such as Raney nickel or silver nitrate.

In the case of conversion to a cyclic ketal using, for example, formalin or acetone as protection of a diol, an acid may be used for reconversion to the diol.
The acid used for this reaction is not particularly restricted so long as it is an acid ordinarily used for reconversion to a diol from a cyclic or acyclic ketal formed for the purpose of protecting the diol, and for example, it may be a Bronsted acid, e.g. an inorganic acid such as hydrochloric acid, hydrobromic acid, sulfuric acid, perchloric acid or phosphoric acid, or an organic acid such as acetic acid, formic acid, oxalic acid, methanesulfonic acid, p-toluenesulfonic acid, camphor sulfonic acid, trifluoroacetic acid or trifluoromethanesulfonic acid; a Lewis acid such as zinc chloride, tin tetrachloride, boron trichloride, boron trifluoride or boron tribromide; or an acidic ion exchange resin, among which inorganic acids and organic acids (most preferably hydrochloric acid and p-toluenesulfonic acid) are preferred.
There are no particular restrictions on the inert solvent used for the first stage of the reaction so long as it is inert to the reaction, and as examples there may be mentioned aliphatic hydrocarbons such as hexane, heptane, ligroin and petroleum ether; aromatic hydrocarbons such as benzene, toluene and xylene; halogenated hydrocarbons such as chloroform, dichloromethane, 1,2-dichloroethane and carbon tetrachloride; esters such as methyl acetate, ethyl acetate, propyl acetate, butyl acetate and diethyl carbonate; ethers such as diethyl ether, diisopropyl ether, tetrahydrofuran, dioxane, dimethoxyethane and diethyleneglycol dimethyl ether; alcohols such as methanol, ethanol, n-propanol, isopropanol, n-butanol, isobutanol, t-butanol, isoamyl alcohol, diethylene glycol, glycerin, octanol, cyclohexanol and methyl cellosolve; amides such as formamide, dimethylformamide, dimethylacetamide and hexamethylphosphoric triamide; water; and mixtures of these solvents, among which ethers, alcohols and water (most preferably acetone, tetrahydrofuran and water) are preferred.
The reaction temperature will differ depending on the starting compounds and the acid and solvent used, but usually it will be between -20°C and the boiling point (preferably between 0°C and 100°C).
The reaction time will differ depending on the starting compounds, the acid and inert solvent used and the reaction temperature, but usually it will be between 5 minutes and 48 hours (preferably between 10 minutes and 24 hours).

Removal of the protecting groups for amino, hydroxyl, carbonyl or carboxyl groups, or a diol, may be carried out in an appropriate order.

After completion of the reactions of each of the methods and steps described above, the target compound of each step may be recovered from the reaction mixture according to conventional procedures.

For example, when the entire reaction mixture is a liquid, it may be returned to room temperature if necessary, or cooled on ice, then allowed to neutralization of an acid, an alkali, an oxidizing agent or a reducing agent if necessary, and then water and an organic solvent such as ethyl acetate which is immiscible with water and which does not react with the target compound may be added, and the layer containing the target compound is separated. Next, there may be added a solvent which is immiscible with the resultant layer and which does not react with the target compound, and the layer containing the target compound may be washed and separated. If the layer is an organic layer, it may be dried using a desiccant such as anhydrous magnesium sulfate or anhydrous sodium sulfate, the solvent may be distilled off to recover the target compound. If the layer is an aqueous layer, it may be electrically desalted and then lyophilized to recover the target compound.
When the entire reaction mixture is a liquid, in some cases, the substances other than the target compound (for example, solvents, reagents, etc.) may be simply distilled off at atmospheric pressure or under reduced pressure to recover the target compound.
When the target compound alone precipitates as a solid, or when the entire reaction mixture is a liquid and the target compound alone precipitates as a solid during the recovery procedure, the target compound may be first filtered by a filtration method and the filtered target compound washed with a suitable organic or inorganic solvent and dried to allow treatment of the mother liquor in the same manner as when the entire reaction mixture is a liquid, in order to recover the target compound.
When only the reagent or catalyst is present in solid form, or when the entire reaction mixture is a liquid and the reagent or catalyst alone precipitates as a solid during the recovery procedure, with the target compound dissolved in the solution, the reagent or catalyst may be first filtered by a filtration method and the filtered reagent or catalyst washed with a suitable organic or inorganic solvent, and then the obtained wash liquids combined as the mother liquor and the obtained mixture treated in the same manner as when the entire reaction mixture is a liquid, in order to recover the target compound.

Particularly when substances other than the target compound in the reaction mixture do not inhibit the reaction of the subsequent step, the reaction mixture may be used directly for the subsequent step without isolation of the target compound.

The purity of the target compound recovered by the method described above may be improved by appropriately employing a recrystallization method, chromatography method or distillation method.
When the recovered target compound is a solid, it will usually be possible to improve the purity of the target compound by recrystallization. For recrystallization, a single solvent which do not react with the target compound or a mixture of multiple solvents which do not react with the target compound may be used. Specifically, the target compound is first dissolved in the single or multiple solvents which do not react therewith, either at room temperature or with heating. The resulting solution is either cooled on ice or allowed to stand at room temperature to crystallization of the target compound from the solution.
When the recovered target compound is a liquid or a solid, the purity of the target compound may be improved by any of various chromatography methods. A weak acid silica gel such as Silica Gel 60 (340-400 mesh) by Merck Co. or BW-300 (300 mesh) by Fuji Silysia Chemical Ltd. may be used in most cases. When the target compound is basic and adsorption is too strong on the aforementioned silica gels, Propylamine Coating Silica Gel (200-300 mesh) by Fuji Silysia Chemical Ltd. or the like may be used. When the target compound is dipolar or must be eluted with a polar solvent such as methanol, NAM-200H or NAM-300H by Nam Research Co. may be used. These silica gels may be used for elution of the target compound with a single solvent or multiple solvents which do not react with the target compound, followed by distilling off of the solvent, to yield the target compound with improved purity.
When the recovered target compound is a liquid, its purity may be improved by a distillation method. For distillation, the target compound is subjected to reduced pressure at room temperature or with heating to distill off the target compound.

Representative examples of production methods for compounds (1) and (100) according to the present invention have been described above, but the starting compounds and reagents used for production of the compounds of the invention may also form salts or solvates (hydrates, etc.), which will differ depending on the starting materials and solvents used, and are not particularly restricted so long as they do not inhibit the reaction. The solvents used will also differ depending on the starting materials and reagents, but of course they are not particularly restricted so long as they dissolve the starting materials to some extent and do not inhibit the reaction.

When compound (1) or (100) of the invention is obtained in the free form, a conventional procedure may be carried out to convert it to a salt or hydrate which compound (1) or (100) may form.
When compound (1) or (100) of the invention is obtained as a salt of compound (1) or (100), or a hydrate of compound (1) or (100), it may be converted to the free form of compound (1) or (100) according to a conventional procedure.

Also, the various isomers obtained for compound (1) or (100) according to the invention (for example, geometric isomers, optical isomers based on asymmetric carbons, rotational isomers, stereoisomers and tautomers, etc.) may be purified and isolated using ordinary separation means such as recrystallization, diastereomer salt methods, enzyme fractionation methods, and various chromatography (for example, thin-layer chromatography, column chromatography, gas chromatography and the like).

The starting compounds for Method A, Method B, Method C, Method D, Method E, Method F, Method G, Method H, Method K, Method M, Method N, Method P, Method Q, Method R, Method S, Method T, Method U and Method V described above may be commercially available compounds, or they may be easily produced from commercially available compounds by methods which are well known in the field. They may also be produced by the following methods.

(Production method for compound (2A)) (Method 1-1)

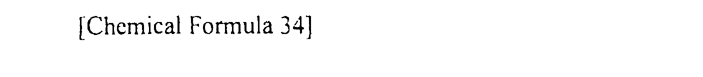

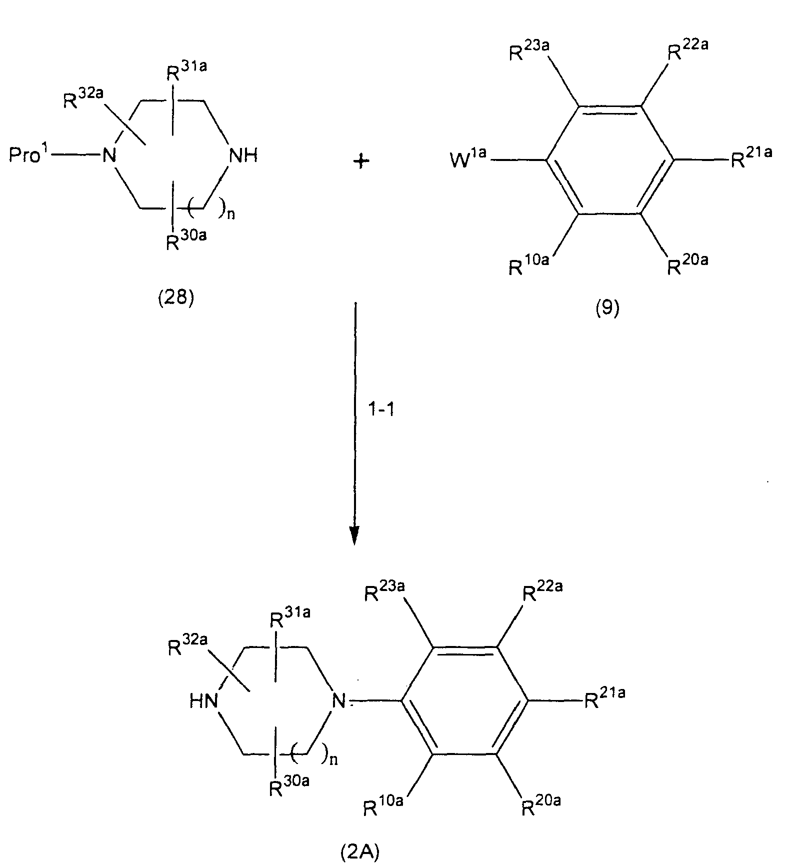

This method is a method of producing compound (2A) (the compound (2)
wherein X¹ is nitrogen) by reacting compound (28) with compound (9) (amination or amidation) in an inert solvent, in the presence of a palladium(0) catalyst or copper catalyst, in the presence or in the absence of a base, in the presence or in the absence of an additive, under or not under an inert gas atmosphere, and then removing the protecting group Pro¹.
In this scheme, n, W^{1a}, R^{10a}, R^{20a}, R^{21a}, R^{22a}, R^{23a}, R^{30a}, R^{31a} and R^{32a} have the same definitions as above. Also, Pro¹ is an amino-protecting group, and for example, it may be an optionally substituted silyl group such as trimethylsilyl, triethylsilyl or t-butyldiphenylsilyl, an optionally substituted aliphatic acyl group such as formyl or acetyl, an optionally substituted aromatic acyl group such as benzoyl, an optionally substituted alkoxycarbonyl group such as ethoxycarbonyl or t-butoxycarbonyl, a substituted methylene group which forms a Schiff base, an optionally substituted aralkyl group such as benzyl, 4-methoxybenzyl or 4-nitrobenzyl, an optionally substituted aralkyloxycarbonyl group such as benzyloxycarbonyl, 4-methoxybenzyloxycarbonyl or 4-nitrobenzyloxycarbonyl, an optionally substituted alkenyloxycarbonyl group such as vinyloxycarbonyl or allyloxycarbonyl, an optionally substituted alkyl group, an optionally substituted alkenyl group or an optionally substituted sulfonyl group, preferably a lower alkoxycarbonyl group such as methoxycarbonyl, ethoxycarbonyl, t-butoxycarbonyl or isobutoxycarbonyl, a lower alkoxycarbonyl group substituted with halogen or tri lower alkyl silyl, such as 2,2,2-trichloroethoxycarbonyl or 2-trimethylsilylethoxycarbonyl, an alkenyloxycarbonyl group such as vinyloxycarbonyl or allyloxycarbonyl, an optionally substituted aralkyloxycarbonyl group such as benzyloxycarbonyl, 4-methoxybenzyloxycarbonyl, 3,4-dimethoxybenzyloxycarbonyl, 2-nitrobenzyloxycarbonyl or 4-nitrobenzyloxycarbonyl, or an optionally substituted aralkyl group such as benzyl, 4-methoxybenzyl, 3,4-dimethoxybenzyl, 2-nitrobenzyl or 4-nitrobenzyl, and more preferably benzyl, 4-methoxybenzyl, 4-nitrobenzyl, ethoxycarbonyl, t-butoxycarbonyl or benzyloxycarbonyl.
The method may be carried out in a manner similar to Method F above.

(Production method for compound (2A)) (Method 1-2)

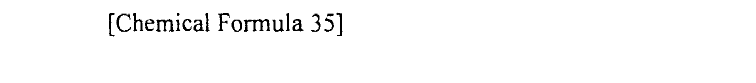

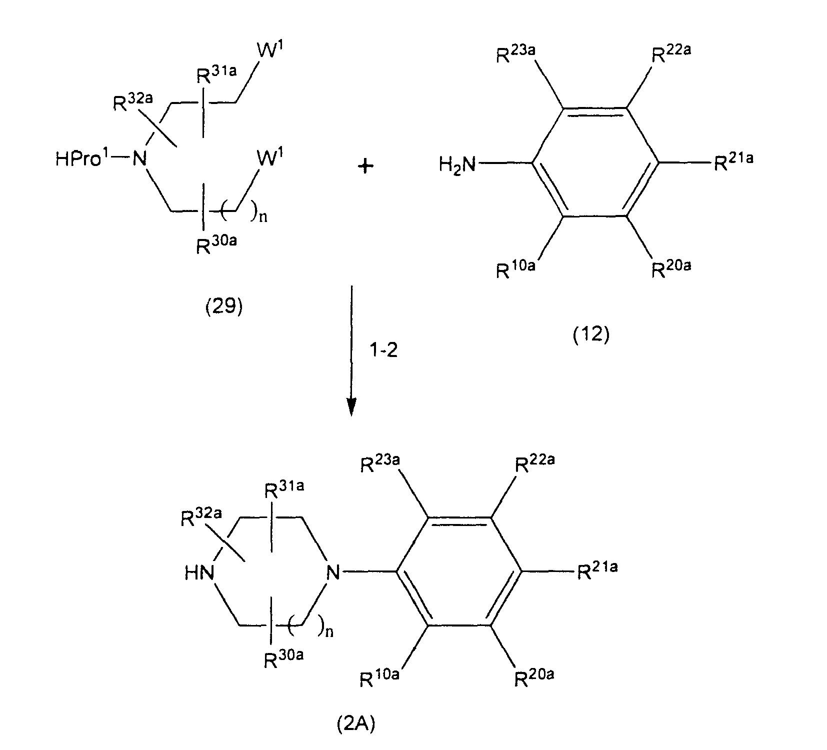

This method is a method of producing compound (2A) (the compound (2)
wherein X¹ is nitrogen) by reacting compound (29) with compound (12) in an inert solvent or without a solvent, under or not under an inert gas atmosphere, in the presence or in the absence of a base, in the presence or in the absence of an additive to yield compound (2A), and afterwards removing the protecting group Pro¹.
In this scheme, n, W¹, R^{10a}, R^{20a}, R^{21a}, R^{22a}, R^{23a}, R^{30a}, R^{31a} and R^{32a} have the same definitions as above.
HPro¹ is hydrogen or has the same definition as Pro¹ above.
This method may be carried out in a manner similar to Method H above.

(Production method for compound (2C)) (Method 1-3)

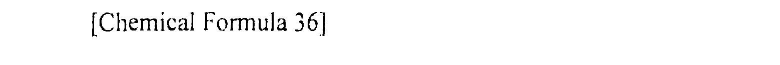

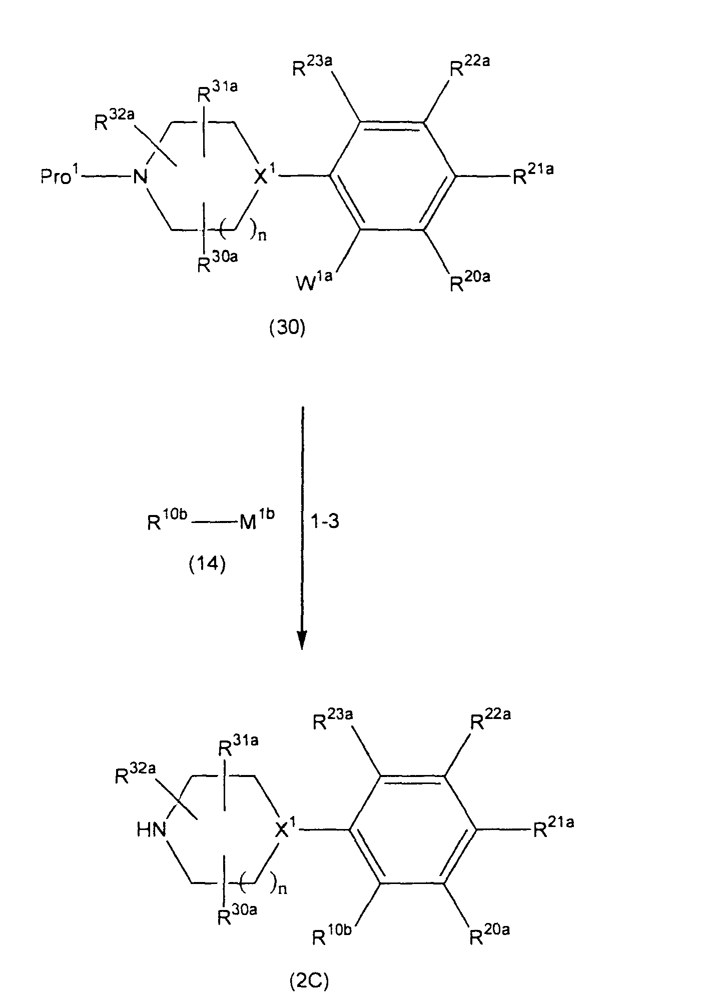

This method is a method of producing compound (2C) (the compound (2)
wherein R^{10a} is R^{10b}) by reacting compound (30) with compound (14) in an inert solvent, in the presence of a palladium(0) catalyst, under or not under an inert gas atmosphere, in the presence or in the absence of a base, in the presence or in the absence of an additive, and then removing the protecting group Pro¹.
In this scheme, X¹, n, W^{1a}, M^{1b}, Pro¹, R^{10b}, R^{20a}, R^{21a}, R^{22a}, R^{23a}, R^{30a}, R^{31a} and R^{32a} have the same definitions as above.
This method may be carried out in a manner similar to Method K described above.

(Production method for compound (2C)) (Method 1-4)

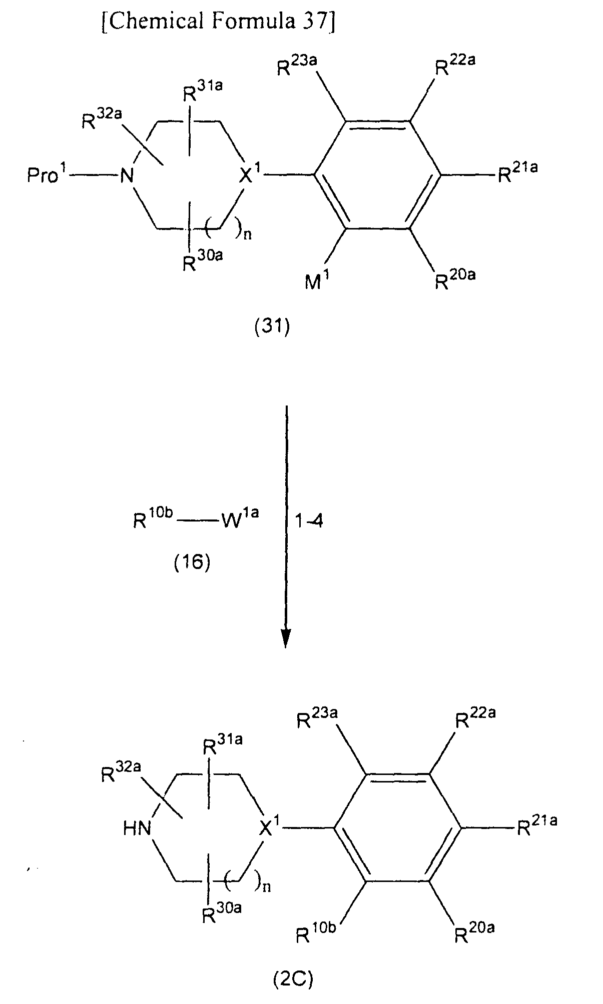

This method is a method of producing compound (2c) (the compound (2)
wherein R^{10a} is R^{10b}) by reacting compound (31) with compound (16) in an inert solvent, in the presence of a palladium(0) catalyst, under or not under an inert gas atmosphere, in the presence or in the absence of a base, in the presence or in the absence of an additive, and then removing the protecting group Pro¹.
In this scheme, X¹, n, W^{1a}, M¹, Pro¹, R^{10b}, R^{20a}, R^{21a}, R^{22a}, R^{23a}, R^{30a}, R^{31a} and R^{32a} have the same definitions as above.
This method may be carried out in a manner similar to Method K described above.

(Production method for compound (2A)) (Method 1-5)

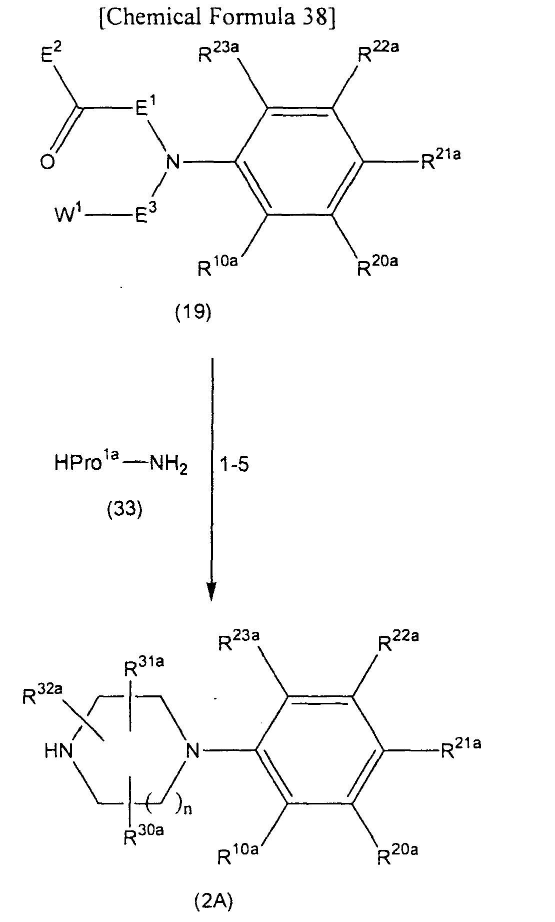

This method is a method of producing compound (2A) (the compound (2)
wherein X¹ is nitrogen) by reacting compound (19) with compound (33) in an inert solvent, in the presence of a reducing agent, in the presence or in the absence of an acid, in the presence or in the absence of an additive, to yield compound (2A), and afterward removing the protecting group Pro^{1a} by the method described above.
In this scheme, n, W¹, E¹, E², E³, R^{10a}, R^{20a}, R^{21a}, R^{22a}, R^{23a}, R^{30a}, R^{31a} and R^{32a} have the same definitions as above.
Also, HPro^{1a} is hydrogen or a group represented by Pro^{1a} below.
Group Pro^{1a} is an optionally substituted aralkyl group such as benzyl, 4-methoxybenzyl, 3,4-dimethoxybenzyl, 2-nitrobenzyl or 4-nitrobenzyl, and is preferably benzyl.
This method may be carried out in a manner similar to Method C described above.

(Production method for compound (2A)) (Method 1-6)

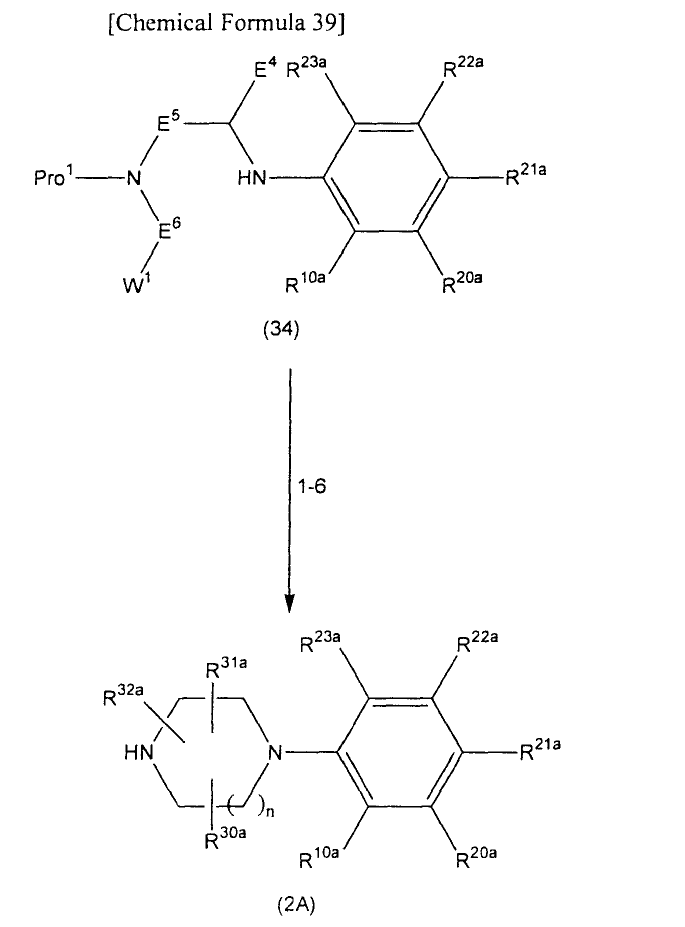

This method is a method of producing compound (2A) (the compound (2)
wherein X¹ is nitrogen) by reacting a base with compound (34) in an inert solvent, and then removing the protecting group Pro¹ by the method described above.
In this scheme, n, W¹, Pro¹, E⁴, E⁵, E⁶, R^{10a}, R^{20a}, R^{21a}, R^{22a}, R^{23a}, R^{30a}, R^{31a} and R^{32a} have the same definitions as above.
This method may be carried out in a manner similar to Method A described above.

(Production method for compounds (2B) and (200)) (Method 1-7)

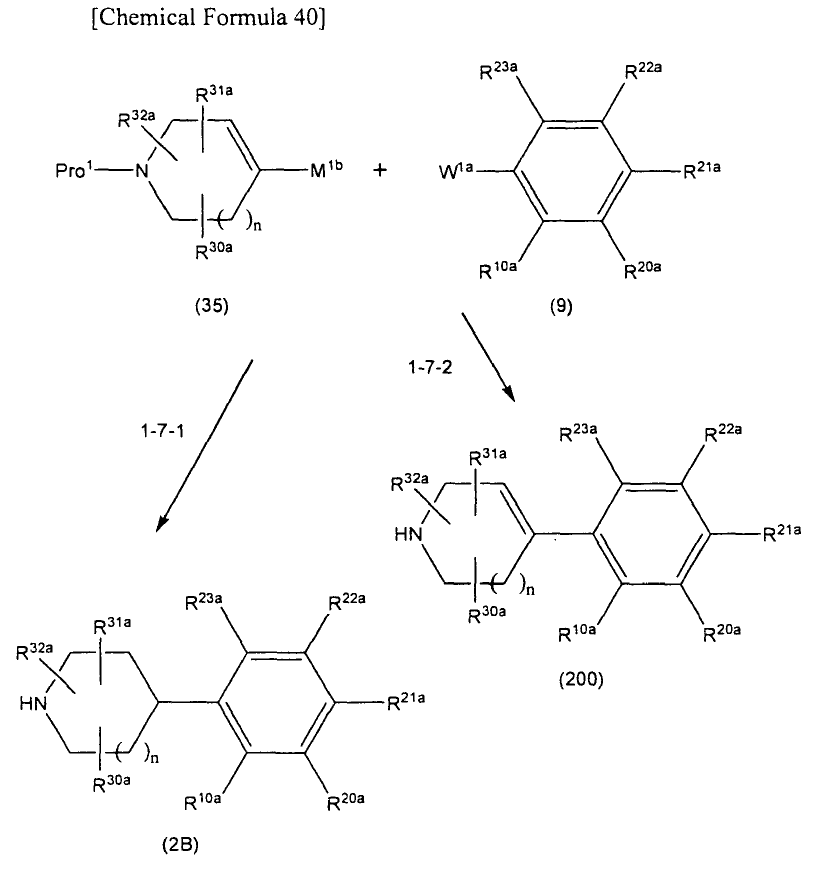

This method is a method of producing compound (2B) (the compound (2)
wherein X¹ is CH) by reacting compound (35) with compound (9) in an inert solvent, in the presence of a palladium(0) catalyst, subjecting it to hydrogenation reaction, and then removing the protecting group Pro¹ (Method 1-7-1), or a method of producing compound (200) by reacting compound (35) with compound (9) in the same manner, then removing the protecting group Pro¹ (Method 1-7-2).
In this scheme. n. W^{1a}, M¹, Pro¹, R^{10a}, R^{20a}, R^{21a}, R^{22a}, R^{23a}, R^{30a}, R^{31a} and R^{32a} have the same definitions as above.
This method may be carried out in a manner similar to Method K described above and the hydrogenation reaction in Method A above.

(Production method for compounds (2B) and (200C)) (Method 1 -8)

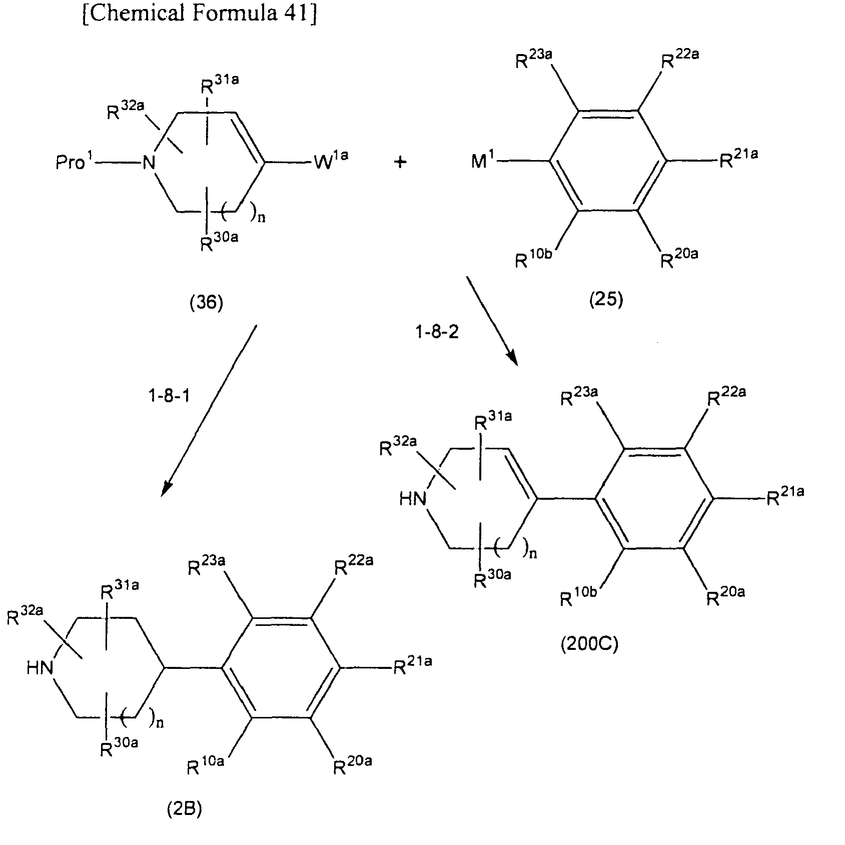

This method is a method of producing compound (2B) (the compound (2)
wherein X¹ is CH) by reacting compound (36) with compound (25) in an inert solvent, in the presence of a palladium(0) catalyst, subjecting it to hydrogenation reaction, and then removing the protecting group Pro¹ (Method 1-8-1), or a method of producing compound (200C) (the compound (200) wherein R^{10a} is R^{10b}) by reacting compound (36) with compound (25) in the same manner, then removing the protecting group Pro¹ (Method 1-8-2).
In this scheme, n, W^{1a}, M¹, Pro¹, R^{10a}, R^{10b}, R^{20a}, R^{21a}, R^{22a}, R^{23a}, R^{30a}, R^{31a} and R^{32a} have the same definitions as above.
This method may be carried out in a manner similar to Method K described above and the hydrogenation reaction in Method A above.

(Production method for compounds (2B) and (200C)) (Method 1-9)

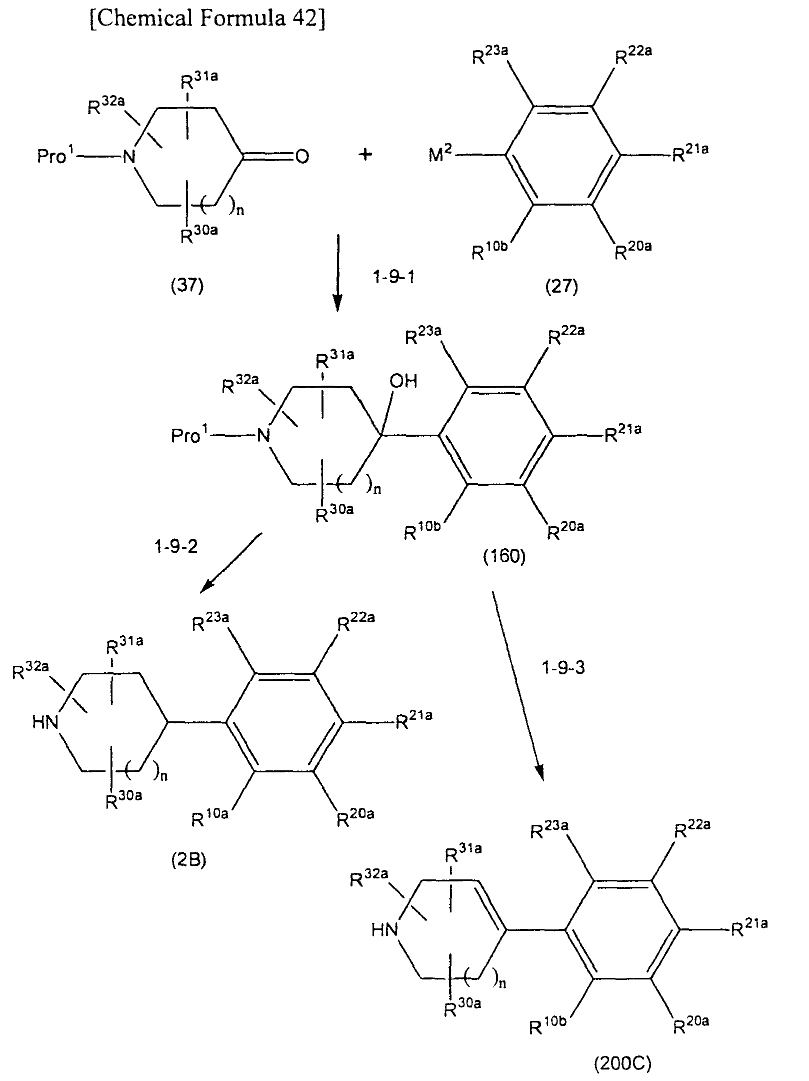

This method is a method of producing compound (2B) (the compound (2)
wherein X¹ is CH) by reacting compound (37) with compound (27) (i.e., a lithium reagent or Grignard reagent) in an inert solvent, in the presence of an inert gas to yield an adduct (160) (Step 1-9-1), and then reducing hydroxyl at the benzyl position of the resultant adduct (160) (Step 1-9-2), and removing the protecting group Pro¹, or a method of producing compound (200C) (the compound (200)
wherein R^{10a} is R^{10b}) by reacting in the same manner to yield an adduct (160) (Step 1-9-1), then dehydrating hydroxyl at the benzyl position of the resultant adduct (160) in the presence of or in the absence of acid (Step 1-9-3), and further removing the protecting group Pro¹.
In this scheme, n, M², Pro¹, R^{10a}, R^{10b}, R^{20a}, R^{21a}, R^{22a}, R^{23a}, R^{30a}, R^{31a} and R^{32a} have the same definitions as above.
This method may be carried out in a manner similar to Method U described above.

(Production method for compound (19)) (Method 2)

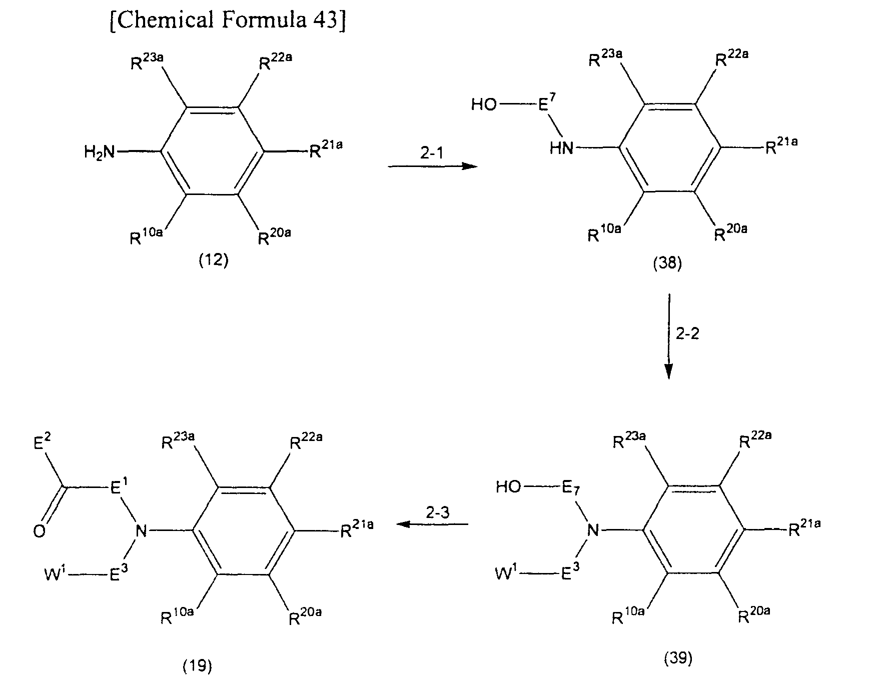

This method is a method of producing compound (19) by reacting an N-alkylating agent with compound (12) in an inert solvent to yield compound (38) (Step 2-1), and then reacting an N-alkylating agent or N-carbonylating agent with compound (38) to yield compound (39) (Step 2-2), and reacting an oxidizing agent with compound (39) in the presence or in the absence of an additive (Step 2-3).
In this scheme, W¹, E¹, E², E³, R^{10a}, R^{20a}, R^{21a}, R^{22a} and R^{23a} have the same definitions as above. Also, E⁷ is a group suitable for obtaining the desired group of the formula E²(CO)E¹-.
Step 2-1 and Step 2-2 may be carried out in a manner similar to Method A described above.

### (Step 2-3)

There are no particular restrictions on the solvent used so long as it dissolves the starting compound to some extent and does not inhibit the reaction of this step, and specifically there may be mentioned halogenated hydrocarbons such as chloroform, dichloromethane, 1,2-dichloroethane and carbon tetrachloride, and nitriles such as acetonitrile and isobutyronitrile, among which halogenated hydrocarbons (particularly dichloromethane) are preferred.
There are no particular restrictions on the oxidizing agent used so long as it can yield the target compound and does not produce any unseparable by-products, and specifically there may be mentioned chromic acids such as pyridinium chlorochromate (PCC) and pyridinium dichromate (PDC), Dess-Martin reagent (Dess D. B., Martin J. C., J. Am. Chem. Soc. (1991), 113, 7277), or catalytic amounts of dimethylsulfoxide oxidizing agents such as tetrapropylammonium perruthenate(VII) (TPAP; Ley S. V. et al., Synthesis (1994), 639) and dimethylsulfoxide-oxalyl chloride (Swern oxidizing agent; D. Swern et al., Synthesis (1981), 165), in the presence ofN-methylmorpholine-N-oxide (NMO) as an auxiliary oxidizing agent, among which dimethylsulfoxide-oxalyl chloride (Swern oxidizing agent) is preferred.
There are no particular restrictions on the additive used so long as it can yield the target compound and does not produce any unseparable by-products, and specifically there may be mentioned Celite and molecular sieve, among which molecular sieve is preferred.
The reaction temperature will differ depending on the starting materials, solvent and reagents, but will usually be between -80°C and 60°C, and is preferably between -80°C and 40°C.
The reaction time will differ depending on the starting materials, solvent, reagents and reaction temperature, but will usually be 0.1 to 100 hours, and is preferably 1 to 12 hours.
Compound (38), (39) or (19) may be isolated or purified from the reaction mixtures obtained above, by the methods described above.

(Production method for compounds (21) and (34)) (Method 3)

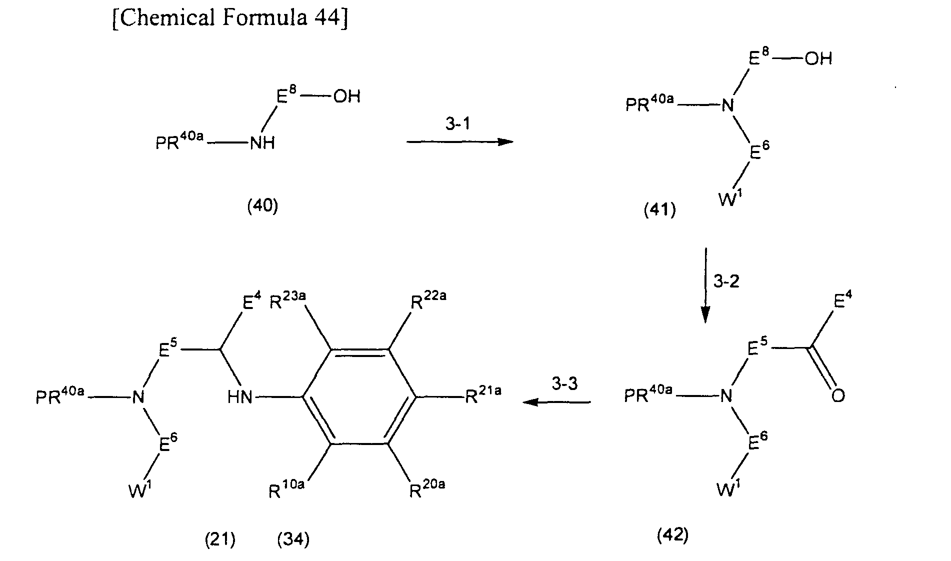

This method is a method of producing compound (21) or (34) by reacting an N-alkylating agent or N-carbonylating agent with compound (40) in an inert solvent to yield compound (41) (Step 3-1), and then oxidizing compound (41) to yield compound (42) (Step 3-2), and reacting compound (42) with compound (12) (Step 3-3).
In this scheme, W¹, E⁴, E⁵, E⁶, R^{10a}, R^{20a}, R^{21a}, R^{22a} and R^{23a} have the same definitions as above. Also, E⁸ is a group suitable for obtaining the desired group of the formula E⁴(CO)E⁵-. PR^{40a} has the same definition as R^{40a} above, or is an amino-protecting group (preferably t-butoxycarbonyl or benzyl).
Step 3-1 in this method may be carried out in a manner similar to Method A above, Step 3-2 may be carried out in a manner similar to Method 2 above (Step 2-3), and Step 3-3 may be carried out in a manner similar to Method C above.

(Production method for compounds (24A), (36A), (22), (35), (24B) and (36B)) (Method 4)

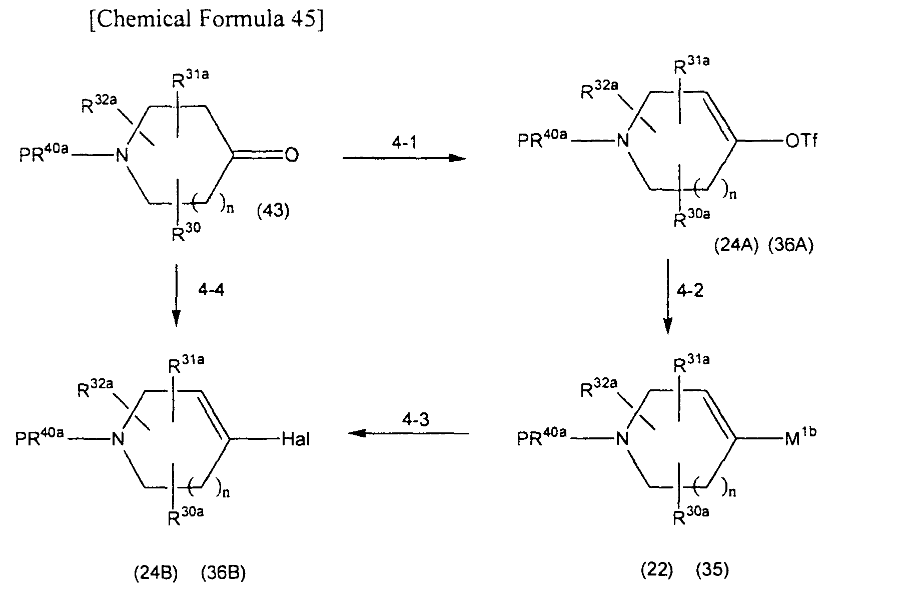

This method is a method of introducing a trifluoromethanesulfonyl group into compound (43) in an inert solvent under an inert gas atmosphere to yield compound (24A) or (36A) (Step 4-1), and then reacting compound (24A) or (36A) with boron metal reagent or tin metal reagent in the presence of a palladium(0) catalyst to yield compound (22) or (35) (Step 4-2), and reacting compound (22) or (35) with a halogenating reagent, in the presence or in the absence of a base, to yield compound (24B) or (36B) (Step 4-3). Compound (43) may also be directly reacted with a halogenating agent to produce compound (24B) or (36B) (Step 4-4).
In this scheme, n, R^{30a}, R^{31a}, R^{32a} and PR^{40a} have the same definitions as above.
Also, Hal represents chlorine, bromine or iodine.
M^{1b} is a group of the formula B(OE^{10c})₂ or Sn(E^{10b})₃ (wherein E^{10c} represents C1-6 alkyl or the two of E^{10c} bond together to form C2-3 alkylene optionally substituted with methyl, and E^{10b} represents C1-6 alkyl).
Tf is trifluoromethanesulfonyl.

### (Step 4-1)

There are no particular restrictions on the solvent used so long as it dissolves the starting compound to some extent and does not inhibit the reaction of this step, and specifically there may be mentioned ethers such as diethyl ether, tetrahydrofuran and dioxane, among which tetrahydrofuran is preferred.
This step is preferably carried out under a dried inert gas atmosphere. The inert gas is preferably argon or nitrogen.
There are no particular restrictions on the base used so long as it can yield the target compound and does not produce any unseparable by-products, and specifically there may be mentioned alkali metal amides such as lithium bis(trimethylsilyl)amide, sodium bis(trimethylsilyl)amide and lithium diisopropylamide, among which lithium bis(trimethylsilyl)amide or lithium diisopropylamide is preferred.
There are no particular restrictions on the trifluoromethanesulfonylating reagent used so long as it can yield the target compound and does not produce any unseparable by-products, but it is preferably N-phenyl bis(trifluoromethanesulfonimide).
The reaction temperature for enolation will differ depending on the starting materials, solvent and reagents, but will usually be between -100 and 20°C, and is preferably between -80 and -30°C.
The reaction temperature for conversion to a leaving group will differ depending on the starting materials, solvent and reagents, but will usually be between -100°C and 50°C, and is preferably between -80°C and 30°C.
The reaction time for enolation will differ depending on the starting materials, solvent, reagents and reaction temperature, but will usually be 0.1 to 5 hours, and is preferably 0.1 to 3 hours.
The reaction time for conversion to a leaving group will differ depending on the starting materials, solvent, reagents and reaction temperature, but will usually be 0.1 to 24 hours, and is preferably 0.5 to 12 hours.
In addition to the method described above, compound (44) may be produced by reacting a ketone compound (43) with trifluoromethanesulfonic anhydride in an inert solvent such as dichloromethane, in the presence of an organic base such as 2,6-di-t-butyl-4-methylpyridine, as described in David Crich ct al., Synthesis (2001), 2, 323, for example.

### (Step 4-2)

### (Production method for compounds (22) and (35) as boronate derivatives)

There are no particular restrictions on the solvent used so long as it dissolves the starting compound to some extent and does not inhibit the reaction of this step, and specifically there may be mentioned amides such as formamide, dimethylformamide, dimethylacetamide and hexamethylphosphoric triamide, sulfoxides such as dimethylsulfoxide and sulfolane, ethers such as diethyl ether, diisopropyl ether, tetrahydrofuran, dioxane, dimethoxyethane and diethyleneglycol dimethyl ether, and aromatic hydrocarbons such as benzene, toluene and xylene, among which dimethylsulfoxide and dioxane are preferred.
There are no particular restrictions on the metal catalyst used so long as it can yield the target compound and does not produce any unseparable by-products, and specifically there may be mentioned divalent palladium compounds such as [1,1'-bis(diphenylphosphino)ferrocene]dichloropalladium(II), and 0-valent palladium compounds such as tetrakis(triphenylphosphine)palladium, among which [1,1'-bis(diphenylphosphino)ferrocene]dichloropalladium(II) is preferred.
As bases to be used there may be mentioned potassium phenoxide, triethylamine, potassium phosphate, potassium carbonate and potassium acetate, among which potassium acetate is preferred.
The catalyst used may be triphenylarsine.
The reaction temperature will differ depending on the starting materials, solvent and reagents, but will usually be between 50°C and 80°C.
The reaction time will differ depending on the starting materials, solvent, reagents and reaction temperature, but will usually be 1 to 6 hours, and is preferably 2 to 3 hours.

### (Production method for compounds (22) and (35) as tin derivatives)

There are no particular restrictions on the solvent used so long as it dissolves the starting compound to some extent and does not inhibit the reaction of this step, and specifically there may be mentioned amides such as formamide, dimethylformamide, dimethylacetamide and hexamethylphosphoric triamide, and ethers such as diethyl ether, diisopropyl ether, tetrahydrofuran, dioxane, dimethoxyethane and diethyleneglycol dimethyl ether, among which ethers (particularly tetrahydrofuran) are preferred.
There are no particular restrictions on the metal catalyst used so long as it can yield the target compound and does not produce any unseparable by-products, and specifically there may be mentioned 0-valent palladium compounds such as tetrakis(triphenylphosphine)palladium(0) and tris(dibenzylideneacetone)dipalladium(0).
As tin reagents to be used there may be mentioned hexamethylditin(IV), hexabutylditin(IV) and hexaphenylditin(IV), among which hexamethylditin(IV) is preferred.
The reaction temperature will differ depending on the starting materials, solvent and reagents, but will usually be between -70°C and 80°C, and is preferably between 50°C and 80°C.
The reaction time will differ depending on the starting materials, solvent, reagents and reaction temperature, but will usually be 1 to 8 hours, and is preferably 2 to 4 hours.
As supplementary literature to be used as reference for carrying out this method, there may be mentioned Kurt Ritter et al., Synthesis 1993; 735-762.

### (Step 4-3)

There are no particular restrictions on the solvent used so long as it dissolves the starting compound to some extent and does not inhibit the reaction of this step, and specifically there may be mentioned halogenated hydrocarbons such as chloroform, dichloromethane, 1,2-dichloroethane and carbon tetrachloride, and ethers such as diethyl ether, diisopropyl ether, tetrahydrofuran, dioxane, dimethoxyethane and diethyleneglycol dimethyl ether, among which dichloromethane, carbon tetrachloride, diethyl ether and tetrahydrofuran are preferred.
There are no particular restrictions on the halogenating reagent used so long as it can yield the target compound and does not produce any unseparable by-products, and specifically there may be mentioned chlorine, bromine, iodine, N-chlorosuccinimide, N-bromosuccinimide, N-iodosuccinimide and copper chloride, among which chlorine, bromine and iodine are preferred.
As bases to be used there may be mentioned sodium hydroxide, pyridine and sodium methoxide.
The reaction temperature will differ depending on the starting materials, solvent and reagents, but will usually be between -78°C and 25°C, and is preferably between 0°C and 25°C.
The reaction time will differ depending on the starting materials, solvent, reagents and reaction temperature, but will usually be 1 to 24 hours, and is preferably 1 to 6 hours.

### (Step 4-4)

There are no particular restrictions on the solvent used so long as it dissolves the starting compounds to some extent and does not inhibit the reaction of this step, and specifically there may be mentioned halogenated hydrocarbons such as chloroform, dichloromethane, 1,2-dichloroethane and carbon tetrachloride, and aromatic hydrocarbons such as benzene, toluene and xylene, among which chloroform, dichloromethane and carbon tetrachloride are preferred.
There are no particular restrictions on the halogenating agent used so long as it can yield the target compound and does not produce any unseparable by-products, and specifically there may be mentioned halogenating agents such as chlorine, oxalic chloride, thionyl chloride, phosphorus oxychloride, phosphorus trichloride, phosphorus pentachloride, bromine, oxalic bromide, thionyl bromide, phosphorus tribromide, 2,2,2-tribromo-1,3,2-benzodioxaphosphol, iodine and phosphorus triiodide, among which phosphorus trichloride, phosphorus tribromide, 2,2,2-tribromo-1,3,2-benzodioxaphosphol and phosphorus triiodide are preferred.
The reaction temperature will differ depending on the starting materials, solvent and reagents, but will usually be between 0°C and 70°C.
The reaction time will differ depending on the starting materials, solvent, reagents and reaction temperature, but will usually be 1 to 24 hours.

(Production method for compounds (25), (27) and (9A)) (Method 5)

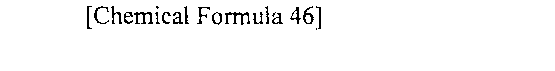

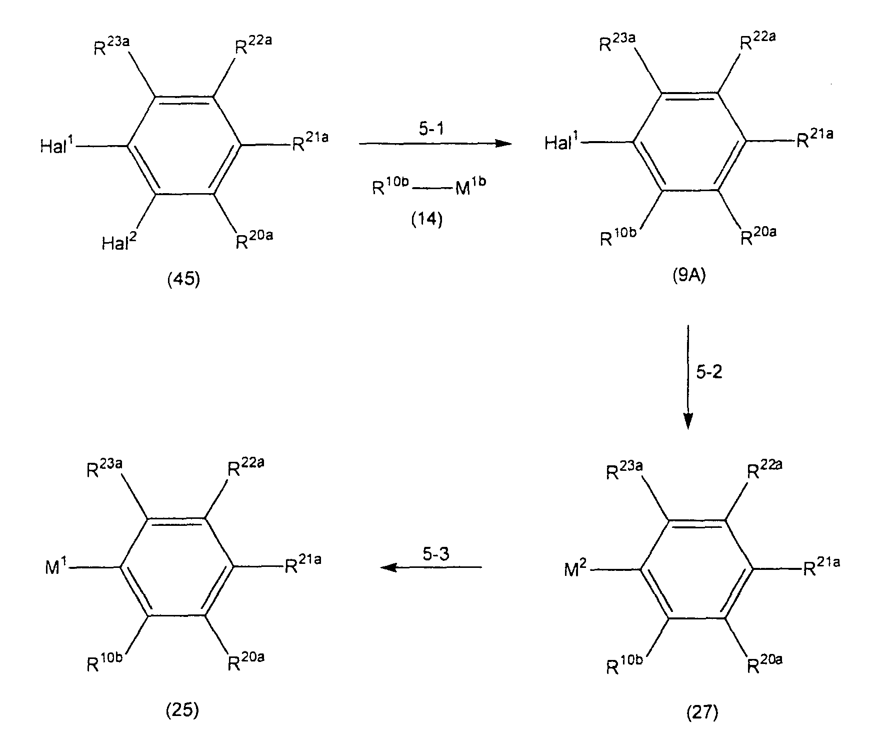

In this method, compound (45) is reacted with compound (14) in an inert solvent in the presence of a palladium(0) catalyst to yield compound (9A) (Step 5-1), and then compound (9A) is reacted with a lithiating agent or Grignard reagent-producing agent to yield compound (27) (Step 5-2) and compound (27) is reacted with a boron metal reagent or tin metal reagent to produce compound (25) (Step 5-3).
In this scheme, M¹, M^{1b}, M², R^{10b}, R^{20a}, R^{21a}, R^{22a} and R^{23a} have the same definitions as above.
Hal¹ is chlorine or bromine, and Hal² is iodine when Hal¹ is bromine, and bromine or iodine when Hal¹ is chlorine.
Step 5-1 of this method may be carried out in a manner similar to Method K above.

### (Step 5-2)

This step will differ depending on the nature of M².

### (Grignard reagent production step)

In this step, compound (9A) is directly reacted with magnesium metal in an inert solvent (direct method), or magnesium-halogen exchange reaction is carried out between compound (9A) and another Grignard reagent (indirect method) to produce compound (27) (i.e., a Grignard reagent).

### (1) Direct method

There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, and phosphoric amides such as hexamethylphosphoric triamide, among which ethers (particularly diethyl ether and tetrahydrofuran) are preferred.
The reaction method may be conducted according to a common procedure, and specifically magnesium metal is suspended in the solvent under an atmosphere of an inert gas such as nitrogen or argon, in the presence or in the absence of a catalytic amount of iodine or dibromoethane as an activating agent, and compound (9A) is slowly added to the reaction system. Upon completion of the reaction, compound (27) is produced in the supernatant, and it is usually used for the next step without isolation.
The reaction temperature will differ depending on the starting materials, solvent and reagents, but will usually be between -20°C and 150°C, and is preferably between 0°C and 100°C.
The reaction time will differ depending on the starting materials, solvent, reagents and reaction temperature, but will usually be 1 to 10 hours.

### (2) Indirect method

The solvent used, the reaction temperature and the reaction time are the same as for the direct method.
The reaction method may be carried out according to a common procedure, but compound (27) (i.e., an organic magnesium compound) may also be produced by reacting the halogen compound (9A) with isopropylmagnesium bromide or the like under an atmosphere of an inert gas such as nitrogen or argon. The resultant compound (27) is usually used for the next step without isolation.

### (Lithiating step)

In this step. lithium-halogen exchange reaction is carried out between the halogen compound (9A) and another alkyllithium reagent in an inert solvent, under an atmosphere of an inert gas such as nitrogen or argon, to produce an aryllithium reagent (27).
There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, and phosphoric amides such as hexamethylphosphoric triamide, among which ethers (particularly diethyl ether and tetrahydrofuran) are preferred.
There are no particular restrictions on the alkyllithium reagent used so long as it can yield the target compound and does not produce any unseparable by-products, and specifically there may be mentioned alkyllithium compounds such as n-butyllithium, sec-butyllithium and t-butyllithium, among which n-butyllithium is preferred.
The reaction temperature will differ depending on the starting materials, solvent and reagents, but will usually be between -100°C and 0°C.
The reaction time will differ depending on the starting materials, solvent, reagents and reaction temperature, but will usually be between 10 minutes and 2 hours.
The resultant compound (27) is usually used for the next step without isolation.

### (Step 5-3)

This step will differ depending on the nature of M¹.

### (Step for the production of a boronic acid reagent)

In this step, the lithium agent or Grignard reagent (27) produced in Step 5-2 is reacted with a borate reagent mentioned below to produce a boronic acid reagent compound (25).
There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, and phosphoric amides such as hexamethylphosphoric triamide, among which ethers (particularly diethyl ether and tetrahydrofuran) are preferred.
There are no particular restrictions on the borate reagent used so long as it can yield the target compound and does not produce any unseparable by-products, and specifically there may be mentioned trialkylborates such as triisopropylborate and trimethylborate, among which triisopropylborate are preferred.
The trialkylborate obtained may be easily hydrolyzed in water or aqueous ammonium chloride to produce a boronic acid reagent compound (25).
The reaction temperature will differ depending on the starting materials, solvent and reagents, but will usually be between -80°C and 50°C, and is preferably between -80°C and 30°C.
The reaction time will differ depending on the starting materials, solvent, reagents and reaction temperature, but will usually be 1 to 10 hours, and is preferably 2 to 6 hours.

### (Step for the production of a tin reagent)

In this step, the lithium agent or Grignard reagent (27) produced in Step 5-2 is reacted with the halogenated trialkyltin reagent mentioned below to produce a tin reagent compound (25).
There are no particular restrictions on the solvent used so long as it dissolves the starting compound to some extent and does not inhibit the reaction of this step, and specifically there may be mentioned ethers such as diethyl ether, diisopropyl ether, tetrahydrofuran, dioxane, dimethoxyethane and diethyleneglycol dimethyl ether, and phosphoric amides such as hexamethylphosphoric triamide, among which ethers (particularly diethyl ether and tetrahydrofuran) are preferred.
There are no particular restrictions on the halogenated trialkyltin reagent used so long as it can yield the target compound and does not produce any unseparable by-products, and specifically there may be mentioned halogenated trialkyltin compounds such as tributyltin chloride and trimethyltin chloride, among which tributyltin chloride is preferred.
The reaction temperature will differ depending on the starting materials, solvent and reagents, but will usually be between -80°C and 50°C, and is preferably between -80°C and 30°C.
The reaction time will differ depending on the starting materials, solvent, reagents and reaction temperature, but will usually be 1 to 10 hours, and is preferably 1 to 6 hours.

(Production method for compound (9B)) (Method 6)

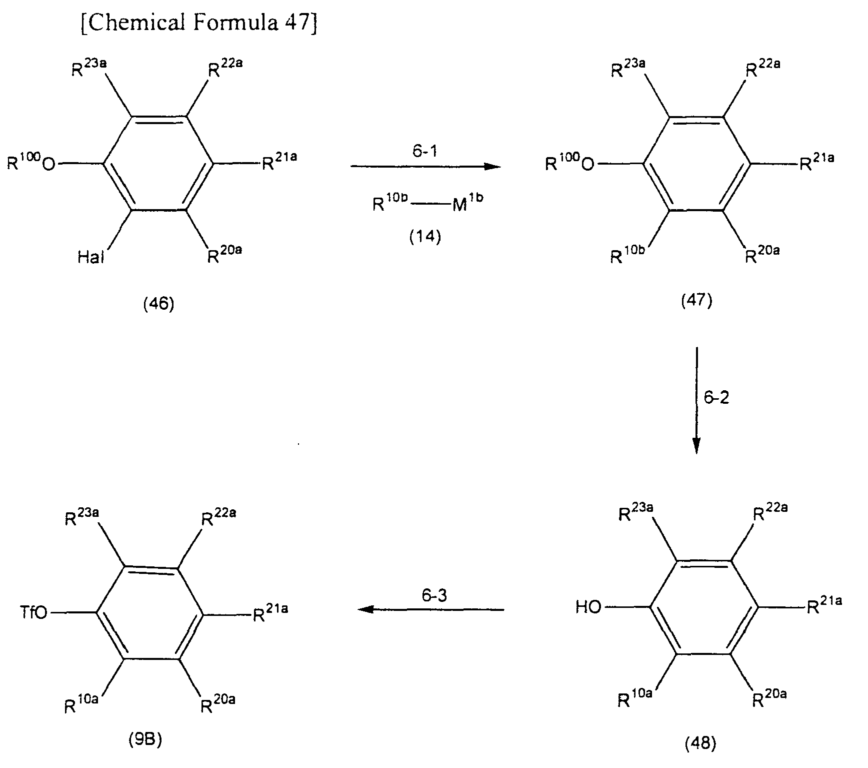

In this step, compound (46) is reacted with compound (14) in an inert solvent in the presence of a palladium(0) catalyst, to yield compound (47) (Step 6-1), and then compound (47) is reacted with a de-alkylating agent or de-aralkylating agent to yield compound (48) (Step 6-2), and a trifluoromethanesulfonyl group is introduced at the phenolic hydroxyl group of compound (48) to produce compound (9B) (Step 6-3).
In this scheme, Tf, M^{1b}, Hal, R^{10a}, R^{10b}, R^{20a}, R^{21a}, R^{22a} and R^{23a} have the same definitions as above. Also, R¹⁰⁰ represents C1-6 alkyl or optionally substituted aralkyl, and is preferably methyl or benzyl.
Step 6-1 of this method may be carried out in a manner similar to Method K above.

### (Step 6-2)

There are no particular restrictions on the solvent used so long as it dissolves the starting compounds to some extent and does not inhibit the reaction of this step, and specifically there may be mentioned alcohols such as methanol, ethanol and isopropanol, ethers such as diethyl ether, diisopropyl ether, tetrahydrofuran, dioxane, dimethoxyethane and diethyleneglycol dimethyl ether, aromatic hydrocarbons such as benzene, toluene and xylene, organic acids such as carbon disulfide, acetic acid and hydrogen bromide in acetic acid solution, organic bases such as quinoline and pyridine, and water. These may be selected as appropriate for the de-alkylating agent or de-aralkylating agent used.
There are no particular restrictions on the de-alkylating agent or de-aralkylating agent used so long as it can yield the target compound and does not produce any unseparable by-products, and specifically there may be mentioned Lewis acids such as boron tribromide, boron trichloride, boron triiodide and aluminum chloride, Bronsted acids such as hydrobromic acid, hydrochloric acid and hydrogen bromide in acetic acid solution, metal salts such as lithium iodide, and halogenated silanes such as trimethylsilane iodide.
The reaction temperature will differ depending on the starting materials, solvent and reagents, but will usually be between -80°C and 250°C.
The reaction time will differ depending on the starting materials, solvent, reagents and reaction temperature, but will usually be 0.1 to 100 hours.
When R¹⁰⁰ is an optionally substituted aralkyl group, the de-aralkylating step may be carried out according to the hydrogenation method described for Method A above.
The conditions may be selected as suitable for the starting materials, in order to allow selective deprotection.
As supplementary literature to be used as reference for carrying out this method, there may be mentioned: M. Vivekananda Bhatt, Surendra U. Kulkarni et al., "Cleavage of Ethers" Synthesis (1983), 249; T.W. Greene, (Protective Groups in Organic Synthesis), John Wiley & Sons; and J.F.W. McOmis, (Protective Groups in Organic Chemistry), Plenum Press.
Step 6-3 may be carried out in a manner similar to Method A or Method B above.

(Production method for compound (25A)) (Method 7)

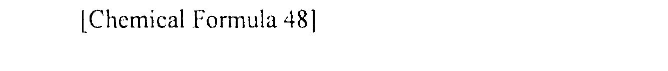

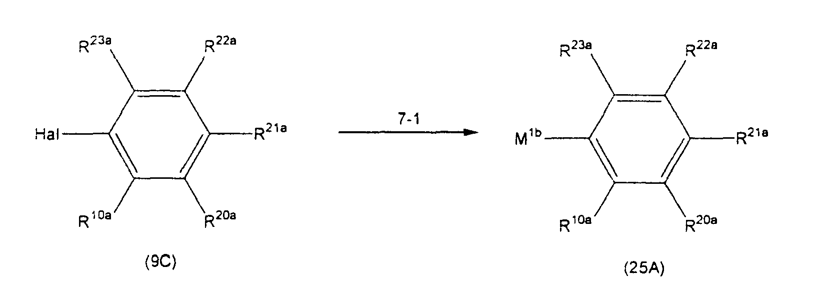

In this method, compound (9C) is reacted with a boron metal reagent or tin metal reagent in an inert solvent in the presence of a palladium(0) catalyst, to produce compound (25A).
In this scheme, M^{1b}, Hal, R^{10a}, R^{20a}, R^{21a}, R^{22a} and R^{23a} have the same definitions as above.
This method may be carried out in a manner similar to Step 4-2 of Method 4.

(Production method for compound (13A)) (Method 8)

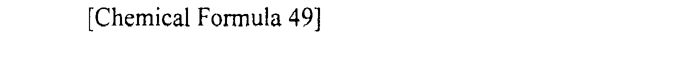

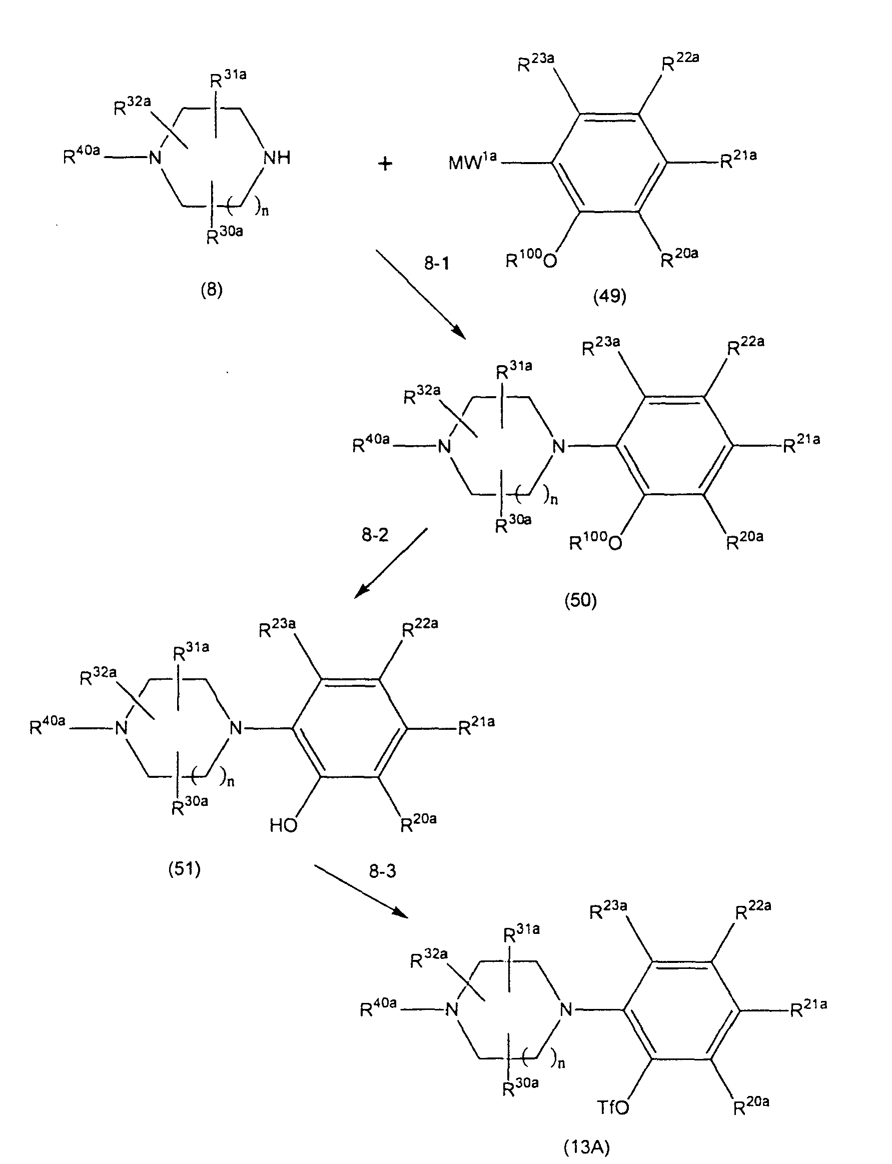

In this method, compound (49) is reacted with compound (8) (amination or amidation), in an inert solvent in the presence of a palladium(0) catalyst or copper catalyst, in the presence or in the absence of a base, in the presence or in the absence of an additive, under or not under an inert gas atmosphere, to yield compound (50) (Step 8-1), and then compound (50) is reacted with a de-alkylating agent or de-aralkylating agent to yield compound (51) (Step 8-2), and a trifluoromethanesulfonyl group is introduced at the phenolic hydroxyl of compound (51) to produce compound (13A) (Step 8-3).
In this scheme, Tf, n, R^{20a}, R^{21a}, R^{22a}, R^{23a}, R^{30a}, R^{31a}, R^{32a}, R^{40a} and R¹⁰⁰ have the same definitions as above. Also, MW^{1a} has the same definition as M^{1a} or W^{1a} above.
Step 8-1 of this method may be carried out in a manner similar to Method F or Method G described above, Step 8-2 may be carried out in a manner similar to Step 6-2 described above, and Step 8-3 may be carried out in a manner similar to Method A or Method B described above.

(Production method for compounds (15) and (13B)) (Method 9)

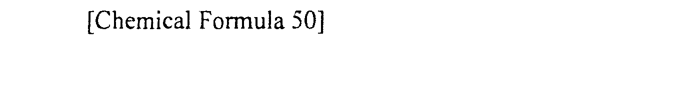

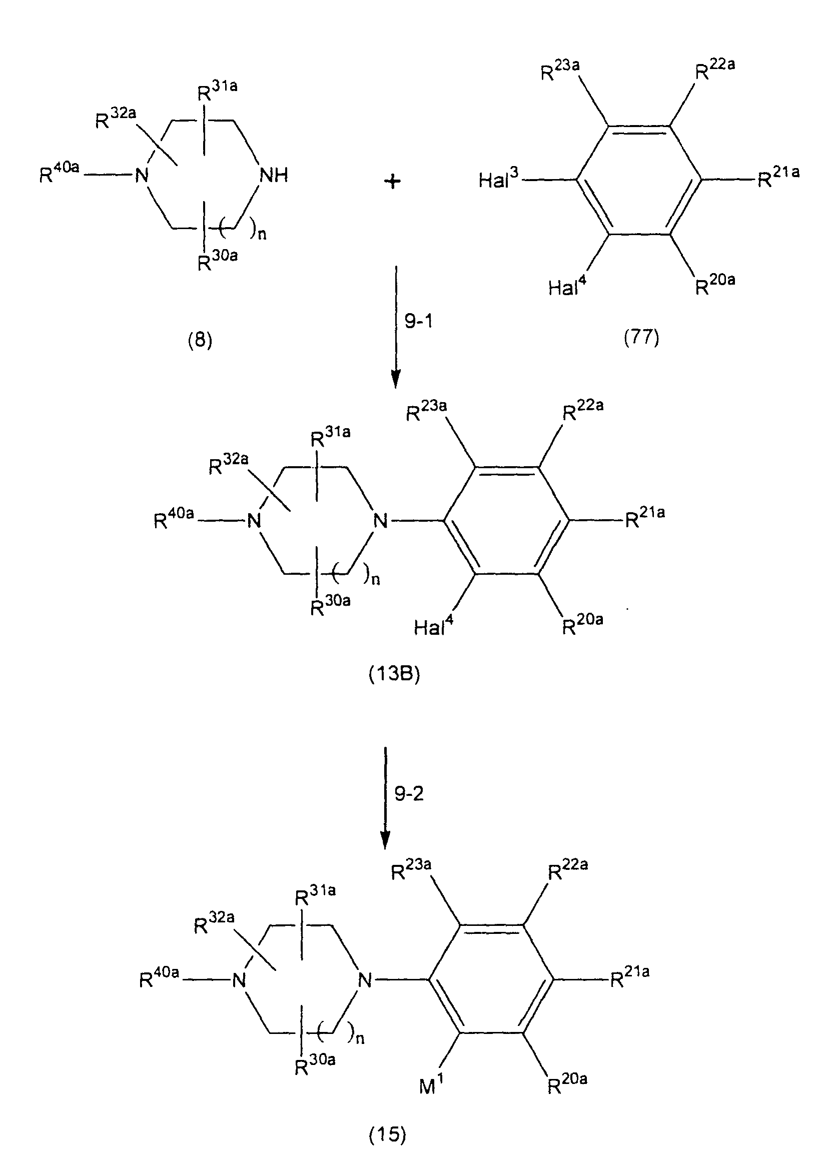

In this method, compound (77) is reacted with compound (8), in an inert solvent, in the presence of a palladium(0) catalyst or copper catalyst, in the presence or in the absence of a base, in the presence or in the absence of an additive, under or not under an inert gas atmosphere, to yield compound (13B) (Step 9-1), and then compound (13B) is reacted with a metal reagent to produce compound (15) (Step 9-2).
In this scheme, n, M¹, R^{20a}, R^{21a}, R^{22a}, R^{23a}, R^{30a}, R^{31a}, R^{32a} and R^{40a} have the same definitions as above.
Hal⁴ is chlorine or bromine, and Hal³ is iodine when Hal⁴ is bromine, and bromine or iodine when Hal⁴ is chlorine.
Step 9-1 of this method may be carried out in a manner similar to Method F above, and Step 9-2 may be carried out in a manner similar to Step 5-2 and Step 5-3, or Method 7, above.

(Production method for compounds (16A), (14) and (16B)) (Method 10)

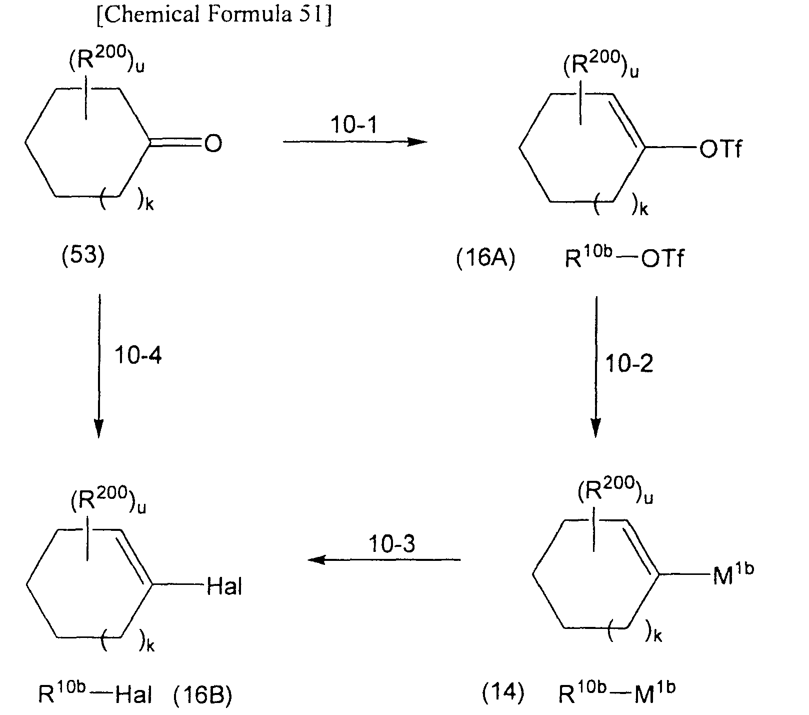

In this method, a leaving group is introduced into compound (53), in an inert solvent, under or not under inert gas atmosphere, to yield compound (16A) (Step 10-1), and then compound (16A) is reacted with a boron metal reagent or tin metal reagent in the presence of a palladium(0) catalyst to yield compound (14) (Step 10-2), and compound (14) is reacted with a halogenating reagent to produce compound (16B) (Step 10-3).
Alternatively, compound (53) is reacted directly with a halogenating agent to produce compound (16B) (Step 10-4).
In this scheme, Tf, R^{10b}, M^{1b} and Hal have the same definitions as above. Also, R²⁰⁰ is a substituent in Group A1, or a substituent in Group A1, which is protected. u is an integer of 0, 1, 2, 3, 4 or 5. k is an integer of 0, 1, 2, 3, 4 or 5.
Step 10-1 of this method may be carried out in a manner similar to Method 4-1 above, Step 10-2 may be carried out in a manner similar to Step 4-2 above, and Step 10-3 may be carried out in a manner similar to Step 4-3 above. Step 10-4 may be carried out in a manner similar to Step 4-4 above.

(Production method for compound (12)) (Method 11)

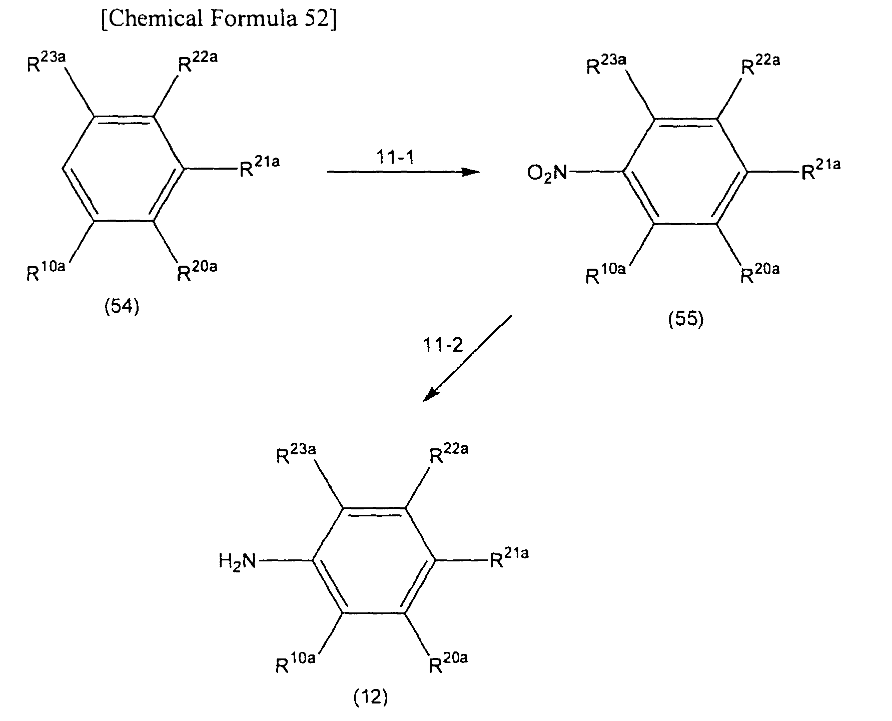

In this step, compound (54) is reacted with a nitrating reagent to yield compound (55) (Step 11-1), and then a metal or metal salt is used in the presence of an acid for reduction of compound (55) to produce compound (12) (Step 11-2).
In this scheme, R^{10a}, R^{20a}, R^{21a}, R^{22a} and R^{23a} have the same definitions as above.

### (Step 11-1)

There are no particular restrictions on the solvent used so long as it dissolves the starting compounds to some extent and does not inhibit the reaction of this step, and specifically there may be mentioned a sulfuric acid and nitric acid mixture or an acetic acid and nitric acid mixture, where the nitric acid solvent reacts as a nitrating agent.
The reaction temperature will differ depending on the starting materials, solvent and reagents, but will usually be between -20°C and 150°C, and is preferably between 0°C and 80°C.
The reaction time will differ depending on the starting materials, solvent, reagents and reaction temperature, but will usually be 0.5 to 48 hours, and is preferably 1 to 12 hours.

### (Step 11-2)

There are no particular restrictions on the solvent used so long as it dissolves the starting compounds to some extent and does not inhibit the reaction of this step, and specifically there may be mentioned alcohols such as methanol and ethanol, amides such as formamide, dimethylformamide, dimethylacetamide and hexamethylphosphoric triamide, organic acids such as acetic acid, water, and mixtures of these solvents, among which a mixed solvent of ethanol and water, a mixed solvent of ethanol dimethylformamide and water, or acetic acid is preferred.
There are no particular restrictions on the metal or metal salt used so long as it can yield the target compound and does not produce any unseparable by-products, and specifically there may be mentioned metals such as iron powder, tin powder and zinc powder, and metal salts such as tin(II) chloride, among which a metal (particularly iron powder) is preferred.
There are no particular restrictions on the acid used so long as it can yield the target compound and does not produce any unseparable by-products, and specifically there may be mentioned organic acids such as acetic acid, and inorganic acids such as hydrochloric acid and ammonium chloride, among which ammonium chloride is preferred.
The equivalents of the metal or metal salt used will differ depending on the starting materials, solvent and reagents, but will usually be a proportion of 2-15 and preferably 3-6, in terms of the molar ratio with respect to the starting material.
The reaction temperature will differ depending on the starting materials, solvent and reagents, but will usually be between 0°C and 150°C, and is preferably between 0°C and 100°C.
The reaction time will differ depending on the starting materials, solvent, reagents and reaction temperature, but will usually be 0.5 to 48 hours, and is preferably 1 to 12 hours.
Compound (55) or (12) may be isolated or purified from the reaction mixture obtained above, by the method described above.

(Production method for compound (55A)) (Method 12)

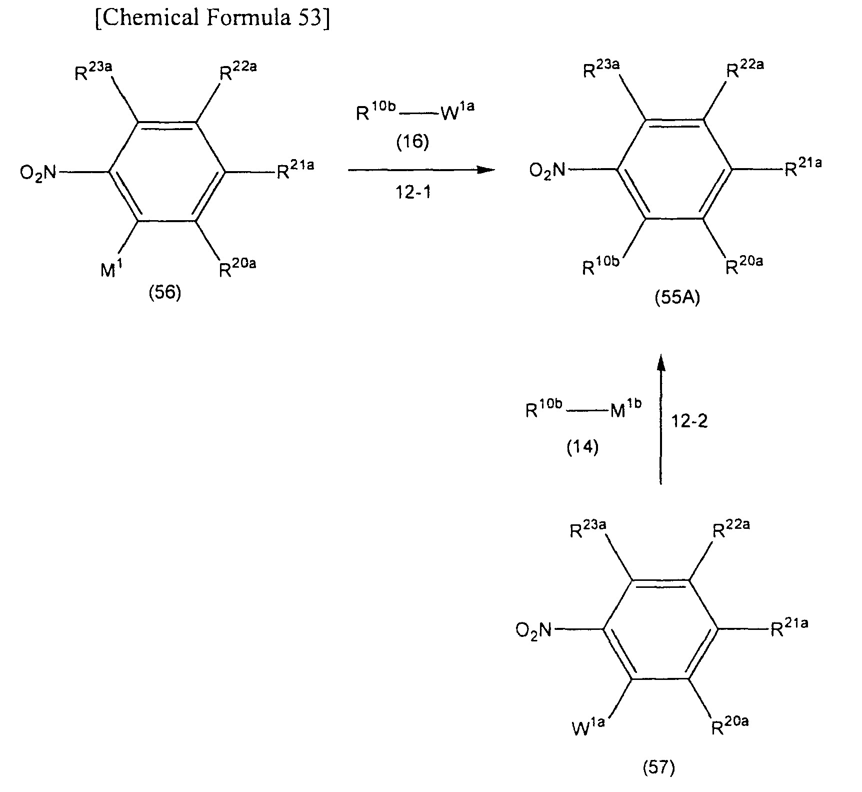

In this method, compound (56) is reacted with compound (16) above to produce compound (55A) (Method 12-1), or compound (57) is reacted with compound (14) above to produce compound (55A) (Method 12-2), in an inert solvent, in the presence of a palladium(0) catalyst, under or not under an inert gas atmosphere, in the presence or in the absence of a base, in the presence or in the absence of an additive.
In this scheme, W^{1a}, M¹, M^{1b}, R^{10b}, R^{20a}, R^{21a}, R^{22a} and R^{23a} have the same definitions as above.
Method 12-1 of this method may be carried out in a manner similar to Method K above, and Method 12-2 may be carried out in a manner similar to Method K described above.

The starting compounds or intermediates for each of the steps in the general production methods described above for obtaining compound (1) or (100) may be purchased as commercial products, or can be obtained as starting compounds or intermediates having various substituents introduced at the R²⁰ to R²³ positions from commercially available compounds by well-known methods (substituent conversion, substituent introduction, protecting group introduction, deprotection, etc.) which are ordinarily carried out by those skilled in the art.
By using such starting compounds or intermediates with various substituents introduced at the R²⁰ to R²³ positions for the general production methods described above, it is possible to produce compound (1) or (100) having various R²⁰ to R²³ substituents introduced therein.
When the compound of the present invention is to be used as a medicament, it will normally be mixed with appropriate additives for use as a formulation. However, this does not preclude the use of the compound of the invention by itself as a medicament.

Such additives may include excipients, binders, lubricants, disintegrators, coloring agents, taste correctives, emulsifiers, surfactants, dissolving aids, suspending agents, isotonizing agents, buffering agents, antiseptics, antioxidants, stabilizers, absorption accelerators and the like which are commonly used in pharmaceuticals, and they may be added in appropriate combinations as desired.
As examples of such excipients there may be mentioned lactose, white soft sugar, glucose, corn starch, mannitol, sorbitol, starch, alpha starch, dextrin, crystalline cellulose, soft silicic anhydride, aluminum silicate, calcium silicate, magnesium aluminometasilicate, calcium hydrogenphosphate, and the like.
As examples of binders there may be mentioned polyvinyl alcohol, methylcellulose, ethylcellulose, gum Arabic, tragacanth, gelatin, shellac, hydroxypropylmethylcellulose, hydroxypropylcellulose, carboxymethylcellulose sodium, polyvinylpyrrolidone, macrogol, and the like.
As examples of lubricants there may be mentioned magnesium stearate, calcium stearate, sodium stearyl fumarate, talc, polyethylene glycol, colloidal silica, and the like.
As examples of disintegrators, there may be mentioned crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch, carboxymethyl starch sodium, and the like.
As coloring agents there may be mentioned those approved for addition to pharmaceuticals, such as iron sesquioxide, yellow iron sesquioxide, carmine, caramel, β-carotene, titanium oxide, talc, riboflavin sodium phosphate, yellow aluminum lake, and the like.
As taste correctives there may be mentioned cocoa powder, menthol, aromatic powders, mentha oil, borneol, powdered cinnamon bark, and the like.
As emulsifiers or surfactants there may be mentioned stearyl triethanolamine, sodium lauryl sulfate, lauryl aminopropionic acid, lecithin, glycerin monostearate, sucrose fatty acid esters, glycerin fatty acid esters, and the like.
As dissolving aids there may be mentioned polyethylene glycol, propylene glycol, benzyl benzoate, ethanol, cholesterol, triethanolamine, sodium carbonate, sodium citrate, polysorbate 80, nicotinamide, and the like.
As suspending agents there may be mentioned the surfactants referred to above, as well as hydrophilic polymers such as polyvinyl alcohol, polyvinylpyrrolidone, methylcellulose, hydroxymethylcellulose, hydroxyethylcellulose, hydroxypropylcellulose, and the like.
As isotonizing agents there may be mentioned glucose, sodium chloride, mannitol, sorbitol, and the like.
As buffering agents there may be mentioned buffering solutions of phosphate, acetate, carbonate, citrate and the like.
As antiseptics there may be mentioned methylparaben, propylparaben, chlorobutanol, benzyl alcohol, phenethyl alcohol, dehydroacetic acid, sorbic acid, and the like.
As antioxidants there may be mentioned sulfite, ascorbic acid, α-tocopherol, and the like.
As stabilizers there may be mentioned those commonly used in pharmaceuticals.
As absorption accelerators there may also be mentioned those commonly used in pharmaceuticals.

The formulation may be in an oral form such as tablets, powders, granules, capsules, syrups, lozenges or inhalanst, an external application form such as suppository, ointment, eye salve, tape, eye drop, nasal drop, ear drop, pap or lotion, or an injection.
An oral formulation will be formulated using an appropriate combination of additives among those mentioned above. The surface thereof may also be coated if necessary.
An external application will be formulated using an appropriate combination of additives among those mentioned above, and particularly excipients, binders, taste correctives, emulsifiers, surfactants, dissolving aids, suspending agents, isotonizing agents, antiseptics, antioxidants, stabilizers and absorption accelerators.
An injection will be formulated using an appropriate combination of additives among those mentioned above, and particularly emulsifiers, surfactants, dissolving aids, suspending agents, isotonizing agents, buffering agents, antiseptics, antioxidants, stabilizers and absorption accelerators.

When the compound of the invention is to be used as a drug, the dosage thereof will differ depending on the symptoms and age of the patient, but it will ordinarily be 0.15 to 5000 mg (preferably 0.5 to 1500 mg) in the case of an oral formulation, 0.5 to 1500 mg (preferably 1.5 to 500 mg) in the case of an external application, and 0.3 to 5000 mg (preferably 1 to 500 mg) in the case of an injection, per day; administered at once or divided over 2 to 6 times. For an oral formulation or injection, this represents the actually administered dose, while for an external application this represents the actually absorbed dose.

### Examples

Compounds (1) and (100) according to the invention may be produced, for example, by the process described in the following examples. It is to be understood, however, that these examples merely serves as illustration and are not intended to restrict the compounds of the invention under any circumstances.
Also, unless otherwise specified, the silica gel mentioned throughout the examples is Silica Gel 60 by Merck & Co. or BW300 by Fuji Silysia Chemical Ltd., and the NH silica gel is propylamine-coated Chromatorex-NH silica gel by Fuji Silysia Chemical Ltd.
In the TLC analysis as well, unless otherwise specified, the silica gel is Silica Gel 60 by Merck & Co. and the NH is a propylamine-coated NH silica gel plate by Fuji Silysia Chemical Ltd.

### (Production Example 1A)

### Trifluoromethanesulfonic acid 4,4-dimethylcyclohex-1-enyl ester

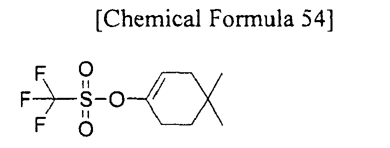

A mixture of lithium bis(trimethylsilyl)amide (1M solution in tetrahydrofuran, 172 mL, 172 mmol) and anhydrous tetrahydrofuran (400 mL) was stirred, and then cooled to below -70°C in a dry ice-acetone bath under a nitrogen atmosphere. A solution of 4,4-dimethylcyclohexanone (18 g, 143 mmol) in anhydrous tetrahydrofuran (100 mL) was added dropwise to the solution over 30 minutes. After stirring for 2 hours and 10 minutes under the same conditions, N-phenyl bis(trifluoromethanesulfonimide) (54 g, 150 mmol) was added to the reaction mixture, and stirring was continued for 16 hours while slowly warmed to room temperature.
Saturated aqueous ammonium chloride was added to the reaction mixture to quench the reaction. Hexane and water were added to the mixture, and the organic layer and aqueous layer were separated. The organic layer was washed with brine and then dried over anhydrous magnesium sulfate. The aqueous layer was re-extracted with hexane and treated in the same manner as the organic layer. The two organic layers were combined, the desiccant was filtered off and the filtrate was concentrated under reduced pressure. The residue was purified by silica gel column chromatography (ethyl acetate/hexane) to give 26.8 g of the title compound as a light yellow oil.
¹H-NMR (400MHz, CDCl₃)
δ: 0.97 (s, 6H), 1.54 (t, J= 6.4Hz, 2H), 1.96-1.98 (m, 2H), 2.31-2.36 (m, 2H), 5.66-5.69 (m, 1H).

### (Production Example 1B)

### 1-(4,4-Dimethylcyclohex-1-enyl)-2-nitrobenzene

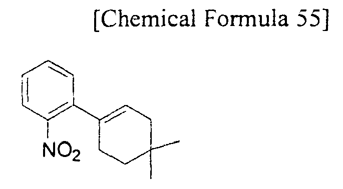

To a solution of 2-nitrophenylboronic acid (14.2 g, 85.19 mmol) in toluene (250 mL)-ethanol(125 mL) were added trifluoromethanesulfonic acid 4,4-dimethylcyclohex-1-enyl ester (20 g, 77.44 mmol) prepared in Example (1a), tetrakis(triphenylphosphine)palladium(0) (4.5 g, 3.87 mmol) and 2N aqueous sodium carbonate (128 mL, 256 mmol). The mixture was stirred at an external temperature of 100°C for 1 hour and 45 minutes under a nitrogen atmosphere.
After air-cooling the reaction mixture to room temperature, it was passed through Celite and insoluble matters were filtered off. Ethyl acetate and water were added to the resultant filtrate and the filtrate was extracted with ethyl acetate. The collected organic layer was washed with water and brine in that order and then dried over anhydrous magnesium sulfate. The desiccant was filtered off, and the filtrate was concentrated under reduced pressure. The resultant residue was purified by silica gel column chromatography (ethyl acetate/hexane) to give 16.3 g of the title compound as a brown oil.
¹H-NMR (400MHz, CDCl₃)
δ: 1.00 (s, 6H), 1.51 (t, J= 6.4Hz, 2H), 1.92-1.94 (m, 2H), 2.24-2.29 (m, 2H), 5.55-5.57 (m, 1H), 7.27 (dd, J= 7.6, 1.6Hz, 1H), 7.34 (ddd, J= 7.6, 7.6, 1.6Hz, 1 H), 7.50 (ddd, J= 7.6, 7.6, 1.6Hz, 1H), 7.77 (dd, J= 7.6, 1.6Hz, 1H).

### (Production Example 1C)

### 2-(4,4-Dimethylcyclohexyl)phenylamine

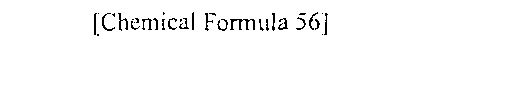

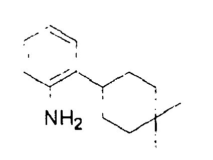

A mixture of 1-(4,4-dimethylcyclohex-1-enyl)-2-nitrobenzene (16.3 g, 70.5 mmol), 10% palladium on carbon (1 g, wet) and ethyl acetate (100 mL) was stirred for 14 hours and 30 minutes under a hydrogen atmosphere at atmospheric pressure and room temperature.
The reaction mixture was filtered and the filtrate was concentrated under reduced pressure. A mixture of the resultant residue, 10% palladium on carbon (3 g, wet) and ethyl alcohol (200 mL) was stirred for 30 hours and 30 minutes under a hydrogen atmosphere at atmospheric pressure and room temperature.
After the reaction completed, the reaction mixture was filtered and the filtrate was concentrated under reduced pressure. The resultant residue was purified by silica gel column chromatography (ethyl acetate/hexane) to give 11.79 g of the title compound as a light yellow solid.
¹H-NMR (400MHz, CDCl₃)
δ: 0.97 (s, 3H), 0.99 (s, 3H), 1.36 (td, J= 13.2, 4.0Hz, 2H), 1.47-1.73 (m, 6H), 2.38 (tt, J= 11.6, 3.6Hz, 1H), 3.63 (brs, 2H), 6.68 (dd, J= 7.6, 1.6Hz, 1H), 6.77 (ddd, J= 7.6, 7.6, 1.6Hz, 1H), 7.01 (ddd, J= 7.6, 7.6, 1.6Hz, 1H), 7.14 (dd, J= 7.6, 1.6Hz, 1H).

### (Production Example 1D)

### 1-[2-(4,4-Dimethylcyclohexyl)phenyl]piperazine

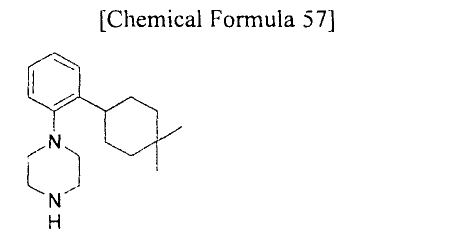

To a solution of 2-(4,4-dimethylcyclohexyl)phenylamine (11.79 g, 57.98 mmol) in 1,2-dichlorobenzene (30 mL) was added bis(2-chloroethyl)amine hydrochloride (12.42 g, 69.58 mmol), and the mixture was stirred at an external temperature of 200°C for 2 hours and 30 minutes under a nitrogen atmosphere. During the reaction, nitrogen was passed through the reactor several times to remove the hydrogen chloride gas.
After air-cooling the reaction mixture to room temperature, ethyl acetate and saturated aqueous sodium hydrogencarbonate were added and the mixture was extracted with ethyl acetate. The collected organic layer was washed with water and brine in that order and then dried over anhydrous sodium sulfate. The desiccant was filtered off, and the filtrate was concentrated under reduced pressure. The resultant residue was purified by NH silica gel column chromatography (ethyl acetate/hexane) to give 12.15 g of the title compound as a brown oil.
¹H-NMR (400MHz, CDCl₃)
δ: 0.97 (s, 3H), 1.01 (s, 3H), 1.34 (td, J= 12.8, 4.4Hz, 2H), 1.48-1.68 (m, 6H), 2.82-2.84 (m, 4H), 2.95-3.03 (m, 5H), 7.05-7.27 (m, 4H).
The 1H of NH could not be identified.

### (Production Example 1E)

### 4-[2-(4,4-Dimethylcyclohexyl)phenyl]piperazine-1-carboxylic acid t-butyl ester

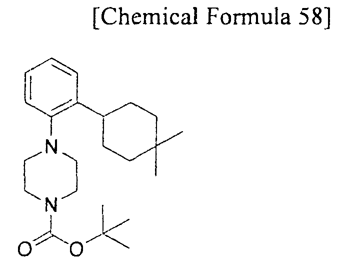

A mixture of 1-[2-(4,4-dimethylcyclohexyl)phenyl]piperazine (11 g. 40.4 mmol), triethylamine (6.2 mL, 44.4 mmol), 4-dimethylaminopyridine (247 mg, 2.02 mmol) and dichloromethane (180 mL) was stirred at an external temperature of 0°C under a nitrogen atmosphere. A mixture of di-t-butyl dicarbonate (9.7 g, 44.4 mmol) and dichloromethane (20 mL) was added thereto.
After stirring for 2 hours and 50 minutes under the same conditions, saturated aqueous sodium hydrogencarbonate was added to the reaction mixture and the mixture was extracted with dichloromethane. The collected organic layer was washed with brine and then dried over anhydrous sodium sulfate. The desiccant was filtered off and the filtrate was concentrated under reduced pressure. The resultant residue was purified by silica gel column chromatography (ethyl acetate/hexane) to give 14.89 g of the title compound as a light yellow oil.
¹H-NMR (400MHz, CDCl₃)
δ: 0.96 (s, 3H), 1.01 (s, 3H), 1.31 (td, J= 12.8, 4.4Hz, 2H), 1.49 (s, 9H), 1.49-1.69 (m, 6H), 2.81 (brs, 4H), 2.95-3.02 (m, 1H), 3.57 (brs, 4H), 7.06 (dd, J= 7.6, 1.6Hz, 1H), 7.10 (ddd, J= 7.6, 7.6, 1.6Hz, 1H), 7.16 (ddd, J= 7.6, 7.6, 2.0Hz, 1H), 7.28 (dd, J= 7.6, 2.0Hz, 1H).

### (Production Example 1F)

### 4-[4-Bromo-2-(4,4-dimethylcyclohexyl)phenyl]piperazine-1-carboxylic acid t-butyl ester

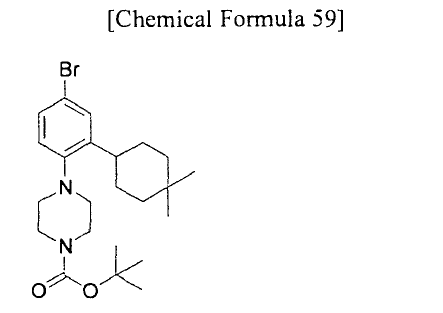

A mixture of 4-[2-(4,4-dimethylcyclohexyl)phenyl]piperazine-1-carboxylic acid t-butyl ester (8 g, 21.5 mmol), sodium acetate (17.6 g, 215 mmol) and methanol (300 mL) was stirred at an external temperature of room temperature under a nitrogen atmosphere. Bromine (1.1 mL, 21.5 mmol) was added dropwise thereto over 20 minutes, and the mixture was stirred for 17 hours under the same conditions. Sodium acetate (8.8 g, 107.5 mmol) was added thereto, and then bromine (0.4 mL, 7.8 mmol) was added dropwise and the mixture was stirred for 1 hour under the same conditions.
A saturated aqueous solution of sodium sulfite was added to the reaction mixture and the mixture was extracted with ethyl acetate. The collected organic layer was washed with brine and then dried over anhydrous magnesium sulfate. The desiccant was filtered off and the filtrate was concentrated under reduced pressure. The resultant residue was purified by silica gel column chromatography (ethyl acetate/hexane) to give 7.97 g of the title compound as a light yellow solid. ¹H-NMR (400MHz, CDCl₃)
δ: 0.96 (s, 3H), 1.01 (s, 3H), 1.24-1.34 (m, 2H), 1.41-1.64 (m, 6H), 1.49 (s, 9H), 2.77 (brs. 4H), 2.89-2.97 (m, 1H), 3.55 (brs, 4H), 6.92 (d, J= 8.4Hz, 1H), 7.25 (dd, J= 8.4, 2.4Hz, 1H), 7.35 (d, J= 2.4Hz, 1H).

### (Production Example 2A)

### Trifluoromethanesulfonic acid 3,3,5,5-tetramethylcyclohex-1-enyl ester

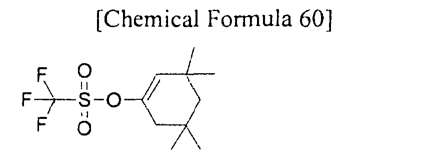

A solution of 3,3,5,5-tetramethylcyclohexanone (12.8 g, 82.98 mmol) in anhydrous tetrahydrofuran (300 mL) was cooled to below -70°C in a dry ice-acetone bath under a nitrogen atmosphere. To the stirred solution was gradually added dropwise lithium bis(trimethylsilyl)amide (1M solution in tetrahydrofuran, 100 mL, 100 mmol) over 15 minutes. After stirring for 40 minutes under the same conditions, a solution of N-phenyl bis(trifluoromethanesulfonimide) (32.51 g, 91 mmol) in anhydrous tetrahydrofuran (150 mL) was added to the reaction mixture, and stirring was continued for 13 hours and 30 minutes with slowly warmed to room temperature.
Ethyl acetate and brine were added to the reaction mixture and the mixture was extracted with ethyl acetate. The collected organic layer was dried over anhydrous sodium sulfate. The desiccant was filtered off and the filtrate was concentrated under reduced pressure. The resultant residue was purified by silica gel column chromatography (ethyl acetate/hexane) to give 23.65 g of the title compound as a colorless oil.
¹H-NMR (400MHz, CDCl₃)
δ: 1.04 (s, 6H), 1.09 (s, 6H), 1.35 (s, 2H), 2.08 (s, 2H), 5.51 (s, 1H).

### (Production Example 2B)

### 4,4,5,5-Tetramethyl-2-(3,3,5,5-tetramethylcyclohex-1-enyl)-[1,3,2]dioxaborolane

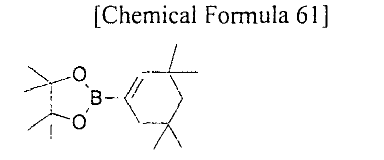

To a solution of trifluoromethanesulfonic acid 3,3,5,5-tetramethylcyclohex-1-enyl ester (45.94 g, 0.16 mol) in dioxane (500 mL) were added bis(pinacolato)diboron (44.9 g, 0.177 mol), [1,1'-bis(diphenylphosphino)ferrocene]dichloropalladium(II) dichloromethane complex (4 g, 4.9 mmol) and potassium acetate (47.3 g, 0.482 mol), and the mixture was stirred at an external temperature of 80°C for 16 hours and 30 minutes.
Ethyl acetate, water and brine were added to the reaction mixture and the mixture was extracted with ethyl acetate. The collected organic layer was dried over anhydrous sodium sulfate. The desiccant was filtered off and the filtrate was concentrated under reduced pressure. The resultant residue was purified by silica gel column chromatography (ethyl acetate/hexane) to give 39.27 g of the title compound as a light yellow solid.
¹H-NMR (400MHz, CDCl₃)
δ: 0.92 (s, 6H), 1.01 (s, 6H), 1.27 (s, 12H), 1.31 (s, 2H), 1.84 (d, J= 1.6Hz, 2H), 6.26 (t, J= 1.6Hz, 1H).

### (Production Example 2C)

### 4-(2-Hydroxyphenyl)piperazine-1-carboxylic acid t-butyl ester

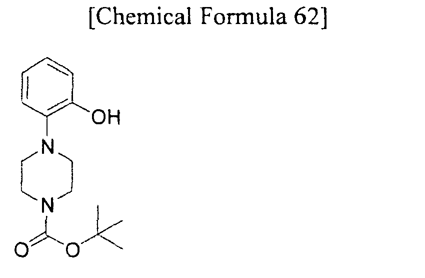

A suspension of 2-(1-piperazino)phenol (3.56 g, 20 mmol) in acetonitrile (15 mL) was stirred at room temperature. A solution of di-t-butyl dicarbonate (4.8 g, 22 mmol) in acetonitrile (15 mL) was added thereto.
After stirring for 1 hour, insoluble matters were filtered out and the filtrate was concentrated. Hexane was added to the residue prior to sonication. The resultant solid was filtered and dried under reduced pressure to give a crude product of the title compound (5.35 g) as a light brown solid.
¹H-NMR (400MHz, CDCl₃)
δ: 1.49 (s, 9H), 2.82 (t, J= 4.8Hz, 4H), 3.59 (t, J= 4.8Hz, 4H), 6.87 (td, J= 7.6, 1.2Hz, 1H), 6.96 (dd, J= 8.0, 1.2Hz, 1H), 7.07-7.14 (m, 2H).
The 1H of OH could not be identified.

### (Production Example 2D)

### 4-(2-Trifluoromethanesulfonyloxyphenyl)piperazine-1-carboxylic acid t-butyl ester

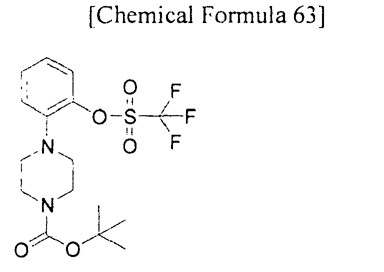

A mixture of 4-(2-hydroxyphenyl)piperazine-1-carboxylic acid t-butyl ester (4.61 g, 16.56 mmol), triethylamine (11.5 mL, 82.5 mmol) and dichloromethane (100 mL) was cooled in an ice bath under a nitrogen atmosphere. Trifluoromethanesulfonic anhydride (4 mL, 23.78 mmol) was gradually added dropwise over 40 minutes with stirring the mixture.
After stirring for 17 minutes under the same conditions, saturated aqueous ammonium chloride, ethyl acetate and water were added to the reaction mixture and the mixture was extracted with ethyl acetate. The collected organic layer was washed twice with saturated aqueous ammonium chloride, subsequently washed once with brine, and then dried over anhydrous sodium sulfate. The desiccant was filtered off and the filtrate was concentrated under reduced pressure. The resultant residue was purified by silica gel column chromatography (ethyl acetate/hexane) to give 5.54 g of the title compound as a light yellow solid.
¹H-NMR (400MHz, CDCl₃)
δ: 1.48 (s, 9H), 2.95 (t, J= 4.8Hz, 4H), 3.62 (t, J= 4.8Hz, 4H), 7.10-7.16 (m, 2H), 7.18 (dd, J= 8.0, 1.6Hz, 1H), 7.33 (ddd, J= 7.2, 7.2, 1.6Hz, 1H).

### (Production Example 2E)

### 4-[2-(3,3,5,5-Tetramethylcyclohex-1-enyl)phenyl]piperazine-1-carboxylic acid t-butyl ester

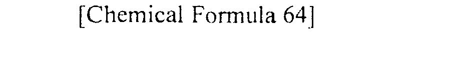

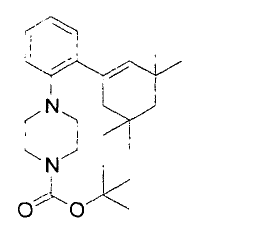

A mixture of 4-(2-trifluoromethanesulfonyloxyphenyl)piperazine-1-carboxylic acid t-butyl ester (6.16 g, 15 mmol), 4,4,5,5-tetramethyl-2-(3,3,5,5-tetramethylcyclohex-1-enyl)-[1,3,2]dioxaborolane (4.6 g, 17.41 mmol), tripotassium phosphate (3.2 g, 15 mmol), 1,2-dimethoxyethane (60 mL) and water (3 mL) was stirred at room temperature under a nitrogen atmosphere. Tetrakis(triphenylphosphine)palladium(0) (1.74 g, 1.5 mmol) was added to the mixture. The mixture was then stirred at an external temperature of 85°C for 2 hours and 20 minutes.
Ethyl acetate and water were added to the reaction mixture and then this was passed through Celite and filtered. The organic extract from the filtrate was dried over anhydrous sodium sulfate. The desiccant was filtered off and the filtrate was concentrated under reduced pressure. The resultant residue was purified by silica gel column chromatography (ethyl acetate/hexane) to give 5.78 g of the title compound as a light yellow oil.
¹H-NMR (400MHz, CDCl₃) δ: 1.02 (s, 6H), 1.07 (s, 6H), 1.40 (s, 2H), 1.48 (s, 9H), 2.16 (d, J= 1.6Hz, 2H), 2.91 (t, J= 5.2Hz, 4H), 3.51 (t, J= 5.2Hz, 4H), 5.50 (t, J= 1.6Hz, 1H), 6.97 (dd, J= 8.0, 1.2Hz, 1H), 7.01 (ddd, J= 8.0, 8.0, 1.2Hz, 1H), 7.09 (dd, J= 8.0, 1.6Hz, 1H), 7.20 (ddd, J= 8.0,8.0, 1.6Hz, 1H).

### (Production Example 3A)

### 4,4-Diethylcyclohexanone

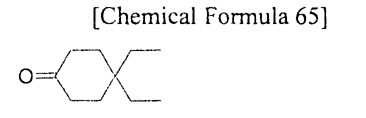

A mixture of 4,4-diethyl-2-cyclohexenone (1 g, 6.57 mmol) (Reference: Michael E. Flaugh, Thomas A. Crowell, and Diane S. Farlow, J. Org. Chem., 1980, 45, 5399.), 10% palladium on carbon (60 mg, wet) and ethyl acetate (15 mL) was stirred for 26 hours under a hydrogen atmosphere at atmospheric pressure and room temperature.
The reaction mixture was filtered and then the filtrate was concentrated under reduced pressure to give a crude product of the title compound (720 mg) as a brown oil.
¹H-NMR (400MHz, CDCl₃)
δ : 0.85 (t, J= 7.6Hz, 6H), 1.43 (q, J= 7.6Hz, 4H), 1.65 (dd, J= 7.2, 7.2Hz, 4H), 2.31 (dd, J= 7.2, 7.2Hz, 4H).

### (Production Example 3B)

### Trifluoromethanesulfonic acid 4.4-diethylcyclohex-1-enyl ester

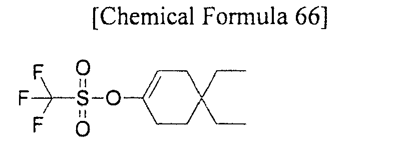

A solution of 4,4-diethylcyclohexanone (720 mg, 4.67 mmol) in anhydrous tetrahydrofuran (20 mL) was cooled to below -70°C in a dry ice-acetone bath under a nitrogen atmosphere, and then stirred. Lithium bis(trimethylsilyl)amide (1M solution in tetrahydrofuran, 5.6 mL, 5.6 mmol) was gradually added dropwise to this solution. After stirring for 60 minutes under the same conditions, N-phenyl bis(trifluoromethanesulfonimide) (1.75 g, 4.9 mmol) was added to the reaction mixture, and stirring was continued for 27 hours with slowly warming to room temperature.
Saturated aqueous ammonium chloride was added to the reaction mixture. Ethyl acetate and brine were then added to the reaction mixture and the organic layer was separated off. After washing the organic layer with diluted hydrochloric acid and saturated aqueous sodium hydrogencarbonate in that order, it was dried over anhydrous magnesium sulfate. The desiccant was filtered off and the filtrate was concentrated under reduced pressure. The resultant residue was purified by silica gel column chromatography (ethyl acetate/hexane) to give 710 mg of the title compound as a yellow oil.
¹H-NMR (400MHz, CDCl₃)
δ: 0.80 (t, J= 7.6Hz, 6H), 1.21-1.40 (m, 4H), 1.55 (t, J= 6.6Hz, 2H), 1.95 (dt, J= 4.0, 2.8Hz, 2H), 2.25-2.30 (m, 2H). 5.63-5.66 (m, 1H).

### (Production Example 3C)

### 2-(4,4-Diethylcyclohex-1-enyl)-4,4,5,5-tetramethyl-[1,3,2]dioxaborolane

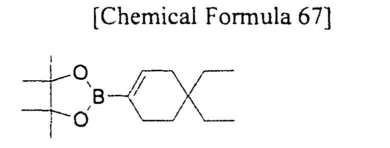

To a solution of trifluoromethanesulfonic acid 4,4-diethylcyclohex-1-enyl ester (5.11 g, 17.8 mmol) in dioxane (60 mL) were added bis(pinacolato)diboron (5.2 g, 20.5 mmol), [1,1'-bis(diphenylphosphino)ferrocene]dichloropalladium(II) dichloromethane complex (580 mg, 0.71 mmol) and potassium acetate (5.3 g, 53.5 mmol), and the mixture was stirred at an external temperature of 90°C for 4 hours.
The reaction mixture was air-cooled to room temperature, and insoluble matters were filtered off. Ethyl acetate and water were added to the resultant filtrate and the organic layer was separated off. The organic layer was washed with brine and then dried over anhydrous magnesium sulfate. The desiccant was filtered off and the filtrate was concentrated under reduced pressure. The resultant residue was purified by silica gel column chromatography (ethyl acetate/hexane) to give 4.16 g of the title compound as white crystals.
¹H-NMR (400MHz, CDCl₃)
δ : 0.76 (t, J= 7.6Hz, 6H), 1.13-1.37 (m, 18H), 1.84-1.86 (m, 2H), 2.05-2.10 (m, 2H), 6.48-6.50 (m, 1H).

### (Production Example 3D)

### 1-(4,4-Diethylcyclohex-1-enyl)-4-methoxy-2-nitrobenzene

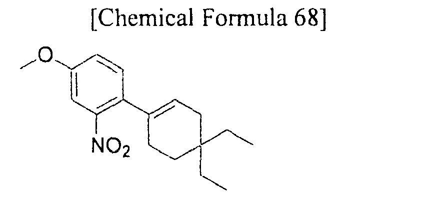

A mixture of 4-bromo-3-nitroanisole (2 g, 8.62 mmol), 2-(4,4-diethylcyclohex-1-enyl)-4,4,5,5-tetramethyl-[1,3,2]dioxaborolane (2.7 g, 10.3 mmol), tripotassium phosphate (2.7 g, 13.0 mmol) and 1,2-dimethoxyethane (20 mL) was stirred at room temperature under a nitrogen atmosphere, and then tetrakis(triphenylphosphine)palladium(0) (0.5 g, 0.43 mmol) was added. The mixture was then stirred at an external temperature of 80°C for 26 hours.
After cooling the reaction mixture, brine was added and the mixture was extracted with ethyl acetate. The organic layer was dried over anhydrous magnesium sulfate. The desiccant was filtered off and the filtrate was concentrated under reduced pressure. The resultant residue was purified by silica gel column chromatography (ethyl acetate/hexane) to give 2.4 g of the title compound as a light yellow oil.
¹H-NMR (400MHz, CDCl₃)
δ: 0.82 (t, J= 7.2Hz, 6H), 1.22-1.54 (m, 6H), 1.87-1.94 (m, 2H), 2.14-2.20 (m, 2H), 3.84 (s, 3H), 5.48-5.54 (m, 1H), 7.04 (dd, J= 8.4, 2.8Hz, 1H), 7.16 (d, J= 8.4Hz, 1H), 7.29 (d, J= 2.8 Hz, 1H).

### (Production Example 3E)

### 2-(4,4-Diethylcyclohex-1-enyl)-5-methoxyphenylamine

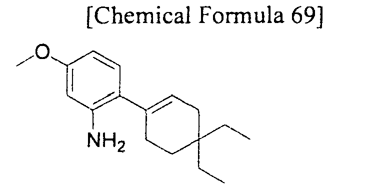

To a solution of 1-(4,4-diethylcyclohex-1-enyl)-4-methoxy-2-nitrobenzene (2.4 g, 8.3 mmol) in ethanol (20 mL) were added an aqueous solution (5 mL) of ammonium chloride (2.2 g, 41 mmol) and iron powder (1.2 g, 20.7 mmol), and the mixture was stirred at an external temperature of 90°C for 1 hour
The reaction mixture was passed through Celite for filtration, and then brine was added to the filtrate and the mixture was extracted with ethyl acetate. The organic layer was dried over anhydrous magnesium sulfate, the desiccant was filtered off, and then the filtrate was concentrated under reduced pressure to give 2.6 g of the title compound as a yellow oil.
¹H-NMR (400MHz, CDCl₃)
δ : 0.82 (t, J= 7.2 Hz, 6H), 1.21-1.56 (m, 6H), 1.92-1.96 (m, 2H), 2.16-2.22 (m, 2H), 3.75 (s, 3H), 5.61-5.65 (m, 1H), 6.24 (d, J= 2.8Hz, 1H), 6.29 (dd, J= 8.4, 2.8Hz, 1H), 6.87 (d, J= 8.4Hz, 1 H).
The 2H of NH₂ could not be identified.

### (Production Example 3F)

### 1-[2-(4,4-Diethylcyclohex-1-enyl)-5-methoxyphenyl]piperazine

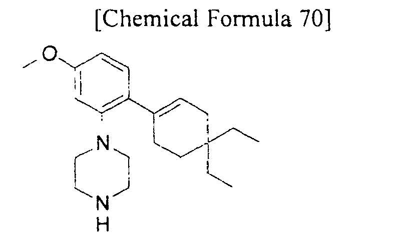

A solution of 2-(4,4-diethylcyclohex-1-enyl)-5-methoxyphenylamine (2.6 g, 10 mmol) and bis(2-chloroethyl)amine hydrochloride (2.2 g, 12 mmol) in 1,2-dichlorobenzene (10 mL) was stirred at an external temperature of 210°C. Nitrogen gas was blown into the reactor several times during the reaction to remove the excess hydrogen chloride gas in the reactor.
After I hour, the reaction mixture was cooled to room temperature, and then saturated aqueous sodium hydrogencarbonate was added and the mixture was extracted with ethyl acetate. The organic layer was dried over anhydrous magnesium sulfate. The desiccant was filtered off and the filtrate was concentrated under reduced pressure. The resultant residue was purified by NH silica gel column chromatography (ethyl acetate/hexane) to give 1.4 g of the title compound as a yellow oil.
¹H-NMR (400MHz, CDCl₃)
δ: 0.82 (t, J= 7.2Hz, 6H), 1.22-1.52 (m, 6H), 1.90-1.96 (m, 2H), 2.38-2.46 (m, 2H), 2.78-3.04 (m, 8H), 3.79 (s, 3H), 5.61-5.66 (m,1H), 6.50 (dd, J= 8.4, 2.8Hz, 1H), 6.52 (d, J= 2.8Hz,1H), 6.99 (d, J= 8.4Hz, 1H).
The 1H of NH could not be identified.
MS m/e (ESI) 329(MH⁺).

### (Production Example 4A)

### 4-Methoxypiperidine hydrochloride

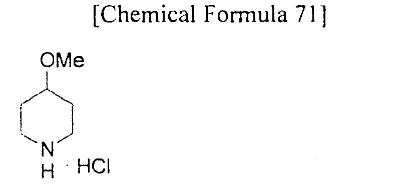

4-hydroxypiperidine-1-carboxylic acid t-butyl ester (25.5 g, 127 mmol) was added to a mixed solution of anhydrous tetrahydrofuran (100 mL) and dimethylformamide (40 mL). The solution was cooled to 0°C in an ice bath with stirring. Sodium hydride (60% dispersion in oil, 7.6 g, 190 mmol) was gradually added over 3 minutes. The reaction mixture was warmed to room temperature, stirred for 70 minutes, and cooled to 0°C again. A solution of methyl iodide (9.5 mL, 152 mmol) in anhydrous tetrahydrofuran (20 mL)-dimethylformamide (5 mL) was gradually added to the reaction mixture over 20 minutes. The ice bath was removed, and the reaction mixture was warmed to room temperature and stirred for 1 hour.
After the reaction, water and diethyl ether were added to the reaction mixture and the organic layer was separated off. The organic layer was washed 3 times with water, ant then once with brine, and dried over anhydrous sodium sulfate. The desiccant was filtered off and the filtrate was concentrated under reduced pressure. Ethyl acetate (200 mL) was added to the residue, and the mixture was cooled to 0°C and stirred. A 4N solution of hydrogen chloride in ethyl acetate (100 mL) was then gradually added over 10 minutes, and then the temperature was slowly raised to room temperature.
After stirring for 13 hours, the reaction mixture was concentrated under reduced pressure. The residue was dissolved in a small amount of dichloromethane. An excess of ethyl acetate was then added and the precipitated solid was filtered out and dried under reduced pressure to give 17.0 g of the title compound as colorless crystals.
¹H-NMR (400MHz, CDCl₃)
δ: 1.95-2.02 (m, 2H), 2.05-2.15 (m, 2H), 3.14-3.30 (m, 4H), 3.32 (s, 3H), 3.52-3.57 (m, 1H).
The 1H ofNH could not be identified.

### (Production Example 4B)

### 5-(4-Methoxypiperidin-1-yl)-2-nitrophenol

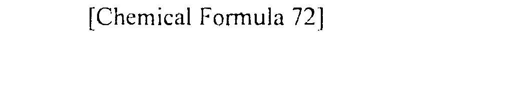

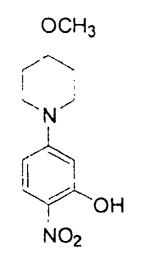

A mixture of 4-methoxypiperidine hydrochloride (9.10 g, 60.01 mmol), 5-fluoro-2-nitrophenol (6.91 g, 43.98 mmol) and dimethylformamide (12 mL) was stirred under a nitrogen atmosphere. Triethylamine (15.24 mL, 109.95 mmol) was added to the reaction mixture and the mixture was stirred at an external temperature of 80°C for 3 hours and 30 minutes.
After the reaction, saturated aqueous ammonium chloride and a mixed solvent of ethyl acetate-diethyl ether were added to the reaction mixture. The organic layer was separated off, and the aqueous layer was extracted with diethyl ether. The obtained organic layers were combined and dried over anhydrous sodium sulfate. The desiccant was filtered off and the filtrate was concentrated under reduced pressure. The resultant residue was purified by silica gel column chromatography (ethyl acetate/hexane) to give 37.36 g of the title compound as orange crystals.
¹H-NMR (400MHz, CDCl₃)
δ:1.60-1.68 (m, 2H), 1.83-1.90 (m, 2H), 3.26 (ddd, J= 13.2, 8.0, 3.6Hz, 2H), 3.32 (s, 3H), 3.42-3.47(m, 1H), 3.62 (ddd, J= 13.2, 7.6, 3.6Hz, 2H), 6.24 (d, J= 2.8Hz, 1H), 6.36 (dd, J= 10.0, 2.8Hz, 1H), 7.87 (d, J= 10.0Hz, 1H).
The 1H of OH could not be identified.

### (Production Example 4C)

### Trifluoromethanesulfonic acid 5-(4-methoxypiperidin-1-yl)-2-nitrophenyl ester

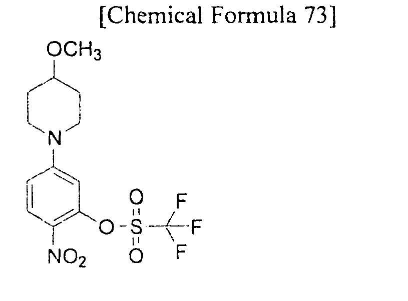

A mixture of 5-(4-methoxypiperidin-1-yl)-2-nitrophenol (2.35 g, 8.16 mmol), triethylamine (5.7 mL, 40.9 mmol) and dichloromethane (50 mL) was stirred while cooling in ice water bath, and then trifluoromethanesulfonic anhydride (2 mL, 12.24 mmol) was gradually added dropwise for 15 minutes, and the mixture was stirred for 10 minutes under the same conditions.
Saturated aqueous ammonium chloride was added to the reaction mixture, and then ethyl acetate and water were added and the organic layer was separated off. The organic layer was dried over anhydrous sodium sulfate. The desiccant was filtered off and the filtrate was concentrated under reduced pressure. The resultant residue was purified by silica gel column chromatography (ethyl acetate/hexane) to give 3.276 g of the title compound as an orange solid.
¹H-NMR (400MHz, CDCl₃)
δ:1.66-1.74 (m, 2H), 1.84-1.92 (m, 2H), 3.27 (ddd, J= 13.2, 7.6, 3.6Hz, 2H), 3.23 (s, 3H), 3.47 (m, 1H), 3.58 (ddd, J= 12, 8.0, 3.6Hz, 2H), 6.54 (d, J= 2.8Hz, 1H), 6.72 (dd, J= 9.6, 2.8Hz. 1H), 8.07 (d. J= 9.6Hz. 1H).

### (Production Example 4D)

### Trifluoromethanesulfonic acid 4-t-butylcyclohex-1-enyl ester

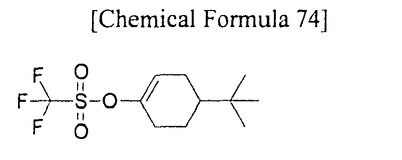

A solution of diisopropylamine (22 mL, 0.157 mol) in anhydrous tetrahydrofuran (500 mL) was cooled to below -70°C in a dry ice-acetone bath under a nitrogen atmosphere. n-Butyllithium (1.56 M solution in hexane, 100 mL, 0.156 mol) was slowly added dropwise to the stirred solution over 15 minutes. The reaction mixture was then warmed to -10°C and then cooled to below -70°C again. After stirring for 10 minutes, a solution of 4-t-butylcyclohexanone (20.05 g, 0.13 mol) in anhydrous tetrahydrofuran (100 mL) was gradually added dropwise to the reaction mixture for 15 minutes. After stirring for 30 minutes, a solution of N-phenyl bis(trifluoromethanesulfonimide) (51.09 g, 0.143 mol) in anhydrous tetrahydrofuran (200 mL) was gradually added dropwise to the reaction mixture over 15 minutes and the mixture was stirred for 30 minutes. The dry ice bath was then exchanged with an ice bath, and stirring was continued for 30 minutes and then for another 30 minutes at room temperature. Ethyl acetate and brine were added to the reaction mixture and the mixture was extracted with ethyl acetate. The collected organic layer was dried over anhydrous sodium sulfate. The desiccant was filtered off and the filtrate was concentrated under reduced pressure. The resultant residue was purified by silica gel column chromatography (ethyl acetate/hexane) to give 33.1 g of the title compound as a light yellow oil, in racemic form at the position of t-butyl.
¹H-NMR (400MHz, CDCl₃)
δ: 0.90 (s, 9H), 1.24-1.44 (m, 2H), 1.90-2.00 (m, 2H), 2.16-2.25 (m, 1H), 2.32-2.46 (m, 2H), 5.72-5.76 (m, 1H).

### (Production Example 4E)

### 2-(4-t-Butylcyclohex-1-enyl)-4,4,5,5-tetramethyl-[1,3,2]dioxaborolane

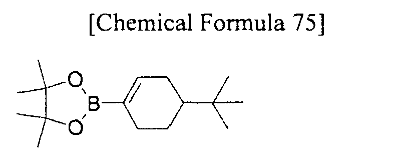

To trifluoromethanesulfonic acid 4-t-butylcyclohex-1-enyl ester (55.0 g, 192.1 mmol) were added bis(pinacolato)diboron (56.1 g, 220.9 mmol), [1,1'-bis(diphenylphosphino)ferrocene]dichloropalladium(II) dichloromethane complex (4.88 g, 5.98 mmol), potassium acetate (56.6 g, 576.3 mmol) and dioxane (400 mL), and the mixture was stirred at an external temperature of 80°C for 16 hours.
After the reaction, the reaction mixture was air-cooled to room temperature, ethyl acetate and water were added to the reaction mixture and the organic layer was separated off. The obtained organic layer was again washed with water, and dried over anhydrous magnesium sulfate. The desiccant was filtered off and the filtrate was concentrated under reduced pressure. The resultant residue was purified by silica gel column chromatography (ethyl acetate/hexane) to give 38.97 g of the title compound as a light yellow solid, in racemic form at the position of t-butyl.
¹H-NMR (400MHz, CDCl₃)
δ: 0.85 (s, 9H), 1.00-1.43 (m, 14H), 1.78-1.90 (m, 2H), 1.98-2.17 (m, 2H), 2.24-2.32 (m, 1H), 6.59 (dd, J= 2.0Hz, 1H).

### (Production Example 5)

### 4-[2-(3,3,5,5-Tetramethylcyclohexyl)phenyl]piperazine-1-carboxylic acid t-butyl ester

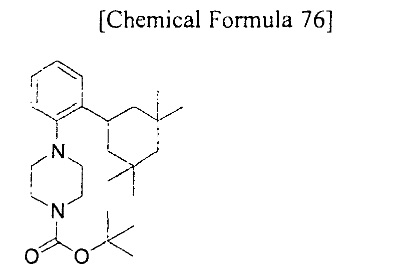

A mixture of 4-[2-(3,3,5,5-tetramethylcyclohex-1-enyl)phenyl]piperazine-1-carboxylic acid t-butyl ester (3.87 g, 9.71 mmol), 10% palladium on carbon (2.3 g, wet), methanol (25 mL) and tetrahydrofuran (25 mL) was stirred for 22 hours and 30 minutes under a hydrogen atmosphere at atmospheric pressure and room temperature.
After filtering the reaction mixture, the filtrate was concentrated. Ethyl acetate was added to the residue, the mixture was filtered again, and the filtrate was concentrated under reduced pressure to give a crude product of the title compound (3.83 g) as a light yellow oil.
¹H-NMR (400MHz, CDCl₃)
δ: 0.92 (s, 6H), 1.12 (s, 6H), 1.12-1.47 (m, 6H), 1.49 (s, 9H), 2.83 (brs, 4H), 3.59 (tt, J= 12.4, 2.8Hz, 1H), 7.07 (td, J= 7.6, 1.2Hz, 1H), 7.10 (dd, J= 7.6, 1.2Hz, 1H), 7.16 (td, J= 7.6, 2.0Hz, 1H), 7.24 (dd, J= 7.6, 2.0Hz, 1H).
The 4H of the piperazine ring could not be identified.
MS m/e (ESI) 401(MH⁺).

### (Production Example 6A)

### 4-Methoxy-2-nitro-1-(3,3,5,5-tetramethylcyclohex-1-enyl)benzene

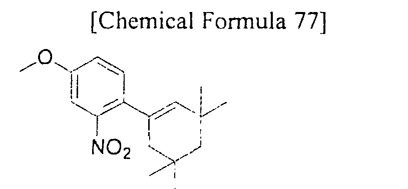

4,4,5,5-Tetramethyl-2-(3,3,5,5-tetramethylcyclohex-1-enyl)-[1,3,2]dioxaborolane (2.7 g, 10.3 mmol) was used instead of 2-(4,4-diethylcyclohex-1-enyl)-4,4,5,5-tetramethyl-[1,3,2]dioxaborolane for reaction in a manner similar to Production Example 3D, and treated in a similar manner, to give 2.5 g of the title compound as a yellow oil.

### (Production Example 6B)

### 5-Methoxy-2-(3,3,5,5-tetramethylcyclohex-1-enyl)phenylamine

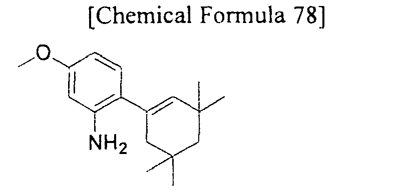

4-Methoxy-2-nitro-1-(3,3,5,5-tetramethylcyclohex-1-enyl)benzene (2.5 g, 8.6 mmol) was reacted and treated in a manner similar to Production Example 3E to give 2.2 g of the title compound as a yellow oil.

### (Production Example 6C)

### 1-[5-Methoxy-2-(3,3,5,5-tetramethylcyclohex-1-enyl)phenyl]piperazine

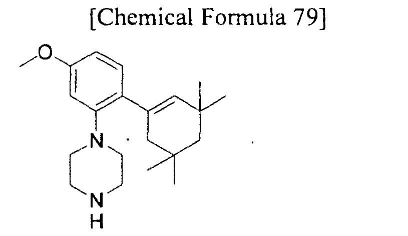

5-Methoxy-2-(3,3,5,5-tetramethylcyclohex-1-enyl)phenylamine (2.2 g, 8.6 mmol) was reacted and treated in a manner similar to Production Example 3F to give 2.0 g of the title compound as a yellow solid.

### (Production Example 7A)

### 4-[2-(4,4-Diethylcyclohex-1-enyl)phenyl]piperazine-1-carboxylic acid t-butyl ester

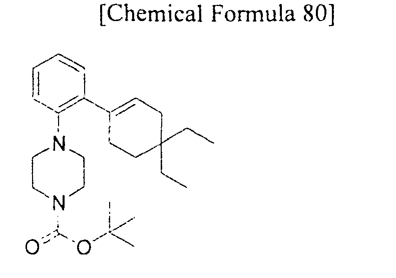

4-(2-Trifluoromethanesulfonyloxyphenyl)piperazine-1-carboxylic acid t-butyl ester (4.71 g, 11.5 mmol) was used as the starting material. 2-(4,4-Diethylcyclohex-1-enyl)-4,4,5,5-tetramethyl[1,3,2]dioxaborolane (3.7 g, 14.0 mmol) was used instead of 4,4,5,5-tetramethyl-2-(3,3,5,5-tetramethylcyclohex-1-enyl)[1,3,2]dioxaborolane for reaction in a manner similar to Production Example 2E and treated in a similar manner, to give 3.94 g of the title compound as a brown oil.

### (Production Example 7B)

### 4-[2-(4,4-Diethylcyclohexyl)phenyl]piperazine-1-carboxylic acid t-butyl ester

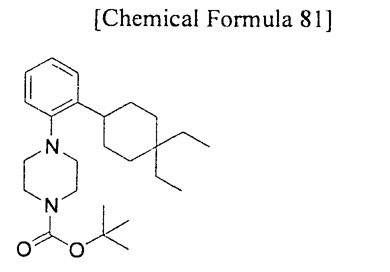

4-[2-(4,4-Diethylcyclohex-1-enyl)phenyl]piperazine-1-carboxylic acid t-butyl ester (3.96 g, 9.93 mmol) was used as the starting material. Methanol was used as the solvent instead of a mixed solvent of tetrahydrofuran-methanol, for reaction in a manner similar to Production Example 5 and treated in a similar manner. The resultant crude product was purified by silica gel column chromatography (ethyl acetate/hexane) to give 3.79 g of the title compound as a yellow oil.

### (Production Example 7C)

### 4-[4-Bromo-2-(4,4-diethylcyclohexyl)phenyl]piperazine-1-carboxylic acid t-butyl ester

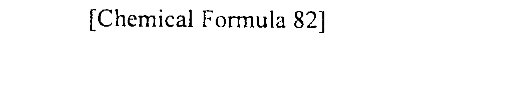

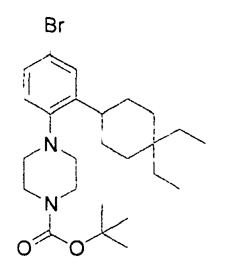

4-[2-(4,4-Diethylcyclohexyl)phenyl]piperazine-1-carboxylic acid t-butyl ester (3.79 g, 9.46 mmol) was used as the starting material. This was reacted and treated in a manner similar to Production Example 1F, to give 2.75 g of the title compound as a white solid.

### (Production Example 8A)

### 1-[4-Bromo-2-(4,4-dimethylcyclohexyl)phenyl]piperazine

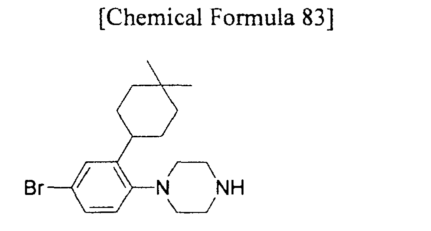

A mixture of 4-[4-bromo-2-(4,4-dimethylcyclohexyl)phenyl]piperazine-1-carboxylic acid t-butyl ester (1.5 g, 3.32 mmol), trifluoroacetic acid (3 mL, 38.7 mmol) and dichloromethane (6 mL) was stirred for 2 hours and 30 minutes at room temperature.
Saturated aqueous sodium carbonate was added to the reaction mixture and extraction was performed with ethyl acetate. The separated organic layer was washed with water and brine in that order and then dried over anhydrous sodium sulfate. The desiccant was filtered off and the filtrate was concentrated under reduced pressure. The residue was dried under reduced pressure to give 1.21 g of a crude product of the title compound as a brown oil.

### (Production Example 8B)

### 1-[4-Bromo-2-(4,4-dimethylcyclohexyl)phenyl]-4-butylpiperazine

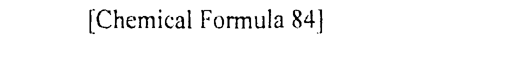

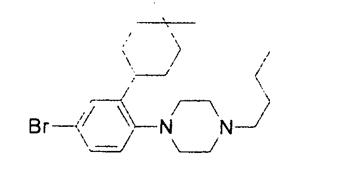

To a mixture of the crude product of 1-[4-bromo-2-(4,4-dimethylcyclohexyl)phenyl]piperazine (1.21 g), butyraldehyde (0.35 mL, 3.98 mmol), acetic acid (0.1 mL, 3.32 mmol) and tetrahydrofuran (8 mL) was added sodium triacetoxyborohydride (1.1 g, 4.98 mmol), followed by stirring for 2 hours and 10 minutes at room temperature.
Saturated aqueous sodium hydrogencarbonate was added to the reaction mixture and extraction was performed with ethyl acetate. The separated organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 901 mg of the title compound as a yellow oil.
¹H-NMR (400MHz, CDCl₃)
δ: 0.94 (t, J= 7.2Hz, 3H), 0.97 (s, 3H), 1.01 (s, 3H), 1.24-1.60 (m, 12H), 2.38-2.44 (m, 2H), 2.59 (brs, 4H), 2.82-2.97 (m, 5H), 6.97 (d, J= 8.8Hz, 1H), 7.24 (dd, J= 8.8, 2.4Hz, 1H), 7.33 (d, J= 2.4Hz, 1H).

### (Production Example 9A)

### 3-Piperazin-1-yl-4-(3,3,5,5-tetramethylcyclohexyl)phenol

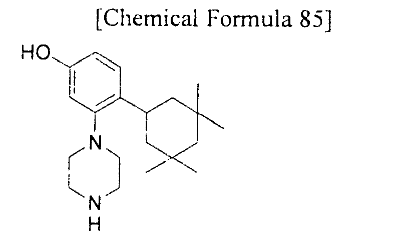

1-[5-Methoxy-2-(3,3,5,5-tetramethylcyclohex-1-enyl)phenyl]piperazine was used as the starting material for N-butyloxycarbonylation and hydrogenation by conventional methods. A mixture of 4-[5-methoxy-2-[5-methoxy-2-(3,3,5,5-tetramethylcyclohexyl)phenyl]piperazine-1-carboxylic acid t-butyl ester (3,41 g, 7.92 mmol) obtained by the reaction, acetic acid (18 mL, 314 mmol) and 48% hydrobromic acid (36 mL, 318 mmol) was stirred for 8 hours and 20 minutes at an external temperature of 130°C.
The reaction mixture was cooled in an ice water bath and stirred, and then 5N aqueous sodium hydroxide was added dropwise thereto to adjust pH of the mixture to 8-9. The produced solid was filtered to give 2.98 g of a crude product of the title compound as a light red solid.
¹H-NMR (400MHz, CD₃OD)
δ: 0.94 (s, 6H), 1.54 (s, 6H), 1.17-1.40 (m, 6H), 3.05 (t, J= 4.8Hz, 4H), 3.42 (tt, J= 12.8, 2.8Hz, 1H), 6.55-6.61 (m, 2H), 7.05 (d, J= 8.0Hz, 1H).
The 4H of the piperazine ring and the 2H of NH and OH could not be identified.

### (Production Example 10A)

### 4-Methoxy-1-nitro-2-(3,3,5,5-tetramethylcyclohex-1-enyl)benzene

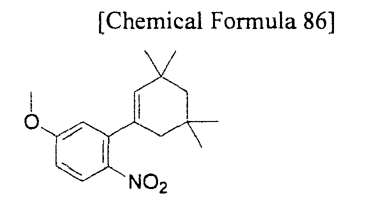

To a solution of 3-iodo-4-nitroanisole (4.21 g, 15.1 mmol) in 1,2-dimethoxyethane (50 mL) were added 4,4,5,5-tetramethyl-2-(3,3,5,5-tetramethylcyclohex-1-enyl)[1,3,2]dioxaborolane (4.78 g, 18.1 mmol), tripotassium phosphate (4.81 g, 22.7 mmol) and water (3 mL). Then, tetrakis(triphenylphosphine)palladium(0) (870 mg, 0.755 mmol) was added to the mixture while stirring at room temperature under a nitrogen atmosphere. The mixture was then further stirred for 13 hours at an external temperature of 70°C. To the reaction mixture were added tetrakis(triphenylphosphine)palladium(0) (870 mg, 0.755 mmol) and water (3 mL), followed by stirring for 26 hours at an external temperature of 100°C.
The reaction mixture was cooled, and then ethyl acetate was added and the mixture was filtered through Celite. The filtrate was concentrated to give a residue, which was subjected to extraction with ethyl acetate, and the organic layer was washed with brine. The organic layer was dried over anhydrous magnesium sulfate and then the desiccant was filtered off and the filtrate was concentrated under reduced pressure. The resultant residue was purified by silica gel column chromatography (ethyl acetate/heptane) to give 1.5 g of the title compound as a yellow solid.
¹H-NMR (400MHz, CDCl₃)
δ:1.05 (s, 6H), 1.07 (s, 6H), 1.41 (s, 2H), 1.99 (d, J= 1.6Hz, 2H), 3.88 (s, 3H), 5.35 (m, 1H), 6.68 (d, J= 2.4Hz, 1H), 6.81 (dd, J= 9.2, 2.4Hz, 1H), 7.91 (d, J= 9.2Hz,
1H).

### (Production Example 10B)

### 4-Methoxy-2-(3,3,5,5-tetramethylcyclohexyl)phenylamine

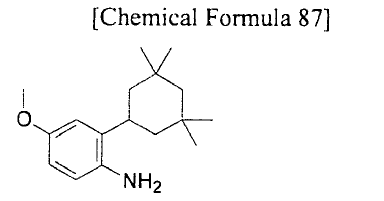

A mixture of 4-methoxy-1-nitro-2-(3,3,5,5-tetramethylcyclohex-1-enyl)benzene (1.0 g, 3.46 mmol), 10% palladium on carbon (500 mg, wet), methanol (8 mL) and tetrahydrofuran (2 mL) was stirred overnight at room temperature and atmospheric pressure under a hydrogen atmosphere.
The mixture was filtered through Celite to remove the catalyst, and the filtrate was concentrated. A crude product of the title compound was obtained as a brown oil. The crude product was used without purification for the following reaction.

### (Production Example 10C)

### 1-[4-Methoxy-2-(3,3,5,5-tetramethylcyclohexyl)phenyl]piperazine

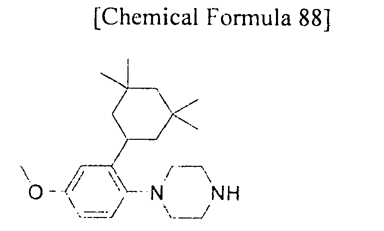

A mixture of the crude product of 4-methoxy-2-(3,3,5,5-tetramethylcyclohexyl)phenylamine, bis(2-chloroethyl)amine hydrochloride (770 mg, 4.33 mmol) and 1.2-dichlorobenzene (10 mL) was stirred for 2 hours at an external temperature of 220°C. During the reaction, the excess hydrogen chloride gas in the reactor was removed several times with nitrogen gas. Bis(2-chloroethyl)amine hydrochloride (180 mg, 1.01 mmol) was further added and the mixture was stirred for 1 hour under the same conditions.
The reaction mixture was cooled to room temperature, saturated aqueous sodium hydrogencarbonate was added and extraction was performed with chloroform. The aqueous layer was again extracted with chloroform, and the organic layers were combined, washed with brine and then dried over anhydrous magnesium sulfate. The desiccant was filtered off and the filtrate was concentrated under reduced pressure. The resultant residue was purified by NH silica gel column chromatography (ethyl acetate/heptane) to give 660 mg of the title compound as a brown solid.
¹H-NMR (400MHz, CDCl₃)
δ:0.92 (s, 6H), 1.12 (s, 6H), 1.15-1.34 (m, 4H), 1.42-1.45 (m, 2H), 2.78-2.81 (m, 4H), 2.99-3.02 (m, 4H), 3.63 (tt, J= 13, 2.8Hz, 1H), 3.78 (s, 3H), 6.69 (dd, J= 8.8, 2.8Hz, 1H), 6.77 (d, J= 2.8Hz, 1H), 7.07 (d, J= 8.8Hz, 1H).

### (Example 1)

### 1-[2-(4,4-Diethylcyclohexyl)-4-(4-methoxymethylpiperidin-1-yl)phenyl]-4-proylpiperazine hydrochloride

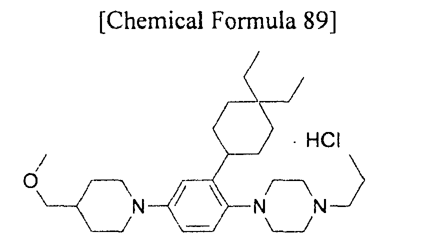

### (1a)

### 4-Methoxymethylpiperidine hydrochloride

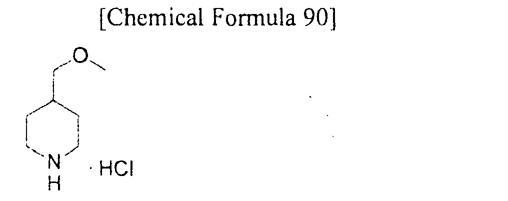

After adding a mixed solvent of anhydrous tetrahydrofuran (6.2 mL) and dimethylformamide (2.5 mL) to sodium hydride (60% dispersion in oil, 83.6 mg, 2.09 mmol), the mixture was cooled to 0°C in an ice bath under a nitrogen atmosphere and stirred for 20 minutes. Next, 4-hydroxymethylpiperidine-1-carboxylic acid t-butyl ester (300 mg, 1.39 mmol) was added to the reaction mixture. The reaction mixture was warmed to room temperature, stirred for 65 minutes, and then cooled again to 0°C. Methyl iodide (0.13 mL, 2.09 mmol) was then added to the reaction mixture. Next, the reaction mixture was warmed to room temperature and stirred for 20 hours and 25 minutes.
After the reaction, to the reaction mixture were added ice and diethyl ether, then sodium chloride, and extraction was performed with diethyl ether. The organic layer was dried over anhydrous sodium sulfate. The desiccant was filtered off and the filtrate was concentrated under reduced pressure to give a crude product of 4-methoxymethylpiperidine-1-carboxylic acid t-butyl ester.
Ethyl acetate (2 mL) was added to the crude product, and the mixture was cooled to 0°C and stirred. Next, a 4N solution of hydrogen chloride in ethyl acetate (10.4 mL, 41.7 mmol) was gradually added and the mixture was warmed to room temperature. After stirring the reaction mixture for 4 hours, it was concentrated under reduced pressure. The residue was dissolved in a small amount of dichloromethane. An excess of ethyl acetate was further added, and the precipitated solid was filtered and dried under reduced pressure to give 206 mg of the title compound as colorless crystals.
¹H-NMR (400MHz, CDCl₃)
δ: 1.61-1.76 (m, 2H), 1.80-2.02 (m, 3H), 2.79-2.97 (m, 2H), 3.26 (d, J= 6.0Hz, 2H), 3.33 (s,3H), 3.42-3.60 (m, 2H).
The 1H of NH could not be identified.

### (1b)

### 1-[4-Bromo-2-(4,4-diethylcyclohexyl)phenyl]piperazine

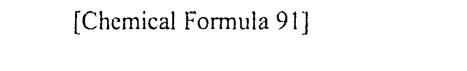

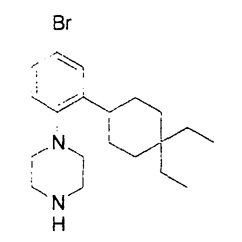

4-[4-Bromo-2-(4,4-diethylcyclohexyl)phenyl]piperazine-1-carboxylic acid t-butyl ester (221 mg, 0.461 mmol) produced in Production Example 7C was dissolved in a mixed solvent of dichloromethane (3 mL)-water (0.3 mL). To the reaction mixture was added dropwise a 4N solution of hydrogen chloride in ethyl acetate (0.710 mL, 9.22 mmol), followed by stirring for 5 hours and 30 minutes under the same conditions.
After the reaction, aqueous potassium carbonate was added to the reaction mixture to make the reaction mixture basic. The reaction mixture was extracted 3 times with ethyl acetate. The collected organic layer was dried over anhydrous sodium sulfate. The desiccant was filtered off and the filtrate was concentrated under reduced pressure. The resultant residue was purified by NH silica gel column chromatography (ethyl acetate/heptane) to give 153 mg of the title compound as a light yellow oil.
¹H-NMR (400MHz, CDCl₃)
δ: 0.79 (t, J= 7.6Hz, 3H), 0.81 (t, J= 7.6Hz, 3H), 1.14-1.28 (m, 4H), 1.45-1.65 (m, 8H), 2.78-2.80 (m, 4H), 2.83-3.03 (m, 5H), 6.96 (d, J= 8.4Hz, 1H). 7.25 (dd, J= 8.4, 2.4Hz, 1H), 7.33 (d, J= 2.4Hz, 1H).
The 1H ofNH could not be identified.

### (1c)

### 1-[4-Bromo-2-(4,4-diethylcyclohexyl)phenyl]-4-propylpiperazine

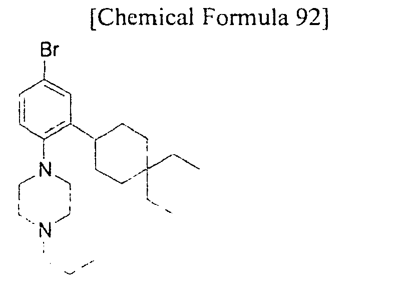

To a solution of 1-[4-bromo-2-(4,4-diethylcyclohexyl)phenyl]piperazine (153 mg, 0.403 mmol) in tetrahydrofuran (5 mL) was added propionaldehyde (0.038 mL, 0.524 mmol), sodium triacetoxyborohydride (111 mg, 0.524 mmol) and acetic acid (0.0438 mL, 0.766 mmol), followed by stirring at room temperature for 13 hours.
After the reaction, saturated aqueous sodium hydrogencarbonate was added to the reaction mixture and extraction was performed 3 times with ethyl acetate. The collected organic layer was dried over anhydrous sodium sulfate. The desiccant was filtered off and the filtrate was concentrated under reduced pressure. The resultant residue was purified by NH silica gel column chromatography (ethyl acetate/heptane) to give 155 mg of the title compound as a white oil.
¹H-NMR (400MHz, CDCl₃)
δ: 0.79 (t, J= 7.6Hz, 3H), 0.81 (t, J= 7.6Hz, 3H), 0.93 (t, J= 7.6Hz, 3H), 1.13-1.28 (m, 4H), 1.43-1.64 (m, 10H), 2.35-2.40 (m, 2H), 2.59 (brs, 4H), 2.84-2.94 (m, 5H), 6.96 (d, J= 8.4Hz, 1H), 7.22 (dd, J= 8.4, 2.4Hz, 1H), 7.31 (d, J= 2.4Hz, 1H).

### (1d)

### 1-[2-(4,4-Diethylcyclohexyl)-4-(4-methoxymethylpiperidin-1-yl)phenyl]-4-propylpiperazine hydrochloride

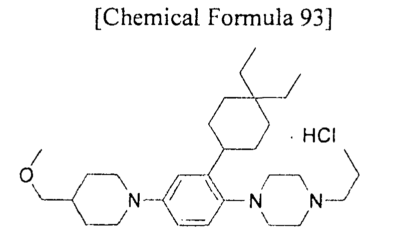

A mixture of 1-[4-bromo-2-(4,4-diethylcyclohexyl)phenyl]-4-propylpiperazine (155 mg, 0.368 mmol), 4-methoxymethylpiperidine hydrochloride (73.2 mg, 0.442 mmol), sodium t-butoxide (184 mg, 1.91 mmol), palladium(II) acetate (16.5 mg, 0.0736 mmol), tri-t-butylphosphonium tetrafluoroborate (64.1 mg, 0.221 mmol) and xylene (4 mL) was stirred at an external temperature of 100°C for 19 hours and 30 minutes under a nitrogen atmosphere.
After air-cooling the reaction mixture to room temperature, the reaction mixture was concentrated under reduced pressure. The resultant residue was purified by silica gel column chromatography (ethyl acetate/heptane) to give 43 mg of 1-[2-(4,4-diethylcyclohexyl)-4-(4-methoxymethylpiperidin-1-yl)phenyl]-4-propylpiperazine as an orange solid.
¹H-NMR (400MHz, CDCl₃)
δ: 0.79 (t, J= 8.0 Hz, 3H), 0.81 (t, J= 8.0Hz, 3H), 0.93 (t, J= 7.6Hz, 3H), 1.14-1.88 (m, 19H), 2.35-2.39 (m, 2H), 2.57 (brs, 4H), 2.65 (ddd, J= 12.0, 12.0, 2.4Hz, 2H), 2.82-2.88 (m, 4H), 2.90-2.98 (m, 1H), 3.27 (d, J= 6.8Hz, 2H), 3.36 (s, 3H), 3.56-3.62 (m, 2H), 6.73 (dd, J= 8.4, 2.8Hz, 1H), 6.84 (d, J= 2.8Hz, 1H), 7.06 (d, J= 8.4Hz, 1H).
This compound was dissolved in dichloromethane (1 mL), and a 4N solution of hydrogen chloride in ethyl acetate (0.0458 mL, 0.183 mmol) was added. The solution was concentrated, diethyl ether was added to the resultant residue, and the resulting precipitate was triturated by sonication. After filtering, drying was performed under reduced pressure to give 47 mg of the title compound as a light brown solid.
MS m/e (ESI) 470(MH⁺).

### (Example 2)

### [4-(4-Butylpiperazin-1-yl)-3-(4,4-dimethylcyclohexyl)phenyl]bis(2-methoxyethyl)amine hydrochloride

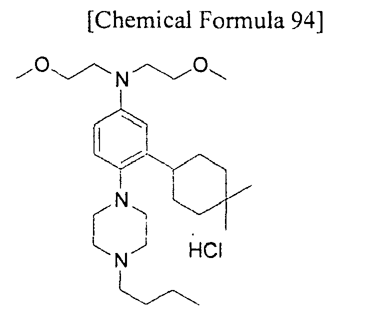

A mixture of 1-[4-bromo-2-(4,4-dimethylcyclohexyl)phenyl]-4-butylpiperazine (50 mg, 0.123 mmol) produced in Production Example 8B, bis(2-methoxyethyl)amine (25 mg, 0.185 mmol), sodium t-butoxide (30 mg, 0.308 mmol), palladium(II) acetate (3 mg, 0.0123 mmol), tri-t-butylphosphonium tetrafluoroborate (11 mg, 0.0369 mmol) and xylene (1 mL) was stirred at an external temperature of 100°C for 1 hour.
The reaction mixture was purified by NH silica gel column chromatography (ethyl acetate/hexane) to give 34 mg of [4-(4-butylpiperazin-1-yl)-3-(4,4-dimethylcyclohexyl)phenyl]bis(2-methoxyethyl)amine as a light yellow oil. ¹H-NMR (400MHz, CDCl₃)
δ: 0.94 (t, J= 7.2Hz, 3H), 0.97 (s, 3H), 1.00 (s, 3H), 1.30-1.70 (m, 12H), 2.38-2.42 (m, 2H), 2.58 (brs, 4H), 2.34-2.38 (m, 2H), 2.84 (brs, 4H), 2.90-3.00 (m, 1H), 3.36 (s, 6H), 3.50-3.60 (m, 8H), 6.52 (dd, J= 8.8, 3.2Hz, 1H), 6.63 (d, J= 3.2Hz, 1H), 7.05 (d, J= 8.8Hz, 1H).
This compound was dissolved in ethyl acetate, and a 4N solution of hydrogen chloride in ethyl acetate was added. The solution was concentrated under reduced pressure. Hexane was added to the residue, and the resulting precipitate was triturated by sonication. After removing the supernatant, the precipitate was dried to give 33 mg of the title compound as a light yellow solid.
MS m/e (ESI) 460(MH⁺).

### (Example 3)

### 1-{4-[4-(4-Butylpiperazin-1-yl)-3-(4,4-dimethylcyclohexyl)phenyl]piperazin-1-yl}ethanone hydrochloride

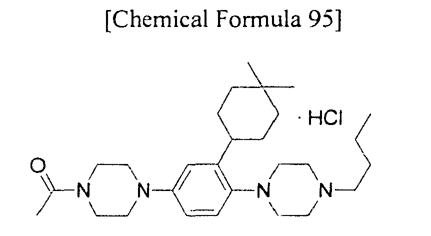

A mixture of the 1-[4-bromo-2-(4,4-dimethylcyclohexyl)phenyl]-4-butylpiperazine (50 mg, 0.123 mmol) produced in Production Example 8B, 1-acetylpiperazine (24 mg, 0.185 mmol), sodium t-butoxide (30 mg, 0.308 mmol), palladium(II) acetate (3 mg, 0.0123 mmol), tri-t-butylphosphonium tetrafluoroborate (11 mg, 0.0369 mmol) and xylene (1 mL) was stirred at an external temperature of 100°C for 1 hour.
The reaction mixture was purified by NH silica gel column chromatography (ethyl acetate/hexane) to give 32 mg of 1-{4-[4-(4-butylpiperazin-1-yl)-3-(4,4-dimethylcyclohexyl)phenyl]piperazin-1-yl}ethanone as colorless crystals.
¹H-NMR (400MHz, CDCl₃)
δ: 0.94 (t, J= 7.6Hz, 3H), 0.97 (s, 3H), 1.02 (s, 3H), 1.28-1.65 (m, 12H), 2.14 (s. 3H), 2.38-2.43 (m, 2H), 2.59 (brs, 4H), 2.78-2.91 (m, 1H), 2.92-3.01 (m, 1H), 3.09 (t, J= 5.2Hz, 2H), 3.12 (t, J= 5.2Hz, 2H), 3.61 (t, J= 5.2Hz, 2H), 3.77 (t, J= 5.2Hz, 2H), 6.72 (dd, J= 8.4, 2.8Hz, 1H), 6.85 (d, J= 2.8Hz, 1H), 7.08 (d, J= 8.4Hz, 1H).
This compound was dissolved in ethyl acetate, and a 4N solution of hydrogen chloride in ethyl acetate was added. The precipitated solid was filtered to give 31 mg of the title compound as a colorless solid.
MS m/e (ESI) 455(MH⁺).

### (Example 4)

### [4-(4-t-Butylcyclohex-1-enyl)-3-(4-butylpiperazin-1-yl)phenyl]-methyl-(tetrahydropyran-4-yl)amine hydrochloride

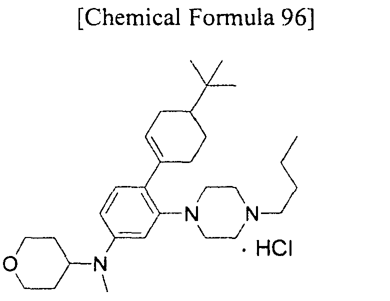

### (4a)

### 1-Butyl-4-(2-methoxy-5-nitrophenyl)piperazine

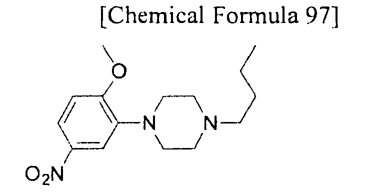

To a solution of 1-(2-methoxy-5-nitrophenyl)piperazine (3.08 g, 13 mmol) and butyraldehyde (1.4 mL, 15.73 mmol) in tetrahydrofuran (40 mL) were added sodium triacetoxyborohydride (3.3 g, 15.57 mmol) and acetic acid (0.75 mL, 13.09 mmol) in that order, followed by stirring at room temperature.
After stirring for 30 minutes, ethyl acetate, saturated aqueous sodium hydrogencarbonate and water were added to the reaction mixture and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 3.88 g of the title compound as a yellow oil.
¹H-NMR (400MHz, CDCl₃)
δ: 0.94 (t, J= 7.2Hz, 3H), 1.30-1.42 (m, 2H), 1.48-1.56 (m, 2H), 2.42 (t, J= 7.6Hz, 2H), 2.66 (brs, 4H), 3.15 (brs, 4H), 3.97 (s, 3H), 6.87 (d, J= 9.2Hz, 1H), 7.78 (d, J= 2.4Hz, 1H), 7.93 (dd, J= 9.2, 2.4Hz, 1H).

### (4b)

### 2-(4-Butylpiperazin-1-yl)-4-nitrophenol

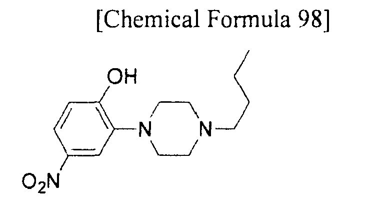

A mixture of 1-butyl-4-(2-methoxy-5-nitrophenyl)piperazine (3.61 g, 12.3 mmol) and 48% hydrobromic acid (50 mL) was heated to reflux. After 134 hours and 30 minutes, the reaction mixture was poured into ice water and made alkaline with 5N aqueous sodium hydroxide.
Next, saturated aqueous ammonium chloride was added to the mixed solution to make it weakly acidic and extraction was performed with a mixed solvent of ethyl acetate and tetrahydrofuran. The collected organic layer was washed with brine and then dried over anhydrous sodium sulfate. The desiccant was filtered off, and the filtrate was concentrated under reduced pressure to give 2.58 g of a crude product of the title compound as a reddish-brown oil. This was used without purification for the following reaction.

### (4c)

### Trifluoromethanesulfonic acid 2-(4-butylpiperazin-1-yl)-4-nitrophenyl ester

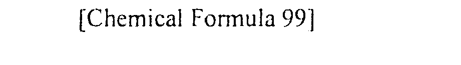

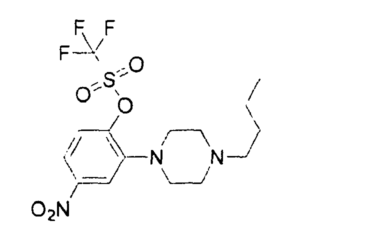

A mixture of a crude product of 2-(4-butylpiperazin-1-yl)-4-nitrophenol (2.58 g), triethylamine (6.5 mL, 46.64 mmol) and anhydrous dichloromethane (50 mL) was cooled under a nitrogen atmosphere in an ice-methanol bath. Trifluoromethanesulfonic anhydride (2.3 mL, 13.67 mmol) was gradually added to the solution for 15 minutes.
After stirring for 20 minutes, ethyl acetate, saturated aqueous ammonium chloride and water were added to the reaction mixture and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 3.01 g of the title compound as a yellow oil.
¹H-NMR (400MHz, CDCl₃)
δ: 0.94 (t, J= 7.2Hz, 3H), 1.30-1.42 (m, 2H), 1.46-1.56 (m, 2H), 2.42 (t, J= 7.6Hz, 2H), 2.66 (brs, 4H), 3.14 (t, J= 4.8Hz, 4H), 7.32 (d. J= 8.8Hz, 1H), 7.94 (dd, J= 8.8, 2.8Hz, 1H), 7.98 (d, J= 2.8Hz, 1H).

### (4d)

### 1-Butyl-4-[2-(4-t-butylcyclohex-1-enyl)-5-nitrophenyl]piperazine

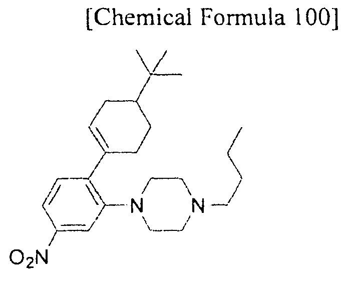

A mixture of 2-(4-t-butylcyclohex-1-enyl)-4,4,5,5-tetramethyl-[1.3.2]dioxaborolane (2.9 g, 10.98 mmol) produced in Production Example 4E, trifluoromethanesulfonic acid 2-(4-butylpiperazin-1-yl)-4-nitrophenyl ester (3 g, 7.29 mmol), tripotassium phosphate (1.55 g, 7.30 mmol), 1,2-dimethoxyethane (40 mL) and water (2 mL) was stirred at room temperature under a nitrogen atmosphere. Tetrakis(triphenylphosphine)palladium(0) (840 mg, 0.73 mmol) was added to the mixture. Next, the mixture was stirred at an external temperature of 85°C for 3 hours.
Ethyl acetate was added to the reaction mixture, which was then filtered. Water was added to the filtrate and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 2.88 g of the title compound as a reddish-yellow oil, in racemic form at the position of t-butyl.
¹H-NMR (400MHz, CDCl₃)
δ: 0.93 (s, 9H), 0.94 (t, J= 7.2Hz, 3H), 1.22-1.40 (m, 4H), 1.48-1.56 (m, 2H), 1.92-2.02 (m, 2H), 2.20-2.42 (m, 4H), 2.52-2.68 (m, 5H), 3.02-3.16 (m, 4H), 5.88 (t, J= 2.8Hz, 1H), 7.18 (dd, J= 8.4Hz, 1H), 7.75 (d, J = 2.4 Hz, 1H), 7.78 (dd, J= 8.4, 2.4Hz, 1H).

### (4e)

### 4-(4-t-Butylcyclohex-1-enyl)-3-(4-butylpiperazin-1-yl)phenylamine

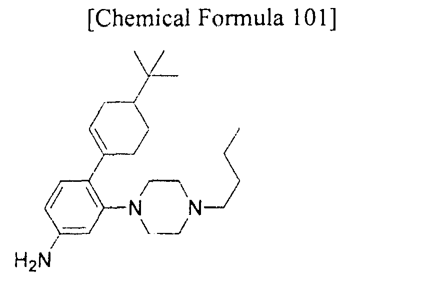

To a solution of 1-butyl-4-[2-(4-t-butylcyclohex-1-enyl)-5-nitrophenyl]piperazine (1.2 g, 3 mmol) in ethanol (18 mL) were added an aqueous solution (6 mL) of ammonium chloride (54 mg, 1 mmol) and iron powder (590 mg, 10.56 mmol), followed by stirring at an external temperature of 90°C for 3 hours.
The reaction mixture was filtered, the insoluble matters were washed with tetrahydrofuran and then the filtrate was concentrated under reduced pressure. The resultant residue was purified by NH silica gel column chromatography (ethyl acetate/hexane) to give 1.04 g of the title compound as light brown crystals, in racemic form at the position of t-butyl.
¹H-NMR (400MHz, CDCl₃)
δ: 0.91 (s, 9H), 0.94 (t, J= 7.2Hz, 3H), 1.15-1.40 (m, 4H), 1.46-1.55 (m, 2H), 1.86-1.97 (m, 2H), 2.11-2.40 (m, 4H), 2.53 (brs, 4H), 2.66-2.75 (m, 1H), 2.96 (brs, 2H), 3.07 (brs, 2H), 3.57(brs, 2H), 5.68 (t, J= 2.8Hz, 1H), 6.26-6.32 (m, 2H), 6.86 (d, J= 8.4Hz, 1H).

### (4f)

### [4-(4-t-Butylcyclohex-1-enyl)-3-(4-butylpiperazin-1-yl)phenyl](tetrahydropyran-4-yl)amine

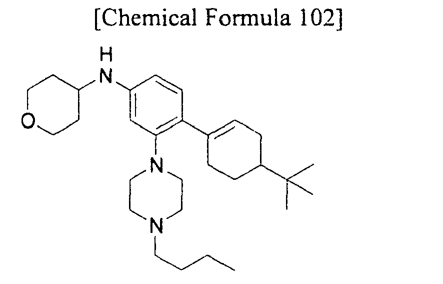

To a mixture of 4-(4-t-butylcyclohex-1-enyl)-3-(4-butylpiperazin-1-yl)phenylamine (80 mg, 0.216 mmol), tetrahydropyran-4-one (0.022 mL, 0.238 mmol), acetic acid (13 mg, 0.216 mmol) and 1,2-dichloroethane (1 mL) was added sodium triacetoxyborohydride (69 mg, 0.325 mmol), followed by stirring at room temperature for 15 hours and 30 minutes.
Saturated aqueous sodium hydrogencarbonate was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 98 mg of the title compound as yellow crystals, in racemic form at the position of t-butyl.
¹H-NMR (400MHz, CDCl₃)
δ: 0.91 (s, 9H), 0.94 (t, J= 7.6Hz, 3H), 1.15-1.60 (m, 10H), 1.87-1.98 (m, 2H), 1.98-2.05 (m, 1H), 2.11-2.43 (m, 4H), 2.54 (brs, 4H), 2.66-2.76 (m, 1H), 2.96 (brs. 2H), 3.07 (brs, 2H), 3.40-3.50 (m, 1H), 3.51 (ddd, J= 11.6. 11.6. 2.0Hz, 2H). 3.90 (ddd. J= 11.6. 3.2, 3.2Hz, 2H). 5.66-5.70 (m. J= 2.4Hz, 1H), 6.15-6.25 (m, 2H). 6.89 (d, J= 7.2Hz, 1 H).

### (4g)

### [4-(4-t-Butylcyclohex-1-enyl)-3-(4-butylpiperazin-1-yl)phenyl]methyl(tetrahydropyran-4-yl)amine hydrochloride

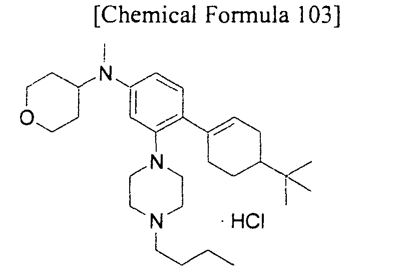

To a mixture of [4-(4-t-butylcyclohex-1-enyl)-3-(4-butylpiperazin-1-yl)phenyl](tetrahydropyran-4-yl)amine (15 mg, 0.0331 mmol), 36% formaldehyde (3.2 mg, 0.0397 mmol), acetic acid (2.9 mg, 0.0331 mmol) and 1,2-dichloroethane (1 mL) was added sodium triacetoxyborohydride (11 mg, 0.0497 mmol), followed by stirring at room temperature for 15 hours and 40 minutes.
Saturated aqueous sodium hydrogencarbonate was added to the reaction mixture and extraction was performed with ethyl acetate. The separated organic layer was washed with water and then filtered through Celite. The filtrate was concentrated under reduced pressure. The resultant residue was purified by NH silica gel column chromatography (ethyl acetate/hexane) to give [4-(4-t-butylcyclohex-1-enyl)-3-(4-butylpiperazin-1-yl)phenyl]methyl(tetrahydropyran-4-yl)amine, in racemic form at the position of t-butyl.
This compound was dissolved in ethyl acetate, and a 4N solution of hydrogen chloride in ethyl acetate was added. The solution was concentrated under reduced pressure. Diethyl ether was added to the residue, and the resulting precipitate was triturated by sonication. After removing the supernatant, the precipitate was dried to give 15.3 mg of the title compound as a light yellow solid.
MS m/e (ESI) 468(MH).

### (Example 5)

### [3-(4-t-Butylcyclohex-1-enyl)-2-(4-butylpiperazin-1-yl)phenyl](tetrahydropyran-4-yl)amine hydrochloride

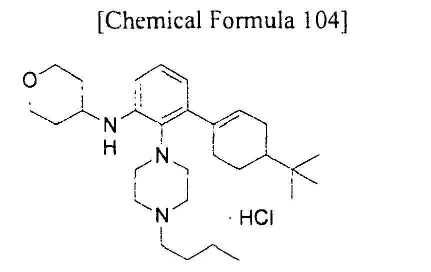

### (5a)

### 4-(2-Chloro-6-nitrophenyl)piperazine-1-carboxylic acid t-butyl ester

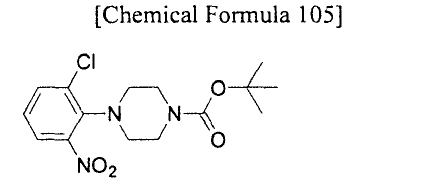

A mixture of 1,2-dichloro-3-nitrobenzene (2 g, 10.4 mmol), piperazine-1-carboxylic acid t-butyl ester (2.91 g, 15.6 mmol), potassium carbonate (2.16 g, 15.6 mmol) and dimethylformamide (10 mL) was stirred at an external temperature of 120°C for 10 hours and 30 minutes.
Water was added to the reaction mixture and extraction was performed with ethyl acetate. The separated organic layer was washed with water and brine in that order and then dried over anhydrous magnesium sulfate. The desiccant was filtered off and the filtrate was concentrated under reduced pressure. The resultant residue was purified by silica gel column chromatography (ethyl acetate/hexane) to give 2.97 g of the title compound as a yellow oil.
¹H-NMR (400MHz, CDCl₃)
δ:1.48 (s, 9H), 3.06 (brs, 4H), 7.14 (dd, J= 8.0, 8.0Hz, 1H), 7.52 (dd, J= 8.0, 1.6Hz, 1 H), 7.5 6 (dd, J= 8.0, 1.6Hz, 1 H).
The 4H of the piperazine ring could not be identified.

### (5b)

### 4-[2-(4-t-Butylcyclohex-1-enyl)-6-nitrophenyl]piperazine-1-carboxylic acid t-butyl ester

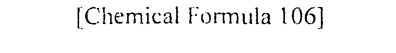

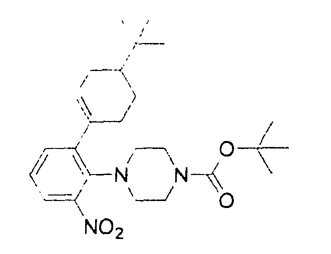

A mixture of 4-(2-chloro-6-nitrophenyl)piperazine-1-carboxylic acid t-butyl ester (370 mg, 1.08 mmol), 2-(4-t-butylcyclohex-1-enyl)-4,4,5,5-tetramethyl-[1,3,2]dioxaborolane produced in Production Example 4E (428 mg, 1.62 mmol), palladium(II) acetate (12 mg, 0.054 mmol), 2-(dicyclohexylphosphino)biphenyl (76 mg, 0.216 mmol), tripotassium phosphate (459 mg, 2.16 mmol), xylene (4 mL) and water (0.3 mL) was stirred at an external temperature of 120°C for 13 hours under a nitrogen atmosphere.
Water and ethyl acetate were added to the reaction mixture, which was filtered through Celite. The filtrate was then extracted with ethyl acetate. The collected organic layer was washed with water and brine in that order and then dried over anhydrous sodium sulfate. The desiccant was filtered off and the filtrate was concentrated under reduced pressure. The resultant residue was purified by silica gel column chromatography (ethyl acetate/hexane) to give 275 mg of the title compound as yellow crystals, in racemic form at the position of t-butyl.
¹H-NMR (400MHz, CDCl₃)
δ: 0.92 (s, 9H), 1.26-1.42 (m, 2H), 1.48 (s, 9H), 1.87-2.01 (m, 2H), 2.14-2.37 (m, 1H), 2.39-2.43 (m, 2H), 3.00 (s, 4H), 3.45 (s, 4H), 5.62-5.69 (m, 1H), 7.11 (dd, J= 8.0, 7.6Hz, 1H), 7.21 (dd, J= 7.6, 2.0Hz, 1H), 7.42 (dd, J= 8.0, 2.0Hz, 1H).

### (5c)

### 1-[2-(4-t-Butylcyclohex-1-enyl)-6-nitrophenyl]piperazine

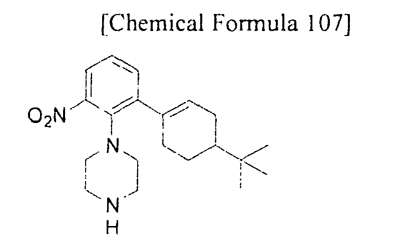

A mixture of 4-[2-(4-t-butylcyclohex-1-enyl)-6-nitrophenyl]piperazine-1-carboxylic acid t-butyl ester (275 mg, 0.62 mmol), trifluoroacetic acid (4 mL, 51.6 mmol) and dichloromethane (4 mL) was stirred at room temperature for 1 hour and 30 minutes.
Saturated aqueous sodium carbonate was added to the reaction mixture, and extraction was performed with ethyl acetate. The collected organic layer was dried over anhydrous sodium sulfate. The desiccant was filtered off and the filtrate was concentrated under reduced pressure to give 225 mg of a crude product of the title compound.

### (5d)

### 1-Butyl-4-[2-(4-t-butylcyclohex-1-enyl)-6-nitrophenyl]piperazine

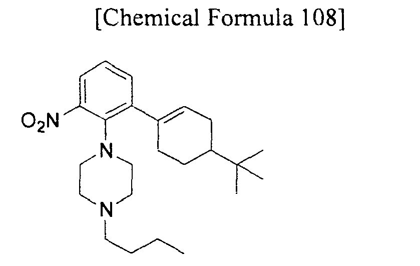

To a mixture of a crude product of 1-[2-(4-t-butylcyclohex-1-enyl)-6-nitrophenyl]piperazine (225 mg), butyraldehyde (0.061 mL, 0.682 mmol), acetic acid (37 mg, 0.620 mmol) and tetrahydrofuran (2 mL) was added sodium triacetoxyborohydride (25 mg, 0.120 mmol), followed by stirring at room temperature for 50 minutes.
Saturated aqueous sodium hydrogencarbonate was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 226 mg of the title compound as a yellow oil, in racemic form at the position of t-butyl.
¹H-NMR (400MHz, CDCl₃)
δ: 0.89-0.96 (m, 12H), 1.24-1.33 (m, 6H), 1.88-2.00 (m, 2H), 2.15-2.26 (m, 1H), 2,31-2,39 (m, 4H), 2,42-2,50 (m, 4H), 3,01-3,16 (m. 4H), 5.61-5.67 (m, 1H), 7.03 (dd, J= 8.0. 7.6Hz, 1H), 7.17 (dd, J= 7.6, 1.6Hz, 1H), 7.38 (dd, J= 8.0. 1.6Hz, 1H).

### (5e)

### 3-(4-t-Butylcyclohex-1-enyl)-2-(4-butylpiperazin-1-yl)phenylamine

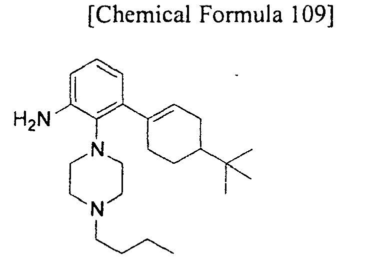

A mixture of 1-butyl-4-[2-(4-t-butylcyclohex-1-enyl)-6-nitrophenyl]piperazine (226 mg, 0.566 mmol), 10% palladium on carbon (30 mg, wet) and methanol (3 mL) was stirred at ordinary temperature and atmospheric pressure for 12 hours under a hydrogen atmosphere.
The reaction mixture was filtered through Celite. The filtrate was concentrated under reduced pressure to give 204 mg of a crude product of the title compound as a red oil.
¹H-NMR (400MHz, CDCl₃)
δ: 0.91 (s, 9H), 0.94 (t, J= 7.6Hz, 3H), 1.28-1.42 (m, 4H), 1.46-1.58 (m, 2H), 1.85-2.00 (m, 2H), 2.08-2.44 (m, 7H), 2.78-3.00 (m, 4H), 3.10-3.24 (m, 2H), 4.07 (brs, 2H), 5.50-5.57 (m, 1H), 6.42 (dd, J= 7.6, 1.6Hz, 1H), 6.63 (dd, J= 8.0, 1.6Hz, 1H), 6.87 (dd, J= 8.0, 7.6Hz, 1H).

### (5f)

### [3-(4-t-Butylcyclohex-1-enyl)-2-(4-butylpiperazin-1-yl)phenyl](tetrahydropyran-4-yl)amine hydrochloride

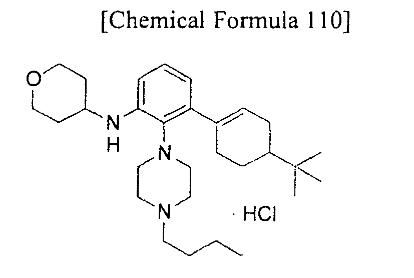

To a mixture of 3-(4-t-butylcyclohex-1-enyl)-2-(4-butylpiperazin-1-yl)phenylamine (56 mg, 0.152 mmol), tetrahydropyran-4-one (0.01 mL, 0.167 mmol), acetic acid (9 mg, 0.152 mmol) and 1,2-dichloroethane (1 mL) was added sodium triacetoxyborohydride (48 mg, 0.228 mmol), followed by stirring at room temperature for 19 hours and 20 minutes.
Saturated aqueous sodium hydrogencarbonate was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 43 mg of [3-(4-t-butylcyclohex-1-enyl)-2-(4-butylpiperazin-1-yl)phenyl](tetrahydropyran-4-yl)amine, in racemic form at the position of t-butyl.
The compound was dissolved in ethyl acetate, and a 4N solution of hydrogen chloride in ethyl acetate was added. The mixture was concentrated under reduced pressure to give 45 mg of the title compound as a colorless solid.
MS m/e (ESI) 454(MH⁺).

### (Example 6)

### 6-(4-t-Butylcyclohexyl)-7-(4-butylpiperazin-1-yl)-4-methyl-4H-benzo[1,4]oxazin-3-one

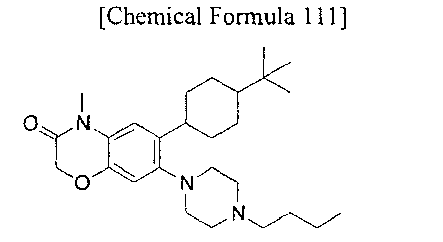

### (6a)

### 6-Chloro-7-nitro-4H-benzo[1,4]oxazin-3-one

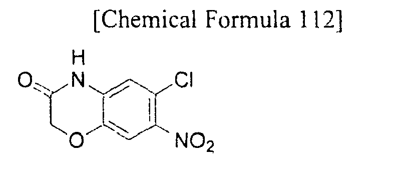

A mixture of 6-chloro-4H-benzo[1,4]oxazin-3-one (5 g, 27.2 mmol) and concentrated sulfuric acid (60 mL) was cooled in an ice bath and stirred. Potassium nitrate (2.89 g, 28.6 mmol) was gradually added to the mixture over 20 minutes, followed by further stirring for 10 minutes.
The reaction mixture was poured into ice water, and the precipitated solid was filtered. A small amount of acetone was added to the resultant solid, and after trituration by sonication, it was filtered. The resultant solid was dried under reduced pressure to give 4.92 g of the title compound as yellow crystals.
¹H-NMR (400MHz, DMSO-d₆)
δ:4.71 (s, 2H), 7.08(s, 1H), 7.77 (s, 1H), 11.28 (s, 1H).

### (6b)

### 6-Chloro-4-methyl-7-nitro-4H-benzo[1,4]oxazin-3-one

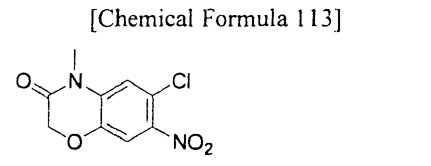

A mixture of 6-chloro-7-nitro-4H-benzo[1,4]oxazin-3-one (1 g, 4.37 mmol), potassium carbonate (1.21 g, 8.74 mmol) and dimethylformamide (10 mL) was stirred at room temperature. Methyl iodide (0.33 mL, 5.25 mmol) was added to the mixture, and the mixture was stirred at room temperature for 4 hours and 30 minutes.
Water was added to the reaction mixture and the precipitated solid was filtered to give 905 mg of the title compound as light yellow crystals.
¹H-NMR (400MHz, CDCl₃)
δ:3.40 (s, 3H), 4.71 (s, 2H), 7.06 (s, 1H), 7.65 (s, 1H).

### (6c)

### 6-(4-t-Butylcyclohex-1-enyl)-4-methyl-7-nitro-4H-benzo[1,4]oxazin-3-one

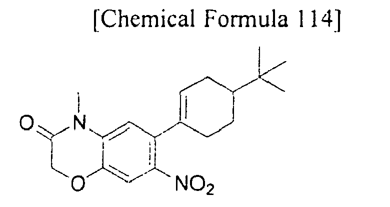

To a solution of 6-chloro-4-methyl-7-nitro-4H-benzo[1,4]oxazin-3-one (905 mg, 3.73 mmol) in 1,2-dimethoxyethane (20 mL) were added 2-(4-t-butylcyclohex-1-enyl)-4,4,5,5-tetramethyl-[1,3,2]dioxaborolane (1.48 g, 5.60 mmol) produced in Production Example 4E, tetrakis(triphenylphosphine)palladium(0) (216 mg, 0.187 mmol) and tripotassium phosphate (1.58 g, 7.46 mmol), followed by stirring at an external temperature of 85°C for 7 hours under a nitrogen atmosphere.
Brine and ethyl acetate were added to the reaction mixture, which was filtered through Celite. The filtrate was then extracted with ethyl acetate. The collected organic layer was washed with water and brine in that order and then dried over anhydrous magnesium sulfate. The desiccant was filtered off and the filtrate was concentrated under reduced pressure. The resultant residue was purified by silica gel column chromatography (ethyl acetate/hexane) to give 1.02 g of the title compound as yellow crystals, in racemic form at the position of t-butyl. ¹H-NMR (400MHz, CDCl₃)
δ: 0.91 (s, 9H), 1.32-1.46 (m, 2H), 1.86-1.99 (m, 2H), 2.14-2.24 (m, 2H), 2.25-2.40 (m, 1H), 3.40 (s, 3H), 4.68 (s, 2H), 5.58-5.66 (m, 1H), 6.77 (s, 1H), 7.57 (s, 1H).

### (6d)

### 7-Amino-6-(4-t-butylevclohexyl)-4-methyl-4H-benzo[1,4]oxazin-3-one

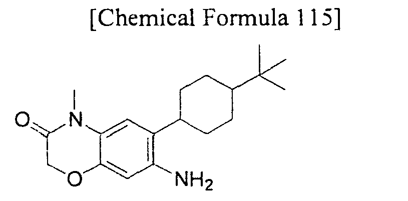

A mixture of 6-(4-t-butylcyclohex-1-enyl)-4-methyl-7-nitro-4H-benzo[1,4]oxazin-3-one (300 mg, 0.871 mmol), 10% palladium on carbon (300 mg, wet), tetrahydrofuran (3 mL) and methanol (3 mL) was stirred at ordinary temperature and atmospheric pressure for 4 hours under a hydrogen atmosphere.
The reaction mixture was filtered and the filtrate was concentrated under reduced pressure. The resultant residue was purified by silica gel column chromatography (ethyl acetate/hexane) to give 260 mg of the title compound as a colorless solid, as a mixture of diastereomers at the position of t-butylcyclohexyl. ¹H-NMR (400MHz, CDCl₃)
δ: 0.87 (s, 9H x 0.6), 0.88 (s, 9H x 0.4), 1.09-2.10 (m, 9H), 2.34-2.48 (m, 1H x 0.4), 2.92 (brs, 1H x 0.6), 3.32 (s, 3H x 0.4), 3.33 (s, 3H x 0.6), 4.53 (s, 2H x 0.4), 4.55 (s, 2H x 0.6), 6.30-6.40 (m, 1H), 6.68 (s, 1H x 0.4), 6.96 (s, 1H x 0.6).

### (6e)

### 6-(4-t-Butylcyclohexyl)-4-methyl-7-piperazin-1-yl-4H-benzo[1,4]oxazin-3-one

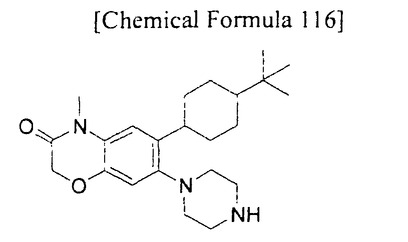

To a solution of 7-amino-6-(4-t-butylcyclohexyl)-4-methyl-4H-benzo[1,4]oxazin-3-one (260 mg, 0.822 mmol) in 1,2-dichlorobenzene (2.6 mL) was added bis(2-chloroethyl)amine hydrochloride (183 mg, 1.03 mmol), followed by stirring at an external temperature of 200°C for 3 hours under a nitrogen atmosphere. During the reaction, nitrogen gas was passed through the reactor several times to remove the hydrogen chloride gas.
After air-cooling the reaction mixture to room temperature, 1N aqueous sodium hydroxide was added, and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 182 mg of the title compound as an orange solid, as a mixture of diastereomers at the position of t-butylcyclohexyl.
¹H-NMR (400MHz, CDCl₃)
δ: 0.895 (s, 9H x 0.6), 0.902 (s, 9H x 0.4), 1.09-1.96 (m, 9H), 2.72-3.06 (m, 8H + 1H x 0.4), 3.35 (brs, 1H), 3.38-3.46 (s, 1H x 0.6), 4.58 (s, 2H x 0.4), 4.59 (s, 2H x 0.6), 6.76 (s, 1H x 0.4), 6.78 (s, 1H x 0.4), 6.80 (s, 1H x 0.6), 7.06 (s, 1H x 0.6).

### (6f)

### 6-(4-t-Butylcyclohexyl)-7-butylpiperazin-1-yl)-4-methyl-4H-benzo[1,4]oxazin-3-one

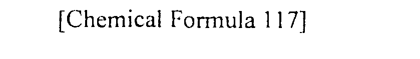

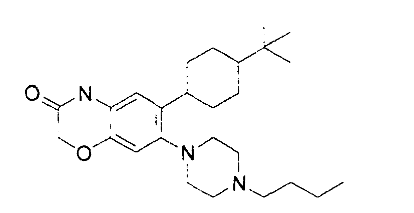

To a mixture of 6-(4-t-butylcyclohexyl)-4-methyl-7-piperazin-1-yl-4H-benzo[1,4]oxazin-3-one (100 mg, 0.259 mmol), butyraldehyde (0.035 mL, 0.389 mmol), acetic acid (0.015 mg, 0.259 mmol) and tetrahydrofuran (2 mL) was added sodium triacetoxyborohydride (82 mg, 0.389 mmol), followed by stirring at room temperature for 1 hour.
To the reaction mixture was added 1N aqueous sodium hydroxide, and then extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 101 mg of the title compound as colorless crystals, as a mixture of diastereomers at the position of t-butylcyclohexyl. ¹H-NMR (400MHz, CDCl₃)
δ: 0.89 (s, 9H x 0.6), 0.90 (s, 9H x 0.4), 0.94 (t, J= 7.6Hz, 3H x 0.6), 0.95 (t, J= 7.6Hz, 3H x 0.4), 1.08-1.96 (m, 13H), 2.30-2.94 (m, 10H), 2.98 (tt, J= 12.0, 3.6Hz, 1H x 0.4), 3.34 (s, 3H), 3.36-3.44 (m, 1H x 0.6), 4.57 (s, 2H x 0.4), 4.58 (s, 2H x 0.6), 6.75-6.78 (m, 2H x 0.4), 6.81 (s, 1H x 0.6), 7.04 (s, 1 H x 0.6).
MS m/e (ESI) 442(MH⁺).

### (Example 7)

### 6-(4-t-Butylcyclohexyl)-7-(4-butylpiperazin-1-yl)-4-methyl-3,4-dihydro-2H-benzo[1,4]oxazine hydrochloride

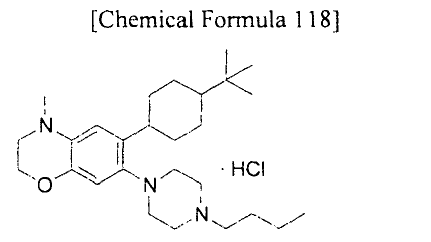

To a mixture of 6-(4-t-butylcyclohexyl)-7-(4-butylpiperazin-1-yl)-4-methyl-4H-benzo[1,4]oxazin-3-one (71 mg, 0.161 mmol), aluminum chloride (47 mg, 0.354 mmol) and tetrahydrofuran (3 mL) was added lithium aluminum hydride (7 mg, 0.177 mmol), followed by stirring at room temperature for 3 hours and 10 minutes.
Saturated aqueous sodium hydrogencarbonate was added to the reaction mixture which was filtered through Celite. The resultant filtrate was then extracted with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 73 mg of 6-(4-t-butylcyclohexyl)-7-(4-butylpiperazin-1-yl)-4-methyl-3,4-dihydro-2H-benzo[1,4]oxazine as a colorless oil, as a mixture of diastereomers at the position of t-butylcyclohexyl.
¹H-NMR (400MHz, CDCl₃)
δ: 0.89 (s, 9H x 0.6), 0.90 (s, 9H x 0.4), 0.93 (t, J= 7.6Hz, 3H x 0.6), 0.94 (t, J= 7.6Hz, 3H x 0.4), 1.07-1.94 (m, 13H), 2.30-2.84 (m, 10H), 2.85 (s, 3H x 0.4), 2.86 (s, 3H x 0.6), 2.96 (tt, J= 12.0, 2.8Hz, 1H x 0.4), 3.18-3.23 (m, 2H), 3.36-3.43 (m, 1H x 0.6), 4.24-4.30 (s, 2H), 6.51 (s, 1H x 0.4), 6.61 (s, 1H x 0.4), 6.65 (s, 1H x 0.6), 6.79 (s, 1H x 0.6).
This compound was dissolved in ethyl acetate, and a 4N solution of hydrogen chloride in ethyl acetate was added. The mixture was concentrated under reduced pressure. Hexane was added to the residue, and the resulting precipitate was triturated by sonication. After removing the supernatant, the precipitate was dried to give 75 mg of the title compound as colorless crystals, as a mixture of diastereomers at the position of t-butylcyclohexyl.
MS m/e (ESI) 428(MH⁺).

### (Example 8)

### 6-(4-t-Butylcyclohex-1-enyl)-4-methyl-7-(4-propylpiperazin-1-yl)-3,4-dihydro-2H-benzo[1,4]oxazine hydrochloride

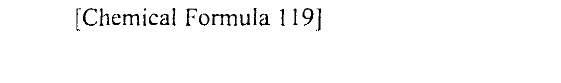

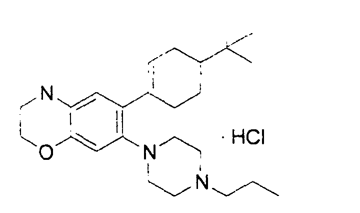

### (8a)

### 7-Amino-6-(4-t-butylcyclohex-1-enyl)-4-methyl-4H-benzo[1,4]oxazin-3-one

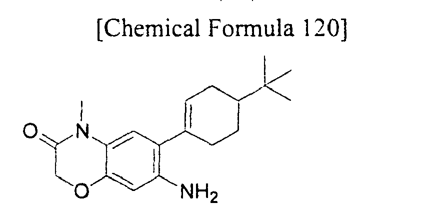

A mixture of 6-(4-t-butylcyclohex-1-enyl)-4-methyl-7-nitro-4H-benzo[1,4]oxazin-3-one (1.14 g, 3.30 mmol), iron powder (553 mg, 9.91 mmol), ammonium chloride (707 mg, 13.2 mmol), ethanol (15 mL), water (5 mL) and dimethylformamide (3 mL) was stirred at an external temperature of 90°C for 7 hours and 30 minutes under a nitrogen atmosphere.
Ethyl acetate and water were added to the reaction mixture, which was filtered through Celite. The filtrate was then extracted with ethyl acetate. The collected organic layer was washed with water and brine in that order and then dried over anhydrous sodium sulfate. The desiccant was filtered off and the filtrate was concentrated under reduced pressure. The resultant residue was purified by silica gel column chromatography (ethyl acetate/hexane) to give 940 mg of the title compound as a light yellow oil, in racemic form at the position of t-butyl.
¹H-NMR (400MHz, CDCl₃)
δ: 0.92 (s, 9H), 1.28-1.46 (m, 2H), 1.88-2.03 (m, 2H), 2.15-2.36 (m, 3H), 3.30 (s, 3H), 4.54 (s, 2H), 5.70-5.80 (m, 1H), 6.34 (s, 1H), 6.57 (s, 1H).
The 2H of NH₂ could not be identified.

### (8b)

### 6-(4-t-Butylcyclohex-1-enyl)-4-methyl-7-piperazin-1-yl-4H-benzo[1,4]oxazin-3-one

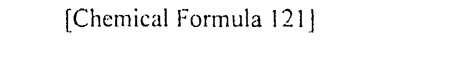

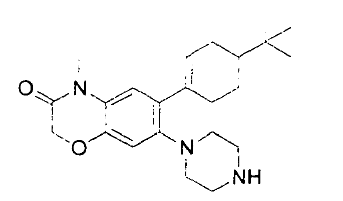

To a solution of 7-amino-6-(4-t-butylcyclohex-1-enyl)-4-methyl-4H-benzo[1.4]oxazin-3-one (940 mg, 2.99 mmol) in 1,2-dichlorobenzene (3 mL) was added bis(2-chloroethyl)amine hydrochloride (694 mg, 3.89 mmol), followed by stirring at an external temperature of 220°C for 2 hours and 30 minutes under a nitrogen atmosphere. During the reaction, nitrogen gas was passed through the reactor several times to remove the hydrogen chloride gas.
After air-cooling the reaction mixture to room temperature, saturated aqueous sodium hydrogencarbonate was added and extraction was performed with ethyl acetate. The collected organic layer was dried over anhydrous sodium sulfate. The desiccant was filtered off and the filtrate was concentrated under reduced pressure. The resultant residue was purified by NH silica gel column chromatography (ethyl acetate/hexane) to give 604 mg of the title compound as light yellow crystals, in racemic form at the position of t-butyl.
¹H-NMR (400MHz, CDCl₃)
δ: 0.92 (s, 9H), 1.20-1.41 (m, 2H), 1.86-2.01 (m, 2H), 2.15-2.27 (m, 1H), 2.31-2.44 (m, 1H), 2.65-2.75 (m, 1H), 2.79-3.06 (m, 8H), 3.34 (s, 3H), 4.59 (s, 2H), 5.65-5.80 (m, 1H), 6.61 (s, 1H), 6.70 (s, 1H).

### (8c)

### 6-(4-t-Butylcyclohex-1-enyl)-4-methyl-7-piperazin-1-yl-3,4-dihydro-2H-benzo[1,4]oxazine

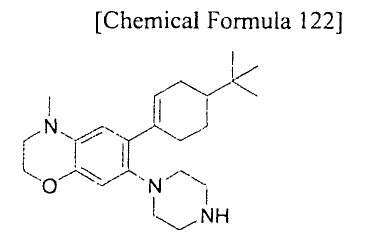

To a mixture of 6-(4-t-butylcyclohex-1-enyl)-4-methyl-7-piperazin-1-yl-4H-benzo[1,4]oxazin-3-one (604 mg, 1.57 mmol), aluminum chloride (840 mg, 6.30 mmol) and tetrahydrofuran (20 mL) was added lithium aluminum hydride (120 mg, 3.15 mmol), followed by stirring at room temperature for 17 hours and 30 minutes.
Diethyl ether, water and 5N aqueous sodium hydroxide were added to the reaction mixture, which was filtered through Celite. The resultant filtrate was then extracted with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 283 mg of the title compound as colorless crystals, in racemic form at the position of t-butyl.
¹H-NMR (400MHz, CDCl₃)
δ: 0.91 (s, 9H), 1.18-1.40 (m, 2H), 1.86-1.98 (m, 2H), 2.13-2.23 (m, 1H), 2.31-2.47 (m, 1H), 2.66-3.00 (m, 12H), 3.15-3.25 (m, 2H), 4.25-4.35 (m, 2H), 5.65-5.70 (m, 1H), 6.46(s, 1H), 6.48(s, 1H).

### (8d)

### 6-(4-t-Butylcyclohex-1-enyl)-4-methyl-7-(4-propylpiperazin-1-yl)-3,4-dihydro-2H-benzo[1,4]oxazine hydrochloride

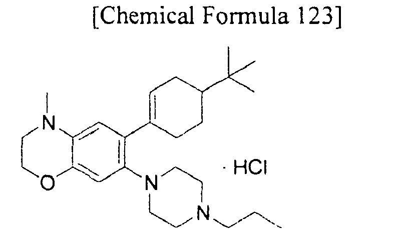

To a mixture of 6-(4-t-butylcyclohex-1-enyl)-4-methyl-7-piperazin-1-yl-3,4-dihydro-2H-benzo[1,4]oxazine (10 mg, 0.0284 mmol), propionaldehyde (5 mg, 0.0852 mmol) and tetrahydrofuran (1 mL) was added sodium triacetoxyborohydride (30 mg, 0.142 mmol), followed by stirring at room temperature for 18 hours and 30 minutes.
Saturated aqueous sodium hydrogencarbonate was added to the reaction mixture and extraction was performed with ethyl acetate. The collected organic layer was washed with water and then filtered through Celite. The solvent was removed by blowing nitrogen gas to the filtrate. The resultant residue was purified by NH silica gel column chromatography (ethyl acetate/hexane) to give 6-(4-t-butylcyclohex-1-enyl)-4-methyl-7-(4-propylpiperazin-1-yl)-3,4-dihydro-2H-benzo[1,4]oxazine, in racemic form at the position of t-butyl.
This compound was dissolved in ethyl acetate, and a 4N solution of hydrogen chloride in ethyl acetate was added. The solvent was removed by blowing nitrogen gas to this. Hexane was added to the resultant residue, and the resultant precipitate was triturated by sonication. After removing the supernatant, the precipitate was dried to give 2.4 mg of the title compound as a colorless solid, in racemic form at the position of t-butyl.
MS m/e (ESI) 412(MH⁺).

### (Example 9)

### 5-(4-t-Butylcyclohex-1-enyl)-6-(4-butylpiperazin-1-yl)-2-methylbenzothiazole dihydrochloride

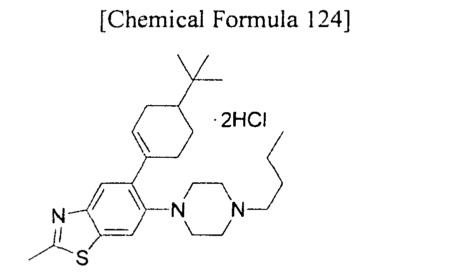

### (9a)

### 5-Chloro-2-methyl-6-nitro-benzothiazole

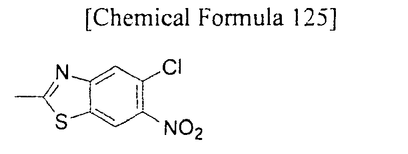

A mixture of 5-chloro-2-methylbenzothiazole (2.5 g, 13.61 mmol) and concentrated sulfuric acid (50 mL) was cooled in an ice bath and stirred. Potassium nitrate (1.38 g, 13.61 mmol) was gradually added to the mixture, and the mixture was allowed to warm up to room temperature and stirred for 5 hours and 30 minutes.
The reaction mixture was cooled in an ice bath, 5N aqueous sodium hydroxide was added thereto, and the reaction mixture was adjusted to pH 7. The mixture was then extracted with ethyl acetate. The collected organic layer was washed with water and brine in that order and then dried over anhydrous sodium sulfate. The desiccant was filtered off and the filtrate was concentrated under reduced pressure. The resultant residue was purified by silica gel column chromatography (toluene) to give 1.67 g of the title compound as light yellow crystals.
¹H-NMR (400MHz, DMSO-d₆)
δ:2.88 (s, 2H), 8.32 (s, I H), 8.96 (s, 1H).

### (9b)

### 5-(4-t-Butylcyclohex-1-enyl)-2-methyl-6-nitro-benzothiazole

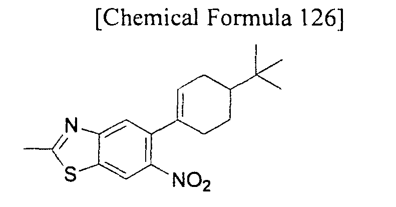

To a mixture of 5-chloro-2-methyl-6-nitro-benzothiazole (1 g, 4.37 mmol), 1,2-dimethoxyethane (30 mL) and water (5 mL) were added 2-(4-t-butylcyclohex-1-enyl)-4,4,5,5-tetramethyl-[1,3,2]dioxaborolane (1.73 g, 6.56 mmol) produced in Production Example 4E, tetrakis(triphenylphosphine)palladium(0) (253 mg, 0.219 mmol) and tripotassium phosphate (1.86 g, 8.74 mmol), followed by stirring at an external temperature of 80°C for 14 hours and 45 minutes under a nitrogen atmosphere.
Brine was added to the reaction mixture, and extraction was performed with ethyl acetate. The separated organic layer was washed with water and brine in that order and then dried over anhydrous sodium sulfate. The desiccant was filtered off and the filtrate was concentrated under reduced pressure. The resultant residue was purified by silica gel column chromatography (ethyl acetate/hexane) to give 634 mg of the title compound as purple crystals, in racemic form at the position of t-butyl.
¹H-NMR (400MHz, CDCl₃)
δ: 0.92 (s, 9H), 1.32-1.52 (m, 2H), 1.86-2.46 (m, 6H), 2.89 (s, 3H), 5.68-5.72 (m, 1H), 7.79 (s, 1H), 8.34 (s, 1H).

### (9c)

### 5-(4-t-Butylcyclohex-1-enyl)-2-methyl-benzothiazol-6-ylamine

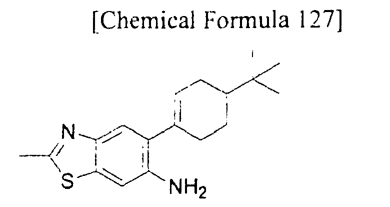

A mixture of 5-(4-t-butylcyclohex-1-enyl)-2-methyl-6-nitro-benzothiazole (634 mg, 2.34 mmol), 10% palladium on carbon (200 mg, wet) and methanol (30 mL) was stirred at ordinary temperature and atmospheric pressure for 12 hours under a hydrogen atmosphere.
The reaction mixture was filtered and the filtrate was concentrated under reduced pressure. To the resultant residue were added 10% palladium on carbon (200 mg, wet) and methanol (30 mL), followed by stirring at ordinary temperature and atmospheric pressure for 8 hours and 30 minutes under a hydrogen atmosphere.
The reaction mixture was filtered, and the filtrate was concentrated under reduced pressure. The resultant residue was dried under reduced pressure to give 542 mg of a crude product of the title compound as a colorless solid, in racemic form at the position of t-butyl.

### (9d)

### 5-(4-t-Butylcyclohex-1-enyl)-2-methyl-6-piperazin-1-ylbenzothiazole

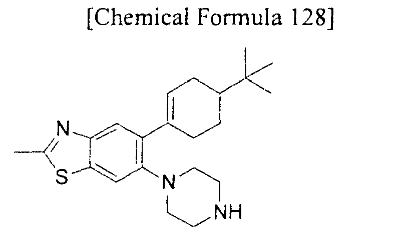

To a solution of a crude product of 5-(4-t-butylcyclohex-1-enyl)-2-methylbenzothiazol-6-ylamine (542 mg) in 1,2-dichlorobenzene (5 mL) was added bis(2-chloroethyl)amine hydrochloride (386 mg, 2.16 mmol), followed by stirring at an external temperature of 200°C for 2 hours and 30 minutes under a nitrogen atmosphere. During the reaction, nitrogen gas was passed through the reactor several times to remove the hydrogen chloride gas.
After air-cooling the reaction mixture to room temperature, saturated aqueous sodium hydrogencarbonate and ethyl acetate were added. The mixture was filtered through Celite, and the filtrate was extracted with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 188 mg of the title compound as brown crystals, in racemic form at the position of t-butyl.
¹H-NMR (400MHz, CDCl₃)
δ: 0.93 (s, 9H), 1.26-1.45 (m, 2H), 1.88-2.03 (m, 2H), 2.18-2.29 (m, 1H), 2.39-2.53 (m, 1H), 2.64-2.75 (m, 1H), 2.78 (s, 3H), 2.88-3.08 (m, 8H), 5.79-5.85 (m, 1H), 7.34 (s, 1H), 7.63 (s, 1H).

### (9e)

### 5-(4-t-Butylcyclohex-1-enyl)-6-(4-butylpiperazin-1-yl)-2-methylbenzothiazole dihydrochloride

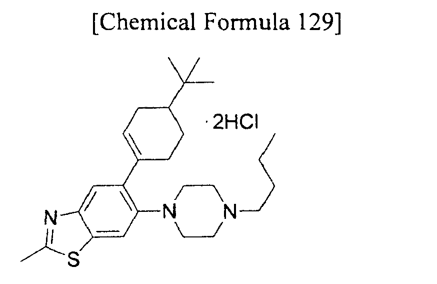

To a mixture of 5-(4-t-butylcyclohex-1-enyl)-2-methyl-6-piperazin-1-ylbenzothiazole (20 mg, 0.0666 mmol), butyraldehyde (5.2 mg, 0.0732 mmol), acetic acid (4 mg, 0.0666 mmol) and tetrahydrofuran (1 mL) was added sodium triacetoxyborohydride (21 mg, 0.100 mmol), followed by stirring at room temperature for 2 hours.
Saturated aqueous sodium hydrogencarbonate was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/hexane) to give 20 mg of 5-(4-t-butylcyclohex-1-enyl)-6-(4-butylpiperarin-1-yl)-2-methylbenzothiazole as a colorless oil, in racemic form at the position of t-butyl.
¹H-NMR (400MHz, CDCl₃)
δ: 0.94 (s, 9H), 0.95 (t, J= 7.2Hz, 3H), 1.18-1.60 (m, 6H), 1.88-2.03 (m, 2H), 2.16-2.29 (m, 1H), 2.32-2.76 (m, 8H), 2.78 (s, 3H), 2.90-3.20 (m, 4H), 5.80-5.85 (m, 1H), 7.34 (s, 1H), 7.62 (s, 1H).
This compound was dissolved in ethyl acetate, and a 4N solution of hydrogen chloride in ethyl acetate was added. This was concentrated under reduced pressure. Hexane was added to the resultant residue, and the resultant precipitate was triturated by sonication. After removing the supernatant, the precipitate was dried to give 23 mg of the title compound as colorless crystals, in racemic form at the position of t-butyl.
MS m/e (ESI) 426(MH⁺).

### (Example 10)

### 1-Butyl-4-[5-(2-methoxyethoxy)-2-(3,3,5,5-tetramethylcyclohexyl)phenyl]piperazine hydrochloride

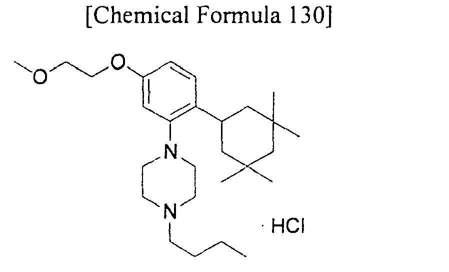

### (10a)

### 3-Piperazin-1-yl-4-(3,3,5,5-tetramethylcyclohexyl)phenol

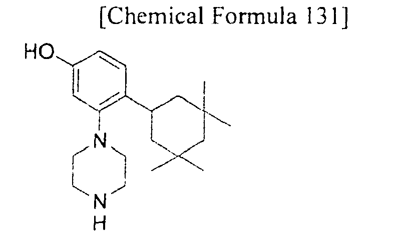

1-[5-Methoxy-2-(3,3,5,5-tetramethylcyclohex-1-enyl)phenyl]piperazine produced in Production Example 6C was used as the starting material for N-t-butyloxycarbonylation and hydrogenation reaction by conventional methods. A mixture of 4-[5-methoxy-2-(3,3,5,5-tetramethylcyclohexyl)phenyl]piperazine-1-carboxylic acid t-butyl ester (3.41 g, 7.92 mmol) obtained in the reaction, acetic acid (18 mL, 314 mmol) and 48% hydrobromic acid (36 mL, 318 mmol) was stirred at an external temperature of 130°C for 8 hours and 20 minutes.
The reaction mixture was cooled in an ice water bath and stirred, and then 5N aqueous sodium hydroxide was added dropwise thereto to adjust the reaction mixture to pH 8-9. The produced solid was filtered to give 2.98 g of a crude product as a light red solid.
¹H-NMR (400MHz, CD₃OD)
δ: 0.94 (s, 6H), 1.54 (s, 6H), 1.17-1.40 (m, 6H), 3.05 (t, J= 4.8Hz, 4H), 3.42 (tt, J= 12.8, 2.8Hz, 1H), 6.55-6.61 (m, 2H), 7.05 (d, J= 8.0Hz, 1H).
The 4H of the piperazine ring and the 2H of NH and OH could not be identified.

### (10b)

### 4-[5-Hydroxy-2-(3,3,5,5-tetramethylcyclohexyl)phenyl]piperazine-1-carboxylic acid t-butyl ester

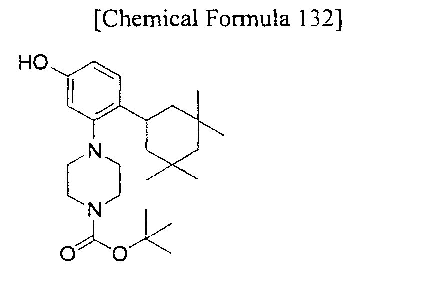

A mixture of a crude product of 3-piperazin-1-yl-4-(3,3,5,5-tetramethylcyclohexyl)phenol (2.98 g) produced in Production Example 9A and a mixed solvent (100 mL) of chloroform-methanol was stirred at an external temperature of 0°C. A solution of di-t-butyl dicarbonate (1.81 g, 8.32 mmol) in chloroform was added dropwise to the mixture.
After stirring the reaction mixture for 2 hours and 30 minutes, the reaction mixture was concentrated under reduced pressure. Saturated aqueous sodium carbonate and ethyl acetate were added to the resultant residue and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/heptane) to give 2.95 g of the title compound as a light red solid.
¹H-NMR (400MHz, CDCl₃)
δ: 0.91 (s, 6H), 1.10 (s, 6H), 1.11-1.43 (m, 6H), 1.49 (s, 9H), 2.80 (brs, 4H), 3.43 (tt, J= 12.4, 2.8Hz, 1H), 3.52 (brs, 4H), 6.55-6.57 (m, 2H), 7.06 (dd, J= 7.2, 1.6Hz, 1H).
The 1H of OH could not be identified.

### (10c)

### 4-[5-(2-Methoxyethoxy)-2-(3,3,5,5-tetramethylcyclohexyl)phenyl]piperazine-1-carboxylic acid t-butyl ester

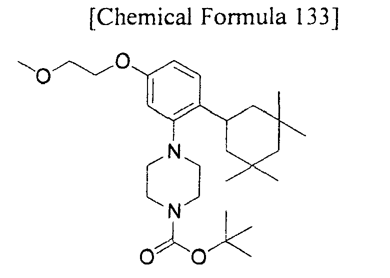

A mixture of 4-[5-hydroxy-2-(3,3,5,5-tetramethylcyclohexyl)phenyl]piperazine-1-carboxylic acid t-butyl ester (200 mg, 0.480 mmol), dimethylformamide (2 mL) and sodium hydride (60% dispersion in oil, 28.8 mg, 0.720 mmol) was stirred at room temperature for 20 minutes under a nitrogen atmosphere. Next, 2-bromoethylmethylether (0.052 mL, 0.528 mmol) was added dropwise to the mixture, followed by stirring for 2 hours and 40 minutes.
Water was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/heptane) to give 232 mg of the title compound as a colorless oil.
¹H-NMR (400MHz, CDCl₃)
δ: 0.91 (s, 6H), 1.10 (s, 6H), 1.11-1.45 (m, 6H), 1.49 (s, 9H), 2.79 (brs, 4H), 3.38-3.49 (m, 4H), 3.55 (brs, 4H). 3.73 (d, J= 4.8, 2H), 4.08 (d, J= 4.8, 2H), 6.64 (dd, J= 8.4, 2.4Hz, 1H), 6.66 (d, J= 2.4Hz, 1H), 7.11 (d, J= 8.4Hz, 1H).

### (10d)

### 1-[5-(2-Methoxyethoxy)-2-(3,3,5,5-tetramethylcyclohexyl)phenyl]piperazine

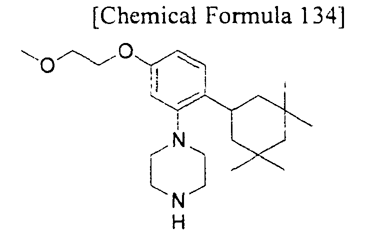

A mixture of 4-[5-(2-methoxyethoxy)-2-(3,3,5,5-tetramethylcyclohexyl)phenyl]piperazine-1-carboxylic acid t-butyl ester (232 mg, 0.489 mmol), trifluoroacetic acid (1 mL, 12.9 mmol) and dichloromethane (2 mL) was stirred at room temperature for 1 hour.
Saturated aqueous sodium carbonate was added to the reaction mixture and extraction was performed with ethyl acetate. The collected organic layer was washed with water and brine in that order and then dried over anhydrous sodium sulfate. The desiccant was filtered off and the filtrate was concentrated under reduced pressure. The resultant residue was purified by NH silica gel column chromatography (ethyl acetate/heptane) to give 158 mg of the title compound as a colorless solid.
¹H-NMR (400MHz, CDCl₃)
δ: 0.91 (s, 6H), 1.11 (s, 6H), 1.13-1.46 (m, 6H), 2.82 (brs, 4H), 2.94-3.06 (m, 4H), 3.37-3.51 (m, 4H), 3.74 (d, J= 4.8, 2H), 4.09 (d, J= 4.8, 2H), 6.63 (dd, J= 8.4, 2.4Hz, 1H), 6.71 (d, J= 2.4Hz, 1H), 7.11 (d, J= 8.4Hz, 1H).
The 1H of NH of the piperazine ring could not be identified.

### (10e)

### 1-Butyl-4-[5-(2-methoxyethoxy)-2-(3,3,5,5-tetramethylcyclohexyl)phenyl]piperazine hydrochloride

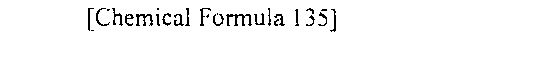

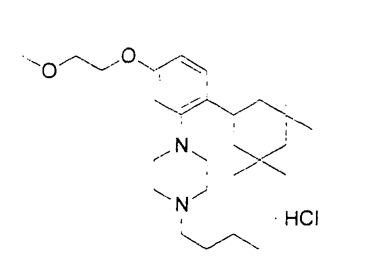

To a mixture of 1-[5-(2-methoxyethoxy)-2-(3,3,5,5-tetramethylcyclohexyl)phenyl]piperazine (15 mg, 0.0400 mmol), butyraldehyde (0.0107 mL, 0.120 mmol) and tetrahydrofuran (1 mL) was added sodium triacetoxyborohydride (25 mg, 0.120 mmol), followed by stirring at room temperature for 1 hour.
Saturated aqueous sodium hydrogencarbonate was added to the reaction mixture and extraction was performed with ethyl acetate. The solvent was removed by blowing nitrogen gas to the collected organic layer. The resultant residue was purified by NH silica gel column chromatography (ethyl acetate/hexane) to give 1-butyl-4-[5-(2-methoxyethoxy)-2-(3,3,5,5-tetramethylcyclohexyl)phenyl]piperazine as a colorless solid.
¹H-NMR (400MHz, CDCl₃) δ: 0.91 (s, 6H), 0.94 (t, J= 7.2Hz, 3H), 1.11 (s, 6H), 1.12-1.57 (m, 10H), 2.34-2.42 (m, 2H), 2.58 (brs, 4H), 2.90 (t, J= 4.4Hz, 4H), 3.36-3.47 (m, 4H), 3.70-3.76 (m, 2H), 4.04-4.11 (m, 2H), 6.61 (dd, J= 8.4, 2.4Hz, 1H), 6.71 (d, J= 2.4Hz, 1H), 7.09 (d, J=8.4Hz, 1H).
This compound was dissolved in dichloromethane, and a 4N solution of hydrogen chloride in ethyl acetate was added. The solvent was removed by blowing nitrogen gas to this. Heptane was added to the resultant residue, and the resultant precipitate was triturated by sonication. After removing the supernatant, the precipitate was dried to give 17.7 mg of the title compound as a colorless solid.
MS m/e (ESI) 431 (MH⁺).

### (Example 11)

### (S)-1-Butyl-4-[2-(4,4-diethylcyclohexyl)-4-(2-methoxymethylpyrrolidin-1-yl)phenyl]piperazine hydrochloride

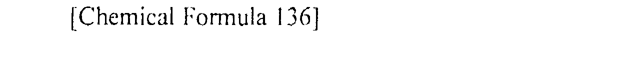

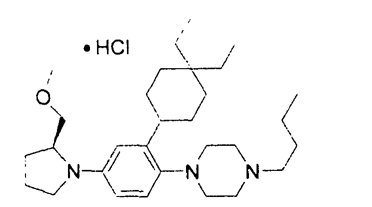

### (11a)

### (S)-4-[2-(4,4-Diethylcyctohexyl)-4-(2-methoxymethylpyrrolidin-1-yl)phenyl]piperazine-1-carboxylic acid t-butyl ester

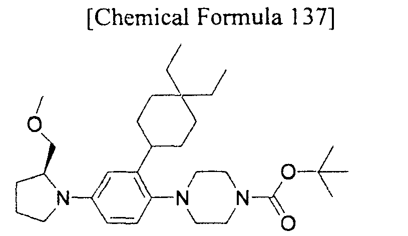

4-[4-Bromo-2-(4,4-diethylcyclohexyl)phenyl]piperazine-1-carboxylic acid t-butyl ester (500 mg, 1.0 mmol) produced in Production Example 7C was dissolved in toluene (10 mL). To the mixture were added (S)-2-(methoxymethyl)pyrrolidine (170 mg, 1.5 mmol), sodium t-butoxide (240 mg, 2.5 mmol), tri-t-butylphosphonium tetrafluoroborate (180 mg, 0.625 mmol) and palladium(II) acetate (56 mg, 0.25 mmol), followed by stirring at an external temperature of 80°C for 15 hours and 30 minutes under a nitrogen atmosphere.
After air-cooling the reaction mixture to room temperature, the insoluble matters were filtered through Celite and the resultant filtrate was concentrated under reduced pressure. The resultant residue was purified by silica gel column chromatography (ethyl acetate/hexane) to give 240 mg of the title compound as a black oil.
¹H-NMR (400MHz, CDCl₃)
TLC Rf= 0.28 (hexane/AcOET= 5/1, UV 254nm)

### (11b)

### (S)-1-[2-(4,4-Diethylcyclohexyl)-4-(2-methoxymethylpyrrolidin-1-yl)phenyl]piperazine

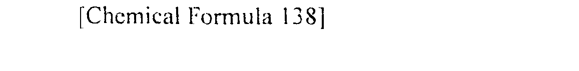

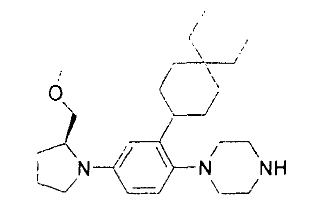

A mixture of (S)-4-[2-(4,4-diethylcyclohexyl)-4-(2-methoxymethylpyrrolidin-1-yl)phenyl]piperazine-1-carboxylic acid t-butyl ester (240 mg, 0.467 mmol), trifluoroacetic acid (1 mL, 13.0 mmol) and dichloromethane (2 mL) was stirred at 0°C for 4 hours.
The reaction mixture was made basic by addition of potassium carbonate while cooled on ice, and then dichloromethane and water were added and extraction was performed with dichloromethane. The collected organic layer was dried over anhydrous magnesium sulfate, the desiccant was filtered off and the filtrate was concentrated under reduced pressure. The resultant residue was purified by NH silica gel column chromatography (ethyl acetate/hexane) to give 145 mg of the title compound as a black oil.
TLC(NH) Rf= 0-0.30 (tailing) (hexane/AcOEt = 2/1, UV 254nm)

### (11c)

### (S)-1-Butyl-4-[2-(4,4-diethylcyclohexyl)-4-(2-methoxymethylpyrrolidin-1-yl)phenyl]piperazine hydrochloride

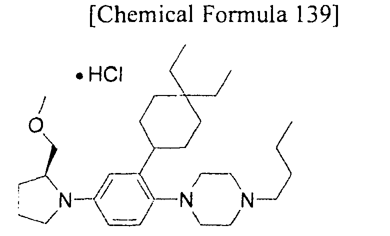

To a solution of (S)-1-[2-(4,4-diethylcyclohexyl)-4-(2-methoxymethylpyrrolidin-1-yl)phenyl]piperazine (20 mg, 0.048 mmol) in tetrahydrofuran (1 mL) were added butyraldehyde (5.2 mg, 0.073 mmol), sodium triacetoxyborohydride (20 mg, 0.097 mmol) and acetic acid (2.9 mg, 0.048 mmol) in that order, followed by stirring at room temperature for 30 minutes.
Saturated aqueous sodium hydrogencarbonate was added to the reaction mixture, and then extraction was performed with ethyl acetate and the organic layer was concentrated. The resultant residue was purified by NH silica gel column chromatography (ethyl acetate/heptane) to give (S)-1-butyl-4-[2-(4.4-diethylcyclohexyl)-4-(2-methoxymethylpyrrolidin-1-yl)phenyl]piperazine. ¹H-NMR (400MHz, CDCl₃) δ:0.76-0.96 (m, 8H), 1.15-1.72 (m, 17H), 1.91-2.04 (m, 4H), 2.37-2.41 (m, 2H), 2.57 (br, 4H), 2.84 (br, 4H), 2.97 (tt, J= 12, 3.6Hz, 1H), 3.06-3.12 (m, 1H), 3.18 (t, J= 9.2Hz, 1H), 3.39 (s, 3H), 3.41-3.45 (m, 1H), 3.51 (dd, J= 9.2, 3.2Hz, 1H), 3.83 (td, J= 8.0, 3.2Hz, 1 H), 6.46 (dd, J= 8.8, 2.8Hz, 1 H), 6.52 (d, J= 2.8Hz, 1 H), 7.08 (d, J= 8.8Hz, 1H).
Thus obtained compound was dissolved in ethyl acetate, and a 4N solution of hydrogen chloride in ethyl acetate was added. The solution was concentrated, hexane was added to the resultant residue, and the resulting precipitate was triturated by sonication. After removing the supernatant, the precipitate was dried to give 22.3 mg of the title compound as a white solid.
MS m/e (ESI) 470(MH⁺).

### (Example 12)

### 1-[4-(2-Methoxyethoxy)-2-(3,3,5,5-tetramethylcyclohexyl)phenyl]-4-propylpiperazine hydrochloride

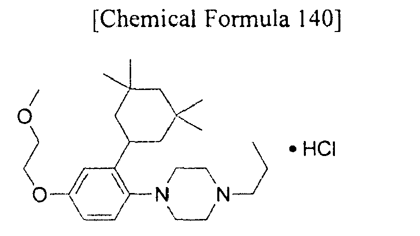

### (12a)

### 4-Piperazin-1-yl-3-(3,3,5,5-tetramethylcyclohexyl)phenol

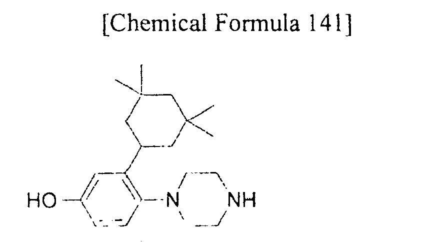

A mixture of 1-[4-methoxy-2-(3,3,5,5-tetramethylcyclohexyl)phenyl]piperazine (450 mg, 1.36 mmol) produced in Production Example 10C, 48% hydrobromic acid (4 mL) and acetic acid (2 mL) was stirred at an external temperature of 130°C for 7 hours and 30 minutes under a nitrogen atmosphere. The reaction mixture was then cooled in an ice water bath and stirred.
The reaction mixture was adjusted to pH 8-9 by addition of 5N aqueous sodium hydroxide. The precipitated solid was filtered and washed with water and then a mixed solvent of methanol, ethyl acetate and dichloromethane was added to the solid. The insoluble matters were filtered off and the filtrate was concentrated under reduced pressure. Diethyl ether was added to the resultant residue for solidification, followed by trituration by sonication. The resultant solid was filtered, washed with heptane and diethyl ether and subjected to through-flow drying to give 460 mg of the title compound as a light red solid. Thus obtained product was used for the following reaction without further purification.

### (12b)

### 4-[4-Hydroxy-2-(3,3,5,5-tetramethylcyclohexyl)phenyl]piperazine-1-carboxylic acid t-butyl ester

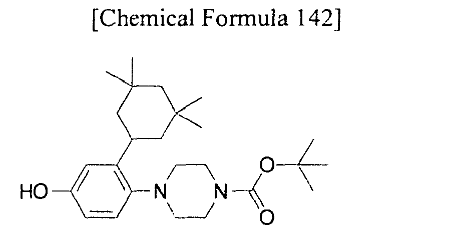

To a solution of 4-piperazin-1-yl-3-(3,3,5,5-tetramethylcyclohexyl)phenol (460 mg, 1.45 mmol) in dimethylformamide (5 mL) were added potassium carbonate (188 mg, 1.36 mmol) and 4-dimethylaminopyridine (20 mg, 0.164 mmol), followed by stirring. Di-t-butyl dicarbonate (300 mg, 1.85 mmol) was added to the mixture in an ice bath, while preventing solidification of the solution.
After stirring the reaction mixture at room temperature for 19 hours and 30 minutes, water was added to the reaction mixture and extraction was performed with ethyl acetate. The collected organic layer was washed with water and brine and then dried over anhydrous magnesium sulfate. The desiccant was filtered off and the filtrate was concentrated under reduced pressure. The resultant residue was purified by NH silica gel column chromatography (ethyl acetate/heptane) to give 340 mg of the title compound as a colorless solid.
¹H-NMR (400MHz, CDCl₃)
δ: 0.86-1.56 (m, 6H), 0.91 (s, 6H), 1.10 (s, 6H), 1.49 (s, 9H), 2.75 (br, 4H), 3.05 (br, 2H), 3.58 (t, J= 12.0Hz, 1H), 4.03 (br, 2H), 5.64 (brs, 1H), 6.62 (d, J= 8.4Hz, 1H), 6.71 (s, 1H), 6.94 (d, J= 8.4Hz, 1H).

### (12c)

### 4-[4-(2-Methoxyethoxy)-2-(3,3,5,5-tetramethylcyclohexyl)phenyl]piperazine-1-carboxylic acid t-butyl ester

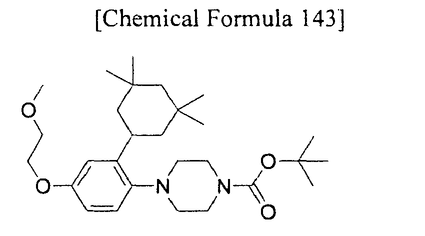

To a solution of 4-[4-hydroxy-2-(3,3,5,5-tetramethylcyclohexyl)phenyl]piperazine-1-carboxylic acid t-butyl ester (290 mg, 0.696 mmol) in dimethylformamide (3 mL) was added sodium hydride (60% dispersion in oil, 100 mg, 2.5 mmol) while stirring at room temperature. After bubbling ceased, 2-bromoethylmethyl ether (0.15 mL, 1.48 mmol) was further added to the reaction mixture.
The mixture was stirred at room temperature for 1 hour and 10 minutes, and then water was added to the reaction mixture and extraction was performed with ethyl acetate. The collected organic layer was washed with water and then dried over anhydrous magnesium sulfate. The desiccant was filtered off and the filtrate was concentrated under reduced pressure. A crude product of the title compound (340 mg) was obtained. The crude product was used for the following reaction without purification.

### (12d)

### 1-[4-(2-Methoxyethoxy)-2-(3,3,5,5-tetramethylcyclohexyl)phenyl]piperazine

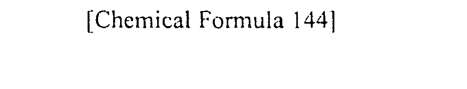

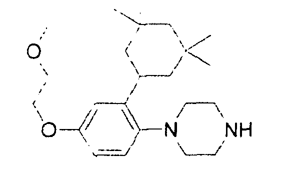

A solution of 4-[4-(2-methoxyethoxy)-2-(3,3,5,5-tetramethylcyclohexyl)phenyl]piperazine-1-carboxylic acid t-butyl ester (400 mg, 0.82 mmol) in dichloromethane (4 mL) was stirred while cooling in an ice bath, followed by gradually adding trifluoroacetic acid (2 mL) dropwise over 10 minutes. The reaction mixture was warmed to room temperature and stirred for 30 minutes.
The reaction mixture was made basic by addition of saturated aqueous sodium hydrogencarbonate while cooling in ice water, and extraction was performed with ethyl acetate. The collected organic layer was washed with water and then dried over anhydrous magnesium sulfate. The desiccant was filtered off and the filtrate was concentrated under reduced pressure. The resultant residue was purified by NH silica gel column chromatography (ethyl acetate/heptane) to give 296 mg of the title compound as a light yellow solid.

### (12e)

### 1-[4-(2-Methoxyethoxy)-2-(3,3,5,3-tetramethylcyclohexyl)phenyl]-4-propylpiperazine hydrochloride

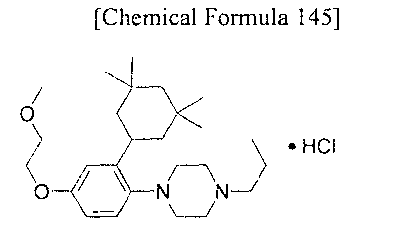

To a solution of 1-[4-(2-methoxyethoxy)-2-(3,3,5,5-tetramethylcyclohexyl)phenyl]piperazine (10 mg, 0.027 mmol) in tetrahydrofuran (1 mL) were added propionaldehyde (2.3 mg, 0.040 mmol), sodium triacetoxyborohydride (11 mg, 0.053 mmol) and acetic acid (1.6 mg, 0.027 mmol) in that order, followed by stirring for 30 minutes at room temperature.
Saturated aqueous sodium hydrogencarbonate was added to the reaction mixture, extraction was performed with ethyl acetate, and the organic layer was concentrated. The resultant residue was purified by NH silica gel column chromatography (ethyl acetate/heptane) to give 1-[4-(2-methoxyethoxy)-2-(3,3,5,5-tetramethylcyclohexyl)phenyl]-4-propylpiperazine.
¹H-NMR (400MHz, CD₃OD)
δ: 0.96 (s, 6H), 1.06 (t, J= 7.2Hz, 3H), 1.18 (s, 6H), 1.18-1.43 (m, 7H), 1.77-1.87 (m, 2H), 3.10-3.22 (m, 8H), 3.42 (s, 3H), 3.57 (tt, J= 12, 2.8Hz, 1H), 3.67 (m, 1H), 3.72-3.74 (m, 2H), 4.07-4.09 (m, 2H), 6.77 (dd, J= 8.8, 3.2Hz, 1H), 6.83 (d, J= 3.2Hz, 1H), 7.14 (d, J= 8.8Hz, 1H).
Thus obtained compound was dissolved in ethyl acetate, and a 4N solution of hydrogen chloride in ethyl acetate was added. The solution was concentrated, hexane was added to the resultant residue, and the resulting precipitate was triturated by sonication. After removing the supernatant, the precipitate was dried to give 6.3 mg of the title compound as a white solid.
MS m/e (ESI) 417(MH⁺).

### (Example 13)

### 1-Butyl-4-[6-(3,3,5,5-tetramethycyclohex-1-enyl)benzo [1,3]dioxol-5-yl]piperazine hydrochloride

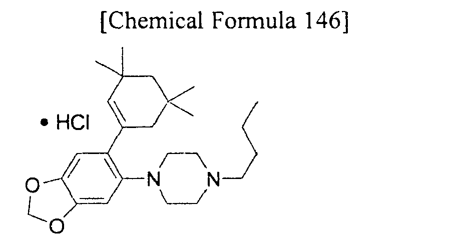

### (13a)

### 5-Bromo-6-nitrobenzo[1,3]dioxole

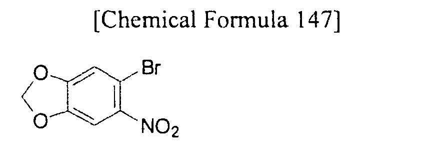

at room temperature. The reaction mixture was cooled to 0°C and then ice water was added while stirring.
Diethyl ether and water were added to the reaction mixture, and the organic layer was collected. The organic layer was washed with saturated sodium hydrogencarbonate water and potassium carbonate, and then washed with water. The organic layer was dried over anhydrous magnesium sulfate and then the desiccant was filtered off and the filtrate was concentrated under reduced pressure. A crude product of the title compound (3.35 g) was obtained as a light yellow solid. ¹H-NMR (400MHz, CDCl₃)
δ: 6.15 (s, 2H), 7.12 (s, 1H), 7.45 (s, 1H).

### (13b)

### 5-Nitro-6-(3,3,5,5-tetramethylcyclohex-1-enyl)benzo[1,3]dioxole

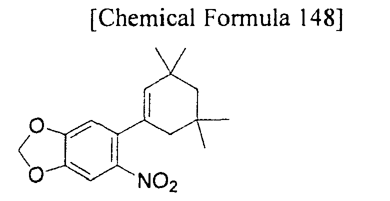

To a solution of 5-bromo-6-nitrobenzo[1,3]dioxole (1.5 g, 6.1 mmol) in 1,2-dimethoxyethane (30 mL) were added 4,4,5,5-tetramethyl-2-(3,3,5,5-tetramethylcyclohex-1-enyl)-[1,3,2]dioxaborolane (1.93 g, 7.3 mmol) produced in Production Example 2B, tripotassium phosphate (1.94 g, 9.14 mmol) and water (1.5 mL).
Tetrakis(triphenylphosphine)palladium (352 mg, 0.305 mmol) was added to the mixture under a nitrogen atmosphere, followed by stirring at an external temperature of 80°C for 4 hours. After then further adding tetrakis(triphenylphosphine)palladium(0) (350 mg, 0.305 mmol) and water (1.5 mL) to the reaction mixture, stirring was continued for 2 hours and 55 minutes. Next, tetrakis(triphenylphosphine)palladium (250 mg, 0.305 mmol), water (2 mL) and tripotassium phosphate (820 mg, 3.9 mmol) were added to the reaction mixture, followed by raising the temperature up to an external temperature of 100°C and stirring for 2 hours and 30 minutes. The reaction mixture was then stirred at an external temperature of 80°C for 18 hours and 30 minutes.
Ethyl acetate was added to the reaction mixture which was filtered through Celite. The filtrate was then concentrated, extraction was performed with ethyl acetate, and the organic layer was washed with water and brine. The organic layer was dried over anhydrous magnesium sulfate, after which the desiccant was filtered off and the filtrate was concentrated under reduced pressure. The resultant residue was purified by silica gel column chromatography (ethyl acetate/heptane) to give 1.35 g of the title compound as a light yellow solid.
¹H-NMR (400MHz, CDCl₃)
δ: 1.04 (s, 12H), 1.39 (s, 2H), 1.97 (d, J= 1.6Hz, 2H), 5.300-5.304 (m, 1H), 6.08 (s, 2H), 6.63 (s, 1H), 7.35 (s, 1H).

### (13c)

### 6-(3,3,5,5-Tetramethylcyclohex-1-enyl)benzo[1,3]dioxol-5-ylamine

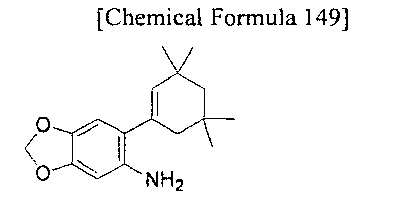

A mixture of 5-nitro-6-(3,3,5,5-tetramethylcyclohex-1-enyl)benzo[1,3]dioxole (900 mg, 2.97 mmol), 10% palladium on carbon (450 mg, wet), methanol (8 mL) and tetrahydrofuran (6 mL) was stirred at ordinary temperature and atmospheric pressure overnight under a hydrogen atmosphere.
The mixture was filtered through Celite to remove the catalyst, and the filtrate was concentrated. A crude product of the title compound (834 mg) was obtained as a yellow oil.

### (13d)

### 1-[6-(3,3,5,5-Tetramethylcyclohex-1-enyl)benzo[1,3]dioxol-5-yl]piperazine

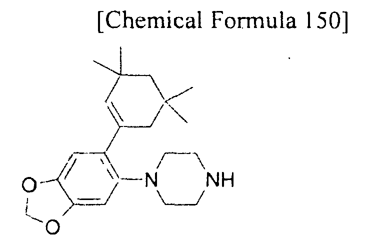

A solution of 6-(3,3,5,5-tetramethylcyclohex-1-enyl)benzo[1,3]dioxol-5-ylamine (834 mg, 3.05 mmol) and bis(2-chloroethyl)amine hydrochloride (680 mg. 3.81 mmol) in 1,2-dichlorobenzene (10 mL) was stirred at an external temperature of 220°C for 7 hours and 35 minutes. The excess hydrogen chloride gas in the reactor was removed several times by nitrogen gas.
After cooling the reaction mixture to room temperature, saturated aqueous sodium hydrogencarbonate was added and extraction was performed with chloroform. The aqueous layer was re-extracted with chloroform, and the organic layers were combined and washed with brine and then dried over anhydrous magnesium sulfate. The desiccant was filtered off and the filtrate was concentrated under reduced pressure. The resultant residue was purified by NH silica gel column chromatography (ethyl acetate/heptane) to give 280 mg of the title compound as a brown oil.
¹H-NMR (400MHz, CDCl₃)
δ:1.01 (s, 6H), 1.05 (s, 6H), 1.37 (s, 2H), 2.13 (s, 2H), 2.81 (br, 4H), 2.93 (br, 4H), 5.37 (s, I H), 5.89 (s, 2H), 6.58 (s, 1H), 6.65 (s, 1H).

### (13e)

### 1-Butyl-4-[6-(3,3,5,5-tetramethylcyclohex-1-enyl)benzo[1,3]dioxol-5-yl]piperazine hydrochloride

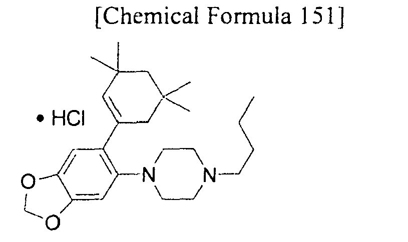

To a solution of 1-[6-(3,3,5,5-tetramethylcyclohex-1-enyl)benzo[1,3]dioxol-5-yl]piperazine (30 mg, 0.088 mmol) in tetrahydrofuran (1 mL) were added butyraldehyde (9.5 mg, 0.13 mmol), sodium triacetoxyborohydride (37 mg, 0.18 mmol) and acetic acid (5.3 mg, 0.088 mmol) in that order, followed by stirring at room temperature for 15 minutes.
Saturated aqueous sodium hydrogencarbonate was added to the reaction mixture, and then extraction was performed with ethyl acetate and the organic layer was concentrated. The resultant residue was purified by NH silica gel column chromatography (ethyl acetate/heptane) to give 1-butyl-4-(6-(3,3,5,5-tetramethylcyclohex-1-enyl)benzo[1,3]dioxo)-5-yl]piperazine.
¹H-NMR (400MHz, CDCl₃)
δ:0.86-0.95 (m, 3H), 1.02 (s, 6H), 1.05 (s, 6H), 1.26-1.37 (m, 4H), 1.43-1.53 (m, 2H), 2.13 (s, 2H), 2.33-2.37 (m, 2H), 2.52 (br, 4H), 2.89 (br, 4H), 5.38 (s, 1H), 5.89 (s, 2H), 6.59 (s, 1H), 6.67 (s, 1H).
Thus obtained compound was dissolved in ethyl acetate, and a 4N solution of hydrogen chloride in ethyl acetate was added. The solution was concentrated, hexane was added to the resultant residue, and the resultant precipitate was triturated by sonication. After removing the supernatant, the precipitate was dried to give 33 mg of the title compound as a white solid.
MS m/e (ESI) 399(MH⁺).

### (Example 14)

### 1-Butyl-4-[6-(3,3,5,5-tetramethylcyclohexyl)benzo[1,3]dioxol-5-yl]piperazine hydrochloride

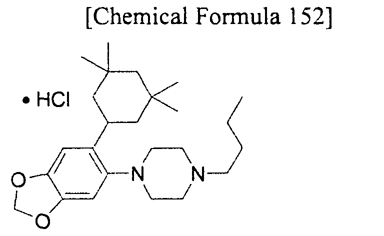

A mixture of 1-butyl-4-[6-(3,3,5,5-tetramethylcyclohex-1-enyl)benzo[1,3]dioxol-5-yl]piperazine hydrochloride (16 mg, 0.037 mmol), 10% palladium on carbon (94 mg, wet), methanol (10 mL) and tetrahydrofuran (5 mL) was stirred at room temperature for 9 hours and 20 minutes under a hydrogen atmosphere at 3 atmospheric pressure.
The mixture was filtered through Celite to remove the catalyst, and the filtrate was concentrated. Hexane was added to the resultant residue, and the resultant precipitate was triturated by sonication. After removing the supernatant, the precipitate was dried to give 10 mg of the title compound as a white solid.
MS m/e (ESI) 401(MH⁺).

### (Example 15)

### 1-Butyl-4-[7-(3,3,5,5-tetramethylcyclohexyl)-2,3-dihydrobenzo[1,4]dioxin-6-yl]piperazine hydrochloride

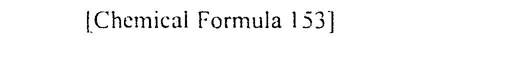

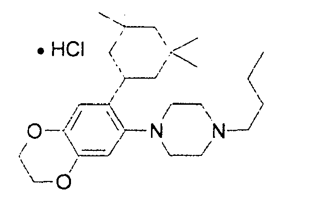

### (15a)

### 6-Bromo-7-nitro-2,3-dihydrobenzo[1,4]dioxin

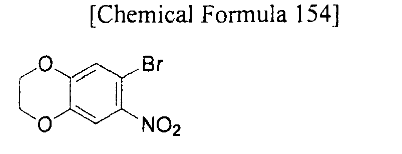

A solution of 3,4-ethylenedioxybromobenzole (785 mg, 3.54 mmol) in acetic acid (30 mL) was cooled to 10°C, and then concentrated nitric acid (10 mL) was gradually added to the reaction mixture while stirring. The reaction mixture was then further stirred for 60 minutes at room temperature. Next, the reaction mixture was cooled to 0°C and ice water was added while stirring.
Diethyl ether and water were added to the reaction mixture and the organic layer was collected. The organic layer was washed with potassium carbonate and 1N aqueous sodium hydroxide and then washed with water and brine. The organic layer was dried over anhydrous magnesium sulfate, and then the desiccant was filtered off and the filtrate was concentrated under reduced pressure. A crude product of the title compound (870 mg) was obtained as a white solid.
¹H-NMR (400MHz, CDCl₃)
δ: 4.29-4.35 (m, 4H), 7.21 (s, I H), 7.59 (s, 1H).

### (15b)

### 6-Nitro-7-(3,3,5,5-tetramethylcyclohex-1-enyl)-2,3-dihydrobenzo[1,4]dioxin

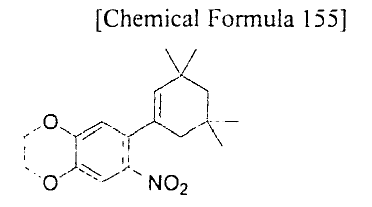

To a solution of 6-bromo-7-nitro-2,3-dihydrobenzo[1,4]dioxin (870 mg. 3.35 mmol) in 1,2-dimethoxyethane (20 mL) were added 4,4,5,5-tetramethyl-2-(3,3,5,5-tetramethylcyclohex-1-enyl)-[1,3,2]dioxaborolane (1.06 g, 4.02 mmol) produced in Production Example 2B, tripotassium phosphate (1.07 g, 5.03 mmol) and water (1 mL).
To the mixture was added tetrakis(triphenylphosphine)palladium(0) (194 mg, 0.168 mmol) under a nitrogen atmosphere, followed by stirring at an external temperature of 80°C for 2 hours and 30 minutes. To the reaction mixture was added tetrakis(triphenylphosphine)palladium(0) (220 mg, 0.20 mmol), followed by stirring at an external temperature of 90°C for 4 hours and 30 minutes. To the reaction mixture were further added an aqueous solution (1.5 mL) of tetrakis(triphenylphosphine)palladium(0) (195 mg, 0.168 mmol) and tripotassium phosphate (1 g, 5.0 mmol), followed by stirring at an external temperature of 75°C for 10 hours and 30 minutes.
After addition of ethyl acetate, the reaction mixture was filtered through Celite. The filtrate was concentrated, and then extraction was performed with ethyl acetate and the organic layer was washed with brine. The organic layer was dried over anhydrous magnesium sulfate, and then the desiccant was filtered off and the filtrate was concentrated under reduced pressure. The resultant residue was purified by silica gel column chromatography (ethyl acetate/heptane) to give 986 mg of the title compound as a light yellow solid.
¹H-NMR (400MHz, CDCl₃)
δ:1.03 (s, 6H), 1.05 (s, 6H), 1.39 (s, 2H), 1.95 (d, J= 1.6Hz, 2H), 4.28-4.34 (m, 4H), 5.30 (t, J= 1.6Hz, 1H), 6.69 (s, 1H), 7.48 (s, 1H).

### (15c)

### 7-(3,3,5,5-Tetramethylcyclohexyl)-2,3-dihydrobenzo[1,4]dioxin-6-ylamine

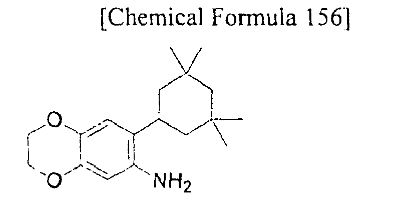

A mixture of 6-nitro-7-(3,3,5,5-tetramethylcyclohex-1-enyl)-2,3-dihydrobenzo[1,4]dioxin (986 mg, 3.11 mmol), 10% palladium on carbon (1 g, wet), methanol (10 mL) and tetrahydrofuran (10 mL) was stirred at room temperature for 6 hours and 30 minutes under a hydrogen atmosphere at 3 atmospheric pressure.
The mixture was filtered through Celite to remove the catalyst, and the filtrate was concentrated. A crude product of the title compound (880 mg) was obtained as a light yellow oil. Thus obtained product was used for the following reaction without further purification.

### (15d)

### 1-[7-(3,3,5,5-Tetramethylcyclohexyl)-2,3-dihydrobenzo[1,4]dioxin-6-yl]piperazine

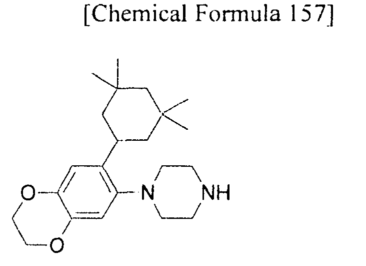

A solution of 7-(3,3,5,5-tetramethylcyclohexyl)-2,3-dihydrobenzo[1,4]dioxyin-6-ylamine (880 mg, 3.04 mmol) and bis(2-chloroethyl)amine hydrochloride (678 mg, 3.8 mmol) in 1,2-dichlorobenzene (10 mL) was stirred at an external temperature of 220°C for 6 hours and 20 minutes. The excess hydrogen chloride gas in the reactor was removed several times by nitrogen gas.
The reaction mixture was cooled to room temperature, saturated aqueous sodium hydrogencarbonate was added and extraction was performed with chloroform. The aqueous layer was re-extracted with chloroform, and the organic layers were combined and washed with brine and then dried over anhydrous magnesium sulfate. The desiccant was filtered off and the filtrate was concentrated under reduced pressure. The resultant residue was purified by NH silica gel column chromatography (ethyl acetate/heptane) to give 511 mg of the title compound as a white solid.
¹H-NMR (400MHz, CDCl₃)
δ: 0.99 (s, 6H), 1.11 (s, 6H), 1.13-1.18 (m, 2H), 1.24-1.32 (m. 2H), 1.39-1.43 (m. 2H), 2.77 (br, 4H), 2.99 (dd. J=4.8, 4.4Hz, 4H), 3.45 (tt. J= 13, 2.8Hz, 1H), 4.22 (s, 4H), 6.65(s, 1H), 6.70 (s, 1H).

### (15e)

### 1-Butyl-4-[7-(3,3,5,5-tetramethylcyclohexyl)-2,3-dihydrobenzo[1,4]dioxin-6-yl]piperazine hydrochloride

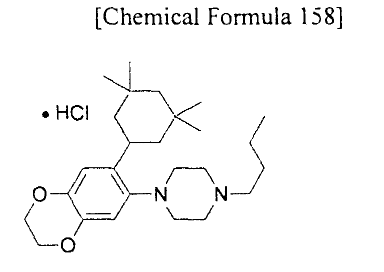

To a solution of 1-[7-(3,3,5,5-tetramethylcyclohexyl)-2,3-dihydrobenzo[1,4]dioxin-6-yl]piperazine (10 mg, 0.028 mmol) in tetrahydrofuran (1 mL) were added butyraldehyde (3.0 mg, 0.042 mmol), sodium triacetoxyborohydride (12 mg, 0.056 mmol) and acetic acid (2 mg, 0.028 mmol) in that order, followed by stirring at room temperature for 30 minutes.
Saturated aqueous sodium hydrogencarbonate was added to the reaction mixture and then extraction was performed with ethyl acetate and the organic layer was concentrated. The resultant residue was purified by NH silica gel column chromatography (ethyl acetate/heptane) to give 1-butyl-4-[7-(3,3,5,5-tetramethylcyclohexyl)-2,3-dihydrobenzo[1,4]dioxin-6-yl]piperazine as a white solid.
¹H-NMR (400MHz, CDCl₃)
δ:0.91 (s, 6H), 0.93 (t, J= 7.2Hz, 3H), 1.10 (s, 6H), 1.25-1.55 (m, 9H), 1.70 (br, 1H), 2.35-2.40 (m, 2H), 2.56 (br, 4H), 2.85 (t, J= 4.6Hz, 4H), 3.47 (tt, J= 13, 2.8Hz, 1H), 4.21 (s, 4H), 6.67 (s, 1H), 6.70 (s, 1H).
Thus obtained compound was dissolved in ethyl acetate, and a 4N solution of hydrogen chloride in ethyl acetate was added. The solution was concentrated, hexane was added to the resultant residue, and the resultant precipitate was triturated by sonication. After removing the supernatant, the precipitate was dried to give 12.6 mg of the title compound as a white solid.
MS m/e (ESI) 415(MH⁺).

### (Example 16)

### 4-(4-Propylpiperazin-1-yl)-3-(3,3,5,5-tetramethylcyclohexyl)benzoic acid methyl ester hydrochloride

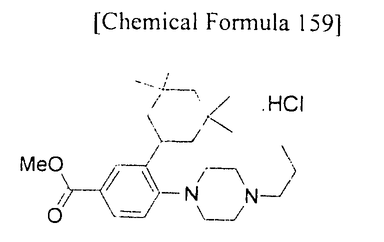

### (16a)

### 4-(2-Bromo-4-methoxycarbonylphenyl)piperazine-1-carboxylic acid t-butyl ester

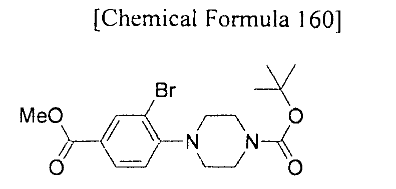

A mixture of 3-bromo-4-fluorobenzoic acid methyl ester (5.1 g, 21.9 mmol), piperazine-1-carboxylic acid t-butyl ester (5.3 g, 28.5 mmol), potassium carbonate (6.05 g, 43.8 mmol) and dimethylsulfoxide (80 mL) was stirred at an external temperature of 130°C.
After stirring the reaction mixture for 5 hours and 30 minutes, ethyl acetate and water were added to the reaction mixture and extraction was performed with ethyl acetate. The collected organic layer was washed twice with water, washed once with brine, and then dried over anhydrous sodium sulfate. The desiccant was filtered off and the filtrate was concentrated under reduced pressure. The resultant residue was purified by silica gel column chromatography (ethyl acetate/hexane) to give 3.12 g of the title compound as a light yellow oil.
¹H-NMR (400MHz, CDCl₃)
δ: 1.49 (s, 9H), 3.07 (t, J= 4.8Hz, 4H), 3.62 (t, J= 4.8Hz, 4H), 3.90 (s, 3H), 7.01 (d, J= 8.4Hz, 1H), 7.94 (dd, J= 8.4, 2.0Hz, 1H), 8.24 (d, J= 2.0Hz, 1H).

### (16b)

### 4-[4-Methoxycarbonyl-2-(3,3,5,5-tetramethylcyclohex-1-enyl)phenyl]piperazine-1-carboxylic acid t-butyl ester

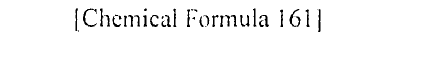

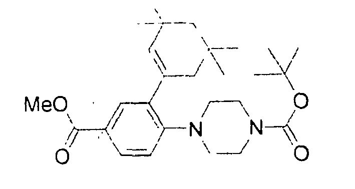

A mixture of 4-(2-bromo-4-methoxycarbonylphenyl)piperazine-1-carboxylic acid t-butyl ester (3.12 g, 7.81 mmol). 4,4,5,5-tetramethyl-2-(3,3,5,5-tetramethylcyclohex-1-enyl)[1,3,2]dioxoborolane (3.1 g, 11.73 mmol) produced in Production Example 2B, tripotassium phosphate (3.3 g, 15.55 mmol) and 1,2-dimethoxyethane (30 mL) was stirred at room temperature under a nitrogen atmosphere. Tetrakis(triphenylphosphine)palladium(0) (905 mg, 0.783 mmol) was added to the mixture. Next, the reaction mixture was stirred at an external temperature of 85°C for 18 hours and 10 minutes.
Ethyl acetate and water were added to the reaction mixture, which was then filtered through Celite. The organic layer obtained from the filtrate was washed with brine and then dried over anhydrous sodium sulfate. The desiccant was filtered off and the filtrate was concentrated under reduced pressure. The resultant residue was purified by NH silica gel column chromatography (ethyl acetate/hexane) to give 3.11 g of the title compound as a light yellow oil.
¹H-NMR (400MHz, CDCl₃)
δ: 1.02 (s, 6H), 1.09 (s, 6H), 1.40 (s, 2H), 1.48 (s, 9H), 2.13 (d, J= 1.2Hz, 2H), 3.01 (t, J= 4.8Hz, 4H), 3.53 (t, J= 4.8Hz, 4H), 3.89 (s, 3H), 5.60 (s, 1H), 6.94 (d, J= 8.4Hz, 1H), 7.73 (d, J= 2.0Hz, 1H), 7.86 (dd, J= 8.4, 2.0Hz, 1H).

### (16c)

### 4-[4-Methoxycarbonyl-2-(3,3,5,5-tetramethylcyclohexyl)phenyl]piperazine-1-carboxylic acid t-butyl ester

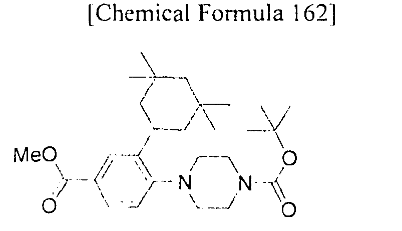

A mixture of 4-[4-methoxycarbonyl-2-(3,3,5,5-tetramethylcyclohex-1-enyl)phenyl]piperazine-1-carboxylic acid t-butyl ester (800 mg, 1.75 mmol), 10% palladium on carbon (400 mg, wet), methanol (10 mL) and tetrahydrofuran (10 mL) was stirred at ordinary temperature and atmospheric pressure for 40 hours under a hydrogen atmosphere.
The reaction mixture was filtered and then the filtrate was concentrated. Ethyl acetate was added to the residue, and after filtration, the filtrate was concentrated. The resultant residue was purified by NH silica gel column chromatography (ethyl acetate/heptane) to give 740 mg of the title compound as colorless crystals.
¹H-NMR (400MHz, CDCl₃)
δ: 0.94 (s, 6H), 1.10 (s, 6H), 1.15-1.46 (m, 6H), 1.49 (s, 9H), 2.87 (t, J= 4.8Hz, 4H), 3.48 (tt, J= 12.4, 3.2Hz, 1H), 3.58 (brs, 4H), 3.89 (s, 3H), 7.04 (d, J= 8.4Hz, 1H), 7.82 (dd, J= 8.4, 2.4Hz, 1H), 7.90 (d, J= 2.4Hz, 1H).

### (16d)

### 4-Piperazin-1-yl-3-(3,3,5,5-tetramethylcyclohexyl)benzoic acid methyl ester

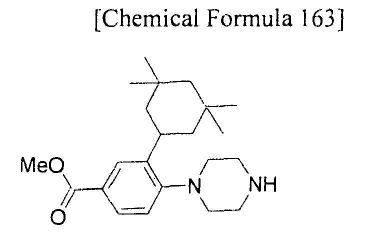

A mixture of 4-[4-methoxycarbonyl-2-(3,3,5,5-tetramethylcyclohexyl)phenyl]piperazine-1-carboxylic acid t-butyl ester (738 mg, 1.61 mmol), trifluoroacetic acid (2 mL, 25.96 mmol) and dichloromethane (10 mL) was stirred at room temperature for 15 hours.
The reaction mixture was poured into ice water, and saturated aqueous sodium hydrogencarbonate was added to make the mixture basic. Next, ethyl acetate, tetrahydrofuran and water were added to the mixture and the organic layer was collected. The collected organic layer was washed with brine and then dried over anhydrous sodium sulfate. The desiccant was filtered off, and the filtrate was concentrated under reduced pressure to give 538 mg of a crude product of the title compound as a light yellow solid.
¹H-NMR (400MHz, CDCl₃)
δ: 0.94 (s, 6H), 1.12 (s, 6H), 1.16-1.46 (m, 6H), 2.98 (t, J= 4.8Hz, 4H), 3.13 (t, J= 4.8Hz, 4H), 3.46 (tt, J= 12.4, 3.2Hz, 1H), 3.90 (s, 3H), 7.08 (d, J= 8.4Hz, 1H), 7.83 (dd, J= 8.4, 2.4Hz, 1H), 7.90 (d, J= 2.4Hz, 1H).

### (16e)

### 4-(4-Propylpiperazin-1-yl)-3-(3,3,5,5-tetramethylcyclohexyl)benzoic acid methyl ester hydrochloride

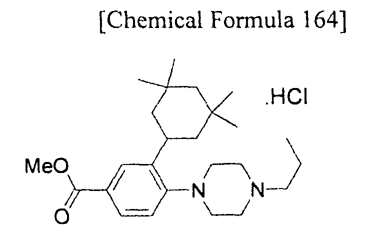

To a mixture of 4-piperazin-1-yl-3-(3,3,5,5-tetramethylcyclohexyl)benzoic acid methyl ester (178 mg, 0.496 mmol), propionaldehyde (0.07 mL, 0.97 mmol) and tetrahydrofuran (7 mL) were added sodium triacetoxyborohydride (260 mg, 1.227 mmol) and acetic acid (0.03 mL, 0.524 mmol) in that order at room temperature.
After stirring the reaction mixture for 1 hour, ethyl acetate, saturated aqueous sodium hydrogencarbonate and water were added to the reaction mixture and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/heptane) to give 168 mg of 4-(4-propylpiperazin-1-yl)-3-(3,3,5,5-tetramethylcyclohexyl)benzoic acid methyl ester as a white solid.
¹H-NMR (400MHz, CDCl₃)
δ: 0.92 (t, J= 8.0Hz, 3H), 0.92 (s, 6H), 1.11 (s, 6H), 1.17-1.46 (m, 6H), 1.51-1.63 (m, 2H), 2.39 (t, J= 8.0Hz, 2H), 2.63 (brs, 4H), 2.98 (t, J= 4.8Hz, 4H), 3.45 (tt, J= 12.4, 2.8Hz, 1H), 3.88 (s, 3H), 7.06 (d, J= 8.4Hz, 1H), 7.80 (dd, J= 8.4, 2.0Hz, 1H), 7.87 (d, J= 2.0Hz, 1H).
This compound was dissolved in ethyl acetate, and a 4N solution of hydrogen chloride in ethyl acetate was added. The solution was concentrated and diethyl ether and hexane were added to the resultant residue. The resultant precipitate was filtered and dried with a vacuum pump to give 124 mg of the title compound as colorless crystals.
MS m/e (ESI) 355(MH⁺).

### (Example 17)

### 4-[4-(4-Butylpiperazin-1-yl)-3-(3,3,5,5-tetramethylcyclohexyl)benzyl]morpholine dihydrochloride

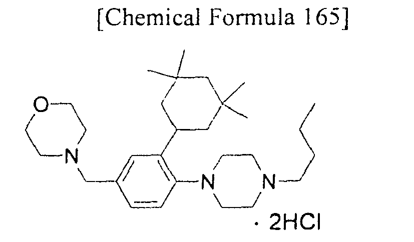

### (17a)

### 4-(4-Butylpiperazin-1-yl)-3-(3,3,5,5-tetramethylcyclohexyl)benzoic acid methyl ester

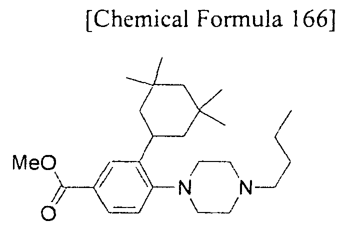

To a mixture of 4-piperazin-1-yl-3-(3,3,5,5-tetramethylcyclohexyl)benzoic acid methyl ester (180 mg, 0.502 mmol), butyraldehyde (0.09 mL, 1.01 mmol) and tetrahydrofuran (7 mL) were added sodium triacetoxyborohydride (265 mg, 1.25 mmol) and acetic acid (0.03 mL, 0.524 mmol) in that order at room temperature.
After stirring the reaction mixture for 1 hour, ethyl acetate, saturated aqueous sodium hydrogencarbonate and water were added to the reaction mixture and extraction was performed with ethyl acetate. The collected organic layer was washed with brine and then dried over anhydrous sodium sulfate. The desiccant was filtered off and the filtrate was concentrated under reduced pressure. The resultant residue was purified by NH silica gel column chromatography (ethyl acetate/heptane) to give 190 mg of the title compound as colorless crystals.
¹H-NMR (400MHz, CDCl₃)
δ: 0.94 (t, J= 8.0Hz, 3H), 0.94 (s, 6H), 1.11 (s, 6H), 1.16-1.57 (m, 10H), 2.37-2.43 (m, 2H), 2.61 (brs, 4H), 2.96 (t, J= 4.8Hz. 4H), 3.45 (tt, J= 12.4, 3.2Hz, 1H), 3.88 (s, 3H), 7.06 (d, J= 8.4Hz, 1H), 7.80 (dd, J= 8.4, 2.4Hz, 1H), 7.87 (d, J= 2.4Hz,
1H).

### (17b)

### 4-(4-Butylpiperazin-1-yl)-3-(3,3,5,5-tetramethycyclohexyl)benzoic acid

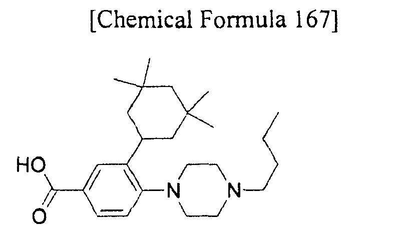

A mixture of 4-(4-butylpiperazin-1-yl)-3-(3,3,5,5-tetramethylcyclohexyl)benzoic acid methyl ester (158 mg, 0.381 mmol), 1N aqueous sodium hydroxide (0.8 mL) and methanol (3 mL) was heated to reflux for 6 hours and 40 minutes.
Saturated aqueous ammonium chloride, ethyl acetate and water were added to the reaction mixture and extraction was performed with ethyl acetate. The collected organic layer was washed with brine and then dried over anhydrous sodium sulfate. The desiccant was filtered off, and the filtrate was concentrated under reduced pressure to give 145 mg of a crude product of the title compound as colorless crystals.
¹H-NMR (400MHz, CDCl₃)
δ: 0.89 (s, 6H), 0.94 (t, J= 7.2Hz, 3H), 1.09 (s, 6H), 1.09-1.42 (m, 8H), 1.56-1.66 (m, 2H), 2.51-2.60 (m, 2H), 2.80 (brs, 4H), 3.01 (brs, 4H), 3.40 (tt, J= 12.0, 3.2Hz, 1H), 7.01 (d, J= 8.0Hz, 1H), 7.80 (dd, J= 8.4, 2.0Hz, 1H), 7.91 (d, J= 2.0Hz, 1H).

### (17c)

### [4-(4-Butylpiperazin-1-yl)-3-(3,3,5,5-tetramethylcyclohexyl)phenyl]morpholin-4-ylmethanone

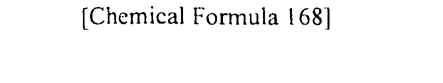

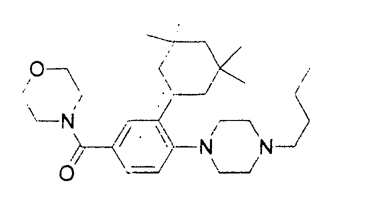

To a mixture of 4-(4-butylpiperazin-1-yl)-3-(3,3,5,5-tetramethylcyclohexyl)benzoic acid (131 mg, 0.327 mmol) and dimethylformamide (7 mL) were added 1-hydroxybenzotriazole monohydrate (137 mg, 1.01 mmol), morpholine (0.059 mL, 0.677 mmol), 1-ethyl-3-(3-dimethylaminopropyl)carbodiimide hydrochloride (194 mg, 1.01 mmol) and triethylamine (0.159 mL, 1.14 mmol) in that order at room temperature.
After stirring the reaction mixture for 67 hours and 45 minutes, ethyl acetate, saturated aqueous sodium hydrogencarbonate and water were added to the reaction mixture and extraction was performed with ethyl acetate. The collected organic layer was washed twice with water, washed once with brine, and then dried over anhydrous sodium sulfate. The desiccant was filtered off and the filtrate was concentrated under reduced pressure. The resultant residue was purified by NH silica gel column chromatography (ethyl acetate/heptane) to give 132 mg of the title compound as a colorless oil.
¹H-NMR (400MHz, CDCl₃)
δ: 0.92 (s, 6H), 0.94 (t, J= 7.2Hz, 3H), 1.11 (s, 6H), 1.15-1.56 (m, 10H), 2.36-2.43 (m, 2H), 2.60 (brs, 4H), 2.93 (t, J= 4.8Hz, 4H), 3.51 (tt. J= 12.4, 2.8Hz, 1H), 3.69 (brs, 8H), 7.07 (d, J= 8.0Hz, 1H), 7.17 (dd, J= 8.0, 2.0Hz, 1H), 7.26 (d, J= 2.0Hz,
1H).

### (17d)

### 4-[4-(4-Butylpiperazin-1-yl)-3-(3,3,5,5-tetramethylcyclohexyl)benzyl]morpholine, and [4-(4-Butylpiperazin-1-yl)-3-(3,3,5,5-tetramethylcyclohexyl)phenyl]methanol

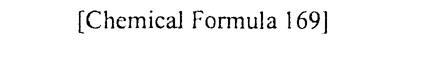

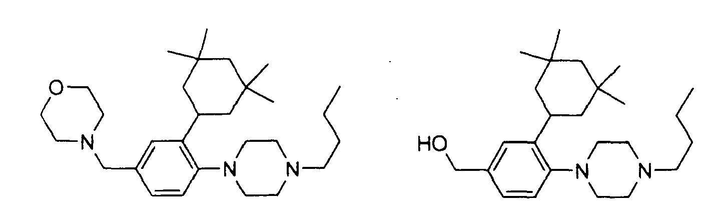

Lithium aluminum hydride (31 mg, 0.817 mmol) was suspended in anhydrous tetrahydrofuran (5 mL). To the suspension was added a solution of [4-(4-butylpiperazin-1-yl)-3-(3,3,5,5-tetramethylcyclohexyl)phenyl]morpholin-4-ylmethanone (97 mg, 0.206 mmol) in anhydrous tetrahydrofuran (5 mL) at room temperature. Next, the reaction mixture was stirred under a nitrogen atmosphere while heating to reflux for 4 hours and 40 minutes, and then lithium aluminum hydride (31 mg, 0.817 mmol) was added and the mixture was further stirred for 4 hours while heating to reflux.
Sodium fluoride (600 mg) was added to the reaction mixture, and water (0.24 mL) was gradually added while blowing nitrogen gas. After stirring the reaction mixture for 50 minutes, the insoluble matters were filtered off and the filtrate was concentrated under reduced pressure. The resultant residue was purified by NH silica gel column chromatography (ethyl acetate/heptane) to give 23 mg of 4-[4-(4-butylpiperazin-1-yl)-3-(3,3,5,5-tetramethylcyclohexyl)benzyl]morpholine as a colorless oil and 53 mg of [4-(4-butylpiperazin-1-yl)-3-(3,3,5,5-tetramethylcyclohexyl)phenyl]methanol as colorless crystals.
4-[4-(4-butylpiperazin-1-yl)-3-(3,3,5,5-tetramethylcyclohexyl)benzyl]morpholine:
¹H-NMR (400MHz, CDCl₃)
δ: 0.93 (s, 6H), 0.94 (t, J= 7.2Hz, 3H), 1.09 (s, 6H), 1.13-1.57 (m, 10H), 2.35-2.46 (m, 6H), 2.59 (brs, 4H), 2.91 (t, J= 4.8Hz, 4H), 3.44 (s, 2H), 3.51 1 (tt, J= 12.4, 3.2Hz, 1H), 3.71 (t, J= 4.8Hz, 4H), 7.06 (d, J= 8.0Hz. 1H), 7.07-7.12 (m, 2H).
[4-(4-butylpiperazin-1-yl)-3-(3,3,5,5-tetramethylcyclohexyl)phenyl]methanol:
¹H-NMR (400MHz, CDCl₃)
δ: 0.93 (s, 6H), 0.95 (t, J= 7.2Hz, 3H), 1.12 (s, 6H), 1.12-1.37 (m, 10H), 2.35-2.43 (m, 2H), 2.60 (brs, 4H), 2.90 (t, J= 4.8Hz, 4H), 3.56 (tt, J= 12.8, 3.2Hz, 1H), 4.63 (s, 2H), 7.10 (d. J= 8.0Hz, 1H), 7.15 (dd. J= 8.0. 1.6Hz. 1H), 7.23 (d. J= 1.6Hz. 1H).

### (17e)

### 4-[4-(4-Butylpiperazin-1-yl)-3-(3,3,5,5-tetramethylcyclohexyl)benzyl]morpholine dihydrochloride

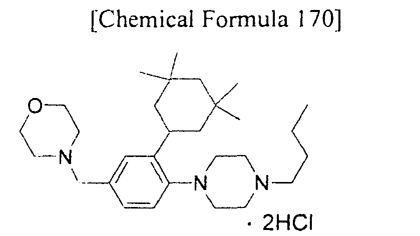

After dissolving 4-[4-(4-butylpiperazin-1-yl)-3-(3,3,5,5-tetramethylcyclohexyl)benzyl]morpholine (23 mg, 0.05 mmol) in dichloromethane (2 mL), a 4N solution of hydrogen chloride in ethyl acetate (0.04 mL) was added thereto. The mixture was concentrated by blowing nitrogen gas, and then diethyl ether was added to the resultant residue and the resultant precipitate was triturated by sonication. After removing the supernatant, the precipitate was dried to give 31 mg of the title compound as a colorless solid.
MS m/e (ESI) 442(MH⁺).

### (Example 18)

### 1-Butyl-4-[4-methoxymethyl-2-(3,3,5,5-tetramethylcyclohexyl)phenyl]piperazine hydrochloride

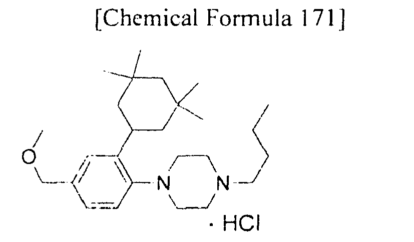

Sodium hydride (60% dispersion in oil, 8 mg, 0.2 mmol) was suspended in dimethylformamide (1 mL), Followed by stirring at room temperature under a nitrogen atmosphere. A solution of [4-(4-butylpiperazin-1-yl)-3-(3,3,5,5-tetramethylcyclohexyl)phenyl]methanol (51 mg, 0.132 mmol) in dimethylformamide (2 mL) was added to the suspension. The reaction mixture was stirred for 20 minutes, and then the reaction mixture was cooled in ice water and iodomethane (0.013 mL, 0.2 mmol) was added. Next, the reaction mixture was stirred at room temperature for 3 hours.
The reaction mixture was cooled in ice water, and then water was added. Next, ethyl acetate was added and extraction was performed with ethyl acetate. The collected organic layer was washed twice with water, washed once with brine, and then dried over anhydrous sodium sulfate. The desiccant was filtered off and the filtrate was concentrated under reduced pressure. The resultant residue was purified by NH silica gel column chromatography (ethyl acetate/heptane) to give 30 mg of 1-butyl-4-[4-methoxymethyl-2-(3,3,5,5-tetramethylcyclohexyl)phenyl]piperazine as a colorless oil.
¹H-NMR (400MHz, CDCl₃)
δ: 0.92 (s, 6H), 0.94 (t, J= 7.2Hz, 3H), 1.12 (s, 6H), 1.12-1.57 (m, 10H), 2.35-2.42 (m, 2H), 2.60 (brs, 4H), 2.91 (t, J= 4.8Hz, 4H), 3.37 (s, 3H), 3.55 (tt, J= 12.4, 2.8Hz, 1H), 4.38 (s, 2H), 7.08 (brs, 2H), 7.17 (brs, 1H).
This compound was dissolved in dichloromethane, and a 4N solution of hydrogen chloride in ethyl acetate was added. The solution was concentrated by blowing nitrogen gas, and then diethyl ether was added to the resultant residue and the resultant precipitate was triturated by sonication. After removing the supernatant, the precipitate was dried to give 33 mg of the title compound as a light brown solid.
MS m/e (ESI) 401(MH⁺).

### (Example 19)

### 1-[4-(4-Butylpiperazin-1-yl)-3-(3,3,5,5-tetramethylcyclohexyl)pheny)]-3-methoxypropan-1-one hydrochloride

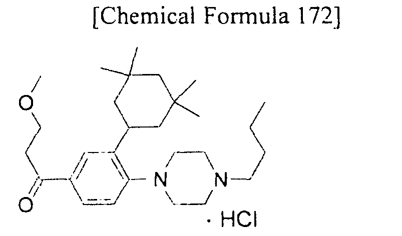

### (19a)

### 4-[4-Bromo-2-(3,3,5,5-tetramethylcyclohexyl)phenyl]piperaxine-1-carboxylic acid t-butyl ester

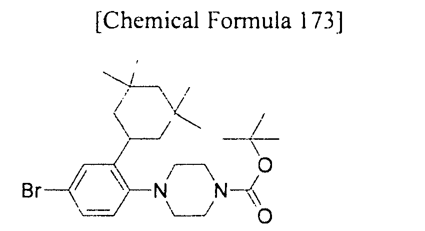

A mixture of 4-[2-(3,3,5,5-tetramethylcyclohexyl)phenyl]piperazine-1-carboxylic acid t-butyl ester (2.93 g, 7.31 mmol) produced in Production Example 5, sodium acetate (6 g, 73.14 mmol) and methanol (50 mL) was stirred at room temperature under a nitrogen atmosphere. Bromine (0.37 mL, 7.22 mmol) was then added to the mixture.
After stirring the reaction mixture for 20 minutes, saturated aqueous sodium thiosulfate, ethyl acetate and water were added to the reaction mixture, and extraction was performed with ethyl acetate. The collected organic layer was washed with brine and then dried over anhydrous sodium sulfate. The desiccant was filtered off and the filtrate was concentrated under reduced pressure. The resultant residue was purified by silica gel column chromatography (ethyl acetate/hexane) to give 2.67 g of the title compound as colorless crystals.
¹H-NMR (400MHz, CDCl₃)
δ: 0.93 (s, 6H), 1.10 (s, 6H), 1. 12-1.45 (m. 6H), 1.49 (s, 9H), 2.79 (brs, 4H), 3.48 (br, 4H), 3.54 (tt, J= 12.4, 2.8Hz, 1H), 6.93 (d, J= 8.4Hz, 1 H), 7.25 (dd, J= 8.4, 2.4Hz, 1H), 7.32 (d. J= 2.4Hz. 1H).

### (19b)

### 4-[4-(3-Methoxyprop-1-ynyl)-2-(3,3,5,5-tetramethylcyclohexyl)phenyl]piperazine-1-carboxylic acid t-butyl ester

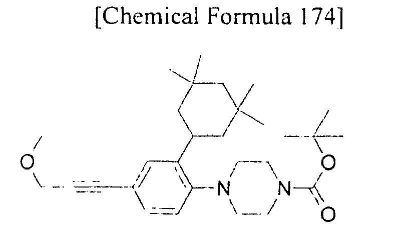

To a mixture of 4-[4-bromo-2-(3,3,5,5-tetramethylcyclohexyl)phenyl]piperazine-1-carboxylic acid t-butyl ester (150 mg, 0.313 mmol), methylpropargyl ether (0.08 mL, 0.947 mmol), copper(I) iodide (5 mg, 0.026 mmol) and triethylamine (3 mL) was added dichlorobis(triphenylphosphine)palladium(II) (11 mg, 0.016 mmol), followed by stirring at an external temperature of 80°C under a nitrogen atmosphere.
After stirring the reaction mixture for 2 hours and 30 minutes, methylpropargyl ether (0.12 mL, 1.42 mmol) and dichlorobis(triphenylphosphine)palladium(II) (15 mg, 0.021 mmol) were further added, followed by stirring for 2 hours and 30 minutes under the same conditions.
Ethyl acetate and water were added to the reaction mixture which was then filtered. To the filtrate was added 5% aqueous potassium hydrogensulfate, and extraction was performed with ethyl acetate. The collected organic layer was washed with 5% aqueous potassium hydrogensulfate, saturated aqueous sodium hydrogencarbonate and brine in that order and then dried over anhydrous sodium sulfate. The desiccant was filtered off and the filtrate was concentrated under reduced pressure. The resultant residue was purified by silica gel column chromatography (ethyl acetate/heptane) to give 106 mg of the title compound as a yellowish-brown solid.
¹H-NMR (400MHz, CDCl₃)
δ: 0.93 (s, 6H), 1.10 (s, 6H), 1.12-1.45 (m, 6H), 1.49 (s, 9H), 2.82 (brs, 4H), 3.45 (s, 3H), 3.49 (tt, J= 12.8, 2.8Hz, 1H), 3.56 (br, 4H), 4.31 (s, 2H), 6.97 (d, J= 8.4Hz, 1H), 7.24 (dd, J= 8.4, 1.6Hz, 1H), 7.34 (d, J= 1.6Hz, 1H).

### (19c)

### 1-[4-(3-Methoxyprop-1-ynyl)-2-(3,3,5,5-tetramethylcyclohexyl)phenyl]piperazine, and 3-Methoxy-1-[4-piperazin-1-yl-3-(3,3,5,5-tetramethylcyclohexyl)phenyl]propan-1-one

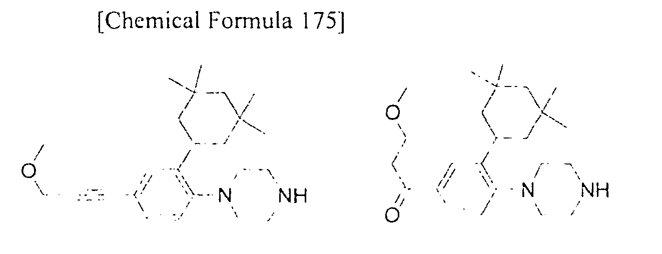

A mixture of 4-[4-(3-methoxyprop-l-yny!)-2-(3.3.5.5-tetramethylcyclohexyl)phenyl]piperazine-1-carboxylic acid t-butyl ester (104 mg, 0.222 mmol), trifluoroacetic acid (0.5 mL, 3.89 mmol) and dichloromethane (1 mL) was stirred at room temperature for 3 hours and 30 minutes.
The reaction mixture was cooled in ice water, and was made basic with addition of 5N aqueous sodium hydroxide. Next, ethyl acetate and water were added to the reaction mixture and extraction was performed with ethyl acetate. The collected organic layer was washed with water and brine and then dried over anhydrous sodium sulfate. The desiccant was filtered off and the filtrate was concentrated under reduced pressure. The resultant residue was purified by NH silica gel column chromatography (ethyl acetate/heptane) to give 26 mg of 1-[4-(3-methoxyprop-1-ynyl)-2-(3,3,5,5-tetramethylcyclohexyl)phenyl]piperazine as a light brown oil and 31 mg of 3-methoxy-1-[4-piperazin-1-yl-3-(3,3,5,5-tetramethylcyclohexyl)phenyl]propan-1-one as a light yellow oil.
1-[4-(3-methoxyprop-1-ynyl)-2-(3,3,5,5-tetramethylcyclohexyl)phenyl]piperazine:
¹H-NMR (400MHz, CDCl₃)
δ: 0.92 (s, 6H), 1.11 (s, 6H), 1.12-1.46 (m, 6H), 2.84 (t, J= 4.8Hz, 4H), 3.03 (t, J= 4.8Hz, 4H), 3.45 (s, 3H), 3.50 (tt, J= 12.8, 2.8Hz, 1H), 4.31 (s, 2H), 7.00 (d, J= 8.0Hz, 1H), 7.24 (dd, J= 8.0, 2.0Hz, 1H), 7.32 (d, J= 2.0Hz, 1H).
3-methoxy-1-[4-piperazin-1-yl-3-(3.3,5,5-tetramethylcyclohexyl)phenyl]propan-1-one:
¹H-NMR (400MHz, CDCl₃)
δ: 0.94 (s, 6H), 1.12 (s, 6H), 1.14-1.45 (m, 6H), 2.91 (t, J= 4.8Hz, 4H), 3.05 (t, J= 4.8Hz, 4H), 3.23 (t, J= 6.8Hz, 2H), 3.39 (s, 3H), 3.47 (tt, J= 12.8, 3.2Hz, 1H), 3.82 (t, J= 6.8Hz, 2H), 7.07 (d, J= 8.4Hz, 1H). 7.76 (dd, J= 8.4, 2.0Hz, 1H), 7.87 (d, J= 2.0Hz, 1H).

### (19d)

### 1-[4-(4-Butylpiperazin-1-yl)-3-(3,3,5,5-tetramethylcyclohexyl)phenyl]-3-methoxypropan-1-one hydrochloride

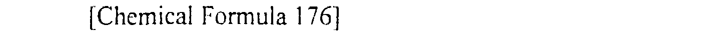

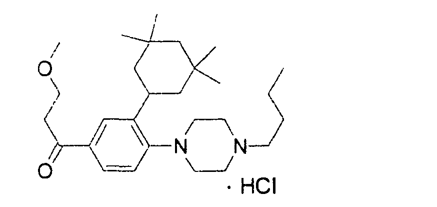

To a mixture of 3-methoxy-1-[4-piperazin-1-yl-3-(3,3,5,5-tetramethylcyclohexyl)phenyl]propan-1-one (10 mg, 0.026 mmol), butyraldehyde (0.006 mL, 0.067 mmol) and tetrahydrofuran (2 mL) were added sodium triacetoxyborohydride (14 mg, 0.066 mmol) and acetic acid (0.002 mL, 0.035 mmol) in that order at room temperature.
After stirring the reaction mixture for 1 hour, ethyl acetate, saturated aqueous sodium hydrogencarbonate and water were added to the reaction mixture and extraction was performed with ethyl acetate. The collected organic layer was concentrated by blowing nitrogen gas. The resultant residue was purified by NH silica gel column chromatography (ethyl acetate/hexane) to give 1-[4-(4-butylpiperazin-1-yl)-3-(3,3,5,5-tetramethylcyclohexyl)phenyl]-3-methoxypropan-1-one.
¹H-NMR (400MHz, CDCl₃)
δ: 0.93 (s, 6H), 0.94 (t, J= 7.2Hz, 3H), 1.11 (s, 6H), 1.15-1.56 (m, 10H), 2.36-2.44 (m, 2H), 2.62 (brs, 4H), 2.98 (t, J= 4.8Hz, 4H), 3.22 (t, J= 6.8Hz, 2H), 3.39 (s, 3H), 3.44 (tt, J= 12.8, 3.2Hz, 1H), 3.82 (t, J= 6.8Hz, 2H), 7.08 (d, J= 8.4Hz, 1H), 7.75 (dd, J= 8.4, 2.0Hz, 1H), 7.86 (d, J= 2.0Hz, 1H).
This compound was dissolved in dichloromethane, and a 4N solution of hydrogen chloride in ethyl acetate was added. The solution was concentrated by blowing nitrogen gas, and then diethyl ether was added to the resultant residue and the resultant precipitate was triturated by sonication. After removing the supernatant, the precipitate was dried to give 9 mg of the title compound as a white solid.
MS m/e (ESI) 443(MH⁺).

### (Example 20)

### 1-Butyl-4-[4-(3-methoxyprop-1-ynyl)-2-(3,3,5,5-tetramethylcyclohexyl)phenyl]piperazine hydrochloride

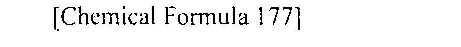

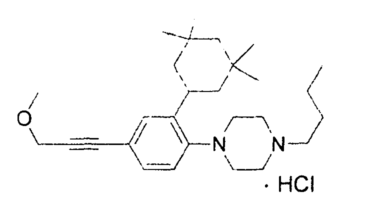

To a mixture of 1-[4-(3-methoxyprop-1-ynyl)-2-(3,3,5,5-tetramethylcyclohexyl)phenyl]piperazine (8.7 mg, 0.024 mmol), butyraldehyde (0.005 mL, 0.056 mmol) and tetrahydrofuran (2 mL) were added sodium triacetoxyborohydride (13 mg, 0.061 mmol) and acetic acid (0.002 mL, 0.035 mmol) in that order at room temperature.
After stirring for 1 hour, ethyl acetate, saturated aqueous sodium hydrogencarbonate and water were added to the reaction mixture and extraction was performed with ethyl acetate. The collected organic layer was concentrated by blowing nitrogen gas. The resultant residue was purified by NH silica gel column chromatography (ethyl acetate/hexane) to give 1-butyl-4-[4-(3-methoxyprop-1-ynyl)-2-(3,3,5,5-tetramethylcyclohexyl)phenyl]piperazine.
¹H-NMR (400MHz, CDCl₃)
δ: 0.92 (s, 6H), 0.94 (t, J= 7.2Hz, 3H), 1.11 (s, 6H), 1.13-1.56 (m. 10H), 2.36-2.43 (m, 2H), 2.60 (brs, 4H), 2.92 (t, J= 4.8Hz, 4H), 3.45 (s, 3H), 3.44 (tt, J= 12.4, 3.2Hz, 1H), 4.31 (s, 2H), 7.01 (d, J= 8.4Hz, 1H), 7.23 (dd, J= 8.4, 2.0Hz, 1H), 7.32 (d, J= 2.0Hz, 1H).
This compound was dissolved in dichloromethane, and a 4N solution of hydrogen chloride in ethyl acetate was added. The solution was concentrated by blowing nitrogen gas, and then diethyl ether was added to the resultant residue and the resultant precipitate was triturated by sonication. After removing the supernatant, the precipitate was dried to give 8 mg of the title compound as a white solid.
MS m/e (ESI) 425(MH⁺).

The following compounds were produced by the general production methods described above, the methods described in the production examples and examples, or combinations of these methods with well-known methods.

### (Example 21)

### {3-(4-t-Butylcyclohex-1-enyl)-4-(4-butylpiperazin-1-yl)phenyl}-methyl-(tetrahydropyran-4-yl)amine hydrochloride

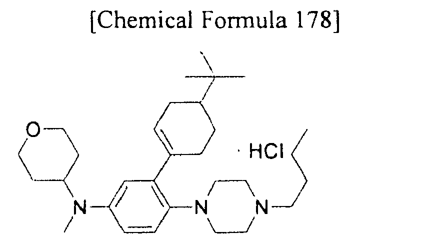

MS m/e (ESI) 468(MH⁺).

### (Example 22)

### {3-(4-t-Butylcyclohexyl)-4-(4-butylpiperazin-1-yl)phenyl}-methyl-(tetrahydropyran-4-yl)amine hydrochloride

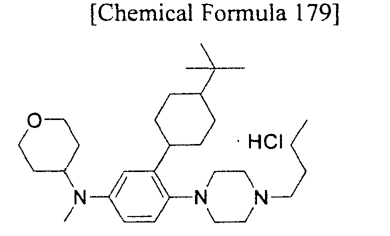

MS m/e (ESI) 470(MH⁺).

### (Example 23)

### 1-Cyclopropylmethyl-4-{2-(4,4-diethylcyclohexyl)-4-(4-methoxymethylpiperidin-1-yl)phenyl}piperazine hydrochloride

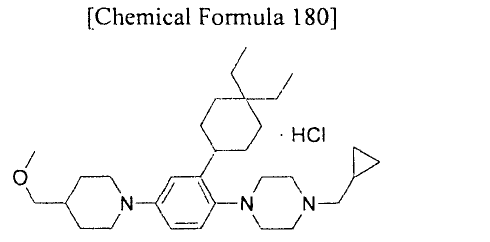

MS m/e (ESI) 482(MH⁺).

### (Example 24)

### 1-{3-(4-t-Butylcyclohexyl)-4-(4-butylpiperazin-1-yl)phenyl}piperidin-4-yloxyacetic acid

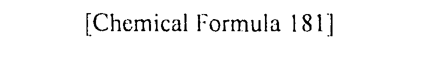

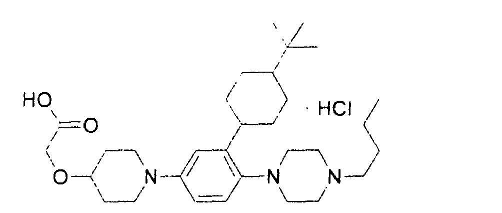

MS m/e (ESI) 514(MH⁺).

### (Example 25)

### 1-Butyl-4-{4-(4-methoxymethylpiperidin-1-yl)-2-(3,3,5,5-tetramethylcyclohexyl)phenyl}piperazine hydrochloride

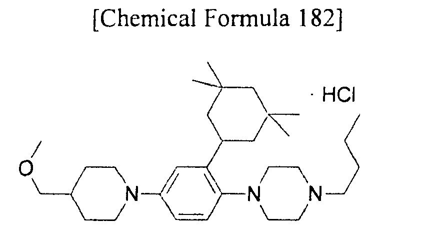

MS m/e (ESI) 484(MH⁺).

### (Example 26)

### 1-Butyl-4-[2-(4-t-butylcyclohexyl)-5-(2-methoxyethoxy)phenyl]piperazine hydrochloride

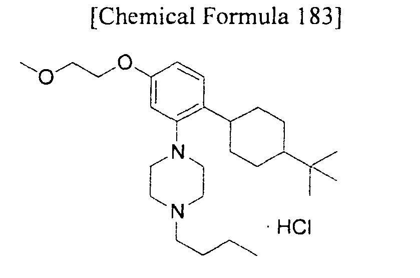

MS m/e (ESI) 431(MH⁺).

### (Example 27)

### [4-(4-t-Butylcyclohexyl)-3-(4-butylpiperazin-1-yl)phenyl]methyl(tetrahydropyran-4-yl)amine hydrochloride

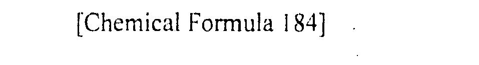

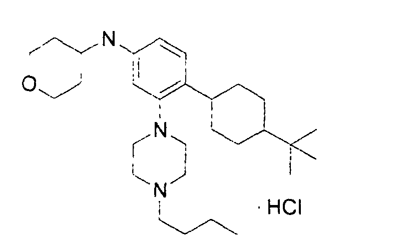

MS m/e (ESI) 470(MH⁺).

### (Example 28)

### 6-(4-t-Butylcyclohexyl)-4-methyl-7-(4propylpiperazin-1-yl)3,4-dihydro-2H-benzo[1,4]oxazine hydrochloride

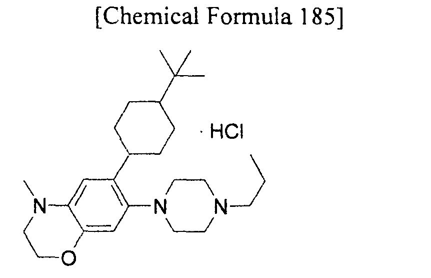

MS m/e (ESI) 414(MH⁺).

### (Example 29)

### 6-(4-t-Butylcyclohex-1-enyl)-7-(4-cyclopropylmethylpiperazin-1-yl)-4-methyl-3,4-dihydro-2H-benzo[1,4]oxazine hydrochloride

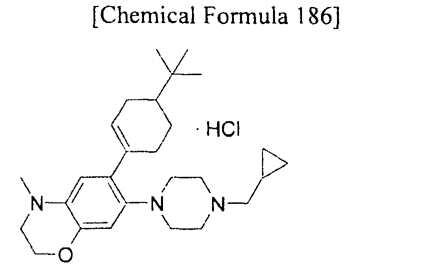

MS m/e (ESI) 424(MH⁺).

### (Example 30)

### 6-(4-t-Butylcyclohex-1-enyl)-4-methyl-7-[4-(tetrahydropyran-4-ylmethyl)piperazin-1-yl)-3,4-dihydro-2H-benzo[1,4]oxazine hydrochloride

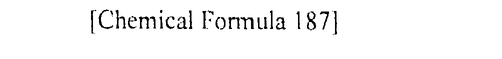

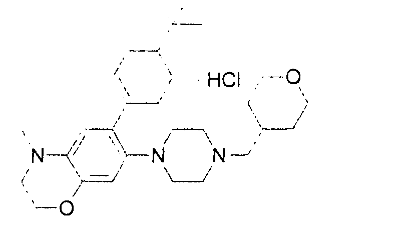

MS m/e (ESI) 468(MH⁺).

### (Example 31)

### [3-(4-t-Butylcyclohexyl)-2-(4-butylpiperazin-1-yl)phenyl](tetrahydropyran-4-yl)amine hydrochloride

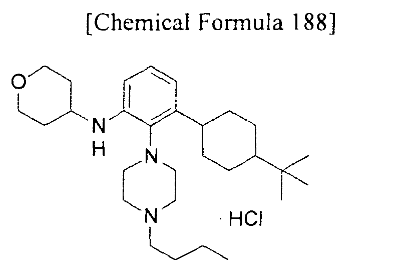

MS m/e (ESI) 456(MH⁺).

### (Example 32)

### 6-(4-t-Butylcyclohexyl)-7-(4-isobutylpiperazin-1-yl)-4-methyl-3,4-dihydro-2H-benzo[1,4]oxazine hydrochloride

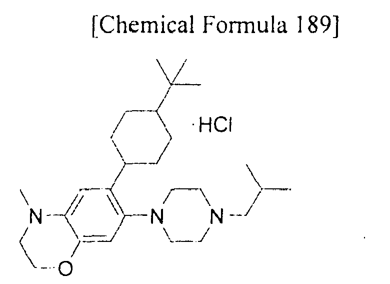

MS m/e (ESI) 428(MH⁺).

### (Example 33)

### 6-(4-t-Butylcyclohexyl)-7-(4-cyclopropylmethylpiperazin-1-yl)-4-methyl-3,4-dihydro-2H-benzo[1,4]oxazine hydrochloride

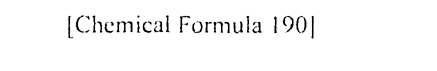

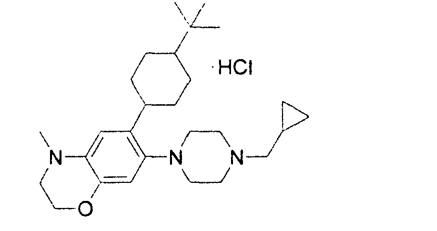

MS m/e (ESI) 426(MH⁺).

### (Example 34)

### 6-(4-t-Butylcyclohexyl)-4-methyl-7-[4-(tetrahydropyran-4-ylmethyl)piperazin-1-yl]-3,4-dihydro-2H-benzo[1,4]oxazine hydrochloride

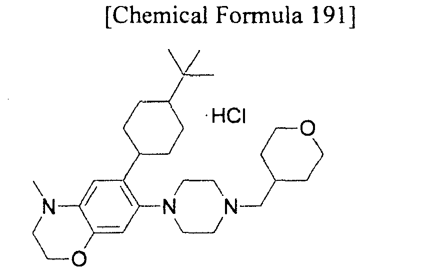

MS m/e (ESI) 470(MH⁺).

### (Example 35)

### 6-(4-t-Butylcyclohexyl)-4-methyl-7-[4-(3-methylbutyl)piperazin-1-yl]-3,4-dihydro-2H-benzo[1,4]oxazine hydrochloride

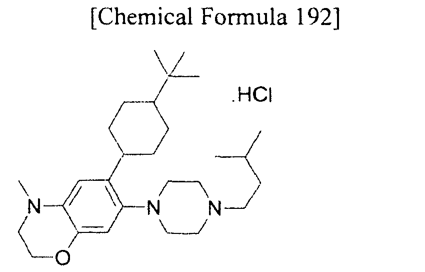

MS m/e (ESI) 442(MH⁺).

### (Example 36)

### 4-Benzyl-6-(4-t-butylcyclohex-1-enyl)-7-(4-butylpiperazin-1-yl)-4H-benzo[1,4]oxazin-3-one hydrochloride

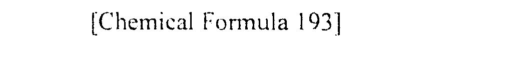

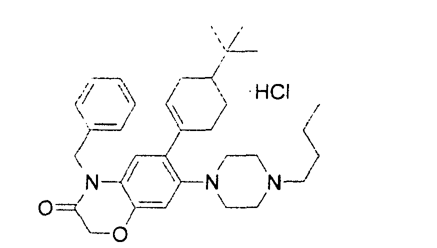

MS m/e (ESI) 516(MH⁺).

### (Example 37)

### 6-(4-t-Butylcyclohex-1-enyl)-7-(4-butylpiperazin-1-yl)-4-methyl-3,4-dihydro-2H-benzo[1,4]oxazine hydrochloride

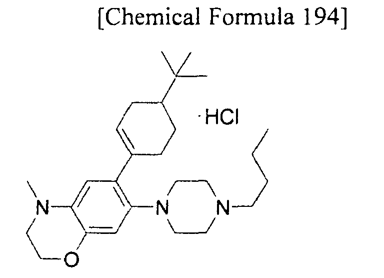

MS m/e (ESI) 426(MH⁺).

### (Example 38)

### 6-(4-t-Butylcyclohex-1-enyl)-4-methyl-7-(4-pentylpiperazin-1-yl)-3,4-dihydro-2H-benzo[1,4]oxazine hydrochloride

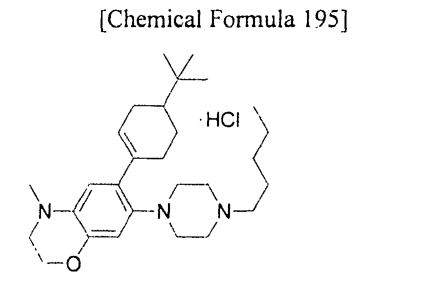

MS m/e (ESI) 440(MH⁺).

### (Example 39)

### 6-(4-t-Butylcyclohex-1-enyl)-7-(4-isobutylpiperazin-1-yl)-4-methyl-3,4-dihydro-2H-benzo[1,4]oxazine hydrochloride

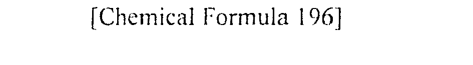

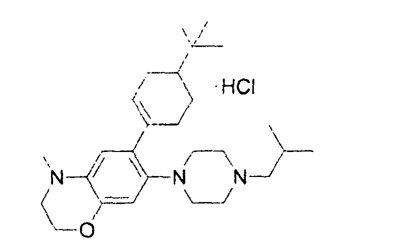

MS m/e (ESI) 426(MH⁺).

### (Example 40)

### 6-(4-t-Butylcyclohex-1-enyl)-4-methyl-7-[4-(3-methylbutyl)piperazin-1-yl]-3,4-dihydro-2H-benzo[1,4]oxazine hydrochloride

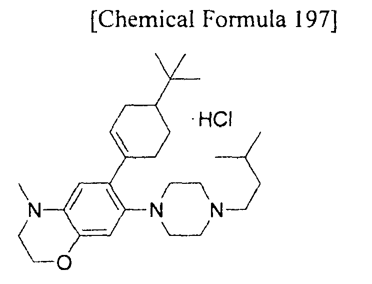

MS m/e (ESI) 440(MH⁺).

### (Example 41)

### (2-Methoxyethyl)methyl[3-(4-propylpiperazin-1-yl)-4-(3,3,5,5-tetramethylcyclohexyl)phenyl]amine hydrochloride

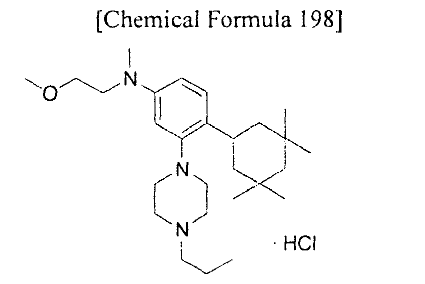

MS m/e (ESI) 430(MH⁺).

### (Example 42)

### [3-(4-Cyclopropylmethylpiperazin-1-yl)-4-(3,3,5,5-tetramethylcyclohexyl)phenyl](2-methoxyethyl)methylamine hydrochloride

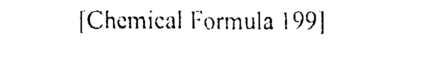

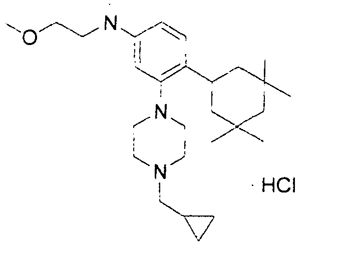

MS m/e (ESI) 442(MH⁺).

### (Example 43)

### 1-[5-(2-Methoxyethoxy)-2-(3,3,5,5-tetramethylcyclohexyl)phenyl]-4-propylpiperazine hydrochloride

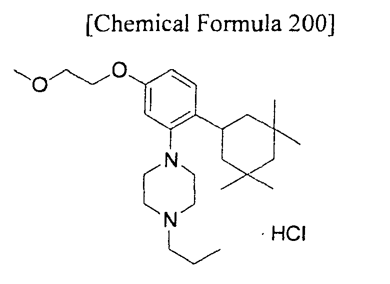

MS m/e (ESI) 417(MH⁺).

### (Example 44)

### (S)-1-[2-(4,4-Diethylcyclohexyl)-4-(2-methoxymethylpyrrolidin-1-yl)phenyl]-4-propylpiperazine hydrochloride

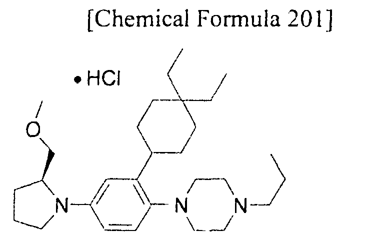

MS m/e (ESI) 456(MH⁺).

### (Example 45)

### (S)-1-Cyclopropylmethyl-4-[2-(4,4-diethylcyclohexyl)-4-(2-methoxymethylpyrrolidin-1-yl)phenyl]piperazine hydrochloride

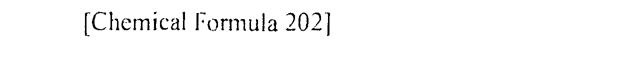

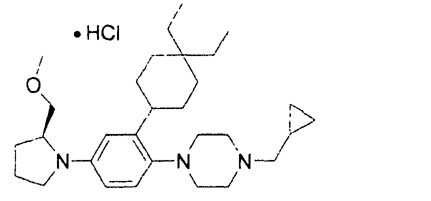

MS m/e (ESI) 468(MH⁺).

### (Example 46)

### 1-Butyl-4-[4-(2-methoxyethoxy)-2-(3,3,5,5-tetramethylcyclohexyl)phenyl]piperazine hydrochloride

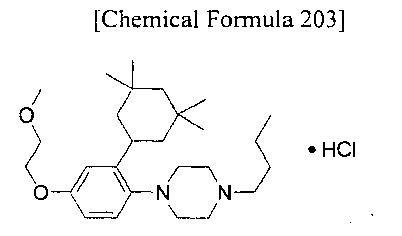

MS m/e (ESI) 431 (MH⁺).

### (Example 47)

### 1-Isobutyl-4-[4-(2-methoxyethoxy)-2-(3,3,5,5-tetramethylcyclohexyl)phenyl]piperazine hydrochloride

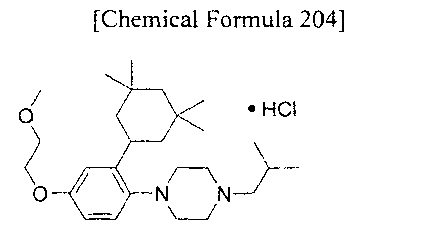

MS m/e (ESI) 431 (MH⁺).

### (Example 48)

### 1-[4-(2-Methoxyethoxy)-2-(3,3,5,5-tetramethylcyclohexyl)phenyl]-4-(tetrahydropyran-4-ylmethyl)piperazine hydrochloride

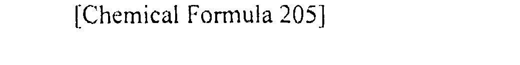

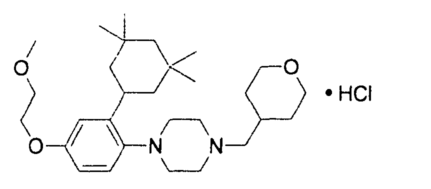

MS m/e (ESI) 473(MH⁺).

### (Example 49)

### 1-Propyl-4-[6-(3,3,5,5-tetramethylcyclohex-1-enyl)benzo[1,3]dioxol-5-yl]piperazine hydrochloride

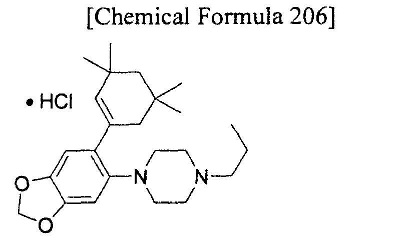

MS m/e (ESI) 385(MH⁺).

### (Example 50)

### 1-Cyclopropylmethyl-4-[6-(3,3,5,5-tetramethylcyclohex-1-enyl)benzo[1,3]dioxol-5-yl]piperazine hydrochloride

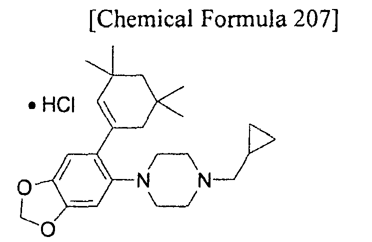

MS m/e (ESI) 397(MH⁺).

### (Example 51)

### 1-Propyl-4-[6-(3,3,5,5-tetramethylcyclohexyl)benzo[1,3]dioxol-5-yl]piperazine hydrochloride

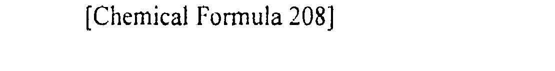

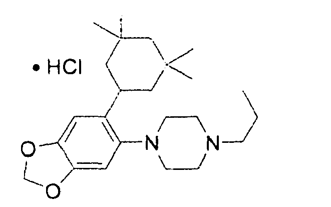

MS m/e (ESI) 387(MH⁺).

### (Example 52)

### 1-Propyl-4-[7-(3,3,5,5-tetramethylcyclohexyl)-2,3-dihydrobenzo[1,4]dioxin-6-yl]piperazine hydrochloride

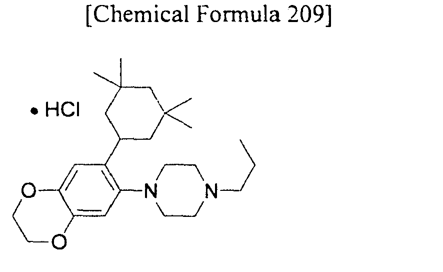

MS m/e (ESI) 401(MH⁺).

### (Example 53)

### 1-Pentyl-4-[7-(3,3,5,5-tetramethylcyclohexyl)-2,3-dihydrobenzo[1,4]dioxin-6-yl]piperazine hydrochloride

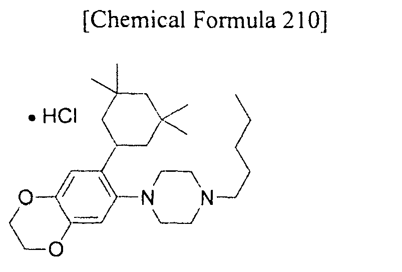

MS m/e (ESI) 429(MH⁺).

### (Example 54)

### 1-Cyclopropylmethyl-4-[7-(3,3,5,5-tetramethylcyclohexl)-2,3-dihydrobenzo[1.4]dioxin-6-yl]piperazine hydrochloride

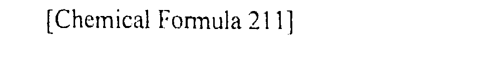

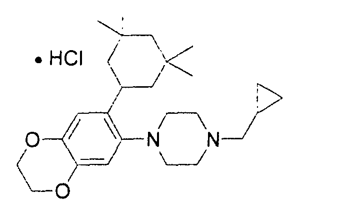

MS m/e (ESI) 413(MH⁺).

### (Example 55)

### 1-(Tetrahydropyran-4-ylmethyl)-4-[7-(3,3,5,5-tetramethylcyclohexyl)-2,3-dihydrobenzo[1,4]dioxin-6-yl]piperazine hydrochloride

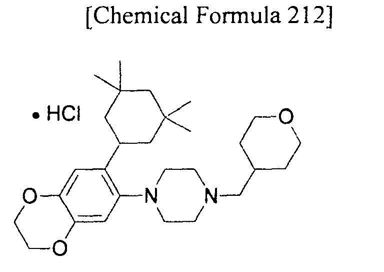

MS m/e (ESI) 457(MH⁺).

### (Example 56)

### 1-Cyclopropylmethyl-4-[6-(3,3,5,5-tetramethylcyclohexyl)benzo[1.3]dioxol-5-yl]piperazine hydrochloride

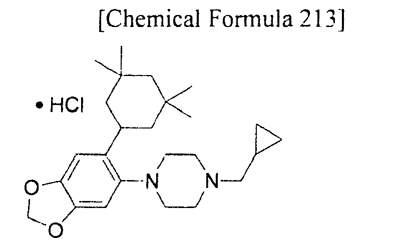

MS m/e (ESI) 399(MH⁺).

### (Example 57)

### 4-(4-Cyclopropylmethylpiperazin-1-yl)-3-(3,3,5,5-tetramethylcyclohexyl)benzoic acid methyl ester hydrochloride

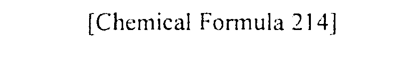

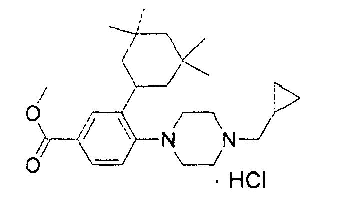

MS m/e (ESI) 413(MH⁺).

### (Example 58)

### 3-Methoxy-1-[4-(4-propylpiperazin-1-yl)-3-(3,3,5,5-tetramethylcyclohexyl)phenyl]propan-1-one hydrochloride

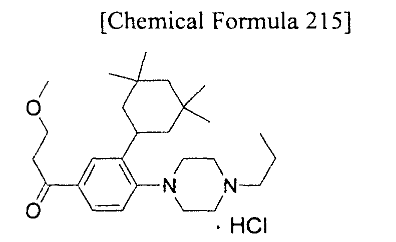

MS m/e (ESI) 429(MH⁺).

### (Example 59)

### 4-[4-(4-Propylpiperazin-1-yl)-3-(3,3,5,5-tetramethylcyclohexyl)benzyl]morpholine dihydrochloride

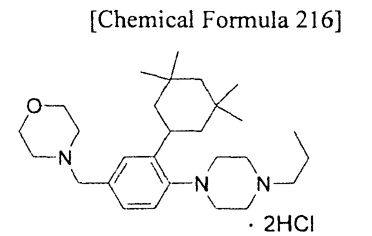

MS m/e (ESI) 442(MH⁺).

### (Example 60)

### 4-[4-(4-Cyclopropylmethylpiperazin-1-yl)-3-(3,3,5,5-tetramethylcyclohexyl)benzyl]morpholine dihydrochloride

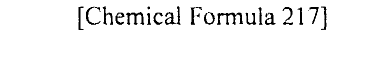

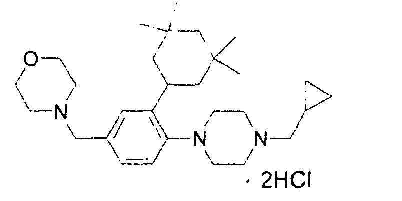

MS m/e (ESI) 454(MH⁺).

### (Example 61)

### 1-[4-Methoxymethyl-2-(3,3,5,5-tetramethylcyclohexyl)phenyl]-4-propylpiperazine hydrochloride

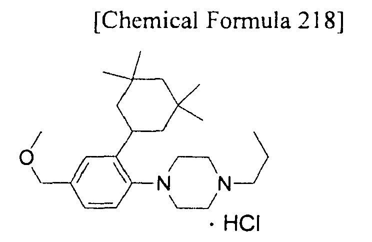

MS m/e (ESI) 387(MH⁺).

### (Example 62)

### 1-Cyclopropylmethyl-4-[4-methoxymethyl-2-(3,3,5,5-tetramethylcyclohexyl)phenyl]piperazine hydrochloride

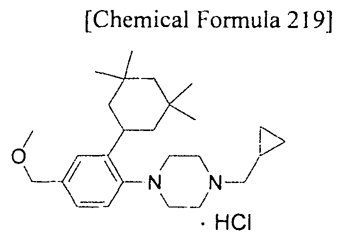

MS m/e (ESI) 399(MH⁺).

### (Example 63)

### 4-(4-Butylerazin-1-yl)-3-(3,3,5,5-tetramethylcyclohexyl)benzoic acid methyl ester hydrochloride

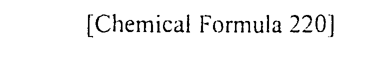

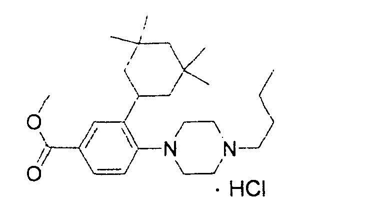

MS m/e (EST) 415(MH⁺).

### (Example 64)

### 1-[4-(3-Methoxyprop-1-ynyl)-2-(3,3,5,5-tetramethylcyclohexyl)phenyl]-4-propylpiperazine hydrochloride

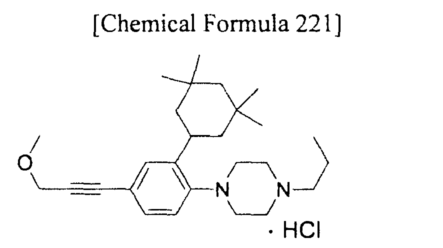

MS m/e (ESI) 411 (MH⁺).

### (Example 65)

### 1-Cyclopropylmethyl-4-[4-(3-methoxyprop-1-ynyl)-2-(3,3,5,5-tetramethylcyclohexyl)phenyl]piperazine hydrochloride

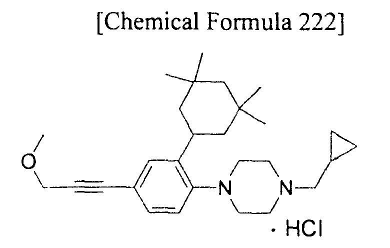

MS m/e (ESI) 423(MH⁺).

### (Example 66)

### 4-(4-t-Butylcyclohexyl)-3-(4-butylpiperazin-1-yl)benzoic acid methyl ester hydrochloride

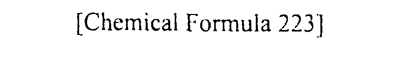

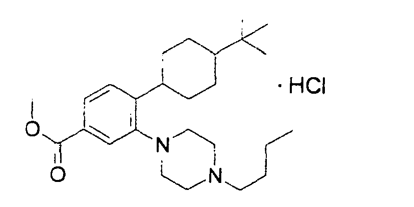

MS m/e (ESI) 415(MH⁺).

### (Example 67)

### 4-(4-t-Butylcyclohexyl)-3-(4-butylpiperazin-1-yl)benzoic acid

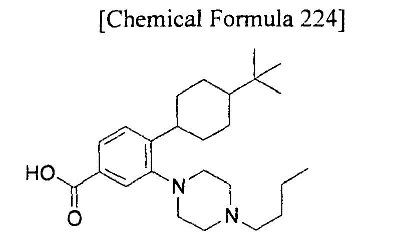

MS m/e (ESI) 401 (MH⁺).

### (Example 68)

### 1-[5-(4-t-Butylcyclohexyl)-4-(4-butylpiperazin-1-yl)-2-methoxyphenyl]piperidin-4-ol hydrochloride

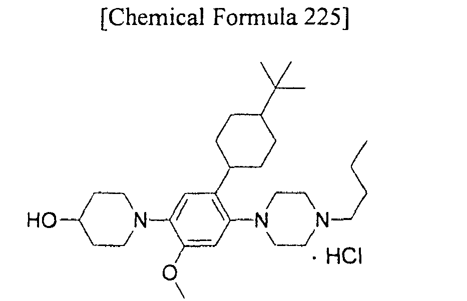

MS m/e (ESI) 486(MH⁺).

### (Example 69)

### 3-(4-t-Butylcyclohexyl)-2-(4-butylpiperazin-1-yl)benzoic acid ethyl ester hydrochloride

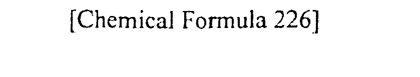

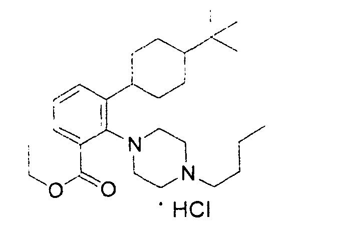

MS m/e (ESI) 429(MH⁺).

### (Example 70)

### 3-(4-t-Butylcyclohexyl)-2-(4-butylpiperazin-1-yl)-N-isopropylbenzamide hydrochloride

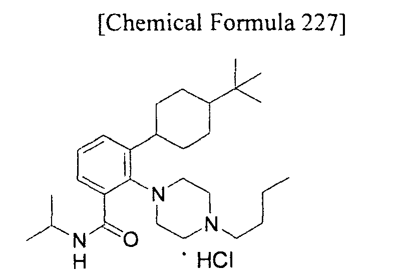

MS m/e (ESI) 442(MH⁺).

### (Example 71)

### 3-(4-t-Butylcyclohex-1-enyl)-4-(4-butylpiperazin-1-yl)benzoic acid ethyl ester hydrochloride

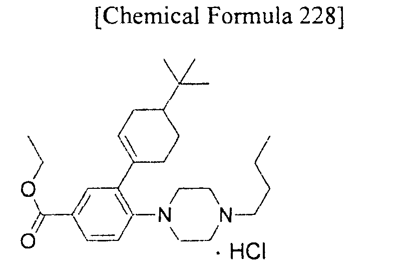

MS m/e (ESI) 427(MH⁺).

### (Example 72)

### 3-(4-t-Butylcyclohexyl)-4-(4-butylpiperazin-1-yl)benzoic acid ethyl ester hydrochloride

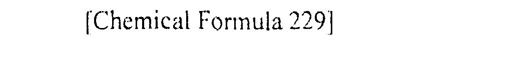

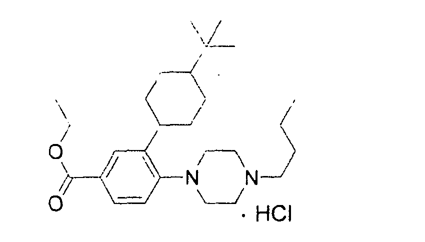

MS m/e (ESI) 429(MH⁺).

### (Example 73)

### 1-Butyl-4-[2-(4-t-butylcyclohexyl)naphthalen-1-yl]piperazine hydrochloride

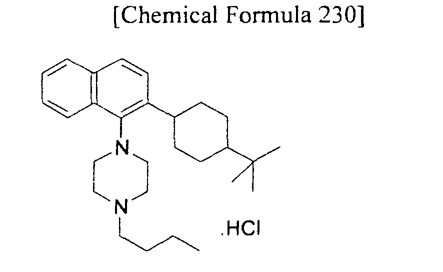

MS m/e (ESI) 407(MH⁺).

### (Example 74)

### 1-Butyl-4-[3-(4-t-butylcyclohex-1-enyl)naphthalen-2-yl]piperazine

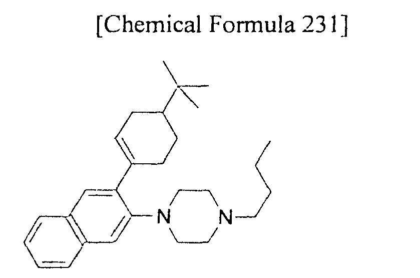

MS m/e (ESI) 405(MH⁺).

### (Example 75)

### 1-Butyl-4-[3-(4-t-butylcyclohexyl)naphthalen-2-yl]piperazine

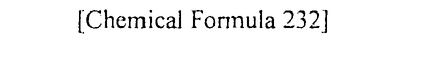

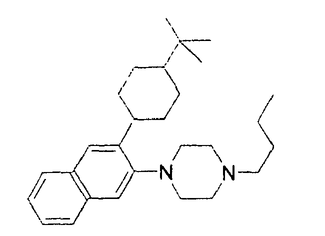

MS m/e (ESI) 407(MH⁺).

### (Example 76)

### 3-(4-t-Butylcyclohexyl)-2-(4-butylpiperazin-1-yl)-N-ethylbenzamide hydrochloride

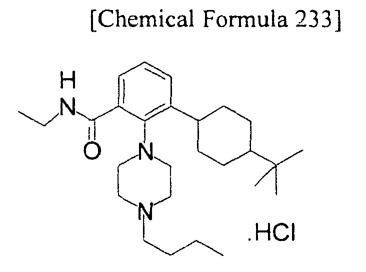

MS m/e (ESI) 428(MH⁺).

### (Example 77)

### 3-(4-t-Butylcyclohexyl)-2-(4-butylpiperazin-1-yl)-N,N-dimethylbenzamide hydrochloride

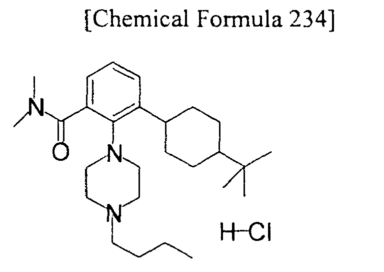

MS m/e (ESI) 428(MH⁺).

### (Example 78)

### [3-(4-t-Butylcyclohexyl)-2-(4-butylpiperazin-1-yl)phenyl]morpholin-4-ylmethanone hydrochloride

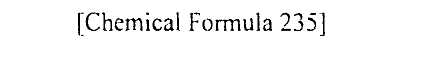

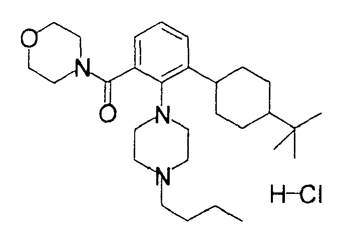

MS m/e (ESI) 470(MH⁺).

### (Example 79)

### 3-(4-t-Butylcyclohexyl)-2-(4-butylpiperazin-1-yl)benzamide hydrochloride

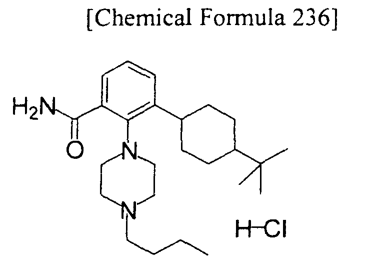

MS m/e (ESI) 400(MH⁺).

### (Example 80)

### 3-(4-t-Butylcyclohexyl)-2-(4-butylpiperazin-1-yl)-N-(tetrahydropyran-4-yl)benzamide hydrochloride

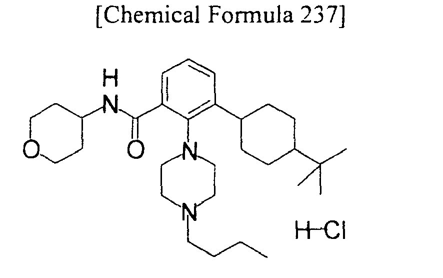

MS m/e (ESI) 484(MH⁺).

### (Example 81)

### 2-[4-(4-t-Butylcyclohexyl)-3-(4-butylpiperazin-1-yl)phenyl]-N-ethylacetamide hydrochloride

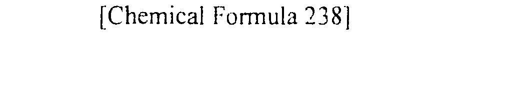

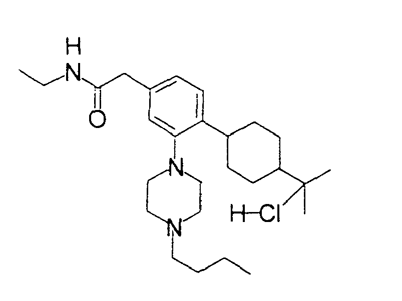

MS m/e (ESI) 442(MH⁺).

### (Example 82)

### 4-(4-t-Butylcyclohexyl)-3-(4-butylpiperazin-1-yl)benzamide hydrochloride

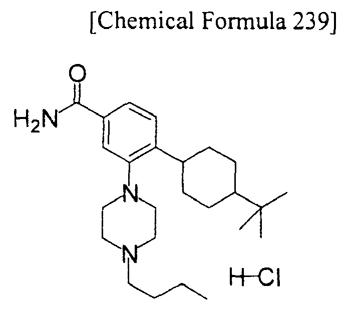

MS m/e (ESI) 400(MH⁺).

### (Example 83)

### 4-(4-t-Butylcyclohexyl)-3-(4-butylpiperazin-1-yl)-N,N-dimethylbenzamide hydrochloride

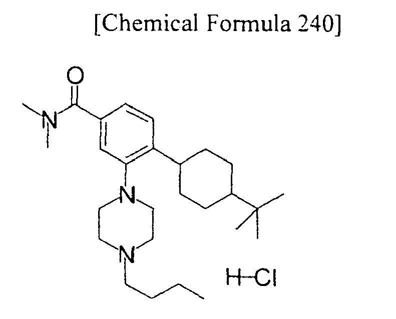

MS m/e (ESI) 428(MH⁺).

### (Example 84)

### 4-(4-t-Butylcyclohexyl)-3-(4-butylpiperazin-1-yl)-N-(2-methoxyethyl)benzamide hydrochloride

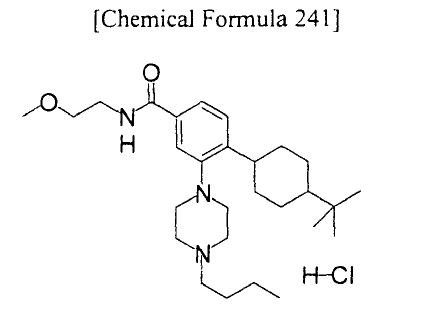

MS m/e (ESI) 458(MH⁺).

### (Example 85)

### [4-(4-t-Butylcyclohexyl)-3-(4-butylpiperazin-1-yl)phenyl]morpholin-4-ylmethanone hydrochloride

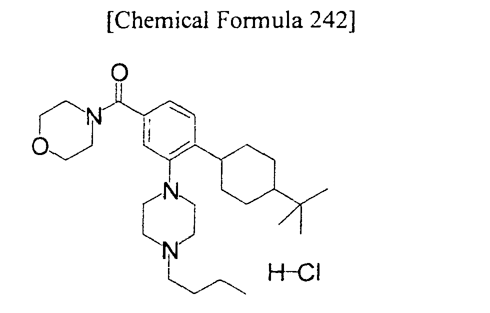

MS m/e (ESI) 470(MH⁺).

### (Example 86)

### N-[3-(4-t-Butylcyclohex-1-enyl)-4-(4-butylpiperazin-1-yl)phenyl]acetamide hydrochloride

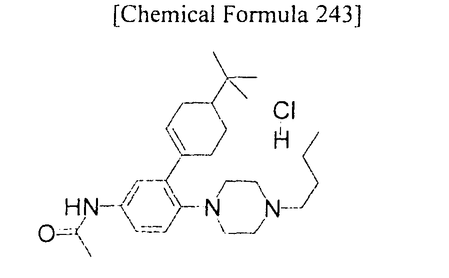

MS m/e (ESI) 412(MH⁺).

### (Example 87)

### [3-(4-t-Butylcyclohex-1-enyl)-4-(4-butylpiperazin-1-yl)phenyl]-(tetrahydropyran-4-yl)amine hydrochloride

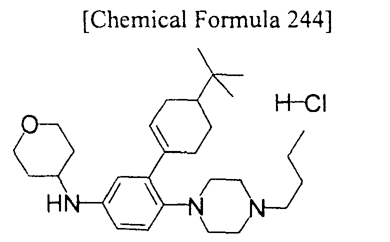

MS m/e (ESI) 454(MH⁺).

### (Example 88)

### 2-[4-(4-t-Butylcyclohexyl)-3-(4-butylpiperazin-1-yl)phenoxy]-N-ethylacetamide hydrochloride

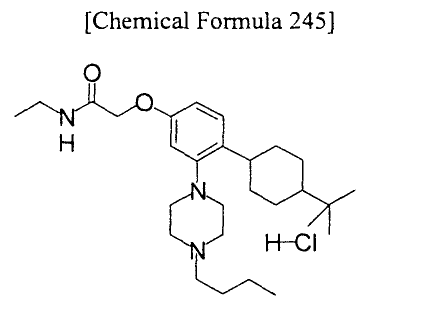

MS m/e (ESI) 458(MH⁺).

### (Example 89)

### 2-[4-(4-t-Butylcyclohexyl)-3-(4-butylpiperazin-1-yl)phenoxy]-N,N-dimethylacetamide hydrochloride

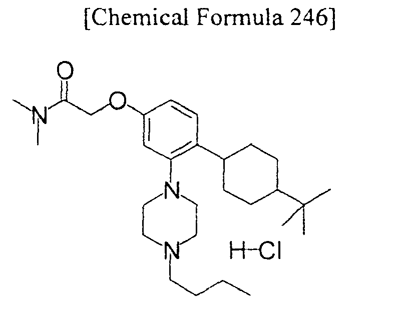

MS m/e (ESI) 458(MH⁺).

### (Example 90)

### [3-(4-t-Butylcyclohex-1-enyl)-2-(4-butylpiperazin-1-yl)phenylamino]acetic acid methyl ester hydrochloride

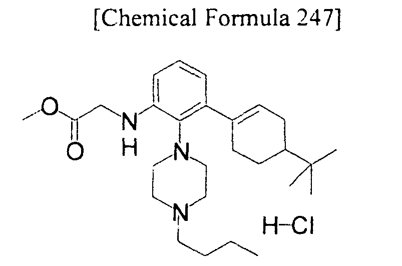

MS m/e (ESI) 442(MH⁺).

### (Example 91)

### 2-[3-(4-t-Butylcyclohex-1-enyl)-2-(4-butylpiperazin-1-yl)phenylamino]-N ethylacetamide hydrochloride

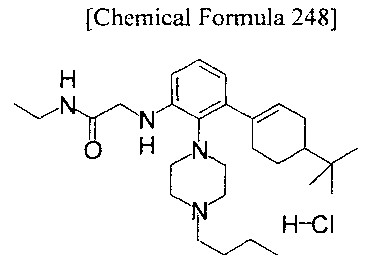

MS m/e (ESI) 455(MH⁺).

### (Example 92)

### 2-[3-(4-t-Butylcyclohex-1-enyl)-2-(4-butylpiperazin-1-yl)phenylamino]-N,N-dimethylacetamide hydrochloride

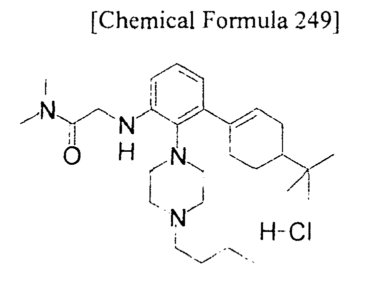

MS m/e (ESI) 455(MH⁺).

### (Example 93)

### 1-[3-(4-t-Butylcyclohex-1-enyl)-4-(4-butylpiperazin-1-yl)phenyl]piperidin-2-one hydrochloride

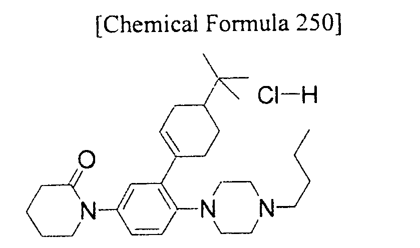

MS m/e (ESI) 452(MH⁺).

### (Example 94)

### 1-[3-(4-t-Butylcyclohex-1-enyl)-4-(4-butylpiperazin-1-yl)phenyl]piperidine-2,6-dione hydrochloride

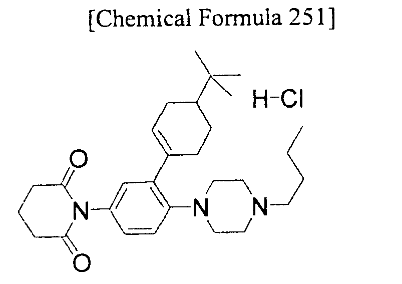

MS m/e (ESI) 466(MH⁺).

### (Example 95)

### 3-[4-(4-t-Butylcyclohexyl)-3-(4-butypiperazin-1-yl)phenyl]propionamide hydrochloride

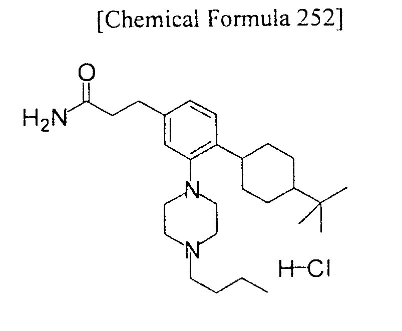

MS m/e (ESI) 428(MH⁺).

### (Example 96)

### 3-[4-(4-t-Butylcyclohexyl)-3-(4-butylpiperazin-1-yl)phenyl]-N-ethylpropionamide hydrochloride

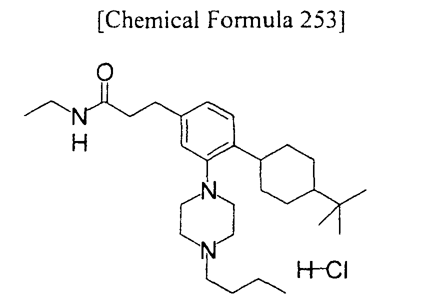

MS m/e (ESI) 456(MH⁺).

### (Example 97)

### 3-[4-(4-t-Butylcyclohexyl)-(4-butylpiperazin-1-yl)phenyl]-N,N-diethylpropionamide hydrochloride

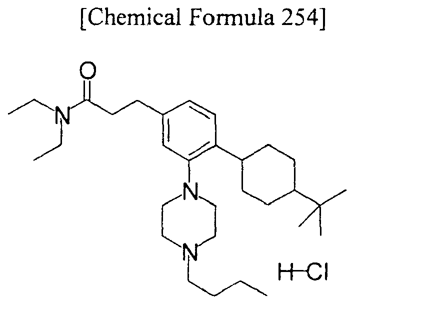

MS m/e (ESI) 484(MH⁺).

### (Example 98)

### [2-[4-(4-t-Butylcyclohexyl)-3-(4-butylpiperazin-1-yl)phenoxy]ethyl]diethylamine dihydrochloride

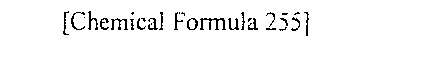

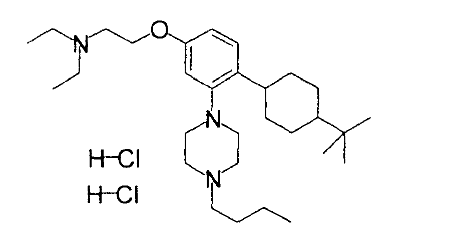

MS m/e (ESI) 472(MH⁺).

### (Example 99)

### 8-[5-(4-t-Butylcyclohexyl)-4-(4-butylpiperazin-1-yl)-2-methoxyphenyl]-1,4-dioxa-8-aza-spiro[4.5]decane hydrochloride

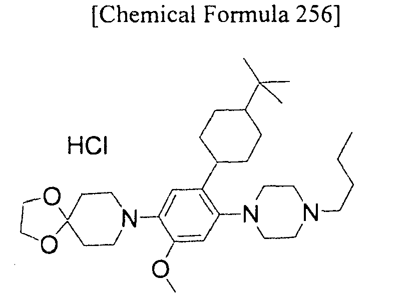

MS m/e (ESI) 528(MH⁺).

### (Example 100)

### 1-[5-(4-t-Butylcyclohexyl)-4-(4-butylpiperazin-1-yl)-2-methoxyphenyl]piperidin-4-one hydrochloride

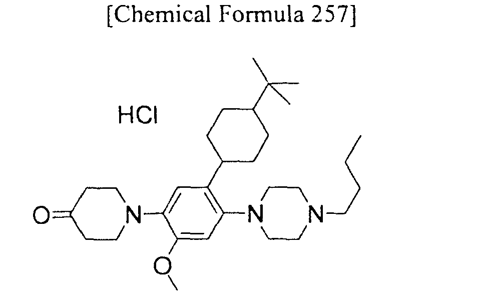

MS m/e (ESI) 484(MH⁺).

### (Example 101)

### [1-[3-(4-t-Butylcyclohexyl)-4-(4-butylpiperazin-1-yl)phenyl]piperidin-4-yloxy]acetic acid ethyl ester hydrochloride

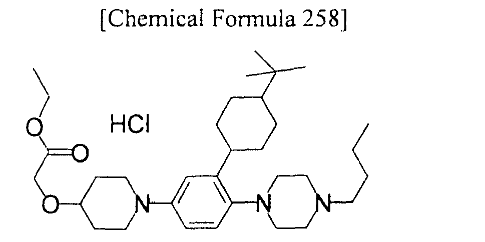

MS m/e (ESI) 542(MH⁺).

### (Example 102)

### 5-(4-t-Butylcyclohexyl)-6-(4-butylpiperazin-1-yl)-2-methylbenzoxazole hydrochloride

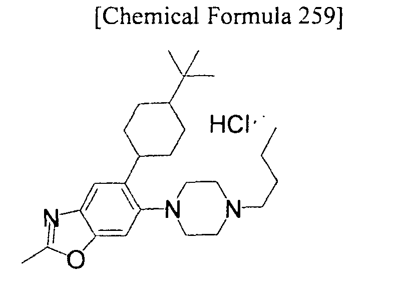

MS m/e (ESI) 412(MH⁺).

### (Example 103)

### [4-(4-t-Butylcyclohex-1-enyl)-3-(4-butylpiperazin-1-yl)phenyl]-(tetrahydropyran-4-yl)amine hydrochloride

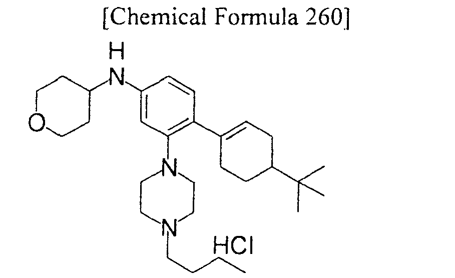

MS m/e (ESI) 454(MH⁺).

### (Example 104)

### 2-[1-1-3-(4-t-Butylcyclohexyl)-4-(4-butylpiperazin-1-yl)phenyl]piperidin-4-yloxy]-N,N-dimethylacetamide hydrochloride

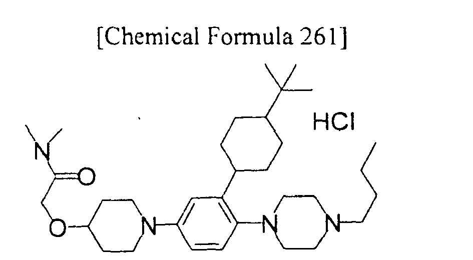

MS m/e (ESI) 541 (MH⁺).

### (Example 105)

### [3-(4-Propylpiperazin-1-yl)-4-(3,3,5,5-tetramethylcyclohexyl)phenoxy]acetonitrile hydrochloride

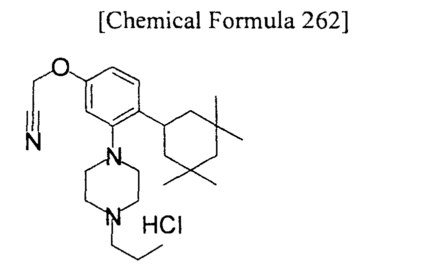

MS m/e (ESI) 398(MH⁺).

### (Example 106)

### [3-(4-Butylpiperazin-1-yl)-4-(3,3,5,5-tetramethylcyclohexyl)phenoxy]acetonitrile hydrochloride

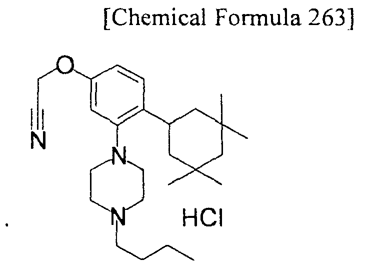

MS m/e (ESI) 412(MH⁺).

### (Example 107)

### Methanesulfonic acid 3-(4-butylpiperazin-1-yl)-4-(3,3,5,5-tetramethylcyclohexyl)phenyl ester hydrochloride

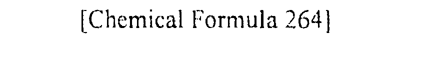

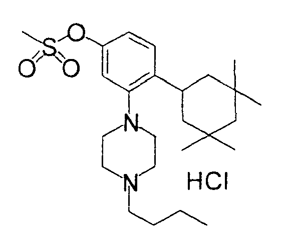

MS m/e (ESI) 451 (MH⁺).

### (Example 108)

### Trifluoromethanesulfonic acid 3-(4-cyclopropylmethylpiperazin-1-yl)-4-(3,3,5,5-tetramethylcyclohexyl)phenyl ester

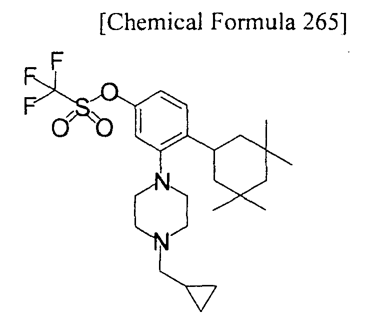

MS m/e (ESI) 503(MH⁺).

### (Example 109)

### 1-[4-(4-Cyclopropylmethylpiperazin-1-yl)-3-(3,3,5,5-tetramethylcyclohexyl)phenyl]-3-methoxypropan-1-one hydrochloride

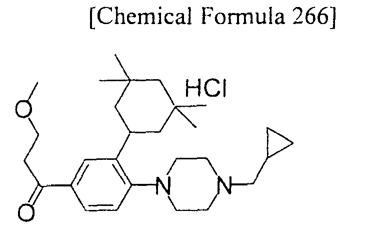

MS m/e (ESI) 441 (MH⁺).

### (Example 110)

### Morpholine-4-carboxylic acid [4-(4-butylpiperazin-1-yl)-3-(3,3,5,5-tetramethylcyclohexyl)phenyl]amide hydrochloride

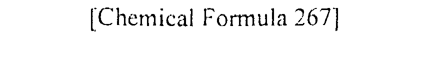

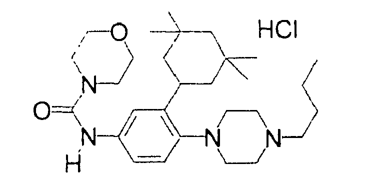

MS m/e (ESI) 485(MH⁺).

### (Example 111)

### Morpholine-4-carboxylic acid [4-(4-butylpiperazin-1-yl)-3-(3,3,5,5-tetramethylcyclohexyl)phenyl]methylamide hydrochloride

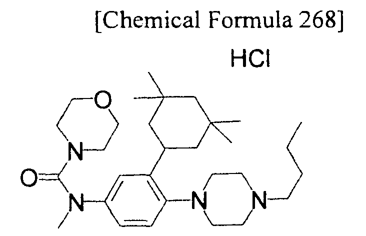

MS m/e (ESI) 499(MH⁺).

### (Example 112)

### 1-Isobutyl-4-[7-(3,3,5,5-tetramethylcyclohexyl)-2,3-dihydrobenzo[1,4]dioxin-6-yl]piperazine hydrochloride

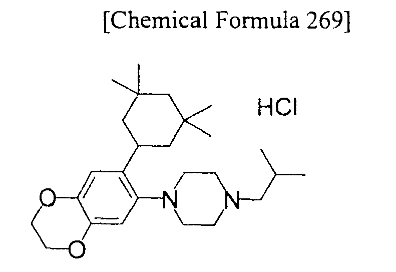

MS m/e (ESI) 415(MH⁺).

### (Example 113)

### 1-Cyclopropylmethyl-4-[3'-methoxy-4-(3,3,5,5-tetramethylcyclohexyl)biphenyl-3-yl]piperazine hydrochloride

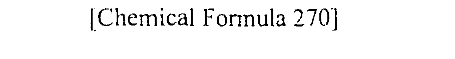

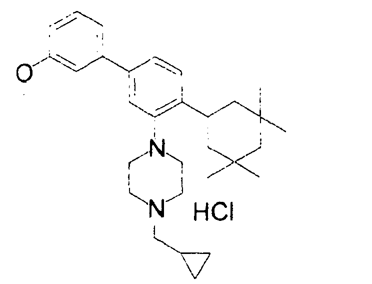

MS m/e (ESI) 461 (MH⁺).

### (Example 114)

### 3'-(4-Cyclopropylmethylpiperazin-1-yl)-4'-(3,3,5,5-tetramethylcyclohexyl)biphenyl-3-carbonitrile hydrochloride

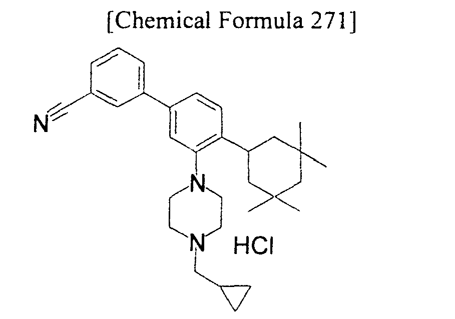

MS m/e (ESI) 456(MH⁺).

### (Example 115)

### 1-Cyclopropylmethyl-4-[5-(2-methoxyethoxy)-2-(3,3,5,5-tetramethylcyclohexyl)phenyl]piperazine hydrochloride

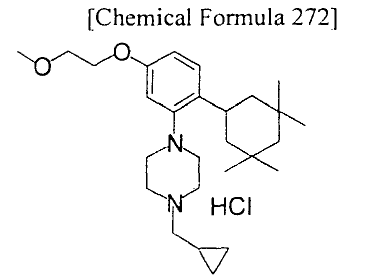

MS m/e (ESI) 429(MH⁺).

### (Example 116)

### 1-Butyl-4-[2-(4-t-butylcyclohexyl)-5-(2-phenoxyethoxy)phenyl]piperazine hydrochloride

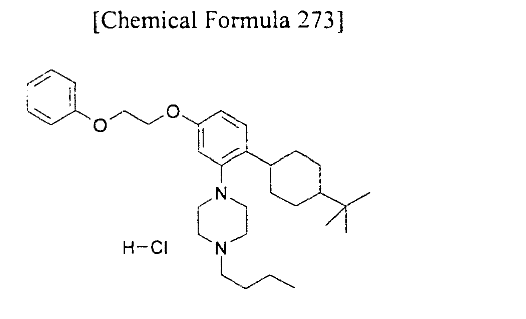

MS m/e (ESI) 493(MH⁺).

### (Example 117)

### 1-Butyl-4-[2-(4-t-butylcyclohexyl)-5-[2-(2-methoxyethoxy)ethoxy]phenyl]piperazine hydrochloride

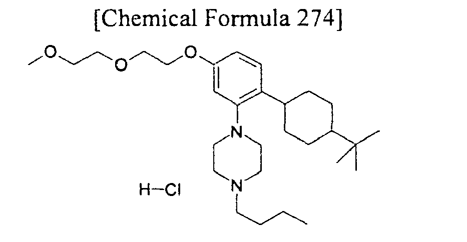

MS m/e (ESI) 475(MH⁺).

### (Example 118)

### 2-[4-(4-t-Butylcyclohexyl)-3-(4-butylpiperazin-1-yl)phenyl]propan-2-ol hydrochloride

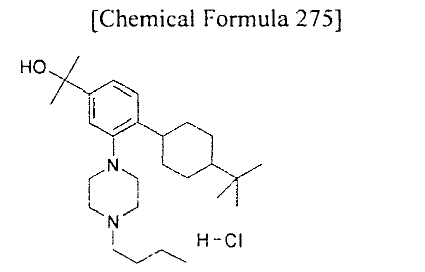

MS m/e (ESI) 415(MH⁺).

### (Example 119)

### [1-[3-(4-t-Butylcyclohexyl)-4-(4-butylpiperazin-1-yl)phenyl]piperidin-4-yl]dimethylamine dihydrochloride

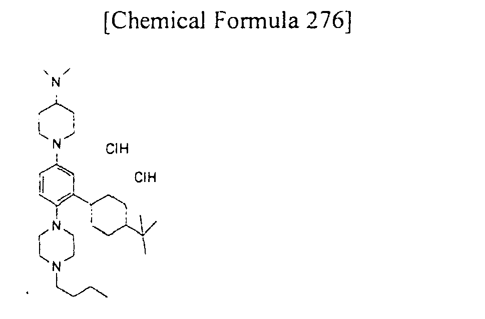

MS m/e (ESI) 483(MH⁺).

### (Example 120)

### 4-[3-(4-t-Butylcyclohexyl)-4-(4-butylpiperazin-1-yl)phenyl]thiomorpholine-1-oxide hydrochloride

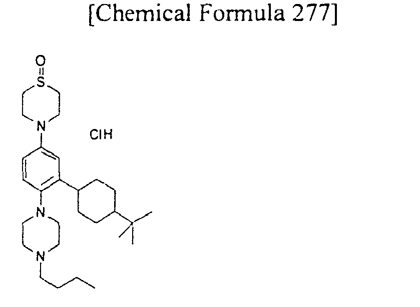

MS m/e (ESI) 474(MH⁺).

### (Example 121)

### 4-[3-(4-t-Butylcyclohexyl)-4-(4-butylpiperazin-1-yl)phenyl]thiomorpholine-1,1-dioxide hydrochloride

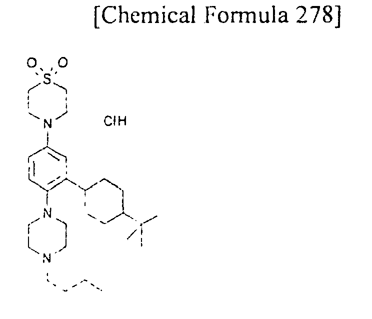

MS m/e (ESI) 490(MH⁺).

### (Example 122)

### 4-[5-(4-t-Butylcyclohexyl)-4-(4-butylpiperazin-1-yl)-2-methoxyphenyl]thiomorpholine-1,1-dioxide hydrochloride

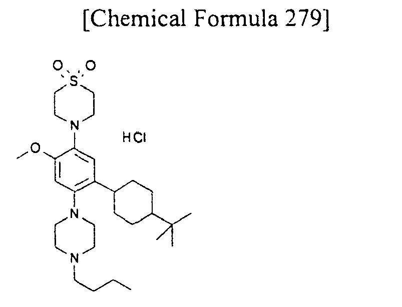

MS m/e (ESI) 520(MH⁺).

### (Example 123)

### 6-[4-(4-Cyclopropylmethylpiperazin-1-yl)-3-(3,3,5,5-tetramethylcyclohexyl)phenyl]-1H-pyridin-2-one hydrochloride

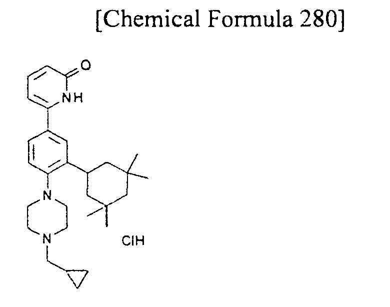

MS m/e (ESI) 448(MH⁺).

### (Example 124)

### 1-[2,4-bis(3,3,5,5-tetramethylcyclohexyl)phenyl]-4-butylpiperazine hydrochloride

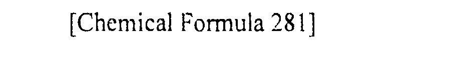

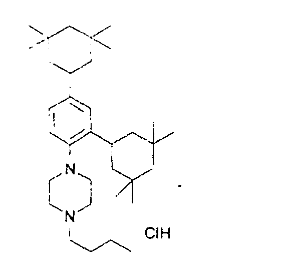

MS m/e (ESI) 495(MH⁺).

### (Example 125)

### 1-Propyl-4-[4-(3,3,5,5-tetramethylcyclohex-1-enyl)-2-(3,3,5,5-tetramethylcyclohexyl)phenyl]piperazine hydrochloride

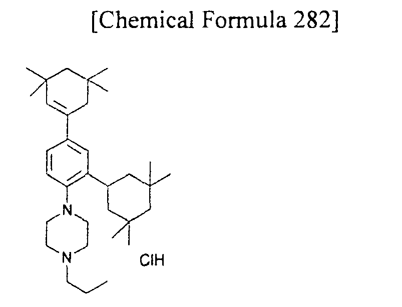

MS m/e (ESI) 479(MH⁺).

### (Example 126)

### 1-Butyl-4-[2-(4-t-butylcyclohexyl)-4-methanesulfonylphenyl]piperazine hydrochloride

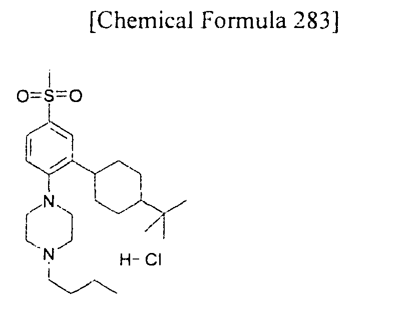

MS m/e (ESI) 435(MH⁺).

### (Example 127)

### Benzyl-[3-(4-t-butylcyclohexyl)-4-(butylpiperazin-1-yl)phenyl]-methylamine hydrochloride

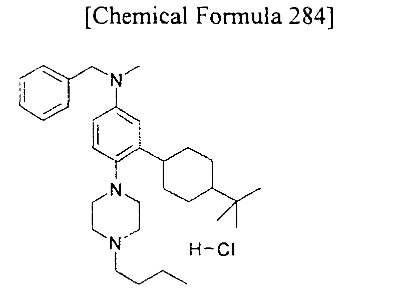

MS m/e (ESI) 476(MH⁺).

### (Example 128)

### 2-[3-(4-t-Butylcyclohex-1-enyl)-2-(4-butylpiperazin-1-yl)phenylamino]acetamide hydrochloride

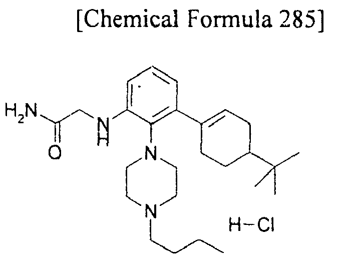

MS m/e (ESI) 427(MH⁺).

### (Example 129)

### [3-(4-t-Butylcyclohex-1-enyl)-2-(4-butylpiperazin-1-yl)phenylamino]acetic acid

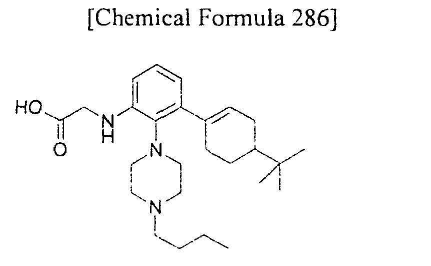

MS m/e (ESI) 428(MH⁺).

### (Example 130)

### 1-Butyl-4-[2-(4-t-butylcyclohexyl)-4-{4-(2-methoxyethoxy)piperidin-1-yl}phenyl]piperazine hydrochloride

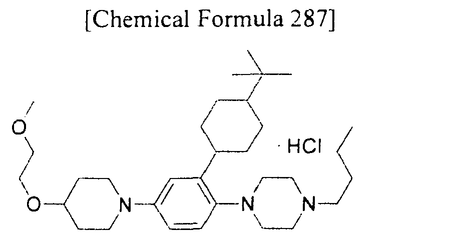

MS m/e (ESI) 514(MH⁺).

### (Example 131)

### [3-(4-Cyclopropylmethylpiperazin-1-yl)-4-(3,3,5,5-tetramethylcyclohexyl)phenoxy]acetonitrile hydrochloride

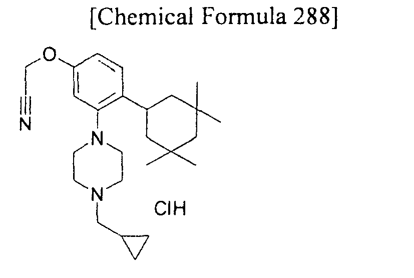

MS m/e (ESI) 410(MH⁺).

### (Example 132)

### 1-[3-(4-t-Butylcyclohex-1-enyl)-4-(4-butylpiperazin-1-yl)phenyl]pyrrolidine-2,5-dione hydrochloride

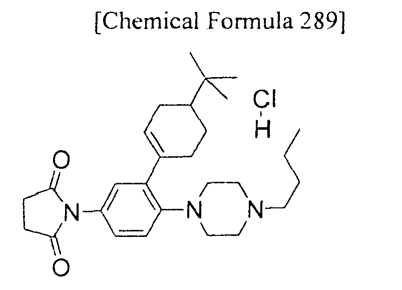

MS m/e (ESI) 452(MH⁺).

### (Example 133)

### 1-Cyclopropylmethyl-4-[5-phenoxy-2-(3,3,5,5-tetramethylcyclohexyl)phenyl]piperazine hydrochloride

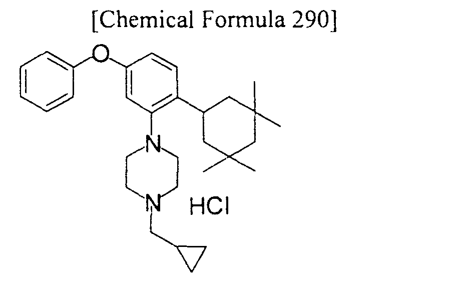

MS m/e (ESI) 447(MH⁺).

### (Example 134)

### 8-{3-(4-t-Butylcyclohexyl)-4-(4-butylpiperazin-1-yl)phenyl}-1,4-dioxa-8-azaspiro[4.5]decane hydrochloride

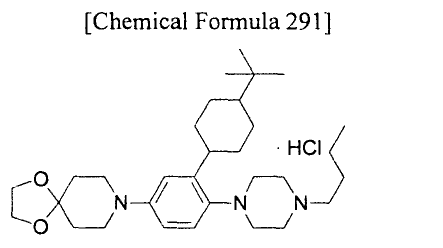

MS m/e (ESI) 498(MH⁺).

### (Example 135)

### 1-[3-(4-t-Butylcyclohexyl)-4-(4-butylpiperazin-1-yl)phenyl]piperidin-4-one

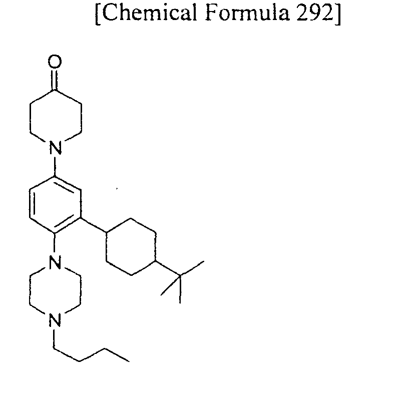

MS m/e (ESI) 454(MH⁺).

### (Example 136)

### 1-[3-(4-t-Butylcyclohexyl)-4-(4-butylpiperazin-1-yl)phenyl]piperidin-4-ol hydrochloride

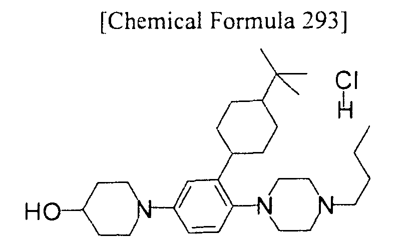

MS m/e (ESI) 456(MH⁺).

### (Example 137)

### 1-{3-(4-t-Butylcyclohexyl)-4-(4-butylpiperazin-1-yl)phenyl}piperidine-4-carboxylic acid ethyl ester hydrochloride

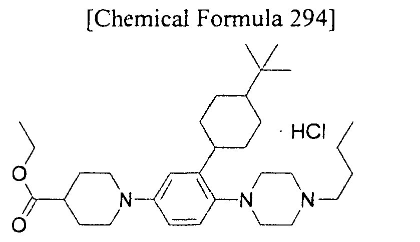

MS m/e (ESI) 512(MH⁺).

### (Example 138)

### 1-{3-(4-t-Butylcyclohexyl)-4-(4-butylpiperazin-1-yl)phenyl}piperidine-4-carboxylic acid

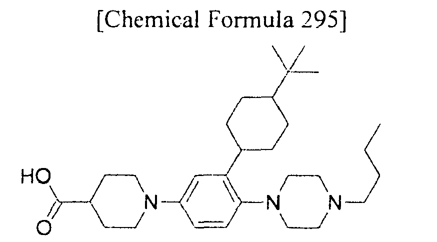

MS m/e (ESI) 484(MH⁺).

### (Example 139)

### [3-(4-Butylpiperazin-1-yl)-4-(3,3,5,5-tetramethylcyclohexyl)phenyl](2-methoxyethyl)methylamine hydrochloride

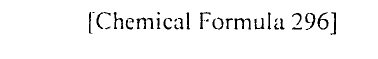

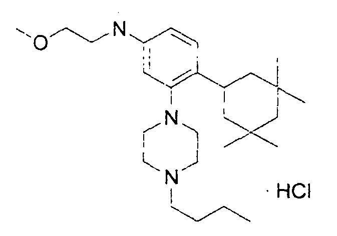

Free form of the title compound:
¹H-NMR (400MHz, CDCl₃)
δ: 0.91 (s, 6H), 0.94 (t, J= 7.2Hz, 3H), 1.10 (s, 6H), 1.12-1.5 (m, 10H), 2.34-2.43 (m, 2H), 2.59 (brs, 4H), 2.93 (t, J= 4.0Hz, 4H), 3.35 (s, 3H), 3.36-3.48 (m, 3H), 3.50-3.56 (m, 2H), 6.45 (dd, J= 8.4, 2.8Hz, 1H), 6.52 (d, J= 2.8Hz, 1H), 7.04 (d, J= 8.4Hz, 1H).
The title compound, i.e. hydrochloride form:
MS m/e (ESI) 444(MH⁺).

### (Example 140)

### 1-[3-(4-t-Butylcyclohex-1-enyl)-4-(4-butylpiperazin-1-yl)phenyl]pyrrolidin-2-one hydrochloride

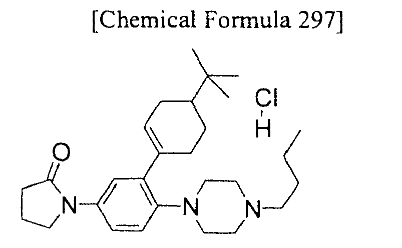

MS m/e (ESI) 438(MH⁺).

### (Example 141)

### 1-Butyl-4-[1-(4-t-butylcyclohex-1-enyl)naphthalen-2-yl]piperazine hydrochloride

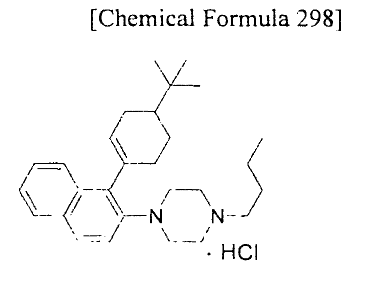

MS m/e (ESI) 405(MH⁺).

### (Example 142)

### 1-Butyl-4-[2-(4-t-butylcyclohexyl)-4-methylsulfanylphenyl]piperazine hydrochloride

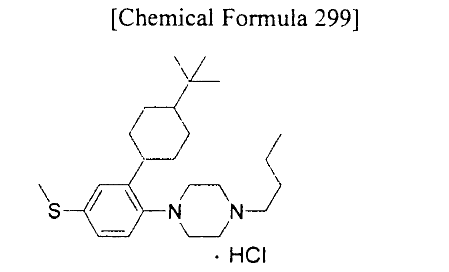

MS m/e (ESI) 403(MH⁺).

### (Example 143)

### 1-Butyl-4-[4-(4-t-butylcyclohexyl)biphenyl-3-yl]piperazine hydrochloride

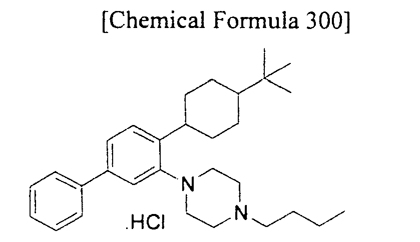

MS m/e (ESI) 433(MH⁺).

### (Example 144)

### 1-[3'-Methoxy-4-(3,3,5,5-tetramethylcyclohexyl)biphenyl-3-yl]-4-propylpiperazine hydrochloride

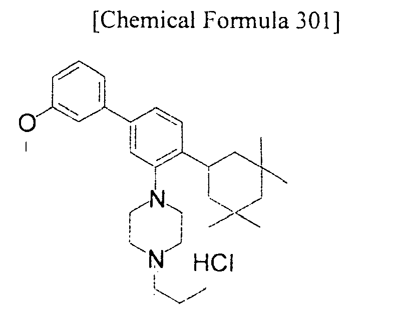

MS m/e (ESI) 449(MH⁺).

### (Example 145)

### 3'-(4-Propylpiperazin-1-yl)-4'-(3,3,5,5-tetramethylcyclohexyl)biphenyl-3-carbonitrile hydrochloride

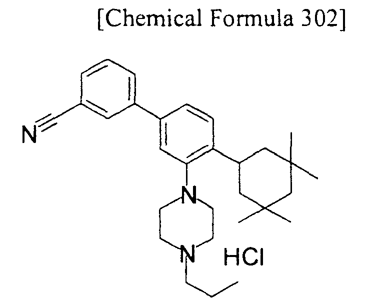

MS m/e (ESI) 444(MH⁺).

### (Example 146)

### 3-[4-(4-t-Butylcyclohexyl)-3-(4-butylpiperazin-1-yl)phenyl]acrylic acid ethyl ester hydrochloride

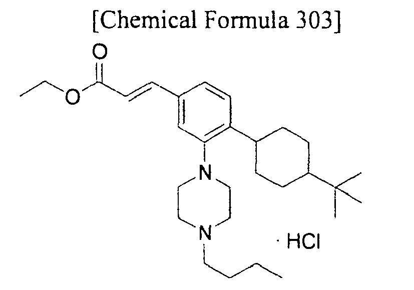

MS m/e (ESI) 455(MH⁺).

### (Example 147)

### 3-[4-(4-t-Butylcyclohexyl)-3(4-butylpierazin-1-yl)phenyl]acrylic acid

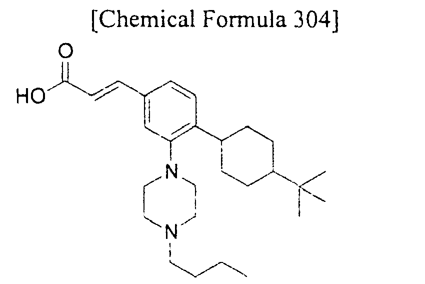

MS m/e (ESI) 427(MH⁺).

### (Test Example 1: Evaluation of compounds in Jurkat cell adhesion system) (Immobilization of human fibronectin in 96-well plate)

Human fibronectin (Becton Dickinson Biosciences) was diluted with phosphate-buffered saline (hereinafter abbreviated as PBS; Sigma) to 0.1-0.01 µg/mL, and the diluted solution was added to a 96-well plate (Becton Dickinson) at 50 µL/well, and allowed to stand overnight at 4°C. On the following day, the supernatant was removed from the plate, and then PBS containing 1% bovine serum albumin (hereinafter abbreviated as BSA; Sigma) was added thereto at 100 µL/well and incubation was performed at 37°C for 2 hours in a CO₂ incubator (Hirasawa).

### (Adhesion assay)

The supernatant was removed from each plate and Jurkat cells suspended in RPMI-1640 (Sigma) containing 1 mg/mL BSA were added at 80 µL/well for 2.5 x 10⁵ cells/well. The compound diluted to different concentrations with RPMI-1640 containing 1 mg/mL BSA was immediately added at 10 µL/well, and then 100 nM phorbol myristate acetate (hereinafter abbreviated as PMA; Sigma) in RPMI-1640 containing 1 mg/mL BSA was added at 10 µL/well and the plate was incubated in a CO₂ incubator at 37°C for 45-60 minutes. The supernatant was removed from the plate and each well was washed several times with 100 µL/well of RPMI-1640, after which 50 mM citrate buffer (pH 5.0) containing 3.75 mM p-nitrophenol-N-acetyl-β-D-glucosaminide (Sigma) and 0.25% Triton X-100 (Sigma) were added at 60 µL/well, and the mixture was placed in a CO₂ incubator and incubated at 37°C for 45 minutes. After incubation, 50 mM glycine buffer (pH 10.4) containing 5 mM EDTA was added at 90 µL/well, and the absorbance at 405 nm was measured with an EL340 Automated Microplate Reader (BIO-TEK) to determine the adhered cell count. The concentration of each compound which inhibited the increase in the number of adhered cells by the PMA-stimulation by 50% is shown as the IC50 (µM) in the table below.

**[Table 1]**

| Example No. | IC50 (µM) |
|---|---|
| 1 | 6.0 |
| 2 | 16.7 |
| 4 | 3.4 |
| 5 | 2.9 |
| 6 | 2.9 |
| 7 | 4.9 |
| 8 | 3.4 |
| 10 | 3.3 |
| 12 | 14.7 |
| 13 | 3.8 |
| 14 | 7.1 |
| 15 | 4.7 |
| 16 | 5.6 |
| 17 | 1.3 |
| 18 | 2.3 |
| 19 | 4.7 |
| 134 | 4.0 |
| 44 | 2.8 |

### (Test Example 2: Evaluation of compounds in human peripheral blood neutrophil adhesion system)

### (Preparation of human peripheral blood neutrophils)

To a plastic centrifugation tube containing 100 units of heparin sodium (Shimizu Pharmaceutical) was added 25 mL of fresh blood sampled from a healthy human. After adding and mixing therewith 8 mL of physiological saline (Otsuka Pharmaceutical) containing 6% Dextran (Nacalai), the mixture was allowed to stand at room temperature for 45 minutes for sedimentation of the erythrocytes. The resultant supernatant was transferred to another plastic centrifugation tube and combined with an equivalent volume of phosphate-buffered saline (hereinafter abbreviated as PBS; Sigma), and then centrifuged at 1600 rpm for 7 minutes at room temperature. The obtained hematocyte fraction was suspended in 4 mL of PBS, and the suspension was superposed on 4 mL of Ficoll-Paque^{™} PLUS (Amersham Biosciences). The resultant bilayer liquid was centrifuged at 2000 rpm for 30 minutes at room temperature, after which the supernatant was removed and the precipitate was suspended in 10 mL of PBS and centrifuged at 1200 rpm for 7 minutes, and the supernatant was removed. The resulting precipitate was suspended in 0.5 mL of PBS again, and then 10 mL of distilled water (Otsuka Pharmaceutical) was added, 0.5 mL of an aqueous solution containing 3 M NaCl was immediately added to restore isotonicity, the mixture was centrifuged at 1200 rpm for 7 minutes, and the obtained precipitate was suspended in PBS containing I mg/mL bovine serum albumin (hereinafter abbreviated as BSA; Sigma) again and stored in ice until being used for the experiment.

### (Fluorescent labeling of human peripheral blood neutrophils)

The obtained neutrophils were suspended in PBS containing 1 mg/mL BSA at 2 x 10⁷ cells/mL. BCECF-AM (Dojin) was added to a final concentration of 5 µM, and the mixture was incubated at 37°C for 45 minutes. It was then rinsed twice with PBS containing 1 mg/mL BSA by centrifugation, suspended again in PBS containing 1 mg/mL BSA at 5 x 10⁷ cells/mL, and stored in ice until use.

### (Preparation of HUVEC immobilized plate)

Human umbilical vein endothelial cells (hereinafter abbreviated as HUVEC) were suspended in MCDB131 medium (Chlorella Industries) containing 10% fetal calf serum and 30 µg/mL endothelial cell growth supplement (Becton Dickinson Bioscience). The suspension was added at 7.5 x 10³ cells/well to a 96-well plate (Iwaki) immobilized with type I collagen, and cultured for 3 days in a CO₂ incubator (Hirasawa). Upon confirming confluency of the cells, the supernatant was discarded, the plate was rinsed twice with PBS, and then PBS containing 0.1% glutaraldehyde (Kanto Kagaku) was added at 100 µl/well and the HUVECs were immobilized for 5 minutes. The supernatant was discarded and the plate was washed twice with PBS, and then PBS was added at 100 µL/well and the mixture was stored at 4°C until use.

### (Adhesion assay)

To 6.5 mL of RPMI-1640 medium (Sigma) containing 1 mg/mL of BSA were added 0.5 mL of a suspension of BCECF-AM labeled neutrophils at 5 x 10⁷/mL stored in ice, which was mixed, and the mixture was added at 80 µL/well to a HUVEC immobilized plate. To this plate were immediately added 10 µl/well of a solution of the compound diluted at different concentrations with RPMI-1640 containing 1 mg/mL BSA, and 10 µl/well of 100 nM phorbol myristate acetate (hereinafter abbreviated as PMA; Sigma) in RPMI-1640 containing 1 mg/mL BSA, and the mixture was incubated in a CO₂ incubator at 37°C for 45 minutes. The supernatant was removed from the plate, which was then washed several times with RPMI-1640 at 100 µl/well, and then PBS containing 0.1% NP-40 (Calbiochem) was added thereto at 100 µl/well and the fluorescent intensity was measured with an ARVO_{TM}SX 1420 multi label counter (Wallac) to determine the number of adhered cells. The concentration of each compound which inhibited the increase in the number of adhered cells by the PMA-stimulation by 50% is shown as the IC50 (µM) in the table below.

**[Table 2]**

| Example No. | IC50 (µM) |
|---|---|
| 1 | 10.1 |
| 2 | 16.9 |
| 4 | 33.4 |
| 5 | 11.0 |
| 6 | 11.7 |
| 7 | 17.4 |
| 8 | 16.7 |
| 10 | 7.8 |
| 12 | 12.5 |
| 13 | 21.7 |
| 14 | 22.4 |
| 15 | 11.8 |
| 16 | 18.5 |
| 17 | 11.4 |
| 18 | 7.3 |
| 19 | 16.0 |
| 134 | 6.0 |
| 44 | 19.2 |

### (Test Example 3: Evaluation of compounds in oxazolone-induced colon neutrophil infiltration model)

### (Sensitization with oxazolone)

Five- to six-week-old male Balb/c mice (Charles River Japan) were shaven at the abdomen to an approximately 2 cm square area. A 100% ethanol solution containing 3% 4-ethoxymethylene-2-phenyl-2-oxazolin-5-one (hereinafter referred to as "oxazolone"; Sigma) was applied at 150 µL onto the abdomen of each mouse.

### (Preparation of emulsion containing oxazolone)

Distilled water (Otsuka Pharmaceutical) was added in an equivalent volume to 100% peanut oil (Kanto Kagaku) containing 1% oxazolone, and the components were vigorously mixed with a glass syringe (Top Co.), to prepare an emulsion containing 0.5% oxazolone.

### (Induction with oxazolone)

The mice were fasted on the 3rd day after oxazolone sensitization, and were challenged with 100 µL of the emulsion containing 0.5% oxazolone prepared in the way described above intrarectally at a site approximately 3 cm from the anus under ether anesthesia on the 4th day.

### (Colon-infiltrating neutrophil assay)

Each compound was suspended or dissolved in an aqueous solution containing 0.5% methyl cellulose (Wako), and orally administered at 30 mg/kg 30 minutes prior to the intrarectal challenge of oxazolone emulsion.
Four hours after the intrarectal challenge of oxazolone, mice were sacrificed by cervical dislocation, and colons were extirpated, dissected in the longitudinal direction, washed with physiological saline, and transferred to ice-cooled plastic centrifugation tubes. After adding 1 mL of 50 mM potassium phosphate buffer (hereinafter abbreviated as KPB) (pH 6.0) to the tube, and the tissue were homogenized with PHYSCOTRON (Microtec Nition Co., Ltd.), 2 mL of 50 mM KPB (pH 6.0) was added and the mixture was centrifuged at 3000 rpm, 4°C for 10 minutes and the supernatant was removed. To the resultant precipitate was added 1 mL of 50 mM KPB (pH 6.0) containing 0.5% hexadecyltrimethyl-ammonium bromide (Sigma), and freeze-thawed 3 to 5 times using liquid nitrogen and hot water, centrifuged at 3000 rpm, 4°C for 10 minutes to yield a supernatant.
The myeloperoxidase enzyme activity in the supernatant was assayed in the following manner. Specifically, to 10 µL of the obtained supernatant was added 200 µL of 50 mM KPB (pH6.0) containing 0.017% o-dianisidine (Sigma) and 0.0005% hydrogen peroxide (Wako), incubated at 37°C, and the change in absorbance at 450 nm (the rate of change in absorbance per minute (mO.D./min.)) was continuously measured for 1 minute using an EL340 Automated Microplate Reader (BIO-TEK) in kinetic mode. The myeloperoxidase enzyme activity inhibitory rate (%) in each of the compound-administered groups in relation to the oxazolone control group (the oxazolone emulsion-intrarectally challenged/compound-free group) is shown in the table below.

**[Table 3]**

| Example No. | Ihibitory Rate (%) |
|---|---|
| 1 | 69.0 |
| 5 | 3.0 |
| 6 | 48.0 |
| 7 | 24.0 |
| 134 | 18.0 |
| 44 | 38.0 |

### (Test Example 4: Evaluation of compounds in DSS-induced colitis model)

A 1-3% solution of dextran sulfate sodium (hereinafter abbreviated as DSS; ICN) in purified water (Otsuka Pharmaceutical) is fed freely to 6- to 7-week-old male Balb/c mice (Charles River Japan) for 5-7 days to induce colitis. Disease Activity Index (hereinafter abbreviated as DAI) scored based on fecal hardness, blood content in feces and body weight change, the number of neutrophils infiltrating the colon and the length of the colon are used as indexes to evaluate compounds. Each compound is suspended or dissolved in an aqueous solution containing 0.5% methyl cellulose (Wako), and orally administered at 30 mg/kg once a day, for 5-7 successive days.

### Industrial Applicability

The compounds of the invention have excellent cell adhesion inhibitory action or cell infiltration inhibitory action, and can therefore serve as pharmaceuticals which are useful as therapeutic or prophylactic agents for various inflammatory diseases and autoimmune diseases associated with adhesion and infiltration of leukocytes, such as inflammatory bowel diseases (particularly ulcerative colitis or Crohn's disease), irritable bowel syndrome, rheumatoid arthritis, psoriasis, multiple sclerosis, asthma and atopic dermatitis.

## Claims

1. A compound represented by the following general formula (1), a salt thereof or a hydrate of the foregoing: 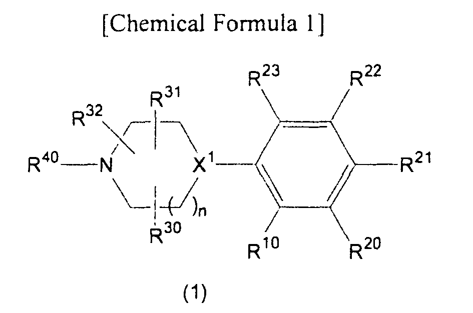 wherein R¹⁰ represents 5- to 10-membered cycloalkyl optionally substituted with a substituent selected from Group A1 or 5- to 10-membered cycloalkenyl optionally substituted with a substituent selected from Group A1,
R³⁰, R³¹ and R³² may be the same or different and each represents hydrogen, hydroxyl, halogen, cyano, carboxyl, C1-6 alkyl, C1-6 alkoxy or C2-7 alkoxycarbonyl, or
two of R³⁰, R³¹ and R³² bond together to form oxo (=O) or methylene (-CH₂-) and the other represents hydrogen, hydroxyl, halogen, cyano, carboxyl, C1-6 alkyl, C1-6 alkoxy or C2-7 alkoxycarbonyl,
R⁴⁰ represents C1-10 alkyl optionally substituted with a substituent selected from Group D1, 3- to 8-membered cycloalkyl optionally substituted with a substituent selected from Group E1, a 4- to 8-membered heterocyclic group optionally substituted with a substituent selected from Group E1, C2-7 alkenyl optionally substituted with a substituent selected from Group F1, C2-7 alkynyl optionally substituted with a substituent selected from Group F1, C2-7 alkylcarbonyl optionally substituted with a substituent selected from Group G1, mono(C1-6 alkyl)aminocarbonyl, 4- to 8-membered heterocyclic carbonyl, C2-7 alkoxycarbonyl or C1-6 alkylsulfonyl,
n represents an integer of 0, 1 or 2,
X¹ represents CH or nitrogen, and
R²⁰, R²¹, R²² and R²³ may be the same or different and each represents hydrogen, hydroxyl, halogen, nitro, cyano, carboxyl, C1-6 alkylthio optionally substituted with a substituent selected from Group F1 C2-7 alkoxycarbonyl, phenoxy, -SO₃H, C1-6 alkyl optionally substituted with a substituent selected from Group W1, C1-6 alkyl optionally substituted with a substituent selected from Group K1, C1-6 alkoxy optionally substituted with a substituent selected from Group W1, a 4- to 8-membered heterocyclic group optionally substituted with a substituent selected from Group W1, a 4- to 8-membered heterocyclic group optionally substituted with a substituent selected from Group V1, a 5- to 10-membered heteroaryl ring group optionally substituted with a substituent selected from Group W1, a 6- to 10-membered aryl ring group optionally substituted with a substituent selected from Group W1, C2-7 alkenyl optionally substituted with a substituent selected from Group W1, C2-7 alkynyl optionally substituted with a substituent selected from Group W1, 3- to 8-membered cycloalkyl optionally substituted with a substituent selected from Group W1, 5- to 8-membered cycloalkenyl optionally substituted with a substituent selected from Group W1, -NR^{1X}R^{2X}, -CO-R^{1X}, -CO-NR^{1X}R^{2X}, -NR^{1X}-CO-R^{2X}, -SO₂-R^{3X} or -O-SO₂-R^{3X},
wherein R^{1X} and R^{2X} may be the same or different and each represents hydrogen, C1-6 alkyl optionally substituted with a substituent selected from Group U1 or a 4- to 8-membered heterocyclic group, and
R^{3X} represents C1-6 alkyl optionally substituted with a substituent selected from Group F1; or
(i) R²⁰ and R²¹, (ii) R²¹ and R²² or (iii) R²² and R²³ bond together to form a ring selected from Group Z1,
wherein Group A1 consists of hydroxyl, halogen, cyano, C1-6 alkoxy, phenyl optionally substituted with a substituent selected from Group C1, C1-6 alkyl, C1-6 haloalkyl and C2-7 alkylene where C2-7 alkylene is permissible only in the case that a spiro union is formed together with the substituted 5- to 10-membered cycloalkyl or the substituted 5- to 10-membered cycloalkenyl,
Group C 1 consists of cyano, halogen, C1-6 alkyl and C1-6 alkoxy,
Group D1 consists of hydroxyl, halogen, cyano, C1-6 alkoxy, C1-6 alkylthio, C1-6 alkylsulfonyl, C1-6 alkylsulfinyl, mono(C1-6 alkyl)amino, di(C1-6 alkyl)amino, C2-7 alkylcarbonylamino, 3- to 8-membered cycloalkyl optionally substituted with a substituent selected from Group H1, C2-7 alkoxycarbonyl. carboxyl, a 4- to 8-membered heterocyclic group, a 5- to 10-membered heteroaryl ring group, a 6- to 10-membered aryl ring group, C2-7 alkylcarbonyl, 6- to 10-membered aryl ring carbonyl, aminocarbonyl, mono(C1-6 alkyl)aminocarbonyl optionally substituted with halogen, mono(3- to 8-membered cycloalkyl)aminocarbonyl, mono(C2-7 alkoxyalkyl)aminocarbonyl, di(C1-6 alkyl)aminocarbonyl, mono(5- to 10-membered heteroaryl ring)aminocarbonyl, 4-to 8-membered heterocyclic carbonyl optionally substituted with C1-6 alkyl, and 5-to 10-membered heteroaryl ring carbonyl,
Group E1 consists of halogen, C1-6 alkoxy, oxo (=O) and C1-6 alkyl,
Group F1 consists of halogen and C 1-6 alkoxy,
Group G1 consists of 3- to 8-membered cycloalkyl,
Group H 1 consists of hydroxyl, C1-6 haloalkyl, C1-6 alkyl, C2-7 alkoxyalkyl, mono(C1-6 alkyl)aminocarbonyl, di(C1-6 alkyl)aminocarbonyl, C2-7 alkoxycarbonyl, carboxyl and C2-7 cyanoalkyl,
Group W1 consists of halogen, hydroxyl, cyano, carboxyl, C1-6 alkyl, C2-7 alkoxyalkyl, C1-6 alkoxy optionally substituted with a substituent selected from Group T1, phenoxy, C2-7 alkoxycarbonyl, C2-7 alkylcarbonyl, -NR^{6X}R^{7X} and -CO-NR^{6X}R^{7X} wherein R^{6X} and R^{7X} may be the same or different and each represents hydrogen or C 1-6 alkyl,
Group T1 consists of C1-6 alkoxy, carboxyl, C2-7 alkoxycarbonyl and -CONR^{4X}R^{5X} wherein R^{4X} and R^{5X} may be the same or different and each represents hydrogen or C1-6 alkyl,
Group V1 consists of oxo (=O) and ethylenedioxy (-O-CH₂CH₂-O-) where ethylenedioxy is permissible only in the case that a spiro union is formed together with the substituted 4- to 8-membered heterocyclic group,
Group K1 consists of a 4- to 8-membered heterocyclic group,
Group U1 consists of carboxyl, C1-6 alkoxy, C2-7 alkoxycarbonyl, halogen, a 6- to 10-membered aryl ring group and -CO-NR^{8x}R^{9x} wherein R^{8x} and R^{9x} may be the same or di fferent and each represents hydrogen or C1-6 alkyl, and
Group Z1 consists of 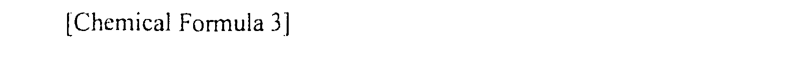 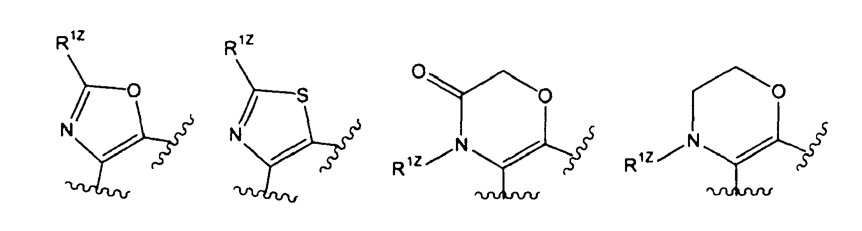 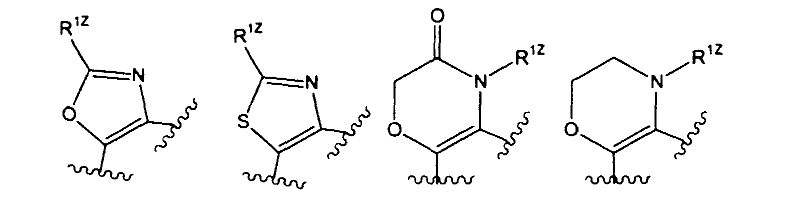 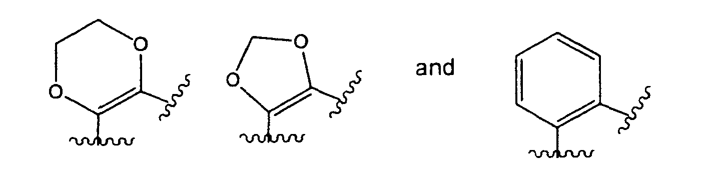 wherein R^{1Z} represents hydrogen, C1-6 alkyl or benzyl,
with the proviso that a compound represented by the following formula: 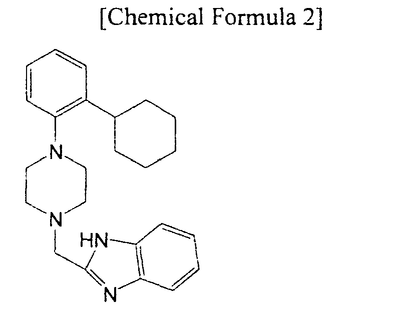 is excepted.

2. A compound represented by the following general formula (100), a salt thereof or a hydrate of the foregoing: 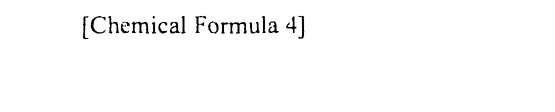 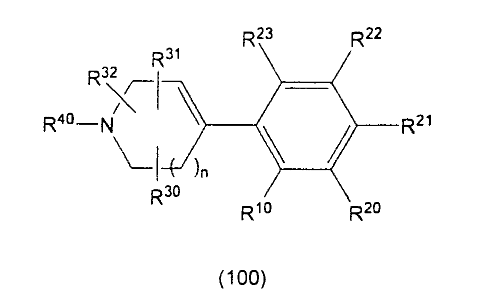 wherein R¹⁰, R²⁰, R²¹, R²², R²³, R³⁰, R³¹ R³², R⁴⁰ and n have the same respective definitions as R¹⁰, R²⁰, R²¹, R²², R²³, R³⁰, R³¹, R³², R⁴⁰ and n in claim 1.

3. The compound according to claim 1, the salt thereof or the hydrate of the foregoing, wherein R¹⁰ is 5- to 10-membered cycloalkyl optionally substituted with a substituent from Group A2, or 5- to 10-membered cycloalkenyl optionally substituted with a substituent from Group A2,
wherein Group A2 consists of hydroxyl, phenyl, C1-6 alkyl, C1-6 haloalkyl and C2-7 alkylene where C2-7 alkylene is permissible only in the case that a spiro union is formed together with the substituted 5- to 10-membered cycloalkyl or the substituted 5- to 10-membered cycloalkenyl.

4. The compound according to claim 1, the salt thereof or the hydrate of the foregoing, wherein R¹⁰ is 5- to 10-membered cycloalkyl optionally substituted with hydroxyl, phenyl, C1-6 alkyl, C1-6 haloalkyl, 1,2-ethylene, trimethylene, tetramethylene or pentamethylene, or 5- to 10-membered cycloalkenyl optionally substituted with hydroxyl, phenyl, C1-6 alkyl, C1-6 haloalkyl, 1,2-ethylene, trimethylene, tetramethylene or pentamethylene, where 1,2-ethylene, trimethylene, tetramethylene or pentamethylene is permissible only in the case that a spiro union is formed together with the 5- to 10-membered cycloalkyl or 5- to 10-membered cycloalkenyl.

5. The compound according to claim 1, the salt thereof or the hydrate of the foregoing, wherein R¹⁰ is cyclohexyl, 4-t-butylcyclohexyl, 4,4-dimethylcyclohexyl; 4,4-diethylcyclohexyl, 3,3,5,5-tetramethylcyclohexyl, 3,5-dimethylcyclohexyl, 4-phenylcyclohexyl, 4-tritluoromethylcyclohexyl, 4-n-butylcyclohexyl, cyclopentyl, 3,3,4,4-tetramethylcyclopentyl, cycloheptyl, cyclooctyl, or a group represented by the following formula: 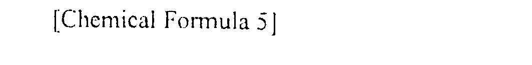 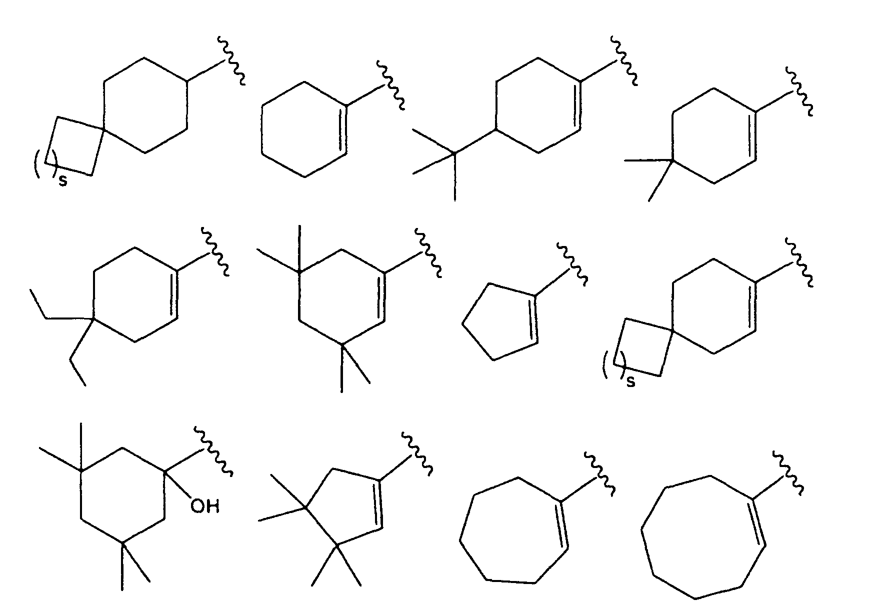 or 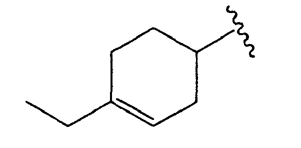 wherein s is an integer of 0, 1, 2 or 3.

6. The compound according to claim 1, the salt thereof or the hydrate of the foregoing, wherein R³⁰, R³¹ and R³² may be the same or different and each represents hydrogen or C1-6 alkyl, or R³⁰ and R³¹ bond together to form oxo (=O) and R³² is hydrogen or C1-6 alkyl.

7. The compound according to claim 1. the salt thereof or the hydrate of the foregoing, wherein R³⁰, R³¹ and R³² may be the same or different and each represents hydrogen or methyl, or R³⁰ and R³¹ bond together to form oxo (=O) and R³² is hydrogen or methyl.

8. The compound according to claim 1, the salt thereof or the hydrate of the foregoing, wherein R³⁰, R³¹ and R³² are all hydrogen.

9. The compound according to claim 1, the salt thereof or the hydrate of the foregoing, wherein R⁴⁰ is C1-6 alkyl optionally substituted with a substituent selected from Group D1, 3- to 8-membered cycloalkyl optionally substituted with a substituent selected from Group E1, C2-7 alkenyl, C2-7 alkynyl or C2-7 alkylcarbonyl wherein Group D1 and Group E1 have the same respective definitions as Group D1 and Group E1 in claim 1.

10. The compound according to claim 1. the salt thereof or the hydrate of the foregoing, wherein R⁴⁰ is C1-6 alkyl optionally substituted with a substituent selected from Group D2.
wherein Group D2 consists of hydroxyl, halogen, cyano, C1-6 alkoxy, 3- to 8-membered cycloalkyl, a 4- to 8-membered heterocyclic group, mono(C1-6 alkyl)aminocarbonyl, di(C1-6 alkyl)aminocarbonyl, C2-7 alkylcarbonyl, a 5-membered heteroaryl ring group, 4- to 8-membered heterocyclic carbonyl and phenyl.

11. The compound according to claim 1, the salt thereof or the hydrate of the foregoing, wherein R⁴⁰ is n-propyl, n-butyl, n-pentyl, isobutyl, ethylcarbonylmethyl, methoxyethyl, ethoxyethyl, cyclopropylmethyl or tetrahydropyran-4-ylmethyl.

12. The compound according to claim 1, the salt thereof or the hydrate of the foregoing, wherein n is an integer of 1.

13. The compound according to claim 1, the salt thereof or the hydrate of the foregoing, wherein X¹ is nitrogen.

14. The compound according to claim 1, the salt thereof or the hydrate of the foregoing, wherein (i) R²⁰ and R²¹, (ii) R²¹ and R²² or (iii) R²² and R²³ bond together to form a ring selected from Group Z1 wherein Group Z1 has the same definition as Group Z1 in claim 1.

15. The compound according to claim 1, the salt thereof or the hydrate of the foregoing, wherein (i) R²¹ and R²² or (ii) R²² and R²³ bond together to form a ring selected from Group Z2,
wherein Group Z2 consists of 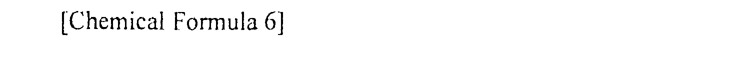 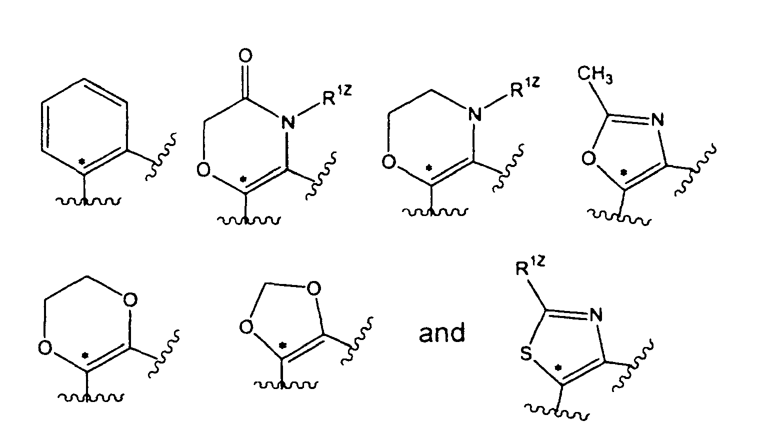 wherein R^{1Z} represents hydrogen, C1-6 alkyl or benzyl, and the carbon atom indicated by "*" is the carbon atom on benzene ring to which R²² is bonded.

16. The compound according to claim 1, the salt thereof or the hydrate of the foregoing, wherein (i) R²¹ and R²² or (ii) R²² and R²³ bond together to form a ring selected from Group Z3,
wherein Group Z3 consists of 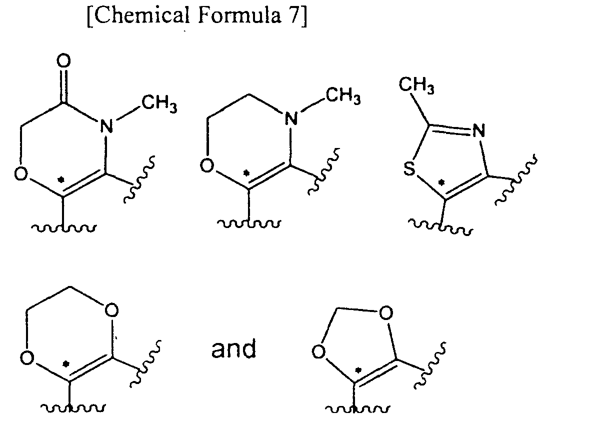 wherein the carbon atom indicated by "*" is the carbon atom on benzene ring to which R²² is bonded.

17. The compound according to claim 14, the salt thereof or the hydrate of the foregoing, wherein R²⁰ and R²³ are hydrogen.

18. The compound according to claim 1, the salt thereof or the hydrate of the foregoing, wherein at least one of R²⁰, R²¹, R²² and R²³ is carboxyl, C1-6 alkylthio optionally substituted with a substituent selected from Group F1, C2-7 alkoxycarbonyl; phenoxy, -SO₃H, C1-6 alkyl substituted with a substituent selected from Group W2, C1-6 alkyl substituted with a substituent selected from Group K1, C1-6 alkoxy substituted with a substituent selected from Group W2, a 4- to 8-membered heterocyclic group substituted with a substituent selected from Group W3, a 4- to 8-membered heterocyclic group substituted with a substituent selected from Group V1, a 5- to 10-membered heteroaryl ring group substituted with a substituent selected from Group W3, a 6- to 10-membered aryl ring group optionally substituted with a substituent selected from Group W1, C2-7 alkenyl optionally substituted with substituent selected from Group W1, C2-7 alkynyl optionally substituted with a substituent selected from Group W1, 3- to 8-membered cycloalkyl optionally substituted with a substituent selected from Group W1, 5- to 8-membered cycloalkenyl optionally substituted with a substituent selected from Group W1, -NR^{10X}R^{2X}, -CO-R^{11X}, -CO-NR^{1X}R^{2X}, -NR^{1X}-CO-R^{2X}, -SO₂-R^{3X} or -O-SO₂-R^{3x},
R^{1X} and R^{2X} may be the same or different and each represents hydrogen, C1-6 alkyl optionally substituted with a substituent selected from Group U1 or a 4-to 8-membered heterocyclic group,
R^{3X} represents C1-6 alkyl optionally substituted with a substituent selected from Group F1,
R^{10X} is C1-6 alkyl substituted with a substituent selected from Group U1, or a 4- to 8-membered heterocyclic group, and
R^{11X} is hydrogen, C1-6 alkyl substituted with a substituent selected from Group U1, or a 4- to 8-membered heterocyclic group,
wherein Group W2 consists of hydroxyl, cyano, C1-6 alkyl, C2-7 alkoxyalkyl. C1-6 alkoxy optionally substituted with a substituent selected from Group T1, phenoxy, C2-7 alkylcarbonyl, -NR^{6X}R^{7X} and -CO-NR^{6X}R^{7X} wherein R ^{6X} and R^{7X} may be the same or different and each represents hydrogen or C1-6 alkyl,
Group W3 consists of hydroxyl, carboxyl, C2-7 alkoxyalkyl, C1-6 alkoxy substituted with a substituent selected from Group T1, , phenoxy, C2-7 alkoxycarbonyl, C2-7 alkylcarbonyl, -NR^{6X}R^{7X} and -CO-NR^{6X}R^{7X} wherein R^{6X} and R^{7X} may be the same or different and each represents hydrogen or C1-6 alkyl, and
Group F1, Group G1, Group H1, Group W1, Group T1, Group V1, Group K1 and Group U1 have the same respective definitions as Group F1, Group G1, Group H1, Group W1, Group T1, Group V1, Group K1 and Group U1 in claim 1.

19. The compound according to claim 1, the salt thereof or the hydrate of the foregoing, wherein one of R²⁰, R²¹, R²² and R²³ represents phenyl optionally substituted with one substituent selected from Group P, C2-7 alkenyl optionally substituted with one substituent selected from Group P, C2-7 alkynyl optionally substituted with one substituent selected from Group P, carboxyl, C1-6 alkylsulfonyloxy optionally having 1-3 fluorine, C1-6 alkylthio, C2-7 alkoxycarbonyl, C1-6 alkoxy-C1-6 alkoxy, C2-7 alkoxyalkyl, morpholin-4-yl-C 1-6 alkyl, pyrrolidin-1-yl optionally substituted with one substituent selected from Group Q, piperidin-1-yl optionally substituted with one substituent selected from Group Q, a group represented by the following formula: 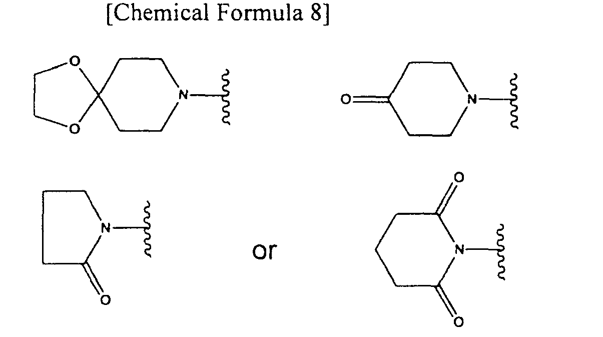 -NR⁸⁰R⁸¹, -CO-R⁸², -CO-NR⁸³R⁸⁴ or -NR⁸⁵CO-R⁸⁶,
wherein R⁸⁰ represents hydrogen, C1-6 alkyl, C2-7 alkoxyalkyl or tetrahydropyran-4-yl,
R⁸¹ represents C2-7 alkoxyalkyl or tetrahydropyran-4-yl,
R⁸² represents C2-7 alkoxyalkyl or morpholin-4-yl,
R⁸³ and R⁸⁴ may be the same or different and each represents hydrogen, C1-6 alkyl, tetrahydropyran-4-yl or C2-7 alkoxyalkyl,
R⁸⁵ represents hydrogen or C1-6 alkyl, and
R⁸⁶ represents C1-6 alkyl,
wherein Group P consists of carboxyl, C2-7 alkoxycarbonyl, C2-7 alkoxyalkyl, C1-6 alkoxy and cyano, and
Group Q consists of carboxyl, C2-7 alkoxycarbonyl, C1-6 alkoxy-C1-6 alkoxy, carboxyl-C1-6 alkoxy, C2-7 alkoxyalkyl and hydroxyl.

20. The compound according to claim 1, the salt thereof or the hydrate of the foregoing, wherein one of R²⁰, R²¹, R²² and R²³ represents phenyl, C2-7 alkoxycarbonyl, C1-6 alkoxy-C1-6 alkoxy, C2-7 alkoxyalkyl, morpholin-4-yl-C1-6 alkyl, pyrrolidin-1-yl optionally having one substituent selected from Group R, piperidin-1-yl optionally having one substituent selected from Group R, a group represented by the following formula: 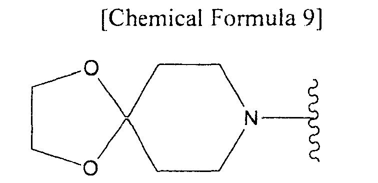 -NR⁹⁰R⁹¹, CO-R⁹² or -CO-NR⁹³R⁹⁴,
wherein R⁹⁰ represents hydrogen, C1-6 alkyl, C2-7 alkoxyalkyl or tetrahydropyran-4-yl,
R⁹¹ represents C2-7 alkoxyalkyl or tetrahydropyran-4-yl,
R⁹² represents C2-7 alkoxyalkyl, and
R⁹³ and R⁹⁴ may be the same or different and each represents hydrogen or C1-6 alkyl,
wherein Group R consists of C1-6 alkoxy-C1-6 alkoxy and C2-7 alkoxyalkyl.

21. The compound according to claim 18, the salt thereof or the hydrate of the foregoing, wherein R²⁰ is hydrogen.

22. The compound according to claim 18, the salt thereof or the hydrate of the foregoing, wherein two of R²⁰, R²¹, R²² and R²³ are hydrogen, and one of the other two is hydrogen or C1-6 alkoxy.

23. The compound according to claim 18, the salt thereof or the hydrate of the foregoing, wherein three of R²⁰, R²¹, R²² and R²³ are hydrogen.

24. A medicament comprising the compound according to claim 1, the salt thereof or the hydrate of the foregoing.

25. A cell adhesion or cell infiltration inhibitor comprising the compound according to claim 1, the salt thereof or the hydrate of the foregoing.

26. A therapeutic or prophylactic agent for an inflammatory disease or an autoimmune disease, comprising the compound according to claim 1, the salt thereof or the hydrate of the foregoing.

27. A therapeutic or prophylactic agent for an inflammatory bowel disease, irritable bowel syndrome, rheumatoid arthritis, psoriasis, multiple sclerosis, asthma or atopic dermatitis, comprising the compound according to claim 1, the salt thereof or the hydrate of the foregoing.

28. A therapeutic or prophylactic agent for an inflammatory bowel disease, comprising the compound according to claim 1, the salt thereof or the hydrate of the foregoing.

29. A therapeutic or prophylactic agent for ulcerative colitis or Crohn's disease, comprising the compound according to claim 1, the salt thereof or the hydrate of the foregoing.
